# EUROPÄISCHE PATENTSCHRIFT

(11) **EP 2 493 845 B1**
(45) Veröffentlichungstag und Bekanntmachung des Hinweises auf die Patenterteilung: **25.12.2013**
(21) Anmeldenummer: 10768933.3
(22) Anmeldetag: 21.10.2010
(51) Int. Cl.: C07C 233/55, C07C 255/60, C07D 305/06, A61K 31/277, A61K 31/167, A61K 31/337, A61P 9/00

(54) **SUBSTITUIERTE 3-PHENYLPROPIONSÄUREN UND IHRE VERWENDUNG**
SUBSTITUTED 3-PHENYLPROPIONIC ACIDS AND THE USE THEREOF
ACIDES 3-PHÉNYLPROPIONIQUES SUBSTITUÉS ET LEUR UTILISATION

(30) Priorität: 28.10.2009 DE 102009046115
(43) Veröffentlichungstag der Anmeldung: 05.09.2012
(73) Patentinhaber: Bayer Intellectual Property GmbH, 40789 Monheim (DE)
(72) Erfinder: LAMPE, Thomas, 40545 Düsseldorf (DE); HAHN, Michael, 40764 Langenfeld (DE); STASCH, Johannes-Peter, 42651 Solingen (DE); SCHLEMMER, Karl-Heinz, 42113 Wuppertal (DE); WUNDER, Frank, 42117 Wuppertal (DE); EL SHEIKH, Sherif, 45219 Essen (DE); LI, Volkhart, Min-Jian, 42553 Velbert (DE); BECKER, Eva-Maria, 42327 Wuppertal (DE); STOLL, Friederike, 40215 Düsseldorf (DE); KNORR, Andreas, 40699 Erkrath (DE)
(74) Vertreter: BIP Patents
(86) Internationale Anmeldenummer: PCT/EP2010/065910
(87) Internationale Veröffentlichungsnummer: WO 2011/051165

(56) Entgegenhaltungen:
- WO-A1-2009/127338

## Beschreibung

Die vorliegende Anmeldung betrifft neue 3-Phenylpropionsäure-Derivate, Verfahren zu ihrer Herstellung, die Verbindungen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann,

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid.*]*.* Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid.*]*.* Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

In WO 00/64888-A1, EP 1 216 980-A1, EP 1 375 472-A1, EP 1 452 521-A1, US 2005/0187266-A1 und US 2005/0234066-A1 werden verschiedene Arylalkancarbonsäure-Derivate als PPAR-Agonisten zur Behandlung von Diabetes, Dyslipidämie, Arteriosklerose, Obesitas und anderen Erkrankungen beschrieben. In EP 1 312 601-A1 und EP 1 431 267-A1 werden substituierte Arylalkancarbonsäuren als PGE₂-Rezeptorantagonisten zur Behandlung beispielsweise von Schmerzzuständen, urologischen Erkrankungen, der Alzheimer'schen Krankheit und Krebs offenbart. Weiterhin werden Arylalkancarbonsäuren in WO 2005/086661-A2 als GPR40-Modulatoren für die Behandlung von Diabetes und Dyslipidämien beansprucht, und in WO 2004/099170-A2, WO 2006/050097-A1 sowie WO 2006/055625-A2 werden Phenyl-substituierte Carbonsäuren als PTP-1B-Inhibitoren für die Behandlung von Diabetes, Krebs und neurodegenerativen Erkrankungen beschrieben. Ferner sind aus WO 96/12473-A1 und WO 96/30036-A1 einzelne Phenylacetamidosubstituierte Phenylalkancarbonsäuren bekannt, die in Form nicht-kovalenter Mischungen die Zuführung von Peptid-Wirkstoffen innerhalb des Körpers verbessern. Vor kurzem wurden in WO 2009/127338-A1 oxo-heterocyclisch substituierte Carbonsäure-Derivate offenbart, welche als Aktivatoren der löslichen Guanylatcyclase wirken.

Gegenstand der vorliegenden Erfindung sind Verbindungen der allgemeinen Formel (I) 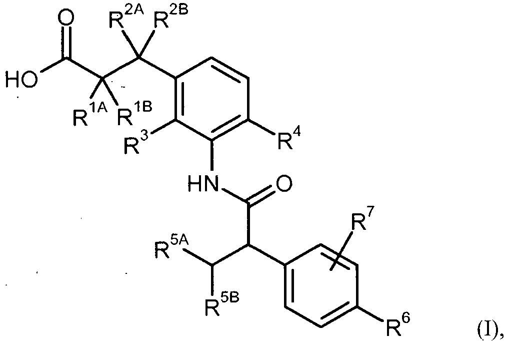 in welcher
- R^{1A}: für Wasserstoff, Fluor, Methyl, Trifluormethyl, Ethyl, 1,1-Difluorethyl, 2,2,2-Trifluorethyl, *n*-Propyl, Cyclopropyl oder Cyclobutyl steht,
- R^{1B}: für Wasserstoff oder Methyl steht,
- R^{2A}: für Wasserstoff, Methyl, Trifluormethyl, Ethyl, 1,1-Difluorethyl, 2,2,2-Trifluorethyl oder n-Propyl steht,
- R^{2B}: für Wasserstoff oder Methyl steht,
oder
- R^{1A} und R^{2A}: miteinander verknüpft sind und gemeinsam mit den Kohlenstoffatomen, an die sie gebunden sind, einen Cyclopropyl-Ring der Formel 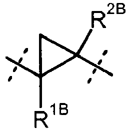 bilden, worin R^{1B} und R^{2B} die zuvor genannten Bedeutungen haben,
oder
- R^{2A} und R^{2B}: miteinander verknüpft sind und gemeinsam mit dem Kohlenstoffatom, an das sie gebunden sind, eine cyclische Gruppe der Formel 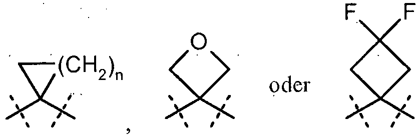 bilden, worin
- n: die Zahl 1, 2 oder 3 bedeutet,
- R³: für Wasserstoff, Fluor, Methyl oder Trifluormethyl steht,
- R⁴: für Wasserstoff, Fluor, Chlor, Cyano, Methyl, Trifluormethyl oder Ethyl steht,
- R^{5A}: für Methyl, Trifluormethyl oder Ethyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cycloalkyl-Ring der Formel 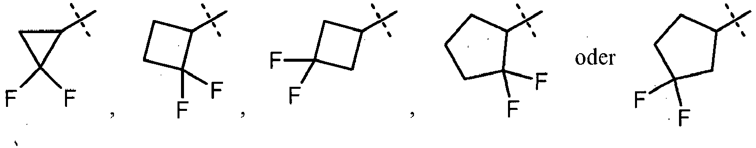 bilden,
- R⁶: für Wasserstoff, Fluor, Chlor, Brom, Cyano, (C₁-C₄)-Alkyl, (C₂-C₄)-Alkenyl, Cyclopropyl oder Cyclobutyl steht, wobei (C₁-C₄)-Alkyl und (C₂-C₄)-Alkenyl bis zu dreifach mit Fluor
und
Cyclopropyl und Cyclobutyl bis zu zweifach mit Fluor substituiert sein können,
und
- R⁷: für Wasserstoff, Fluor, Chlor, Cyano, Methyl, Trifluormethyl, Ethyl, Methoxy oder Tri-fluormethoxy steht,
sowie ihre Salze, Solvate und Solvate der Salze.

Erfindungsgemäße Verbindungen sind die Verbindungen der Formel (I) und deren Salze, Solvate und Solvate der Salze, die von Formel (1) umfassten Verbindungen der nachfolgend genannten Formeln und deren Salze, Solvate und Solvate der Salze sowie die von Formel (1) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen insbesondere die Salze üblicher Basen, wie beispielhaft und vorzugsweise Alkalimetall-Salze (z.B. Natrium- und Kaliumsalze), Erdalkali-Salze (z.B. Calcium- und Magnesiumsalze) und Ammonium-Salze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, *N*-Methylpiperidin, Arginin, Lysin und Ethylendiamin.

Als Solvate werden im Rahmen der Erfmdung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *in*-Butyl, *iso*-Butyl, *sec*.-Butyl und *tert*.-Butyl.

(C₂-C₄)-Alkenyl und (C₂-C₃)-Alkenyl stehen im Rahmen der Erfindung für einen geradkettigen oder verzweigten Alkenylrest mit einer Doppelbindung und 2 bis 4 bzw. 2 oder 3 Kohlenstoffatomen. Bevorzugt ist ein geradkettiger oder verzweigter Alkenylrest mit 2 oder 3 Kohlenstoffatomen. Beispielhaft und vorzugsweise seien genannt: Vinyl, Allyl, *n*-Prop-1-en-1-yl, Isopropenyl, *n*-But-1-en-1-yl, *n*-But-2-en-1-yl, *n*-But-3-en-1-yl, 2-Methylprop-1-en-1-yl und 2-Methylprop-2-en-1-yl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder zwei oder drei gleichen oder verschiedenen Substituenten ist bevorzugt. Besonders bevorzugt ist die Substitution mit einem oder zwei gleichen oder verschiedenen Substituenten.

In einer bestimmten Ausführungsform umfasst die vorliegende Erfindung Verbindungen der Formel (I), in welcher
- R^{1A}: für Wasserstoff, Fluor, Methyl, Trifluormethyl, Ethyl, 1,1-Difluorethyl, 2,2,2-Trifluorethyl oder *n*-Propyl steht,
- R^{1B}: für Wasserstoff oder Methyl steht,
- R^{2A}: für Wasserstoff, Methyl, Trifluormethyl, Ethyl, 1,1-Difluorethyl, 2,2,2-Trifluorethyl oder *n-*Propyl steht,
- R^{2B}: für Wasserstoff oder Methyl steht,
oder
- R^{1A} und R^{2A}: miteinander verknüpft sind und gemeinsam mit den Kohlenstoffatomen, an die sie gebunden sind, einen Cyclopropyl-Ring der Formel 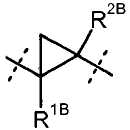 bilden, worin R^{1B} und R^{2B} die zuvor genannten Bedeutungen haben, oder R^{2A} und R^{2B} miteinander verknüpft sind und gemeinsam mit dem Kohlenstoffatom, an das sie gebunden sind, eine cyclische Gruppe der Formel 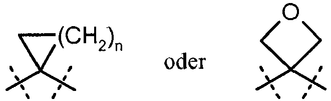 bilden, worin
- n: die Zahl 1, 2 oder 3 bedeutet,
- R³: für Wasserstoff, Fluor, Methyl oder Trifluormethyl steht,
- R⁴: für Wasserstoff, Fluor, Chlor, Cyano, Methyl, Trifluormethyl oder Ethyl steht,
- R^{5A}: für Methyl, Trifluormethyl oder Ethyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cycloalkyl-Ring der Formel 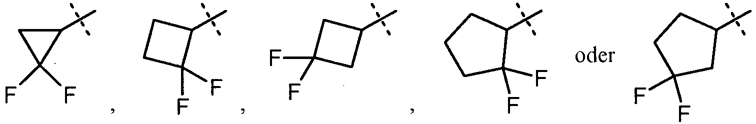 bilden, R⁶ für Wasserstoff, Fluor, Chlor, Brom, Cyano, (C₁-C₄)-Alkyl oder (C₂-C₄)-Alkenyl steht, wobei (C₁-C₄)-Alkyl und (C₂-C₄)-Alkenyl ihrerseits bis zu dreifach mit Fluor substituiert sein können,
und
- R⁷: für Wasserstoff, Fluor, Chlor oder Methyl steht,
sowie ihre Salze, Solvate und Solvate der Salze.

Bevorzugt im Rahmen der vorliegenden Erfindung sind Verbindungen der Formel (I), in welcher
- R^{1A}: für Wasserstoff, Methyl, Trifluormethyl, Ethyl, *n*-Propyl, Cyclopropyl oder Cyclobutyl steht,
- R^{1B}: für Wasserstoff oder Methyl steht,
- R^{2A}: für Wasserstoff, Methyl, Trifluormethyl, Ethyl oder *n*-Propyl steht,
- R^{2B}: für Wasserstoff oder Methyl steht, oder
- R^{2A} und R^{2B}: miteinander verknüpft sind und gemeinsam mit dem Kohlenstoffatom, an das sie gebunden sind, eine cyclische Gruppe der Formel 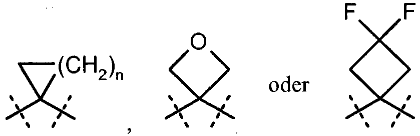 bilden, worin
- n: die Zahl 1 oder 2 bedeutet,
- R³: für Wasserstoff, Fluor oder Methyl steht,
- R⁴: für Wasserstoff, Fluor, Chlor, Cyano, Methyl oder Trifluormethyl steht,
- R^{5A}: für Methyl oder Ethyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cycloalkyl-Ring der Formel 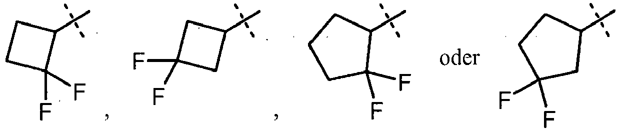 bilden, R⁶ für Fluor, Chlor, (C₁-C₄)-Alkyl, (C₂-C₃)-Alkenyl, Cyclopropyl oder Cyclobutyl steht, wobei (C₁-C₄)-Alkyl und (C₂-C₃)-Alkenyl bis zu dreifach mit Fluor
und
Cyclopropyl und Cyclobutyl bis zu zweifach mit Fluor substituiert sein können,
und
- R⁷: für Wasserstoff, Fluor, Chlor, Methyl oder Methoxy steht,
sowie ihre Salze, Solvate und Solvate der Salze.

Eine weitere bevorzugte Ausführungsfonn der vorliegenden Erfindung umfasst Verbindungen der Formel (I), in welcher
- R^{1A}: für Wasserstoff, Methyl, Trifluormethyl, Ethyl oder *n*-Propyl steht,
- R^{1B}: für Wasserstoff oder Methyl steht,
- R^{2A}: für Wasserstoff, Methyl, Trifluormethyl oder Ethyl steht,
- R^{2B}: für Wasserstoff oder Methyl steht,
oder
- R^{2A} und R^{2B}: miteinander verknüpft sind und gemeinsam mit dem Kohlenstoffatom, an das sie gebunden sind, eine cyclische Gruppe der Formel 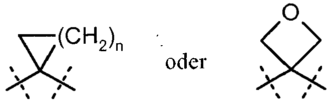 bilden, worin
- n: die Zahl 1 oder 2 bedeutet,
- R³: für Wasserstoff oder Fluor steht,
- R⁴: für Wasserstoff, Fluor, Chlor, Cyano, Methyl oder Trifluormethyl steht,
- R^{5A}: für Methyl oder Ethyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cycloalkyl-Ring der Formel 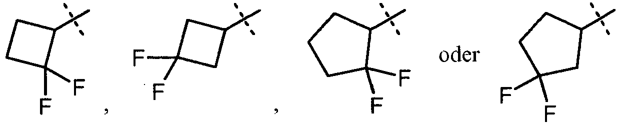 bilden,
- R⁶: für Fluor, Chlor, (C₁-C₄)-Alkyl oder (C₂-C₃)-Alkenyl steht, wobei (C₁-C₄)-Akyl und (C₂-C₃)-Alkenyl ihrerseits bis zu dreifach mit Fluor substituiert sein können,
und
- R⁷: für Wasserstoff, Fluor oder Chlor steht,
sowie ihre Salze, Solvate und Solvate der Salze.

Besonders bevorzugt im Rahmen der vorliegenden Erfindung sind Verbindungen der Formel (I), in welcher
- R^{1A}: für Wasserstoff, Methyl oder Ethyl steht,
- R^{1B}: für Wasserstoff steht,
- R^{2A}: für Wasserstoff, Methyl, Trifluormethyl, Ethyl oder *n*-Propyl steht,
- R^{2B}: für Wasserstoff oder Methyl steht,
oder
- R^{2A} und R^{2B}: miteinander verknüpft sind und gemeinsam mit dem Kohlenstoffatom, an das sie gebunden sind, eine cyclische Gruppe der Formel 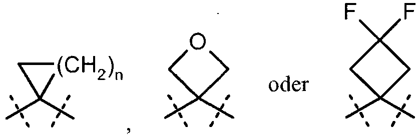 bilden, worin
- n: die Zahl 1 oder 2 bedeutet,
- R³: für Wasserstoff steht,
- R⁴: für Fluor, Chlor oder Methyl steht,
- R^{5A}: für Methyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cyclopentyl-Ring der Formel 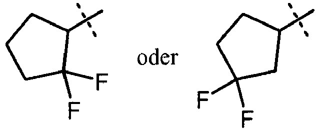 bilden,
- R⁶: für Fluor, Chlor, Methyl, Trifluormethyl, Ethyl, 1,1-Difluorethyl, 2,2,2-Trifluorethyl, Isopropyl, *tert*.-Butyl, 1,1,1-Trifluor-2-methylpropan-2-yl, Vinyl, 1-Fluorvinyl, Cyclopropyl, 2,2-Difluorcyclopropyl, Cyclobutyl oder 3,3-Difluorcyclobutyl steht,
und
- R⁷: für Wasserstoff, Fluor, Chlor oder Methyl steht,
sowie ihre Salze, Solvate und Solvate der Salze.

Eine weitere besonders bevorzugte Ausführungsform der vorliegenden Erfindung umfasst Verbindungen der Formel (I), in welcher
- R^{1A}: für Wasserstoff, Methyl oder Ethyl steht,
- R^{1B}: für Wasserstoff steht,
- R^{2A}: für Wasserstoff oder Methyl steht,
- R^{2B}: für Wasserstoff steht,
oder
- R^{2A} und R^{2B}: miteinander verknüpft sind und gemeinsam mit dem Kohlenstoffatom, an das sie gebunden sind, eine cyclische Gruppe der Formel 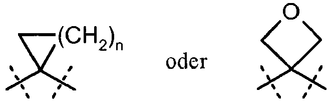 bilden, worin
- n: die Zahl 1 oder 2 bedeutet,
- R³: für Wasserstoff steht,
- R⁴: für Fluor, Chlor oder Methyl steht,
- R^{5A}: für Methyl steht,
- R^{5B}: für Trifluormethyl steht,
oder
- R^{5A} und R^{5B}: miteinander verknüpft sind und gemeinsam mit dem Kohlenstoffatom, an das sie gebunden sind, einen Difluor-substituierten Cyclopentyl-Ring der Formel 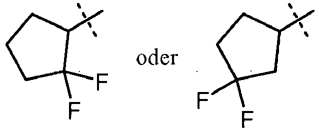 bilden,
- R⁶: für Chlor, Methyl, Trifluormethyl, Ethyl, 1,1-Difluorethyl, 2,2,2-Trifluorethyl, Isopropyl, *tert.*-Butyl, 1,1,1-Trifluor-2-methylpropan-2-yl, Vinyl oder 1-Fluorvinyl steht,
und
- R⁷: für Wasserstoff oder Fluor steht,
sowie ihre Salze, Solvate und Solvate der Salze.

Eine besondere Ausführungsform der vorliegenden Erfindung umfasst Verbindungen der Formel (I), in welcher
- R^{1A}: für Wasserstoff, Methyl oder Ethyl steht
und
- R^{1B}, R^{2A} und R^{2B}: jeweils für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R^{2A}: für Methyl, Trifluormethyl, Ethyl oder *n*-Propyl steht
und
- R^{1A}, R^{1B} und R^{2B}: jeweils für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R^{1A} und R^{1B}: jeweils für Wasserstoff stehen
und
- R^{2A} und R^{2B}: jeweils für Methyl stehen,
sowie ihre Salze, Solvate und Solvate der Salze.

Eine weitere besondere Ausführungsform der vorliegenden Erfindung umfasst Verbindungen der Formel (I), in welcher
- R^{1A} und R^{1B}: jeweils für Wasserstoff stehen
und
- R^{2A} und R^{2B}: miteinander verknüpft sind und gemeinsam mit dem Kohlenstoffatom, an das sie gebunden sind, einen Cyclopropyl- oder Cyclobutyl-Ring der Formel 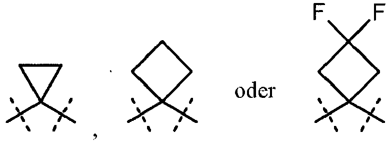 bilden,
sowie ihre Salze, Solvate und Solvate der Salze.

Eine weitere besondere Ausführungsform der vorliegenden Erfindung umfasst Verbindungen der Formel (I), in welcher
- R³: für Wasserstoff steht
und
- R⁴: für Fluor oder Chlor steht,
sowie ihre Salze, Solvate und Solvate der Salze.

Eine weitere besondere Ausführungsform der vorliegenden Erfindung umfasst Verbindungen der Formel (I), in welcher
- R^{5A}: für Methyl steht
und
- R^{5B}: für Trifluormethyl steht,
sowie ihre Salze, Solvate und Solvate der Salze.

Eine weitere besondere Ausführungsform der vorliegenden Erfindung umfasst Verbindungen der Formel (I), in welcher
- R^{5A} und R^{5B}: miteinander verknüpft sind und gemeinsam mit dem Kohlenstoffatom, an das sie gebunden sind, einen Difluor-substituierten Cyclopentyl-Ring der Formel 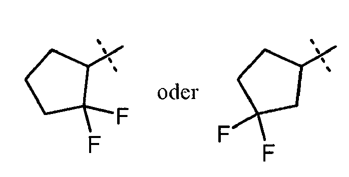 bilden, sowie ihre Salze, Solvate und Solvate der Salze.

Eine weitere besondere Ausführungsform der vorliegenden Erfindung umfasst Verbindungen der Formel (I-A) 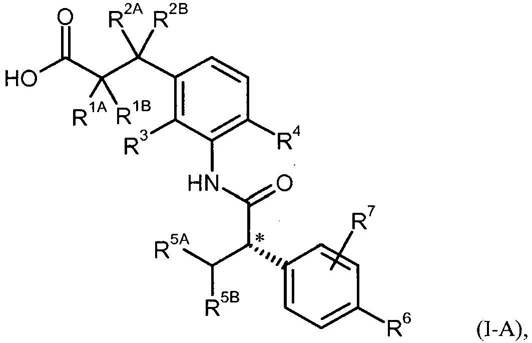 in welcher das mit * gekennzeichnete C-Atom der Phenylacetamid-Gruppierung die abgebildete S-Konfiguration aufweist
und
die Reste R^{1A}, R^{1B}, R^{2A}, R^{2B}, R³, R⁴, R^{5A}, R^{5B}, R⁶ und R⁷ jeweils die oben angegebenen Bedeutungen haben,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Carbonsäure der Formel (II) 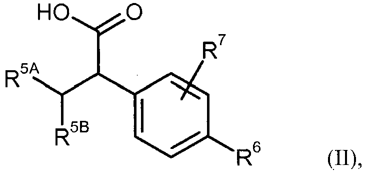 in welcher R^{5A}, R^{5B}, R⁶ und R⁷ die oben angegebenen Bedeutungen haben,
in einem inerten Lösungsmittel mit Hilfe eines Kondensationsmittels oder über die Zwischenstufe des entsprechenden Carbonsäurechlorids in Gegenwart einer Base mit einem Amin der Formel (III) 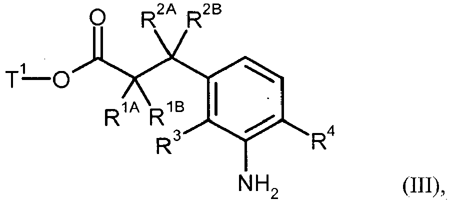 in welcher R^{1A}, R^{1B}, R^{2A}, R^{2B}, R³ und R⁴ die oben angegebenen Bedeutungen haben und
- T¹: für (C₁-C₄)-Alkyl oder Benzyl steht,
zu einem Carbonsäureamid der Formel (IV) 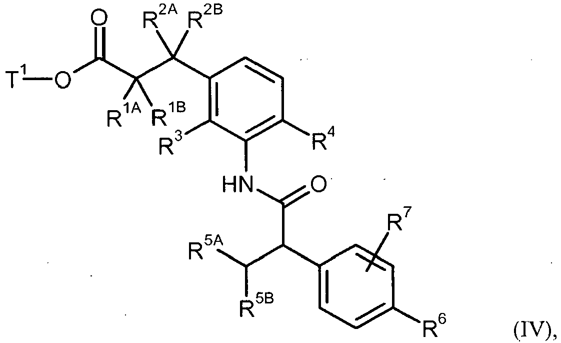 in welcher R^{1A}, R^{1B}, R^{2A}, R^{2B}, R³, R⁴, R^{5A}, R^{5B}, R⁶, R⁷ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen zu ihren Solvaten, Salzen und/oder Solvaten der Salze umsetzt.

Inerte Lösungsmittel für den Verfahrensschritt (II) + (III) → (IV) [Amid-Kupplung] sind beispielsweise Ether wie Diethylether, *tert*.-Butyl-methylether, Tetrahydrofuran, 1,4-Dioxan, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Acetonitril, Ethylacetat, Pyridin, Dimethylsulfoxid (DMSO), *N,N-*Dimethylformamid (DMF), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt werden Dichlormethan, Tetrahydrofuran, Dimethylformamid oder Gemische dieser Lösungsmittel verwendet.

Als Kondensationsmittel für diese Kupplungsreaktion eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N'-*Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, 1-Chlor-2-methyl-1-dimethylamino-1-propen, Propanphosphonsäureanhydrid, Cyanophosphonsäurediethylester, Bis-(2-oxo-3-oxazoli-dinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), O-(Benzotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyl-uronium-hexafluorophosphat (HATU) oder *O*-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Basen wie Triethylamin, *N*-Methylmorpholin, *N*-Methylpiperidin, *N,N*-Diisopropylethylamin, Pyridin oder 4-*N,N*-Dimethylaminopyridin. Bevorzugt eingesetzt werden *O*-(7-Azabenzotriazol-1-yl)-*N,N,N',N'*-tetramethyluronium-hexafluorophosphat (HATU) oder *O*-(Benzotriazol-1-yl)-*N,N,N',N'*-tetramethyluronium-tetrafluoroborat (TBTU), jeweils in Kombination mit Pyridin oder *N,N*-Diisopropylethylamin, oder *N*-(3-Dimethyl-aminoisopropyl)-*N'*-ethylcarbodiimid-Hydrochlorid (EDC) in Verbindung mit 1-Hydroxybenzotriazol (HOBt) und Triethylamin, oder 1-Chlor-2-methyl-1-dimethylamino-1-propen zusammen mit Pyridin.

Die Reaktion (II) + (III) → (IV) wird in der Regel in einem Temperaturbereich von 0°C bis +60°C, bevorzugt bei +10°C bis +40°C durchgeführt.

Bei Einsatz eines der Verbindung (II) entsprechenden Carbonsäurechlorids wird die Kupplung mit der Amin-Komponente (III) in Gegenwart einer üblichen organischen Hilfsbase wie Triethylamin, *N*-Methylmorpholin, *N*-Methylpiperidin, *N,N*-Diisopropylethylamin, Pyridin, 4-*N,N*-Dimethylamino-pyridin, 1,8-Diazabicyclo[5.4.0]undec-7-en (DBU) oder 1,5-Diazabicyclo[4.3.0]non-5-en (DBN) durchgeführt. Bevorzugt wird Triethylamin oder *N,N*-Diisopropylethylamin verwendet.

Die Umsetzung des Amins (III) mit dem Carbonsäurechlorid erfolgt im Allgemeinen in einem Temperaturbereich von -20°C bis +60°C, bevorzugt im Bereich von -10°C bis +30°C.

Die Herstellung der Carbonsäurechloride selbst erfolgt auf übliche Weise durch Behandlung der Carbonsäure (II) mit Thionylchlorid oder Oxalylchlorid.

Die Abspaltung der Ester-Gruppe T¹ im Verfahrensschritt (IV) → (I) wird nach üblichen Methoden durchgeführt, indem man den Ester in inerten Lösungsmitteln mit Säuren oder Basen behandelt, wobei bei letzterer Variante das zunächst entstehende Salz durch Behandeln mit Säure in die freie Carbonsäure überführt wird. Im Falle der *tert*.-Butylester erfolgt die Esterspaltung vorzugsweise mit Säuren. Benzylester werden bevorzugt durch Hydrogenolyse (Hydrierung) in Gegenwart eines geeigneten Katalysators, wie beispielsweise Palladium auf Aktivkohle, abgespalten.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +100°C, bevorzugt bei 0°C bis +60°C.

Die Intermediate der Formel (II) können beispielsweise dadurch hergestellt werden, dass man einen Carbonsäureester der Formel (V) 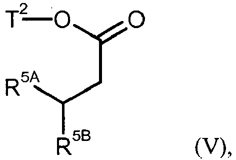 in welcher R^{5A} und R^{5B} die oben angegebenen Bedeutungen haben
und
- T²: für (C₁-C₄)-Alkyl oder Benzyl steht,
in einem inerten Lösungsmittel zunächst mit Hilfe einer Base deprotoniert, anschließend in Gegenwart eines geeigneten Palladium-Katalysators mit einem Phenylbromid der Formel (VI) 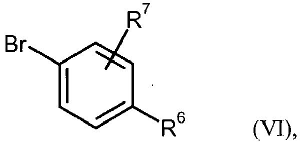 in welcher R⁶ und R⁷ die oben angegebenen Bedeutungen haben,
zu einer Verbindung der Formel (VII) 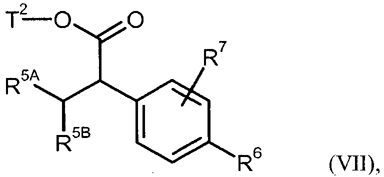 in welcher R^{5A}, R^{5B}, R⁶, R⁷ und T² die oben angegebenen Bedeutungen haben,
aryliert und nachfolgend den Ester-Rest T² durch basische oder saure Solvolyse oder im Fall, dass T² für Benzyl steht, auch durch Hydrogenolyse unter Erhalt der Carbonsäure (II) abspaltet.

Die Arylierungsreaktion im Verfahrensschritt (V) + (VI) → (VII) wird vorzugsweise in Toluol oder Toluol/Tetrahydrofuran-Gemischen in einem Temperaturbereich von +20°C bis +100°C durchgeführt. Als Base zur Deprotonierung des Esters (V) wird hierbei bevorzugt Lithium-bis(trimethylsilyl)amid eingesetzt. Geeignete Palladium-Katalysatoren sind beispielsweise Palladium(II)acetat oder Tris(dibenzylidenaceton)-dipalladium, jeweils in Kombination mit einem elektronenreichen, sterisch anspruchsvollen Phosphin-Liganden wie 2-Dicyclohexylphosphino-2'-(*N,N*-dimethylamino)-biphenyl oder 2-Di-*tert*.-butylphosphino-2'-(*N,N*-dimethylamino)biphenyl [vgl. z.B. W.A. Moradi, S.L. Buchwald, J. Am. Chem. Soc. 123, 7996-8002 (2001)].

Die Abspaltung der Ester-Gruppe T² im Verfahrensschritt (VII) → (II) erfolgt auf analoge Weise wie zuvor für den Ester-Rest T¹ beschrieben.

Intermediate der Formel (II-A) 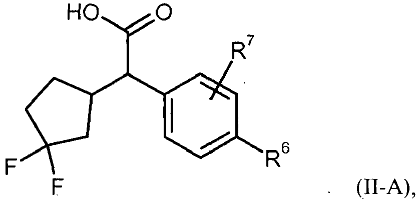 in welcher R⁶ und R⁷ die oben angegebenen Bedeutungen haben,
können alternativ auch dadurch hergestellt werden, dass man einen Phenylessigsäureester der Formel (VIII) 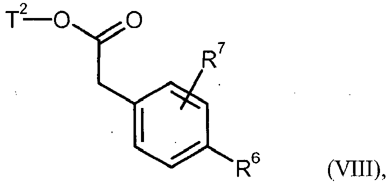 in welcher R⁶, R⁷ und T² die oben angegebenen Bedeutungen haben,
zunächst durch Basen-induzierte Addition an 2-Cyclopenten-1-on in eine Verbindung der Formel (IX) 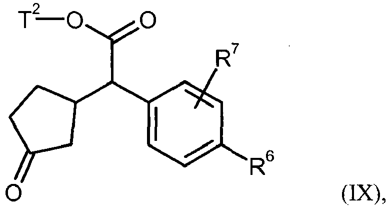 in welcher R⁶, R⁷ und T² die oben angegebenen Bedeutungen haben,
überführt, diese anschließend mit 1,1'-[(Trifluor-λ⁴-sulfanyl)imino]bis(2-methoxyethan) unter Bortrifluorid-Katalyse zu einer Verbindung der Formel (VII-A) 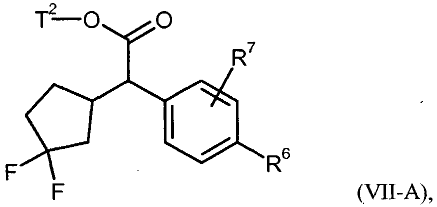 in welcher R⁶, R⁷ und T² die oben angegebenen Bedeutungen haben,
fluoriert und nachfolgend wiederum die Ester-Gruppe T² unter Erhalt der Carbonsäure (II-A) abspaltet.

Im Verfahrensschritt (VIII) → (IX) wird zur Deprotonierung des Esters (VIII) bevorzugt eine Amid-Base wie Lithiumdiisopropylamid oder Lithium-bis(trimethylsilyl)amid verwendet. Zur Deoxy-Fluorierung in der Transformation (IX) → (VII-A) können an Stelle des oben genannten 1,1'-[(Trifluor-λ⁴-sulfanyl)imino]bis(2-methoxyethan) ("Desoxofluor") gegebenenfalls auch andere bekannte Fluorierungsagentien, wie Diethylaminoschwefeltrifluorid (DAST) oder Morpholinoschwefeltrifluorid (Morpho-DAST), eingesetzt werden [zur Reaktionssequenz (VIII) → (IX) → (VII-A) vgl. z.B. T. Mase et al., J. Org. Chem. 66 (20), 6775-6786 (2001)].

Die Intermediate der Formel (III) können beispielsweise dadurch hergestellt werden, dass man entweder
[A] einen Phosphonoessigsäureester der Formel (X) 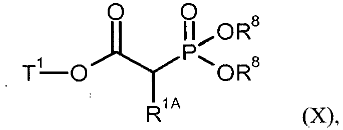 in welcher R^{1A} und T¹ die oben angegebenen Bedeutungen haben
   und
   - R⁸: für (C₁-C₄)-Alkyl steht,
   in einem inerten Lösungsmittel in einer Basen-induzierten Olefinierungsreaktion mit einer 3-Nitrobenzoyl-Verbindung der Formel (XI) 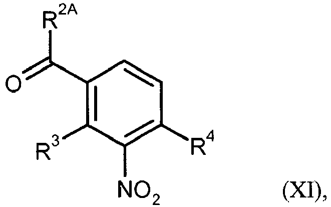 in welcher R^{2A}, R³ und R⁴ die oben angegebenen Bedeutungen haben,
   zu einer Verbindung der Formel (XII) 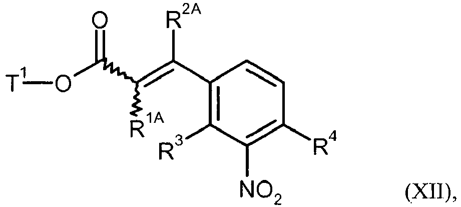 in welcher R^{1A}, R^{2A}, R³, R⁴ und T¹ die oben angegebenen Bedeutungen haben,
   umsetzt und diese dann in Gegenwart eines geeigneten Palladium- oder Platin-Katalysators zu einem 3-(3-Aminophenyl)propionsäureester der Formel (III-A) 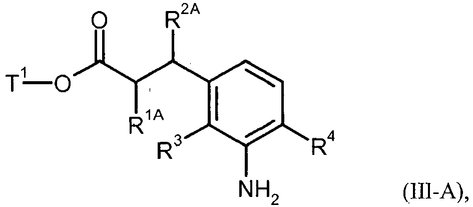 in welcher R^{1A}, R^{2A}, R³, R⁴ und T¹ die oben angegebenen Bedeutungen haben,
   hydriert,
   oder
[B] einen Acrylsäureester der Formel (XIII) 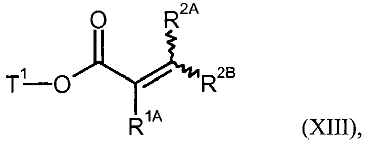 in welcher R^{1A}, R^{2A}, R^{2B} und T¹ die oben angegebenen Bedeutungen haben,
   in einem inerten Lösungsmittel entweder (i) unter Rhodium(I)-Katalyse mit einer Phenylboronsäure der Formel (XIV) 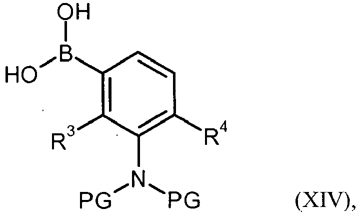 in welcher R³ und R⁴ die oben angegebenen Bedeutungen haben
   und
   - PG: für Benzyl oder *p*-Methoxybenzyl als inerte Amino-Schutzgruppe steht,
   oder (*ii*) unter Kupfer(I)-Katalyse mit einem Phenylmagnesium-Agens der Formel (XV) 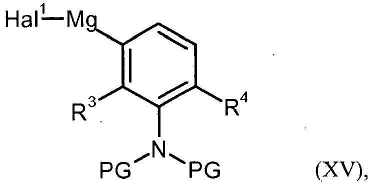 in welcher R³, R⁴ und PG die oben angegebenen Bedeutungen haben und
   - Hal¹: für Chlor oder Brom steht,
   zu einer Verbindung der Formel (XVI) 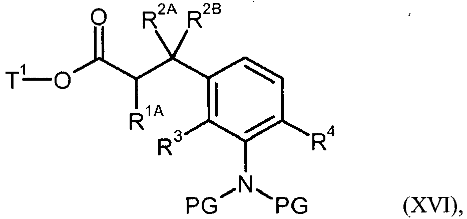 in welcher R^{1A}, R^{2A}, R^{2B}, R³, R⁴, PG und T¹ die oben angegebenen Bedeutungen haben,
   umsetzt und nachfolgend die Amino-Schutzgruppen PG nach üblichen Methoden durch Hydrogenolyse oder auf oxidativem Wege entfernt unter Erhalt eines 3-(3-Aminophenyl)-propionsäureesters der Formel (III-B) 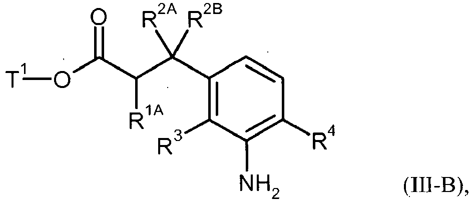 in welcher R^{1A}, R^{2A}, R^{2B}, R³, R⁴ und T¹ die oben angegebenen Bedeutungen haben,
   oder
[C] einen Acrylsäureester der Formel (XVII) 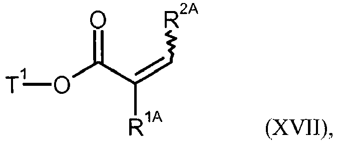 in welcher R^{1A}, R^{2A} und T¹ die oben angegebenen Bedeutungen haben,
   in einem inerten Lösungsmittel unter Palladium-Katalyse mit einem 3-Amino- oder 3-Nitro-phenylbromid der Formel (XVIII) 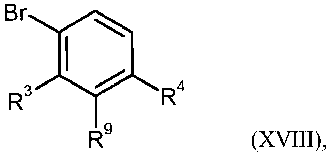 in welcher R³ und R⁴ die oben angegebenen Bedeutungen haben und
   - R⁹: für Amino oder Nitro steht,
   zu einer Verbindung der Formel (XIX) 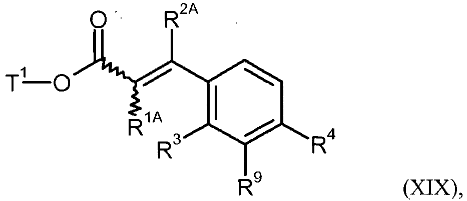 in welcher R^{1A}, R^{2A}, R³, R⁴, R⁹ und T¹ die oben angegebenen Bedeutungen haben,
   kuppelt und diese in Gegenwart eines geeigneten Palladium- oder Platin-Katalysators zum 3-(3-Aminophenyl)propionsäureester der Formel (III-C) 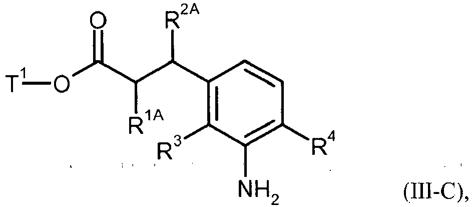 in welcher R^{1A}, R^{2A}, R³, R⁴ und T¹ die oben angegebenen Bedeutungen haben,
   hydriert,
   oder
[D] einen Ester der Formel (XX) 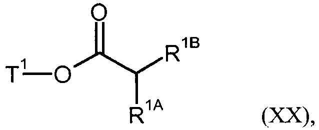 in welcher R^{1A}, R^{1B} und T¹ die oben angegebenen Bedeutungen haben,
   in einem inerten Lösungsmittel nach α-Deprotonierung mit einem 3-Brombenzylhalogenid der Formel (XXI) 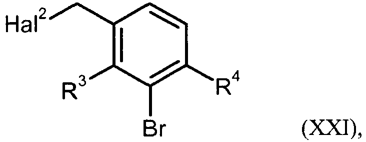 in welcher R³ und R⁴ die oben angegebenen Bedeutungen haben
   und
   - Hal²: für Chlor, Brom oder Iod steht,
   zu einer Verbindung der Formel (XXII) 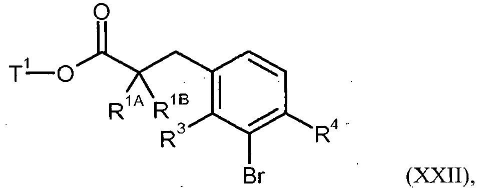 in welcher R^{1A}, R^{1B}, R³, R⁴ und T¹ die oben angegebenen Bedeutungen haben,
   alkyliert, anschließend mit Benzylamin in Gegenwart einer Base und eines Palladium-Katalysators zu einer Verbindung der Formel (XXIII) 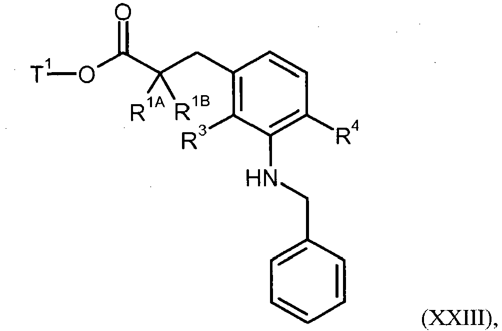 in welcher R^{1A}, R^{1B}, R³, R⁴ und T¹ die oben angegebenen Bedeutungen haben,
   umsetzt und die *N*-Benzylgruppe dann durch Hydrogenolyse unter Erhalt eines 3-(3-Amino-phenyl)propionsäureesters der Formel (III-D) 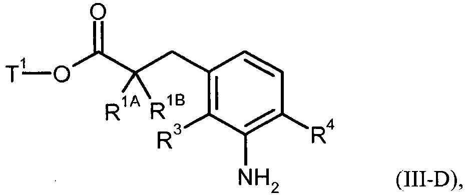 in welcher R^{1A}, R^{1B}, R³, R⁴ und T¹ die oben angegebenen Bedeutungen haben,
   entfernt.

Zur Deprotonierung des Phosphonester (X) in der Olefinierungsreaktion (X) + (XI) (XII) eignen sich insbesondere nicht-nukleophile, starke Basen wie beispielsweise Natrium- oder Kaliumhydrid, Lithium-, Natrium- oder Kalium-bis(trimethylsilyl)amid oder Lithiumdiisopropylamid; bevorzugt wird Natriumhydrid verwendet.

Die Hydrierung im Verfahrensschritt (XII) → (III-A) bzw. (XIX) → (III-C) wird in der Regel unter einer stationären Wasserstoffatmosphäre bei normalem Druck durchgeführt. Als Katalysator wird hierbei bevorzugt Palladium auf Aktivkohle (als Trägennaterial) eingesetzt. Die Entfernung der Amino-Schutzgruppe(n) in den Transformationen (XVI) → (III-B) und (XXIII) → (III-D) erfolgt üblicherweise durch Hydrogenolyse nach der gleichen Prozedur; im Falle, dass PG in (XVI) für *p*-Methoxybenzyl steht, kann dies alternativ auch auf oxidativem Wege geschehen, beispielsweise mit Hilfe von 2,3-Dichlor-5,6-dicyano-1,4-benzochinon (DDQ) oder Ammoniumcer(IV)nitrat.

Als Palladium-Katalysator für die Umsetzung (XVII) + (XVIII) → (XIX) [Heck-Reaktion] wird vorzugsweise Palladiwn(II)acetat in Kombination mit einem Phosphin-Liganden, wie beispielsweise Triphenyl- oder Tri-2-tolylphosphin, eingesetzt [zur Reaktion (XIII) + (XIV) → (XVI) vgl. z.B. N. Miyaura et al., Organometallics 16, 4229 (1997) sowie T. Hayashi, Synlett, Special Issue 2001, 879-887; zur Umsetzung (XIII) + (XV) → (XVI) vgl. z.B. P. Knochel et al., Tetrahedron 56, 2727-2731 (2000), Angew. Chem. 120, 6907-6911 (2008)].

Zur α-Deprotonierung des Esters (XX) in der Alkylierungsreaktion (XX) + (XXI) → (XXII) sind gleichfalls nicht-nukleophile, starke Basen besonders geeignet, wie beispielsweise Natrium- oder Kaliumhydrid, Lithium-, Natrium- oder Kalium-bis(trimethylsilyl)amid oder Lithiumdiisopropylamid; bevorzugt wird hier Lithiumdiisopropylamid verwendet.

Für die Reaktion (XXII) + Benzylamin → (XXIII) [Buchwald-Hartwig-Kupplung] wird bevorzugt Tris(dibenzylidenaceton)dipalladium(0) als Palladium-Katalysator in Verbindung mit (±)-2,2'-Bis-(diphenylphosphino)-1,1'-binaphthyl als Phosphin-Liganden und Natrium- oder Kalium-*tert*.-butylat als Base verwendet [vgl. z.B. J. P. Wolfe und S. L. Buchwald, Organic Syntheses, Coll. Vol. 10, 423 (2004), Vol. 78, 23 (2002)].

Die zuvor beschriebenen Verfahrensschritte können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (III), (IV), (VII), (XVI), (XXII) oder (XXIII) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Vorzugsweise werden chromatographische Verfahren an achiralen bzw. chiralen Trennphasen angewandt; im Falle von Carbonsäuren als Zwischen- oder Endprodukten kann alternativ auch eine Trennung über diastereomere Salze erfolgen.

Die Verbindungen der Formeln (V), (VI), (VIII), (X), (XI), (XIII), (XIV), (XV), (XVII), (XVIII), (XX) und (XXI) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden: 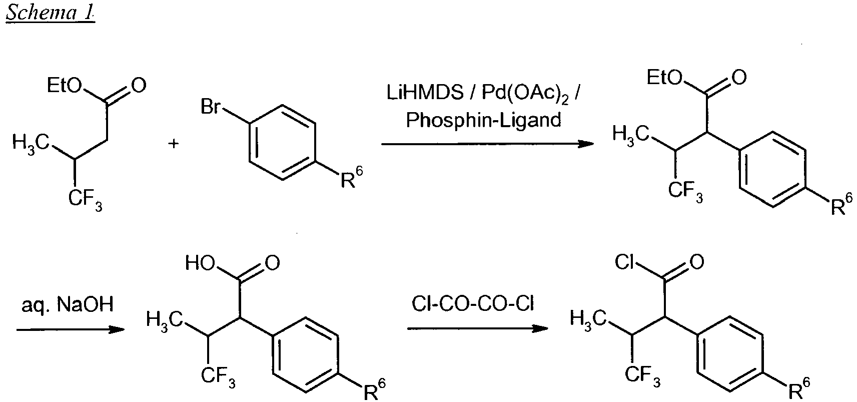 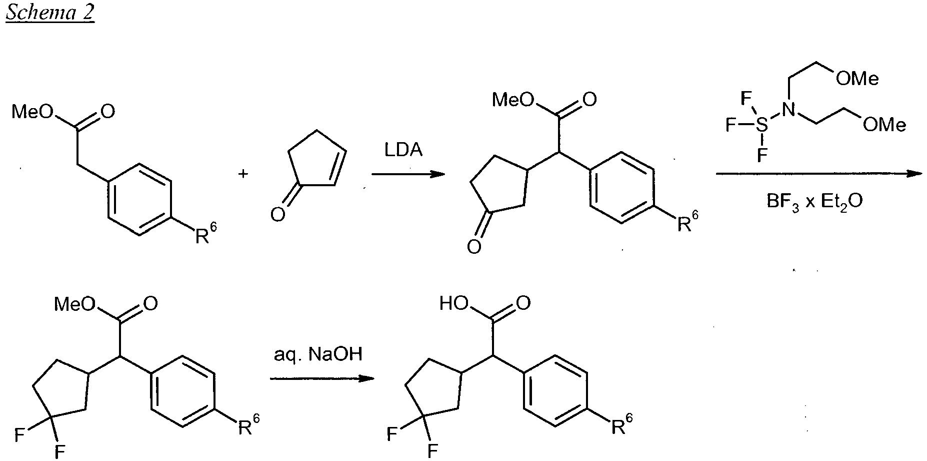 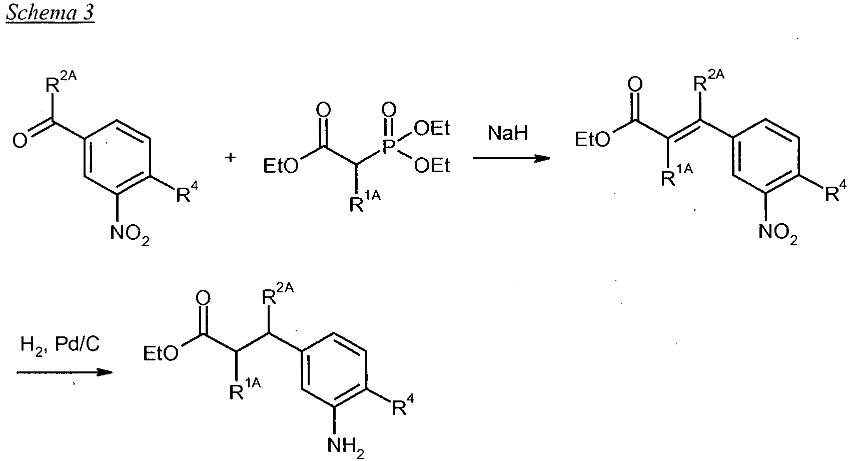 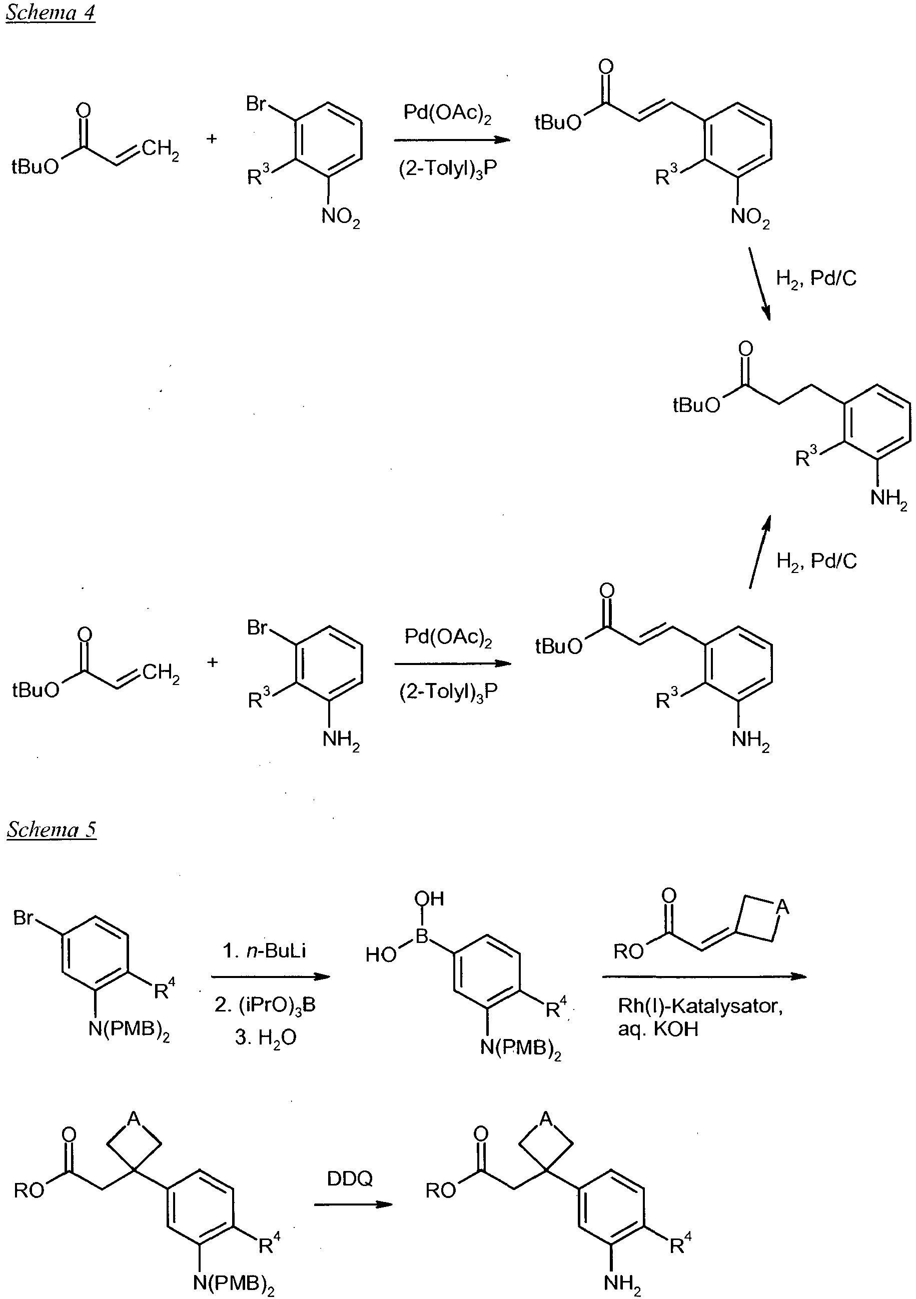 [PMB = *p*-Methoxybenzyl; A = CH₂ oder O; R = Methyl oder Benzyl].

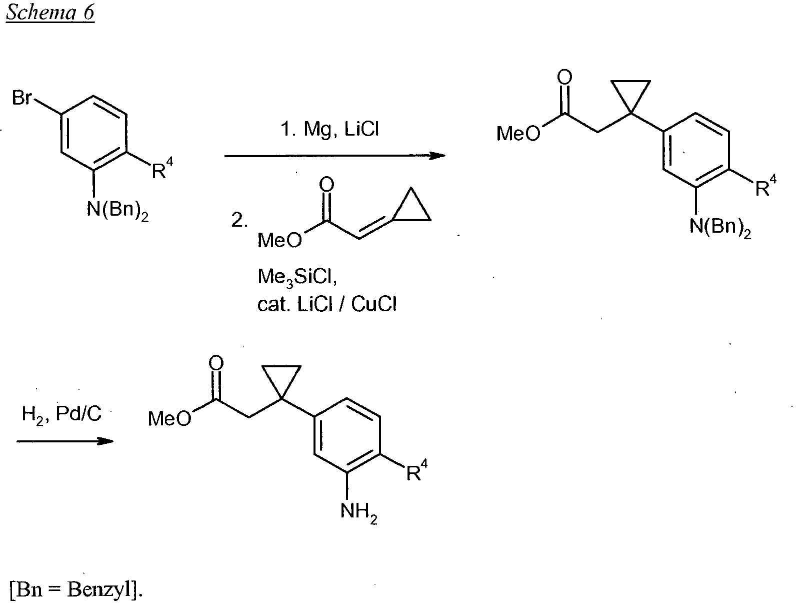

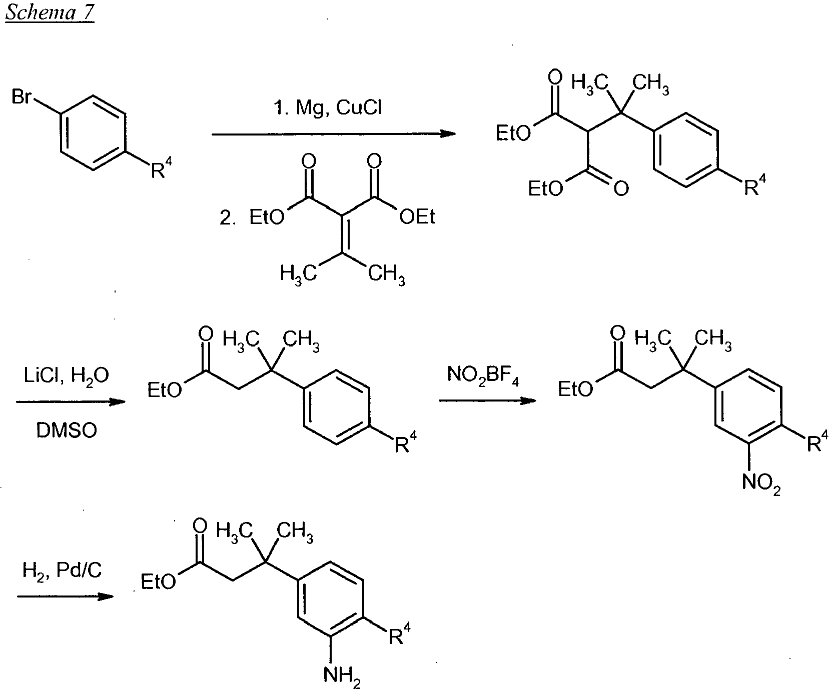

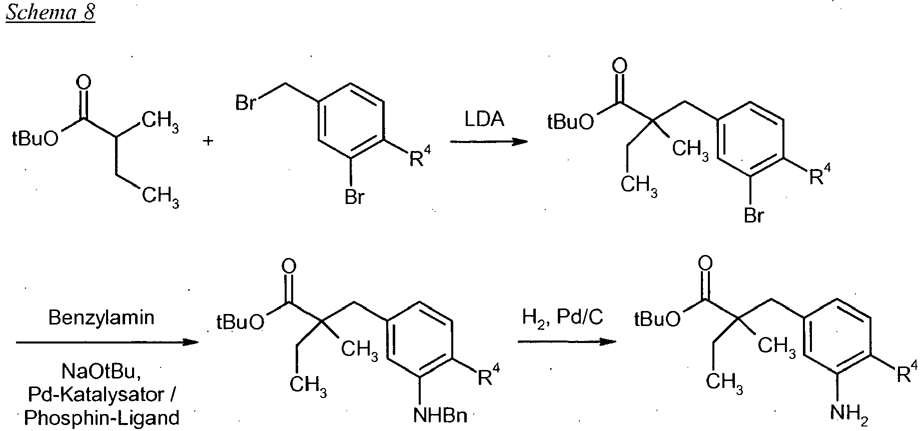

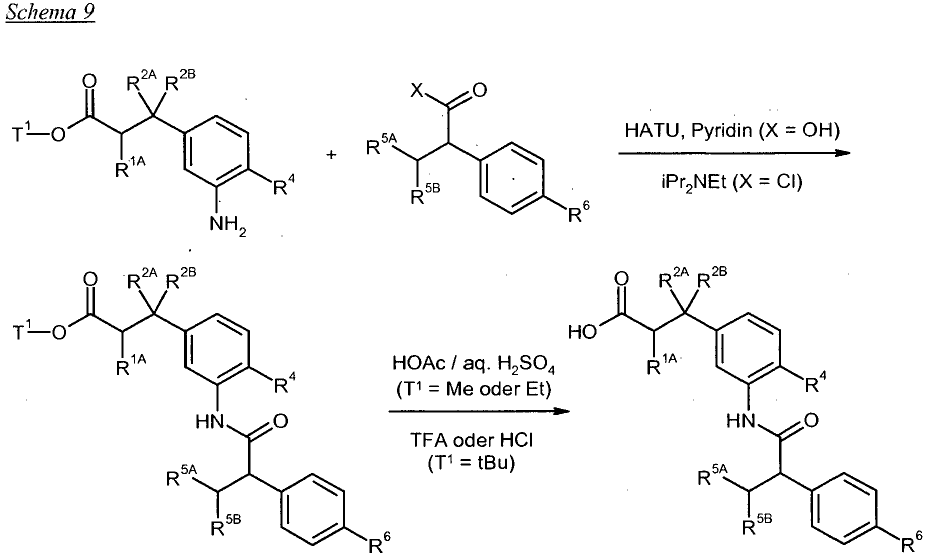

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Darüber hinaus verfügen die erfindungsgemäßen Verbindungen über gute pharmakokinetische Eigenschaften, insbesondere bezüglich ihrer Bioverfügbarkeit und ihrer Halbwertszeit im Körper.

Die erfindungsgemäßen Verbindungen können daher in Arzneimitteln zur Behandlung und/oder Prävention von kardiovaskulären Erkrankungen wie beispielsweise des Bluthochdrucks (Hypertonie) und der Herzinsuffizienz, stabiler und instabiler Angina pectoris, pulmonaler arterieller Hypertonie (PAH) und anderer Formen der pulmonalen Hypertonie (PH), renaler Hypertonie, peripheren und kardialen Gefäßerkrankungen sowie von Arrhythmien, zur Behandlung von thromboembolischen Erkrankungen und Ischämien wie Myokardinfarkt, Hirnschlag, transistorischen und ischämischen Attacken sowie peripheren Durchblutungsstörungen, zur Verhinderung von Restenosen wie nach Thrombolysetherapien, percutan-trinsluininalen Angioplastien (PTA), percutan-transluminalen Koronarangioplastien (PTCA) und Bypass, zur Behandlung von Arteriosklerose, zur Förderung der Wundheilung sowie zur Behandlung von Osteoporose, Glaukom und Gastroparese eingesetzt werden.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Die erfindungsgemäßen Verbindungen können darüber hinaus zur Verhinderung von ischämieund/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis , akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immmkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nepluosklerose und nephrotisches Syndrom, welche diagnostisch beispielsweise durch abnorm verminderte Kreatininund/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise von überaktiver Blase, Blasenentleerungsstörungen, unterem Harnwegssyndrom (LUTS), Inkontinenz, benigner Prostatahyperplasie (BPH), erektiler Dysfunktion und weiblicher sexueller Dysfunktion.

Die erfindungsgemäßen Verbindungen können ferner zur Behandlung von asthmatischen Erkrankungen, chronisch-obstruktiven Atemwegserkrankungen (COPD) und von Respiratory Distress-Syndromen eingesetzt werden.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impainnent", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Kranklneit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannung- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis, multiplem Organversagen, entzündlichen Erkrankungen der Niere, chronischen Darmentzündungen wie Colitis ulcerosa und Morbus Crohn, Pankreatitis, Peritonitis, rheumatoiden Erkrankungen, entzündlichen Hauterkrankungen und entzündlichen Augenerkrankungen eingesetzt werden.

Aufgrund ihres Wirkprofils eignen sich die erfindungsgemäßen Verbindungen insbesondere zur Behandlung und/oder Prävention kardiovaskulärer Erkrankungen wie Herzinsuffizienz, Angina pectoris, Hypertonie, pulmonale Hypertonie, Ischämien, Gefäßerkrankungen, Mikrozirkulationsstörungen, thromboembolische Erkrankungen und Arteriosklerose.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgeinäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Motsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-B locker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absoptionshemmer, Lipase-Inhibitoren, polymeren Gallensäureadsorber, Gallensäure-Reabsoptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezep-tor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranoloi, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie

HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inbibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, phannazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem

Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- AIBN: 2,2'-Azobis-(2-methylpropionitril)
- aq.: wässrig, wässrige Lösung
- ATP: Adenosin-5'-triphosphat
- Bn: Benzyl
- Brij^{®}: Polyethylenglycoldodecylether
- BSA bovines: Serumalbumin
- Bsp.: Beispiel
- Bu: Butyl
- c: Konzentration
- ca.: *circa,* ungefähr
- cat.: katalytisch
- CI: chemische Ionisation (bei MS)
- d: Tag(e)
- DAST: Diethylaminoschwefeltrifluorid
- DC: Dünnschichtchromatographie
- DCI: direkte chemische Ionisation (bei MS)
- DDQ: 2,3-Dichlor-5,6-dicyano-1,4-benzochinon
- de: Diastereomerenüberschuss
- DIBAH: Diisobutylaluminiumilydrid
- DMF: Dimethylformamid
- DMSO: Dimethylsulfoxid
- d. Th.: der Theorie (bei Ausbeute)
- DTT: Dithiothreitol
- EDC: *N*'-(3-Dimethylaminopropyl)-*N*-ethylcarbodiimid-Hydrochlorid
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- GC: Gaschromatographie
- ges.: gesättigt
- GTP: Guanosin-5'-triphosphat
- h: Stunde(n)
- HATU: *O*-(7-Azabenzotriazol-1-yl)-*N*,*N,N',N'*-tetramethyluronium-Hexafluorophosphat
- HOBt: 1-Hydroxy-*1H*-benzotriazol-Hydrat
- HPLC: Hochdruck-, Hochleistungsftüssigchromatographie
- iPr: Isopropyl
- konz.: konzentriert
- LC-MS: Flüssigchromatographie-gekoppelte Massenspektroskopie
- LDA: Lithiumdiisopropylamid
- LiHMDS: Lithiumhexamethyldisilazid [Lithium-bis(trimethylsilyl)amid]
- Me: Methyl
- min: Minute(n)
- MS: Massenspektroskopie
- NBS: *N*-Bromsuccinimid
- NMR: Kernresonanzspektroskopie
- *p*: para
- Pd/C: Palladium auf Aktivkohle
- Ph: Phenyl
- PMB: *p*-ethoxybenzyl
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC)
- s.o.: siehe oben
- tBu: *tert.*-Butyl
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (LC-MS):

Gerätetyp MS: Micromass ZQ; Gerätetyp HPLC: HP 1100 Series; UV DAD; Säule: Phenomenex Gemini 3 µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 4 (LC-MS):

Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 5 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 6 (LC-MS):

Instrument: Waters Acquity SQD UPLC System; Säule: Waters Acquity UPLC HSS T3 1.8 µ, 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 7 (LC-MS):

Gerätetyp MS: Waters ZQ; Gerätetyp HPLC: Agilent 1100 Series; UV DAD; Säule: Thermo Hypersil GOLD 3 µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (12 min halten).

### Methode 9 (LC-MS):

Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.3 min 90% A → 3.0 min 10% A → 4.8 min 10% A; Fluss: 0.33 ml/min; Ofen:50°C; UV-Detektion: 210 nm.

### Methode 10 (GC-MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Ausganasverbindunsen und Intermediate:

### Beispiel 1A

### tert.-Butyl-3-(3-amino-2-methylphenyl)propanoat

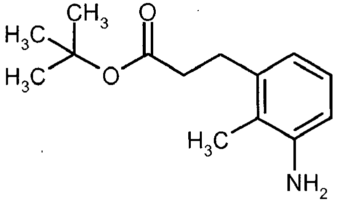

Unter Argon wurden 201 mal (1.39 mol) *tert*.-Butyl-prop-2-enoat zu einer Lösung von 100 g (463 mmol) 1-Brom-2-methyl-3-nitrobenzol, 322 ml (2.31 mol) Triethylamin, 28.18 g (92.58 mmol) Tri-2-tolylphosphin und 10.39 g (46.29 mmol) Palladium(II)acetat in 2 Liter DMF getropft und die Mischung anschließend 36 h bei 125°C gerührt. Nach Abkühlen auf Raumtemperatur wurde das Reaktionsgemisch mit gesättigter wässriger Ammoniumchlorid-Lösung verrührt und die organische Phase abgetrennt. Die wässrige Phase wurde dreimal mit *tert*.-Butylmethylether extrahiert, und die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 9:1). Es wurden 89 g (338 mmol, 73% d. Th.) des Zwischenprodukts *tert*.-Butyl-(2*E*)-3-(2-methyl-3-nitrophenyl)prop-2-enoat als farbloser Feststoff erhalten. 88 g (334 mmol) dieses Feststoffs wurden in 2 Liter Ethanol gelöst, bei Raumtemperatur mit 7 g Palladium auf Kohle (10%) versetzt und 18 h unter Normaldruck hydriert. Nach vollständiger Umsetzung wurde die Reaktionslösung über Kieselgur filtriert und das erhaltene Filtrat im Vakuum eingeengt. Es wurden 61.3 g (260.5 mmol, 78% d. Th.) der Titelverbindung als farbloser Feststoff erhalten.

LC-MS (Methode 2): Rₜ = 1.84 min; m/z = 236 (M+H)⁺.

¹H-NMR (400 MHz, DMSO-d₆, δ/ppm): 6.77 (1H, t), 6.47 (1H, d), 6.36 (1H, d), 4.72 (2H, s), 2.14 (2H, t), 2.36 (2H, t), 1.95 (3H, s), 1.39 (9H, s).

### Beispiel 2A

### Ethyl-3-(3-amino-2-methylphenyl)propanoat

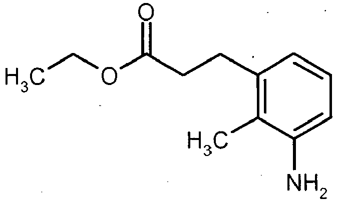

Unter Argon wurden 10.844 g (108 mmol) Ethyl-prop-2-enoat zu einer Lösung von 7.8 g (36.1 mmol) 1-Brom-2-methyl-3-nitrobenzol, 25 ml (180.5 mmol) Triethylamin, 2.197 g (7.22 mmol) Tri-2-tolylphosphin und 810 mg (3.6 mmol) Palladium(II)acetat in 200 ml DMF getropft und die Mischung anschließend 36 h bei 125°C gerührt. Nach Abkühlen auf Raumtemperatur wurde das Reaktionsgemisch mit gesättigter wässriger Ammoniumchlorid-Lösung verrührt und die organische Phase abgetrennt. Die wässrige Phase wurde dreimal mit *tert*.-Butyl-methylether extrahiert, und die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 3:1). Es wurden 6.6 g (27.2 mmol, Gehalt 97%, 75% d. Th.) des Zwischenprodukts Ethyl-(2*E*)-3-(2-methyl-3-nitrophenyl)prop-2-enoat als farbloser Feststoff erhalten. 6.6 g (27.2 mmol, Gehalt 97%) dieses Feststoffs wurden in 200 ml Ethanol gelöst, bei Raumtemperatur mit 500 mg Palladium auf Kohle (10%) versetzt und über Nacht unter Normaldruck hydriert. Nach vollständiger Umsetzung wurde die Reaktionslösung über Kieselgur filtriert und das erhaltene Filtrat im Vakuum eingeengt. Es wurden 5.47 g (26.38 mmol, Gehalt 97%, 97% d. Th.) der Titelverbindung als farbloser Feststoff erhalten.

LC-MS (Methode 3): Rₜ = 1.07 min; m/z = 208 (M+H)⁺.

### Beispiel 3A

### tert.-Butyl-(2E)-3-(4-fluor-3-nitrophenyl)acrylat

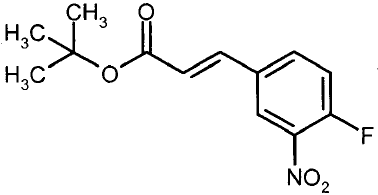

Unter Argon wurden 0.65 g (16.3 mmol) Natriumhydrid (als 60%-ige Suspension in Mineralöl) in 25 ml THF vorgelegt und auf 0°C gekühlt. Anschließend wurden 4.29 g (17 mmol) Diethyl-phosphonoessigsäure-*tert*.-butylester langsam zugetropft. Nach 30 min wurden 2.5 g (14.8 mmol) 4-Fluor-3-nitrobenzaldehyd zugegeben. Das Reaktionsgemisch wurde 3 h bei RT gerührt, dann in 100 ml Wasser gegossen und dreimal mit jeweils 100 ml Ethylacetat extrahiert. Die vereinigten organischen Phasen wurden über Magnesiumsulfat getrocknet und eingeengt. Der Rückstand wurde flash-chromatographisch gereinigt (Kieselgel, Laufmittel Cyclohexan/Ethylacetat 50:1). Es wurden 3.37 g (85% d. Th.) der Titelverbindung erhalten.

GC-MS (Methode 1): Rₜ = 6.45 min; m/z = 211 (M-^{t}But)⁺.

¹H-NMR (400 MHz, DMSO-d₆): δ [ppm] = 1.49 (s, 9H), 6.69 (d, 1H), 7.59-7.76 (m, 2H), 8.19 (ddd, 1H), 8.50 (dd, 1H).

### Beispiel 4A

### 3-(3-Amino-4-fluorphenyl)propansäure-tert.-butylester

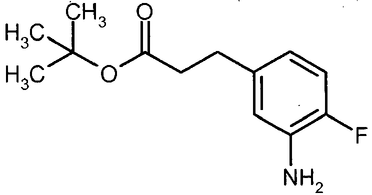

535 mg (2.00 mmol) (2*E*)-3-(4-Fluor-3-nitrophenyl)prop-2-ensäure-*tert*.-butylester wurden in 1 ml Ethanol und 1 ml THF gelöst und mit 21.3 mg Palladium auf Kohle (10%) versetzt. Bei RT wurde über Nacht unter einer Wasserstoffatmosphäre bei Normaldruck hydriert. Das Reaktionsgemisch wurde dann über Kieselgur abgesaugt, der Rückstand mit THF nachgewaschen und das Filtrat eingeengt. Es wurden 479 mg (100% d. Th.) der Titelverbindung erhalten.

LC-MS (Methode 6): Rₜ = 1.06 min; m/z = 184 (M-C₄H₈)⁺.

¹H-NMR (400 MHz, DMSO-d₆): δ = 6.84 (dd, 1H), 6.58 (dd, 1H), 6.36-6.29 (m, 1H), 5.00 (s, 2H), 2.64 (t, 2H), 2.42 (t, 2H), 1.36 (s, 9H).

### Beispiel 5A

### tert.-Butyl-(2E)-3-(4-chlor-3-nitrophenyl)prop-2-enoat

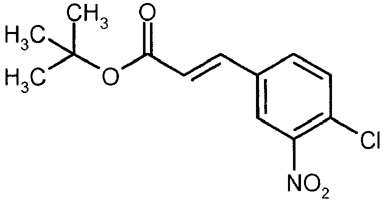

Unter Argon wurden 1.19 g (29.64 mmol, 60%-ig) Natriumhydrid in 25 ml Toluol und 25 ml THF suspendiert und auf 0°C abgekühlt. Anschließend wurden 7.28 ml (30.99 mmol) *tert*.-Butyl-(di-ethoxyphosphoryl)acetat langsam zugetropft und das Gemisch 30 min bei 0°C gerührt. Danach wurden 5 g (26.94 mmol) 4-Chlor-3-nitrobenzaldehyd zur Reaktionsmischung gegeben und der Ansatz dann auf Raumtemperatur erwärmt. Das Gemisch wurde 2 h bei Raumtemperatur gerührt und anschließend mit 50 ml Wasser versetzt. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 9:1). Es wurden 6.77 g (23.86 mmol, 77% d. Th.) der Titelverbindung erhalten.

MS (DCI): m/z = 301 (M+NH₄)⁺.

¹H-NMR (400 MHz, DMSO-d₆): δ = 8.46 (d, 1H), 8.07 (dd, 1H), 7.71 (d, 1H), 7.51 (d, 1H), 6.75 (d, 1H), 1.49 (s, 9H).

### Beispiel 6A

### tert.-Butyl-3-(3-amino-4-chlorphenyl)propanoat

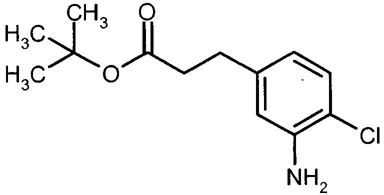

Eine Lösung von 6.74 g (23.76 mmol) *tert*.-Butyl-(2*E*)-3-(4-chlor-3-nitrophenyl)prop-2-enoat in 200 ml Ethanol und 20 ml THF wurde bei Raumtemperatur mit 500 mg Palladium auf Kohle (10%) versetzt und 12 h unter Normaldruck hydriert. Nach vollständiger Umsetzung (DC-Kontrolle; Laufmittel Cyclohexan/Essigsäureethylester 1:1) wurde die Reaktionslösung über Kieselgur filtriert und das Filtrat im Vakuum eingeengt. Das Rohprodukt wurde über Kieselgel chromatographisch gereinigt (Laufmittel Cyclohexan/Essigsäureethylester 4:1 → 2:1). Es wurden 1.40 g (5.47 mmol, 23% d. Th.) der Titelverbindung erhalten.

LC-MS (Methode 6): Rₜ = 1.14 min; m/z = 256 (M+H)⁺.

¹H-NMR (400 MHz, DMSO-d₆): δ = 7.08 (d, 1H), 6.62 (s, 1H), 6.39 (dd, 1H), 5.22 (s, 2H), 2.66 (t, 2H), 2.45 (t, 2H), 1.37 (s, 9H).

### Beispiel 7A

### Methyl-3-(3-amino-4-chlorphenyl)propanoat

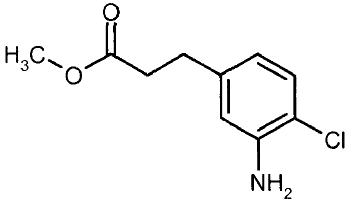

Zu einer Lösung von 1.0 g (3.91 mmol) *tert*.-Butyl-3-(3-amino-4-chlorphenyl)propanoat in 20 ml Methanol wurden unter Rückfluss 0.86 ml (11.7 mmol) Thionylchlorid getropft. Die Mischung wurde 1.5 h unter Rückfluss gerührt und dann nach Abkühlen mit Dichlormethan verdünnt. Die Lösung wurde auf Wasser gegeben, und nach Phasentrennung wurde die organische Phase mit gesättigter Natriumhydrogencarbonat-Lösung und gesättigter Natriumchlorid-Lösung gewaschen, über Natriumsulfat getrocknet und im Vakuum eingeengt. Der Rückstand wurde im Hochvakuum getrocknet. Es wurden 745 mg (90.3% d. Th.) der Zielverbindung erhalten.

LC-MS (Methode 6): Rₜ = 0.91 min; m/z = 213/215 (M+H)⁺.

¹H-NMR (400 MHz, DMSO-d₆): δ [ppm] = 2.52-2.60 (m, 2H), 2.62-2.77 (m, 2H), 5.22 (s, 2H), 6.39 (dd, 1H), 6.62 (d, 1H), 7.06 (d, 1H).

### Beispiel 8A

### Methyl-{1-[3-(dibenzylamino)-4-fluorphenyl]cyclopropyl}acetat

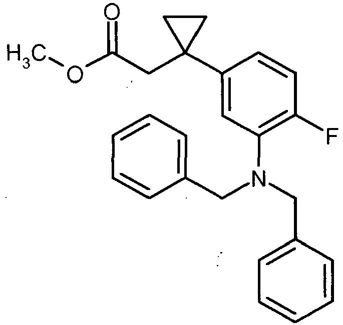

Herstellung von Lösung A: 688 mg (16.2 mmol) Lithiumchlorid wurden unter Argon in 50 ml THF gelöst und anschließend mit 789 mg (32.5 mmol) Magnesiumspäne und 23 µl (0.023 mmol) einer 1 M Lösung von Diisobutylaluminiumhydrid in THF versetzt. Die Reaktionslösung wurde 10 min bei Raumtemperatur gerührt und dann auf -10°C abgekühlt. Nachfolgend wurden 5 g (13.5 mmol) *N,N*-Dibenzyl-5-brom-2-fluoranilin (CAS Reg.-Nr. 869529-97-5) zugesetzt und die Lösung ca. 1 h bei -10°C nachgerührt.

Herstellung von Lösung B: 110 mg (2.6 mmol) Lithiumchlorid und 128 mg (1.3 mmol) Kupfer(I)-chlorid wurden unter Argon bei Raumtemperatur in 10 ml THF suspendiert und anschließend mit 1.65 ml (12.98 mmol) Chlor(trimethyl)silan sowie 1.46 g (12.98 mmol) Methyl-cyclopropyliden-acetat (CAS Reg.-Nr. 110793-87-8) versetzt. Die Lösung wurde danach noch 1 h bei RT nachgerührt.

Die oben erhaltene Lösung A wurde auf -40°C abgekühlt. Anschließend wurde langsam die Lösung B zugetropft. Die nun vereinigten Lösungen wurden langsam auf -20°C erwärmt und 1 h bei dieser Temperatur nachgerührt. Dann wurden 50 ml einer eiskalten, halbgesättigten Ammoniumchlorid-Lösung zur Reaktionsmischung gegeben. Nach Trennung der Phasen wurde die wässrige Phase noch dreimal mit Essigsäureethylester extrahiert, die vereinigten organischen Phasen über Magnesiumsulfat getrocknet und bis zur Trockene eingeengt. Das erhaltene Rohprodukt wurde über Kieselgel chromatographisch gereinigt (Laufmittel Cyclohexan/Essigsäureethylester 10:1). Es wurden 2.1 g (5.2 mmol, 39% d. Th.) der Titelverbindung isoliert.

¹H-NMR (400 MHz, DMSO-d₆, δ/ppm): 7.33-7.25 (8H, m), 7.25-7.18 (2H, m), 7.02-6.94 (1H, m), 6.78-6.69 (2H, m), 4.27 (4H, s), 3.43 (3H, s), 2.48 (2H, s), 0.78-0.73 (2H, m), 0.63-0.58 (2H, m).

LC-MS (Methode 5): Rₜ = 2.99 min; m/z = 404 (M+H)⁺.

### Beispiel 9A

### Methyl-[1-(3-amino-4-fluorphenyl)cyclopropyl]acetat

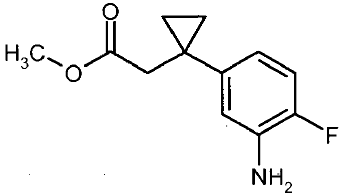

Eine Lösung von 2.1 g (5.2 mmol) Methyl- {1-[3-(dibenzylamino)-4-fluorphenyl]cyclopropyl} acetat in 100 ml Ethanol wurde bei Raumtemperatur mit 200 mg Palladium auf Kohle (10%) versetzt und 12 h bei Normaldruck hydriert. Nach vollständiger Umsetzung (DC-Kontrolle; Laufmittel Cyclohexan/Essigsäureethylester 1:1) wurde die Reaktionslösung über Kieselgur filtriert und das Filtrat im Vakuum eingeengt. Das Rohprodukt wurde über Kieselgel chromatographisch gereinigt (Laufmittel Cyclohexan/Essigsäureethylester 10:1). Es wurden 647 mg (2.9 mmol, 56% d. Th.) der Titelverbindung erhalten.

¹H-NMR (400 MHz, DMSO-d₆, δ/ppm): 6.88-6.78 (1H, m), 6.70-6.62 (1H, m), 6.44-6.35 (1H, m), 4.98 (2H, br. s), 3.51 (3H, s), 2.55 (2H, s), 0.84-0.79 (2H, m), 0.78-0.73 (2H, m).

GC-MS (Methode 1): Rₜ = 5.67 min; m/z = 224 (M+H)⁺.

### Beispiel 10A

### 5-Brom-2-chlor-N,N-bis(4-methoxybenzyl)anilin

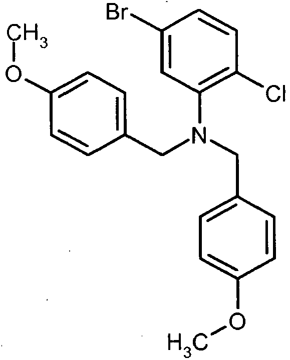

Unter Argon wurden 5.07 g (126.93 mmol, 60%-ig) Natriumhydrid in 150 ml THF suspendiert und auf 0°C abgekühlt. Anschließend wurden 10.70 g (51.81 mmol) 5-Brom-2-chloranilin, gelöst in 10 ml THF, langsam zugetropft und das Gemisch 30 min bei 0°C gerührt. Danach wurden 25 g (124.34 mmol) 4-Methoxybenzylchlorid zur Reaktionsmischung gegeben und der Ansatz dann auf Raumtemperatur erwärmt. Das Gemisch wurde 2 h bei RT gerührt und anschließend langsam auf 150 ml Eiswasser gegossen. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Roh-produkt wurde chromatographisch gereinigt [Säule: Kromasil Si 6012, 350 mm x 30 mm; Eluent A: Isohexan, Eluent B: Essigsäureethylester; Gradient: 0 min 98% A → 4.65 min 98% A → 13 min 87% A → 13.01 min 98% A → 13.28 min 98% A; Fluss: 70 ml/min; Temperatur: 20°C; UV-Detektion: 265 nm]. Es wurden 12.37 g (27.69 mmol, 57% d. Th.) der Titelverbindung erhalten.

¹H-NMR (400 MHz, DMSO-d₆, δ/ppm): 7.37 (1H, d), 7.26-7.19 (5H, m), 7.19-7.14 (1H, m), 6.86 (4H, d), 4.11 (4H, s), 3.71 (6H, s).

LC-MS (Methode 4): Rₜ = 1.68 min; m/z = 446 (M)⁺.

### Beispiel 11A

### {3-[Bis(4-methoxybenzyl)amino]-4-chlorphenyl}boronsäure

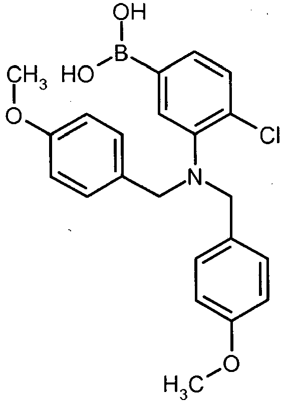

Unter Argon bei -78°C wurden zu einer Lösung von 5.2 g (11.64 mmol) 5-Brom-2-chlor-*N*,*N*-bis(4-methoxybenzyl)anilin in 100 ml THF/Diethylether (1:1) langsam 6.1 ml (15.25 mmol) einer 2.5 M Lösung von n-Butyllithium in Hexan getropft. Nachdem die Reaktionslösung 60 min bei -78°C nachgerührt worden war, wurden langsam 4.3 ml (18.62 mmol) Triisopropylborat hinzugefügt. Die Reaktionslösung wurde anschließend noch 15 min bei -78°C nachgerührt, dann langsam auf Raumtemperatur erwärmt und noch 3 h bei dieser Temperatur gerührt. Danach wurden 150 ml Eiswasser zudosiert. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel: zunächst Cyclohexan/ Essigsäureethylester 10:1 → 9:1 → 4:1, dann Dichlormethan/Methanol 95:5). Es wurden so 2.54 g (6.17 mmol, 53% d. Th.) der Titelverbindung erhalten.

LC-MS (Methode 6): Rₜ = 1.20 min; m/z = 412 (M+H)⁺.

### Beispiel 12A

### Benzyl-oxetan-3-ylidenacetat

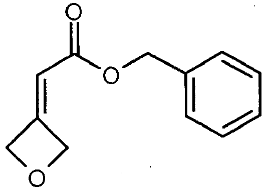

Unter Argon wurden bei 0°C 3.0 g (41.63 mmol) Oxetan-3-on (CAS Reg.-Nr. 6704-31-0) in 50 ml Dichlormethan gelöst und anschließend mit 18.8 g (45.79 mmol) Benzyl(triphenyl-λ⁵-phosphanyliden)acetat versetzt. Danach wurde das Reaktionsgemisch langsam auf Raumtemperatur erwärmt und noch 15 min nachgerührt. Die Reaktionslösung wurde dann bis zur Trockene eingeengt. Der Rückstand wurde in 25 ml Diethylether aufgenommen und verrührt und das Gemisch 12 h bei 4°C gelagert. Das ausgefallene Triphenylphosphinoxid wurde abfiltriert und das Filtrat bis zur Trockene eingeengt. Das erhaltene Rohprodukt wurde über Kieselgel chromatographisch gereinigt (Laufmittel Cyclohexan/Essigsäureethylester 4:1 → 1:1). Es wurden 4.2 g (20.57 mmol, 49% d. Th.) der Titelverbindung erhalten.

¹H-NMR (400 MHz, DMSO-d₆, δ/ppm): 7.42-7.30 (5H, m), 5.85-5.80 (1H, m), 5.39-5.34 (2H, m), 5.27-5.22 (2H, m), 5.13 (2H, s).

MS (DCI): m/z = 205 (M+H)⁺.

### Beispiel 13A

### Benzyl-(3-{3-[bis(4-methoxybenzyl)amino]-4-chlorphenyl}oxetan-3-yl)acetat

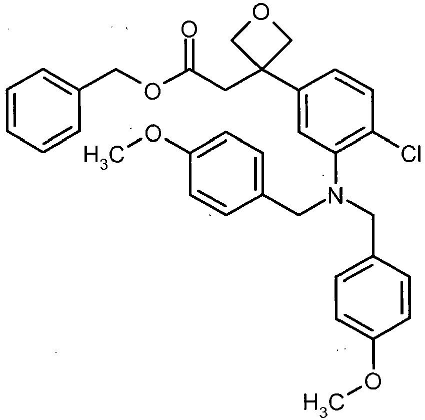

Unter Argon wurde bei Raumtemperatur zu einer Lösung von 45 mg (0.09 mmol) (1Z,5Z)-Cyclo-octa-1,5-dien-Rhodium(I)chlorid-Dimer in 25 ml Dioxan nacheinander 1.6 ml (2.37 mmol) einer 1.5 M wässrigen Kaliumhydroxid-Lösung, 272 mg (1.82 mmol) Benzyl-oxetan-3-ylidenacetat sowie 750 mg (1.82 mmol) {3-[Bis(4-methoxybenzyl)amino]-4-chlorphenyl}boronsäure gegeben. Anschließend wurde die Reaktionslösung 4 h bei Raumtemperatur gerührt. Nach erfolgter Umsetzung wurde die Lösung bis zur Trockene eingeengt und der Rückstand in 25 ml Wasser und 25 ml Essigsäureethylester aufgenommen. Nach Trennung der Phasen wurde die wässrige Phase noch dreimal mit Essigsäureethylester extrahiert, die vereinigten organischen Phasen über Magnesiumsulfat getrocknet und bis zur Trockene eingeengt. Das erhaltene Rohprodukt wurde über Kieselgel chromatographisch gereinigt (Laufmittel Cyclohexan/Essigsäureethylester 4:1 → 1:1). Es wurden 669 mg (1.17 mmol, 64% d. Th.) der Titelverbindung isoliert.

¹H-NMR (400 MHz, DMSO-d₆, δ/ppm): 7.34-7.26 (4H, m), 7.18-7.13 (4H, m), 7.12-7.07 (2H, m), 6.86-6.76 (6H, m), 4.90 (2H, s), 4.72 (2H, d), 4.57 (2H, d), 4.01 (6H, s), 3.08 (2H, s).

LC-MS (Methode 6): Rₜ = 1.45 min; m/z = 572 (M)⁺.

Analog Synthesebeispiel 13A wurde die folgende Verbindung erhalten:

| **Beispiel** | **Name / Struktur / Edukte** | **Analytische Daten** |
|---|---|---|
| **14A** | Methyl-(1-{3-[bis(4-methoxybenzyl)amino]-4-chlorphenyl}cyclobutyl)acetat | ¹H-NMR (400 MHz, DMSO-d₆, δ/ppm): 7.29 (1H, d), 7.19 (4H, |
| | 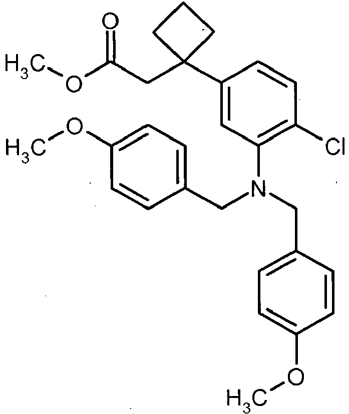 | d), 6.83 (4H, d), 6.79-6.74 (2H, m), 4.04 (4H, s), 3.70 (6H, s), 3.35 (3H, s), 2.69 (2H, s), 2.26-2.17 (2H, m), 2.16-2.06 (2H, m), 2.03-1.89 (1H, m), 1.71-1.58 (1H, m). |
| | | |
| | | LC-MS (Methode 6): Rₜ = 1.50 min; m/z = 494 (M)⁺. |
| | (aus {3-[Bis(4-methoxybenzyl)amino]-4-chlor-phenyl}boronsäure und Methyl-cyclobutylidenacetat [hergestellt nach A. Goti et al., Tetrahedron 48 (25), 5283-5300 (1992)]) | |

### Bespiel 15A

### Benzyl-[3-(3-amino-4-chlorphenyl)oxetan-3-yl]acetat

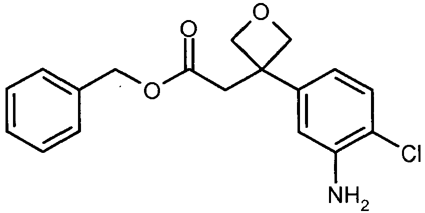

Eine Lösung von 660 mg (1.15 mmol) Benzyl-(3-{3-[bis(4-methoxybenzyl)amino]-4-chlorphenyl}-oxetan-3-yl)acetat in 30 ml Dichlormethan und 6 ml Wasser wurde bei Raumtemperatur mit 576 mg (2.54 mmol) 2,3-Dichlor-5,6-dicyano-1,4-benzochinon (DDQ) versetzt und 2 h gerührt. Nach vollständiger Umsetzung (DC-Kontrolle; Laufmittel Cyclohexan/Essigsäureethylester 2:1) wurde die Reaktionslösung mit 25 ml gesättigter Natriumhydrogencarbonat-Lösung versetzt. Nach Trennung der Phasen wurde die wässrige Phase noch dreimal mit Dichlormethan extrahiert, die vereinigten organischen Phasen über Magnesiumsulfat getrocknet und bis zur Trockene eingeengt. Das erhaltene Rohprodukt wurde über Kieselgel chromatographisch gereinigt (Laufmittel Cyclohexan/Essigsäureethylester 5:1). Es wurden 360 mg (0.98 mmol, Gehalt 90%, 85% d. Th.) der Titelverbindung isoliert.

LC-MS (Methode 4): Rₜ = 1.18 min; m/z = 332 (M+H)⁺.

Analog Synthesebeispiel 15A wurde die folgende Verbindung erhalten:

| **Beispiel** | **Name / Struktur / Edukt** | **Analytische Daten** |
|---|---|---|
| **16A** | Methyl-[1-(3-amino-4-chlorphenyl)-cyclobutyl]acetat | ¹H-NMR (400 MHz, DMSO-d₆, δ/ppm): 7.06 (1H, d), 6.59 (1H, s), |
| | 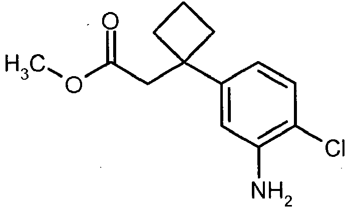 | 6.32 (1H, d), 5.21 (2H, br. s), 3.43 (3H, s), 2.73 (2H, s), 2.31-2.19 (4H, m), 2.09-1.94 (1H, m), 1.82-1.67 (1H, m). |
| | | |
| | (aus Methyl-(1-{3-[bis(4-methoxybenzyl)amino]-4-chlorphenyl}cyclobutyl)acetat) | LC-MS (Methode 4): Rₜ = 1.20 min; m/z = 254 (M+H)⁺. |

### Beispiel 17A

### 3-Brom-2-fluoranilin

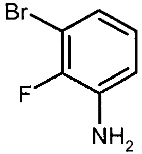

2.0 g (9.09 mmol) 3-Brom-2-fluornitrobenzol wurden in 10 ml Dioxan gelöst und bei RT mit 8.62 g (45.45 mmol) Zinn(II)chlorid versetzt. Nach Zugabe von einigen Tropfen 1 N Salzsäure wurde die Mischung 2 h auf 70°C erhitzt. Nach dem Abkühlen wurde die Reaktionsmischung im Vakuum eingeengt und der Rückstand mit Ethylacetat aufgenommen. Die Lösung wurde nacheinander zweimal mit 1 N Natronlauge, Wasser und gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 10:1). Es wurden 997 mg (57.7% d. Th.) der Zielverbindung erhalten.

LC-MS (Methode 6): Rₜ = 0.88 min; m/z = 189/191 (M+H)⁺.

¹H-NMR (400 MHz, DMSO-d₆): δ [ppm] = 5.43 (s, 2H), 6.66-6.85 (m, 3H).

### Beispiel 18A

### tert.-Butyl-(2E)-3-(3-amino-2-fluorphenyl)acrylat

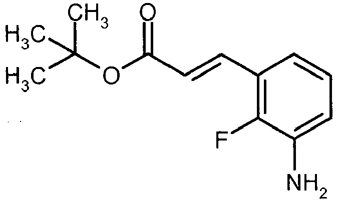

Eine Lösung von 1.41 g (7.42 mmol) 3-Brom-2-fluoranilin und 2.85 g (22.3 mmol) Acrylsäure-*tert*.-butylester in 8 ml DMF wurde mit 5.2 ml (37.1 mmol) Triethylamin versetzt. Der Kolben wurde dreimal evakuiert und mit Argon belüftet, bevor 451 mg (1.48 mmol) Tri-2-tolylphosphin und 166.6 mg (0.74 mmol) Palladium(II)acetat zugesetzt wurden. Das Reaktionsgefäß wurde erneut zweimal evakuiert und mit Argon belüft und die Mischung dann auf ca. 140°C erhitzt. Nach 2 h kräftigem Rühren wurde die Reaktionsmischung abgekühlt und auf gesättigte Natriumhydrogencarbonat-Lösung gegeben. Das Gemisch wurde dreimal mit Ethylacetat extrahiert, und die vereinigten organischen Phasen wurden über Magnesiumsulfat getrocknet und im Vakuum eingeengt. Der Rückstand wurde durch Chromatographie an Silicagel gereinigt (Laufmittel Cyclohexan/ Ethylacetat 10:1). Erhalten wurden 1660 mg des Zielprodukts (94.3% d. Th.).

LC-MS (Methode 6): Rₜ = 1.12 min; m/z = 279 (M+H+CH₃CN)⁺.

¹H-NMR (400 MHz, DMSO-d₆): δ [ppm] = 1.48 (s, 9H), 5.27 (s, 2H), 6.45 (d, 1H), 6.73-7.02 (m, 3H), 7.59 (d, 1H).

### Beispiel 19A

### tert.-Butyl-3-(3-amino-2-fluorphenyl)propanoat

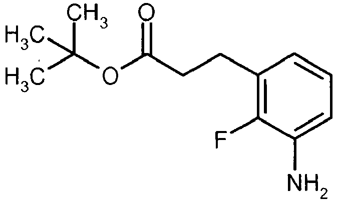

Eine Lösung von 1660 mg (7.0 mmol) *tert*.-Butyl-(2*E*)-3-(3-amino-2-fluorphenyl)acrylat in einer Mischung aus 5 ml Ethanol und 3 ml THF wurde mit Palladium auf Kohle (10%) versetzt und über Nacht unter einer Wasserstoff-Atmosphäre bei Normaldruck kräftig gerührt. Die Reaktionsmischung wurde dann über Kieselgur filtriert und der Filterrückstand mehrfach mit Ethanol/THF gewaschen. Das vereinigte Filtrat wurde im Vakuum eingeengt und der Rückstand durch Chromatographie an Silicagel gereinigt (Laufmittel Cyclohexan/Ethylacetat 20:1 → 10:1). Erhalten wurden 1350 mg des Zielprodukts (80.6% d. Th.).

LC-MS (Methode 6): Rₜ = 1.07 min; m/z = 225.

¹H-NMR (400 MHz, DMSO-d₆): δ [ppm] = 1.36 (s, 9H), 2.45 (t, 2H), 2.74 (t, 2H), 5.00 (s, 2H), 6.24-6.46 (m, 1H), 6.51-6.66 (m, 1H), 6.66-6.82 (m, 1 H).

### Beispiel 20A

### (E/Z)-3-(4-Fluor-3-nitrophenyl)-2-methylprop-2-ensäureethylester

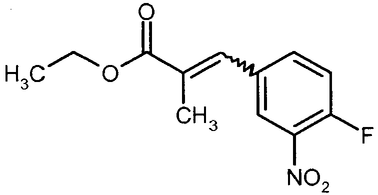

3.17 g Natriumhydrid (60%-ige Suspension in Mineralöl, 79.36 mmol) wurden in 90 ml eines THF/DMF-Gemisches (2:1) suspendiert. Die Mischung wurde auf 0°C gekühlt und tropfenweise mit einer Lösung von 19.76 g (82.96 mmol) 2-Phosphonopropionsäuretriethylester in 60 ml THF/ DMF (2:1) versetzt. Nach 30 min wurde bei 0°C eine Lösung von 12.2 g (72.14 mmol) 4-Fluor-3-nitrobenzaldehyd in 60 ml THF/DMF (2:1) hinzugetropft. Nach Ende der Zugabe wurde die Reaktionsmischung langsam auf RT erwärmt und 2 h bei dieser Temperatur gerührt. Danach wurde die Reaktionsmischung auf Wasser gegeben. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 20:1). Es wurden 15.2 g (83.2% d. Th.) des Zielprodukts als *E*/*Z*-Isomerengemisch (*E*/*Z* 91:9) erhalten.

LC-MS (Methode 6): *Z*-Isomer: Rₜ = 1.11 min; m/z = 254 (M+H)⁺; *E*-Isomer: Rₜ = 1.14 min; m/z = 254 (M+H)⁺.

¹H-NMR (400 MHz, DMSO-d₆): *E*-Isomer: δ [ppm] = 1.28 (t, 3H), 4.22 (q, 2H), 7.59-7.73 (m, 2H), 7.92 (ddd, 1H), 8.24 (dd, 1H).

### Beispiel 21A

### (+/-)-3-(3-Amino-4-fluorphenyl)-2-methylpropansäureethylester

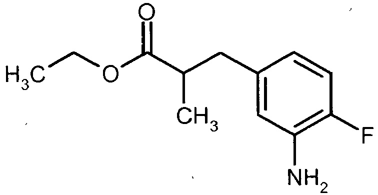

15.2 g (60.02 mmol) (*E*/*Z*)-3-(4-Fluor-3-nitrophenyl)-2-methylprop-2-ensäureethylester (*E*/*Z* 91:9) wurden in einem Gemisch aus 100 ml Ethanol und 100 ml THF mit Palladium auf Kohle (10%) versetzt und über Nacht unter einer Wasserstoffatmosphäre bei Normaldruck kräftig gerührt. Die Reaktionsmischung wurde dann über Celite filtriert, der Rückstand mit Ethanol/Dichlormethan gewaschen und die vereinigten Filtrate im Vakuum eingeengt. Das Produkt wurde im Hochvakuum getrocknet. Erhalten wurden 13.34 g des Zielprodukts (98.7% d. Th).

LC-MS (Methode 6): Rₜ = 0.98 min; m/z = 226 (M+H)⁺.

¹H-NMR (400 MHz, DMSO-d₆): δ [ppm] = 1.04 (d, 3H), 1.12 (t, 3H), 2.46-2.50 (m, 1 H), 2.55-2.66 (m, 1H), 2.66-2.78 (m, 1H), 4.01 (q, 2H), 5.00 (s, 2H), 6.18-6.35 (m, 1H), 6.55 (dd, 1H), 6.84 (dd, 1H).

Das oben erhaltene Racemat wurde durch präparative HPLC an chiraler Phase in die Enantiomere aufgetrennt [Säule: Daicel Chiralpak AD-H, 5 µm, 250 mm x 20 mm; Injektionsvolumen: 0.15 ml; Temperatur: 30°C; Eluent: 90% Isohexan / 10% Ethanol; Fluss: 15 ml/min; Detektion: 220 nm]. Ausgehend von 7.25 g Racemat wurden 3.43 g Enantiomer 1 (*Beispiel 22A*) und 3.35 g Enantiomer *2* (*Beispiel 23A*) erhalten:

### Beispiel 22A

### (+)-(2S)-3-(3-Amino-4-fluorphenyl)-2-methylpropansäureethylester

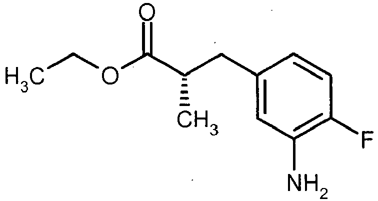

Ausbeute: 3.43 g

LC-MS (Methode 6): Rₜ = 0.97 min; m/z = 226 (M+H)⁺.

¹H-NMR (400 MHz, DMSO-*d*₆): δ [ppm] = 1.04 (d, 3H), 1.12 (t, 3H), 2.46-2.50 (m, 1H), 2.55-2.66 (m, 1H), 2.66-2.78 (m, 1H), 4.01 (q, 2H), 5.00 (s, 2H), 6.18-6.35 (m, 1H), 6.55 (dd, 1H), 6.84 (dd, 1H).

[α]_{D}²⁰ = +18.3°, c = 0.465, Chloroform.

### Beispiel 23A

### (-)-(2R)-3-(3-Amino-4-fluorphenyl)-2-methylpropansäureethylester

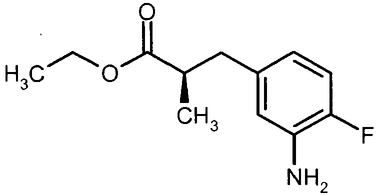

Ausbeute: 3.35 g

LC-MS (Methode 6): Rₜ = 0.97 min; m/z = 226 (M+H)⁺.

¹H-NMR (400 MHz, DMSO-*d*₆): δ [ppm] = 1.04 (d, 3H), 1.12 (t, 3H), 2.46-2.50 (m, 1H), 2.55-2.66 (m, 1H), 2.68-2.79 (m, 1H), 4.01 (q, 2H), 5.00 (br. s, 2H), 6.30 (dd, 1H), 6.55 (dd, 1H), 6.84 (dd, 1H).

[α]_{D}²⁰ = -31.4°, c = 0.520, Chloroform.

### Beispiel 24A

### (E/Z)-3-(4-Chlor-3-nitrophenyl)-2-methylprop-2-ensäureethylester

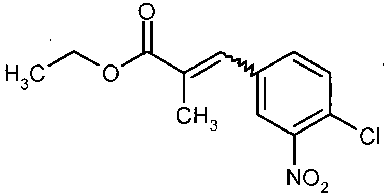

4.74 g Natriumhydrid (60%-ige Suspension in Mineralöl, 118.56 mmol) wurden in 93 ml eines THF/DMF-Gemisches (1: 1) suspendiert. Die Mischung wurde auf 0°C gekühlt und tropfenweise mit 26.6 ml (123.95 mmol) 2-Phosphonopropionsäuretriethylester versetzt. Nach 30 min wurden bei 0°C 20.0 g (107.78 mmol) 4-Chlor-3-nitrobenzaldehyd hinzugefügt. Nach Ende der Zugabe wurde die Reaktionsmischung langsam auf RT erwärmt und noch 3 h bei dieser Temperatur gerührt. Dann wurde die Reaktionsmischung auf Wasser gegeben. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 70:1 → 50:1). Es wurden 26.7 g (91.9% d. Th.) des Zielprodukts als *E*/*Z*-Isomerengemisch (*E*/*Z* 91:9) erhalten.

LC-MS (Methode 4): Z-Isomer: Rₜ = 1.32 min; m/z = 255; *E*-Isomer: Rₜ = 1.36 min; m/z = 270 (M+H)⁺.

¹H-NMR (400 MHz, DMSO-d₆): *E*-Isomer: δ [ppm] = 1.28 (t, 3H), 2.06 (d, 3H), 4.22 (q, 2H), 7.56-7.67 (m, 1H), 7.75-7.87 (m, 2H), 8.17 (d, 1H).

### Beispiel 25A

### (+/-)-3-(3-Amino-4-chlorphenyl)-2-methylpropansäureethylester

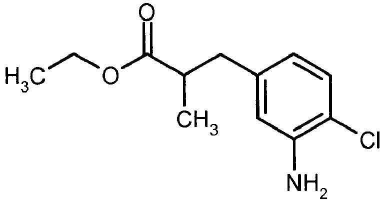

10.0 g (37.08 mmol) (*E*/*Z*)-3-(4-Chlor-3-nitrophenyl)-2-methylprop-2-ensäureethylester (*E*/*Z* 91:9) wurden in 25 ml Ethylacetat und 25 ml Essigsäure gelöst und mit Palladium auf Kohle (10%) versetzt. Die Reaktionsmischung wurde insgesamt 6 h unter einer Wasserstoffatmosphäre bei Normaldruck kräftig gerührt, wobei nach 2 h nochmals 25 ml Essigsäure und weitere Portionen an 10% Palladium auf Kohle hinzugefügt wurden. Es wurde dann über Celite filtriert und der Rückstand mit Ethanol/Dichlormethan gewaschen. Die vereinigten Filtrate wurden mit gesättigter Natriumhydrogencarbonat-Lösung gewaschen, über Natriumsulfat getrocknet und im Vakuum eingeengt. Das Rohprodukt wurde durch Chromatographie an Silicagel gereinigt (Laufmittel Cyclohexan/ Ethylacetat 30:1 → 10:1). Erhalten wurden 4.01 g des Zielprodukts (44.7% d. Th).

LC-MS (Methode 6): Rₜ = 1.06 min; m/z = 242 (M+H)⁺.

¹H-NMR (400 MHz, DMSO-d₆): δ [ppm] = 1.05 (d, 3H), 1.12 (t, 3H), 2.47-2.50 (m, 1H), 2.56-2.67 (m, 1H), 2.67-2.78 (m, 1H), 4.02 (q, 2H), 5.23 (s, 2H), 6.35 (dd, 1H), 6.58 (d, 1H), 7.05 (d, 1H).

Das oben erhaltene Racemat wurde durch präparative HPLC an chiraler Phase in die Enantiomere aufgetrennt [Säule: Daicel Chiralpak OJ-H, 5 µm, 250 mm x 20 mm; Injektionsvolumen: 0.15 ml; Temperatur: 35°C; Eluent: 50% Isohexan / 50% Isopropanol; Fluss: 15 ml/min; Detektion: 220 nm]. Ausgehend von 10.3 g Racemat wurden 4.0 g Enantiomer 1 (*Beispiel 26A)* und 3.7 g Enantiomer 2 *(Beispiel 27A)* erhalten:

### Beispiel 26A

### (-)-(2R)-3-(3-Amino-4-chlorphenyl)-2-methylpropansäureethylester

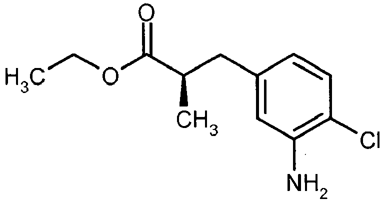

Ausbeute: 4.0 g

LC-MS (Methode 7): Rₜ = 2.27 min; m/z = 196/198.

¹H-NMR (400 MHz, DMSO-*d*₆): δ [ppm] = 1.05 (d, 3H), 1.12 (t, 3H), 2.47-2.50 (m, 1H), 2.54-2.66 (m, 2H), 2.68-2.80 (m, 1H), 4.02 (q, 2H), 5.23 (s, 2H), 6.35 (dd, 1H), 6.58 (d, 1H), 7.05 (d, 1H).

[α]_{D}²⁰ = -35.8°, c = 0.560, Chloroform.

### Beispiel 27A

### (+)-(2S)-3-(3-Amino-4-chlorphenyl)-2-methylpropansäureethylester

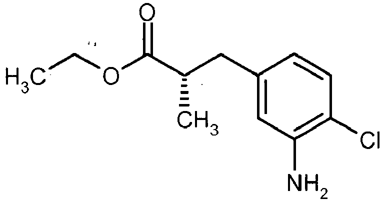

Ausbeute: 3.7 g

¹H-NMR (400 MHz, DMSO-*d*₆): δ [ppm] = 1.05 (d, 3H), 1.12 (t, 3H), 2.47-2.50 (m, 1H), 2.56-2.67 (m, 1H), 2.67-2.81 (m, 1H), 4.02 (q, 2H), 5.23 (br. s, 2H), 6.35 (dd, 1H), 6.58 (d, 1H), 7.05 (d, 1H).

[α]_{D}²⁰ = +35.1 °, c = 0.525, Chloroform.

### Beispiel 28A

### Ethyl-(2E/Z)-2-(4-chlor-3-nitrobenzyliden)butanoat

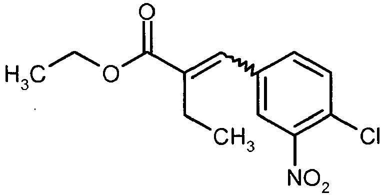

1.19 g Natriumhydrid (60%-ige Suspension in Mineralöl, 29.64 mmol) wurden in 50 ml eines THF/DMF-Gemisches (1:1) suspendiert. Die Mischung wurde auf 0°C gekühlt und tropfenweise mit 7.3 ml (30.99 mmol) 2-Phosphonobuttersäuretriethylester versetzt. Nach 30 min wurden bei -10°C 5.0 g (26.94 mmol) 4-Chlor-3-nitrobenzaldehyd portionsweise hinzugefügt. Nach Ende der Zugabe wurde die Reaktionsmischung 5 h bei 0°C gerührt und dann langsam über Nacht auf RT erwärmt. Danach wurde die Reaktionsmischung auf Wasser gegeben. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 6:1). Es wurden 7.05 g (92.1% d. Th.) des Zielprodukts als *E*/*Z*-Isomerengemisch erhalten.

LC-MS (Methode 6): Rₜ = 1.24 min und 1.26 min; keine Ionisierung.

### Beispiel 29A

### (+/-)-Ethyl-2-(3-amino-4-chlorbenzyl)butanoat

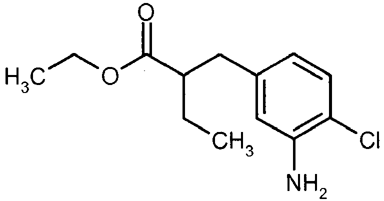

7.05 g (24.84 mmol) Ethyl-(2*E*/*Z*)-2-(4-chlor-3-nitrobenzyliden)butanoat wurden in 35 ml Ethylacetat und 35 ml Essigsäure gelöst und mit Palladium auf Kohle (10%) versetzt. Die Reaktionsmischung wurde insgesamt 6 h unter einer Wasserstoffatmosphäre bei Normaldruck kräftig gerührt, wobei nach 4 h weitere Portionen an 10% Palladium auf Kohle hinzugefügt wurden. Es wurde dann über Celite filtriert und der Rückstand mit Ethylacetat/THF gewaschen. Die vereinigten Filtrate wurden mit gesättigter Natriumhydrogencarbonat-Lösung gewaschen, über Natriumsulfat getrocknet und im Vakuum eingeengt. Das Rohprodukt wurde durch Chromatographie an Silicagel gereinigt (Laufmittel Cyclohexan/Ethylacetat 30:1 → 10:1). Erhalten wurden 4.12 g des Zielprodukts (64.9% d. Th).

LC-MS (Methode 6): Rₜ = 1.14 min; m/z = 210.

¹H-NMR (400 MHz, DMSO-d₆): δ [ppm] = 0.84 (t, 3H), 1.10 (t, 3H), 1.42-1.59 (m, 2H), 2.40-2.80 (m, 4H), 4.01 (q, 2H), 5.23 (s, 2H), 6.34 (dd, 1H), 6.58 (d, 1H), 7.05 (d, 1H).

Das oben erhaltene Racemat wurde durch präparative HPLC an chiraler Phase in die Enantiomere aufgetrennt [Säule: Daicel Chiralpak OJ-H, 5 µm, 250 mm x 20 mm; Injektionsvolumen: 0.43 ml; Temperatur: 30°C; Eluent: Ethanol; Fluss: 15 ml/min; Detektion: 220 nm]. Ausgehend von 3.22 g Racemat wurden 1.22 g Enantiomer 1 (*Beispiel 30A*) und 1.27 g Enantiomer 2 *(Beispiel 31A*) erhalten:

### Beispiel 30A

### (-)-Ethyl-(2R)-2-(3-amino-4-chlorbenzyl)butanoat

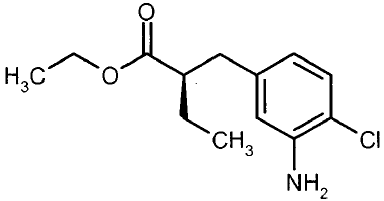

Ausbeute: 1.22 g

LC-MS (Methode 6): Rₜ = 1.14 min; m/z - 210.

¹H-NMR (400 MHz, DMSO-*d*₆): δ [ppm] = 0.84 (t, 3H), 1.10 (t, 3H), 1.42-1.56 (m, 2H), 2.39-2.48 (m, 1H), 2.56-2.73 (m, 3H), 4.01 (q, 2H), 5.11-5.27 (m, 2H), 6.34 (dd, 1H), 6.58 (d, 1H), 7.05 (d, 1H).

[α]_{D}²⁰ = -28.1°, c = 0.510, Chlorofonn.

### Beispiel 31A

### (+)-Ethyl-(2S)-2-(3-amino-4-chlorbenzyl)butanoat

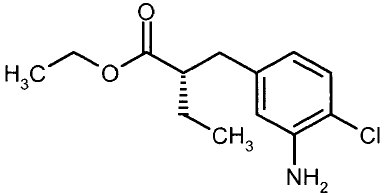

Ausbeute: 1.27 g

LC-MS (Methode 6): Rₜ = 1.15 min; m/z = 210.

¹H-NMR (400 MHz, DMSO-*d*₆): δ [ppm] = 0.84 (t, 3H), 1.10 (t, 3H), 1.46-1.55 (m, 2H), 2.42-2.49 (m, 1H), 2.54-2.69 (m, 3H), 4.01 (q, 2H), 5.22 (s, 2H), 6.34 (dd, 1H), 6.57 (d, 1H), 7.05 (d, 1H).

[α]_{D}²⁰ = +34.1°, c = 0.550, Chloroform.

### Beispiel 32A

### tert.-Butyl-(2E/Z)-3-(4-chlor-3-nitrophenyl)but-2-enoat

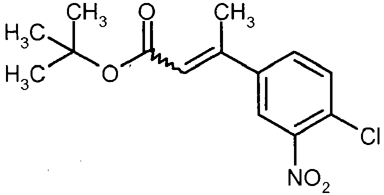

2.87 g Natriumhydrid (60%-ige Suspension in Mineralöl, 71.65 mmol) wurden in 80 ml THF suspendiert. Die Mischung wurde auf 0°C gekühlt und tropfenweise mit 17.6 ml (74.9 mmol) *tert.-*Butyl-(diethoxyphosphoryl)acetat versetzt. Nach 30 min wurden bei 0°C 13.0 g (65.1 mmol) 4-Chlor-3-nitroacetophenon hinzugefügt. Nach Ende der Zugabe wurde die Reaktionsmischung langsam auf RT erwärmt und noch 1.5 h bei RT gerührt, bevor das Gemisch dann auf Wasser gegeben wurde. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 20:1 → 10:1). Es wurden 17.03 g (87.8% d. Th.) des Zielprodukts als *E*/*Z*-Isomerengemisch erhalten (*E*/*Z* ca. 1:1).

LC-MS (Methode 5): Isomer 1: Rₜ = 2.61 min; m/z = 255; Isomer 2: Rₜ = 2.77 min; m/z = 224.

### Beispiel 33A

### (+/-)-3-(3-Amino-4-chlorphenyl)butansäure-tert.-butylester

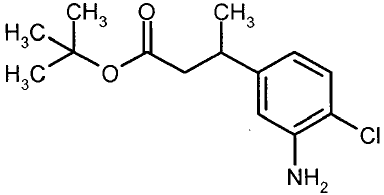

11.5 g (38.62 mmol) *tert*.-Butyl-(2*E*/*Z*)-3-(4-chlor-3-nitrophenyl)but-2-enoat (*E*/*Z* ca. 1:1) wurden in 60 ml Ethylacetat und 60 ml Essigsäure gelöst und mit Palladium auf Kohle (10%) versetzt. Die Reaktionsmischung wurde 6 h bei Normaldruck unter einer Wasserstoffatmosphäre kräftig gerührt. Es wurde dann über Celite filtriert und der Rückstand mit Ethylacetat gewaschen. Die vereinigten Filtrate wurden mit gesättigter Natriumhydrogencarbonat-Lösung gewaschen, über Natriumsulfat getrocknet und im Vakuum eingeengt. Das Rohprodukt wurde durch Chromatographie an Silicagel gereinigt (Laufmittel Cyclohexan/Ethylacetat 30:1). Es wurden 3.90 g (37.4% d. Th.) des Zielprodukts erhalten.

¹H-NMR (400 MHz, DMSO-d₆): δ [ppm] = 1.14 (d, 3H), 1.31 (s, 9H), 2.38 (dd, 2H), 2.95 (q, 1H), 5.21 (br. s, 2H), 6.42 (dd, 1H), 6.65 (d, 1H), 7.06 (d, 1H).

Das oben erhaltene Racemat wurde durch präparative HPLC an chiraler Phase in die Enantiomere aufgetrennt [Säule: Daicel Chiralpak AD-H, 5 µm, 250 mm x 20 mm; Injektionsvolumen: 0.15 ml; Temperatur: 30°C; Eluent: 90% Isohexan / 10% Ethanol; Fluss: 15 ml/min; Detektion: 220 nm]. Ausgehend von 5.0 g Racemat wurden 2.1 g Enantiomer 1 (*Beispiel 34A)* und 1.8 g Enantiomer 2 *(Beispiel 35A)* erhalten:

### Beispiel 34A

### (+)-(3S)-3-(3-Amino-4-chlorphenyl)butansäure-tert.-butylester

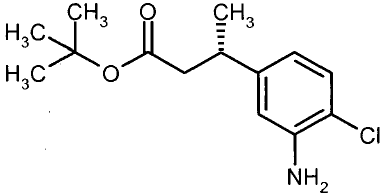

LC-MS (Methode 4): Rₜ = 1.34 min; m/z = 270 (M+H)⁺.

¹H-NMR (400 MHz, DMSO-*d*₆): δ [ppm] = 1.14 (d, 3H), 1.31 (s, 9H), 2.19-2.45 (m, 2H), 2.95 (q, 1H), 5.20 (s, 2H), 6.42 (dd, 1H), 6.65 (d, 1H), 7.06 (d, 1H).

[α]_{D}²⁰ = +20.9°, c = 0.670, Chloroform.

### Beispiel 35A

### (-)-(3R)-3-(3-Amino-4-chlorphenyl)butansäure-tert.-butylester

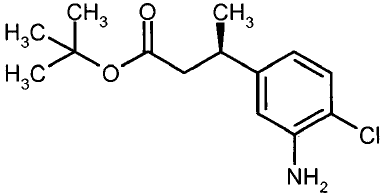

LC-MS (Methode 4): Rₜ = 1.34 min; m/z = 214 (M+H-C₄H₈)⁺.

¹H-NMR (400 MHz, DMSO-*d*₆): δ [ppm] = 1.14 (d, 3H), 1.31 (s, 9H), 2.38 (dd, 2H), 2.95 (q, 1H), 5.20 (br. s, 2H), 6.42 (dd, 1H), 6.65 (d, 1H), 7.06 (d, 1H).

[α]_{D}²⁰ = -24.1°, c = 0.570, Chloroform.

### Beispiel 36A

### tert.-Butyl-(2E/Z)-3-(4-fluor-3-nitrophenyl)but-2-enoat

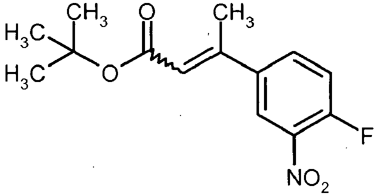

4.81 g Natriumhydrid (60%-ige Suspension in Mineralöl, 120.13 mmol) wurden in einer Mischung aus 120 ml THF und 120 ml DMF suspendiert. Die Mischung wurde auf 0°C gekühlt und tropfenweise mit 29.5 ml (125.59 mmol) *tert*.-Butyl-(diethoxyphosphoryl)acetat versetzt. Nach 30 min wurden bei 0°C 20.0 g (109.21 mmol) 4-Fluor-3-nitroacetophenon hinzugefügt. Nach Ende der Zugabe wurde die Reaktionsmischung langsam auf RT erwärmt und noch 3.5 h bei RT gerührt, bevor das Gemisch dann auf Wasser gegeben wurde. Es wurde dreimal mit Ethylacetat extrahiert, und die vereinigten organischen Phasen wurden im Vakuum eingeengt. Der Rückstand wurde durch Chromatographie an Silicagel gereinigt (Laufmittel Cyclohexan/Ethylacetat 50:1). Es wurden 7.24 g (23.6% d. Th.) des Zielprodukts als *E*/*Z*-Isomerengemisch erhalten (*E*/*Z* ca. 1.2:1).

LC-MS (Methode 4): Isomer 1: Rₜ = 1.34 min; m/z = 208; Isomer 2: Rₜ = 1.42 min; m/z = 208.

### Beispiel 37A

### (+/-)-3-(3-Amino-4-fluorphenyl)butansäure-tert.-butylester

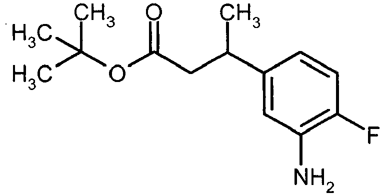

7.24 g (25.74 mmol) *tert*.-Butyl-(2*E*/*Z*)-3-(4-fluor-3-nitrophenyl)but-2-enoat (*E*/*Z* ca. 1.2:1) wurden in 200 ml Ethanol gelöst und mit Palladium auf Kohle (10%) versetzt. Die Reaktionsmischung wurde über Nacht unter einer Wasserstoffatmosphäre bei Normaldruck kräftig gerührt. Es wurde dann über Celite filtriert und der Rückstand zweimal mit Ethylacetat gewaschen. Die vereinigten Filtrate wurden im Vakuum eingeengt und der Rückstand im Hochvakuum getrocknet. Erhalten wurden 6.02 g des Zielprodukts (92.4% d. Th).

¹H-NMR (400 MHz, DMSO-d₆): δ [ppm] = 1.14 (d, 3H), 1.31 (s, 9H), 2.37 (dd, 2H), 2.95 (q, 1H), 4.98 (s, 2H), 6.36 (ddd, 1H), 6.62 (dd, 1H), 6.85 (dd, 1H).

Das oben erhaltene Racemat wurde durch präparative HPLC an chiraler Phase in die Enantiomere aufgetrennt [Säule: Daicel Chiralpak OJ-H, 5 µm, 250 mm x 20 mm; Injektionsvolumen: 0.15 ml; Temperatur: 35°C; Eluent: 65% Isohexan / 35% Ethanol; Fluss: 15 ml/min; Detektion: 220 nm]. Ausgehend von 6.0 g Racemat wurden 2.44 g Enantiomer 1 (*Beispiel 38A*) und 1.92 g Enantiomer 2 (*Beispiel 39A*) erhalten:

### Beispiel 38A

### (+)-(3S)-3-(3-Amino-4-fluorphenyl)butansäure-tert.-butylester

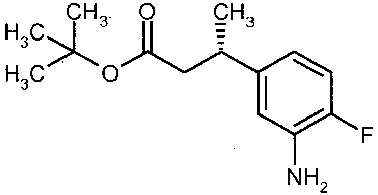

LC-MS (Methode 6): Rₜ = 1.11 min; m/z = 254 (M+H)⁺.

¹H-NMR (400 MHz, DMSO-*d*₆): δ [ppm] = 1.14 (d, 3H), 1.31 (s, 9H), 2.18-2.46 (m, 2H), 2.95 (q, 1 H), 4.99 (br. s, 2H), 6.36 (ddd, 1H), 6.61 (dd, 1H), 6.85 (dd, 1H).

[α]_{D}²⁰ = +22.5°, c = 0.570, Chloroform.

### Beispiel 39A

### (-)-(3R)-3-(3-Amino-4-fluorphenyl)butansäure-tert.-butylester

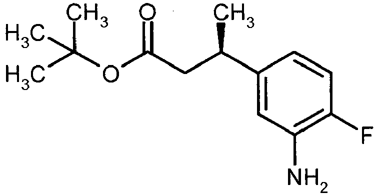

LC-MS (Methode 6): Rₜ = 1.11 min; m/z = 254 (M+H)⁺.

¹H-NMR (400 MHz, DMSO-*d*₆): δ [ppm] = 1.14 (d, 3H), 1.31 (s, 9H), 2.26-2.45 (m, 2H), 2.95 (q, 1H), 4.99 (br. s, 2H), 6.36 (ddd, 1H), 6.62 (dd, 1H), 6.85 (dd, 1H).

[α]_{D}²⁰ = -23.2°, c = 0.510, Chlorofonn.

### Beispiel 40A

### Ethyl-3-(3-brom-4-fluorphenyl)acrylat

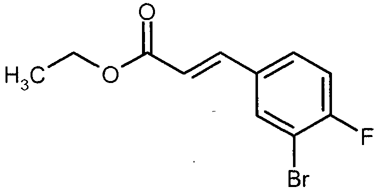

Zu einer Lösung von 6.5 g (31.7 mmol) 3-Brom-4-fluorbenzylalkohol und 13.25 g (38 mmol) Ethoxycarbonylmethylenphosphoran in 390 ml Toluol wurden 9.65 g (111 mmol) Braunstein gegeben. Das Reaktionsgemisch wurde zum Rückfluss erhitzt, nach 1 h mit weiteren 9.65 g Braunstein versetzt und über Nacht weiter unter Rückfluss erhitzt. Nach dem Abkühlen wurde das Gemisch über Celite filtriert und das Filtrat eingeengt. Der Rückstand wurde flash-chromatographisch an Kieselgel gereinigt (Laufmittel Cyclohexan/Ethylacetat 5:1). Es wurden 7.05 g (81% d. Th.) des Zielprodukts in Form eines *E*/*Z*-Isomerengemisches erhalten.

LC-MS (Methode 4): Rₜ = 1.33 min und 1.35 min; m/z = 273/275 (M+H)⁺.

### Beispiel 41A

### rac-Ethyl-2-(3-brom-4-fluorphenyl)-trans-cyclopropancarboxylat

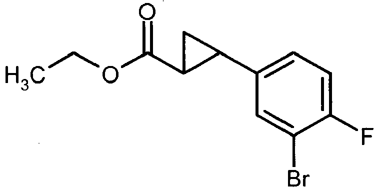

Unter Argon wurden 381 mg (9.52 mmol) Natriumhydrid (60% in Paraffinöl) in 20 ml DMSO vorgelegt und bei RT in einer Portion mit 2.1 g (9.52 mmol) Trimethylsulfoxoniumiodid versetzt. Nach beendeter Gasentwicklung wurden 2.0 g (7.3 mmol) Ethyl-3-(3-brom-4-fluorphenyl)acrylat, in 10 ml DMSO gelöst, langsam zugetropft. Das Reaktionsgemisch wurde über Nacht auf 50°C erwärmt, dann auf RT abgekühlt und ohne weitere Aufarbeitung durch Flash-Chromatographie an Kieselgel gereinigt (Laufmittel Isohexan/Ethylacetat 100:1). Es wurden 907 mg (43% d. Th.) des Zielprodukts erhalten.

LC-MS (Methode 6): Rₜ = 1.20 min; m/z = 289 (M+H)⁺.

GC-MS (Methode 1): Rₜ = 5.85 min; m/z = 287/289 (M+H)⁺.

¹H-NMR (500 MHz, CDCl₃): δ [ppm] = 1.20-1.33 (m, 4H), 1.56-1.63 (m, 1H), 1.80-1.88 (m, 1H), 2.43-2.52 (m, 1H), 4.17 (q, 2H), 7.00-7.06 (m, 2H), 7.28 (d, 1H).

### Beispiel 42A

### rac-Ethyl-2-[3-(benzylamino)-4-fluorphenyl]-trans-cyclopropancarboxylat

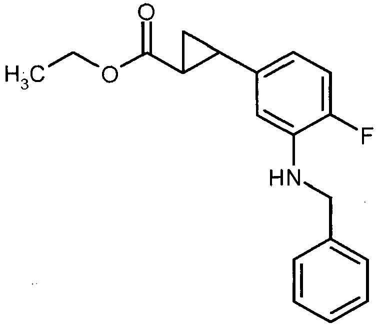

Unter Argon wurden 361.5 mg (3.8 mmol) Natrium-*tert*.-butylat in 12.9 ml Toluol suspendiert und nacheinander mit 900 mg (3.1 mmol) (+/-)-*trans*-Ethyl-2-(3-brom-4-fluorphenyl)cyclopropan-carboxylat, 403 mg (3.8 mmol) Benzylamin, 28.7 mg (0.03 mmol) Tris(dibenzylidenaceton)-dipalladium und 19.5 mg (0.03 mmol) *rac*-2,2'-Bis(diphenylphosphino)-1,1'-binaphthyl versetzt. Die Mischung wurde 4 h lang auf 110°C erhitzt. Anschließend wurde das Reaktionsgemisch auf RT abgekühlt, mit 100 ml Ethylacetat und 50 ml gesättigter Ammoniumchlorid-Lösung versetzt und über Celite filtriert. Die organische Phase wurde abgetrennt, mit jeweils 50 ml gesättigter Ammoniumchlorid-Lösung und gesättigter Natriumchlorid-Lösung gewaschen, über Magnesiumsulfat getrocknet und eingeengt. Das Rohprodukt wurde durch präparative HPLC gereinigt. Es wurden 262 mg der Zielverbindung mit einer Reinheit von 66% erhalten (18% d. Th.).

LC-MS (Methode 6): Rₜ = 1.28 min; m/z = 314 (M+H)⁺.

### Beispiel 43A

### rac-Ethyl-2-[3-amino-4-fluorphenyl]-trans-cyclopropancarboxylat

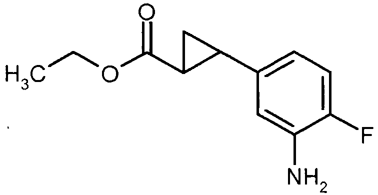

262 mg (Reinheit 66%, 0.55 mmol) (+/-)-Ethyl-2-[3-(benzylamino)-4-fluorphenyl]-*trans*-cyclo-propancarboxylat wurden in 5 ml Ethanol/THF (1:1) gelöst, mit 26 mg Palladium auf Kohle (10%) versetzt und für 24 h bei RT mit 1 bar Wasserstoffdruck hydriert. Anschließend wurde das Reaktionsgemisch über Celite filtriert, der Rückstand mit Ethanol nachgewaschen und das Filtrat eingeengt. Das so erhaltene Rohprodukt wurde durch präparative HPLC gereinigt. Es wurden 87 mg (69% d. Th.) der Zielverbindung erhalten.

LC-MS (Methode 6): Rₜ = 0.96 min; m/z = 224 (M+H)⁺.

¹H-NMR (400 MHz, DMSO-d₆): δ [ppm] = 1.17-1.23 (m, 3H), 1.23-1.28 (m, 1H), 1.39 (dt, 1H), 1.67-1.81 (m, 1H), 2.21-2.31 (m, 1H), 4.09 (q, 2H), 6.31 (ddd, 1H), 6.55 (dd, 1H), 6.86 (dd, 1H).

### Beispiel 44A

### 3-Amino-4-fluoracetophenon

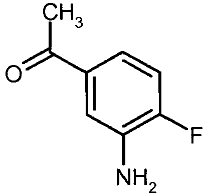

Zu einer Lösung von 3 g (16.4 mmol) 4-Fluor-3-nitroacetophenon in 7.8 ml 12 N Salzsäure wurde bei 0°C eine Lösung von 11.1 g (89 mmol) Zinnchlorid-Dihydrat in 12 ml Wasser innerhalb von 15 min zugetropft. Anschließend wurde das Reaktionsgemisch für 15 min zum Rückfluss erhitzt und dann über Nacht bei RT nachgerührt. Das Reaktionsgemisch wurde danach auf Eis gegossen, mit 50%-iger Natronlauge auf pH 12 gebracht und mit Ethylacetat extrahiert. Die organische Phase wurde mit gesättigter Kochsalz-Lösung gewaschen, über Magnesiumsulfat getrocknet und eingeengt. Es wurden 2.47 g (90% Reinheit, 87% d. Th.) der Zielverbindung erhalten.

LC-MS (Methode 5): Rₜ = 1.32 min; m/z = 154 (M+H)⁺.

### Beispiel 45A und Beispiel 46A

Ethyl-(2*E*)-3-(3-amino-4-fluorphenyl)-2-methylbut-2-enoat und
Ethyl-(2*Z*)-3-(3-amino-4-fluorphenyl)-2-methylbut-2-enoat

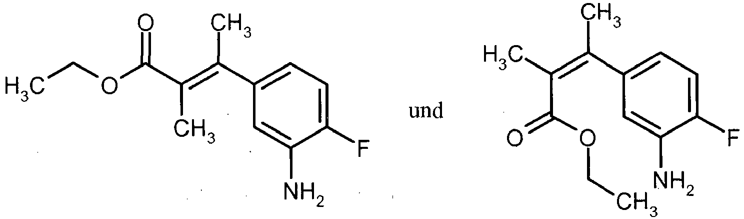

Zu einer Suspension von 1.29 g Natriumhydrid (60% in Paraffinöl; 32.3 mmol) in 24.7 ml THF wurden bei 0°C 6.92 ml (7.68 g; 32.3 mmol) 2-Phosphonopropionsäuretriethylester langsam zugetropft. Das Reaktionsgemisch wurde 30 min gerührt und anschließend mit 2.47 g (90% Reinheit, 14.5 mmol) 3-Amino-4-fluoracetophenon versetzt. Das Reaktionsgemisch wurde zunächst 1 h bei RT, anschließend 2 h unter Rückfluss gerührt, dann wieder auf RT abgekühlt und über Nacht nachgerührt. Der Ansatz wurde dann auf Wasser gegossen und dreimal mit jeweils 100 ml Ethylacetat extrahiert. Die vereinigten organischen Phasen wurden über Magnesiumsulfat getrocknet, eingeengt und der Rückstand flash-chromatographisch an Kieselgel gereinigt (Laufmittel Toluol/ Ethylacetat 5:1). Es wurden in getrennter Form 612 mg (15% d. Th.) des 2*E*-Isomeren (*Beispiel 45A*) und 529 mg (13% d. Th.) des 2Z-Isomeren *(Beispiel 46A)* erhalten.

### 2E-Isomer (Beispiel 45A):

LC-MS (Methode 6): Rₜ = 1.05 min; m/z = 238 (M+H)⁺.

¹H-NMR (400 MHz, DMSO-d₆): δ [ppm] = 1.22-1.29 (m, 3H), 1.69 (d, 3H), 2.11 (d, 3H), 4.17 (d, 2H), 6.30 (ddd, 1H), 6.56 (dd, 1H), 6.97 (dd, 1H).

### 2Z-Isomer (Beispiel 46A):

LC-MS (Methode 6): Rₜ = 0.99 min; m/z = 238 (M+H)⁺.

¹H-NMR (400 MHz, DMSO-d₆): δ [ppm] = 0.85 (t, 3H), 1.86-1.92 (m, 3H), 1.94-2.01 (m, 3H), 3.82 (q, 2H), 6.24 (ddd, 1H), 6.51 (dd, 1H), 6.87 (dd, 1H).

### Beispiel 47A

### rac-threo-Ethyl-3-(3-amino-4-fluorphenyl)-2-methylbutanoat

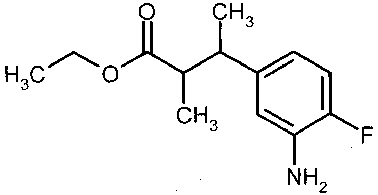

Zu einer Lösung von 48 mg (0.2 mmol) Ethyl-(2*E*)-3-(3-amino-4-fluorphenyl)-2-methylbut-2-enoat in 5 ml Methanol wurden 4.8 mg Palladium auf Kohle (10%) gegeben. Das Reaktionsgemisch wurde über Nacht bei 1 bar Wasserstoffdruck hydriert. Anschließend wurde über Celite filtriert und das Filtrat eingeengt. Es wurden 35.8 mg (74% d. Th.) der Titelverbindung erhalten, welche ca. 20% des *erythro*-Isomeren enthielt.

LC-MS (Methode 6): Rₜ = 1.02 min; m/z = 240 (M+H)⁺.

### Beispiel 48A

### rac-erythro-Ethyl-3-(3-amino-4-fluorphenyl)-2-methylbutanoat

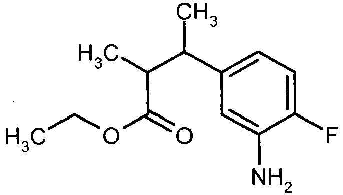

Zu einer Lösung von 30 mg (0.13 mmol) Ethyl-(2*Z*)-3-(3-amino-4-fluorphenyl)-2-methylbut-2-enoat in 3.1 ml Methanol wurden 3 mg Palladium auf Kohle (10%) gegeben. Das Reaktionsgemisch wurde über Nacht bei 1 bar Wasserstoffdruck hydriert. Anschließend wurde über Celite filtriert und das Filtrat eingeengt. Es wurden 22.5 mg (74% d. Th.) der Titelverbindung erhalten, welche ca. 5% des *threo*-Isomeren enthielt.

LC-MS (Methode 6): Rₜ = 1.04 min; m/z = 240 (M+H)⁺.

### Beispiel 49A

### tert.-Butyl-(2E)-3-(4-fluor-3-nitrophenyl)acrylat

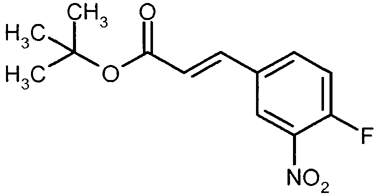

Unter Argon wurden 0.65 g Natriumhydrid (60% in Paraffinöl; 16.3 mmol) in 25 ml THF vorgelegt und auf 0°C gekühlt. Anschließend wurden 4.29 g (17 mmol) Diethylphosphonoessigsäure-*tert*.-butylester langsam zugetropft. Nach 30 min Rühren wurden 2.5 g (14.8 mmol) 4-Fluor-3-nitrobenzaldehyd zugegeben. Das Reaktionsgemisch wurde 3 h bei RT gerührt, dann in 100 ml Wasser gegossen und dreimal mit jeweils 100 ml Ethylacetat extrahiert. Die vereinigten organischen Phasen wurden über Magnesiumsulfat getrocknet und eingeengt. Der Rückstand wurde flash-chromatographisch an Kieselgel gereinigt (Laufmittel Cyclohexan/Ethylacetat 50:1). Es wurden 3.37 g (85% d. Th.) des Zielprodukts erhalten.

GC-MS (Methode 1): Rₜ = 6.45 min; m/z = 211 (M-^{t}Bu)⁺.

¹H-NMR (400 MHz, DMSO-d₆): δ [ppm] = 1.49 (s, 9H), 6.69 (d, 1H), 7.59-7.76 (m, 2H), 8.19 (ddd, 1H), 8.50 (dd, 1 H).

### Beispiel 50A

### tert.-Butyl-(2E)-3-(4-cyano-3-nitrophenyl)acrylat

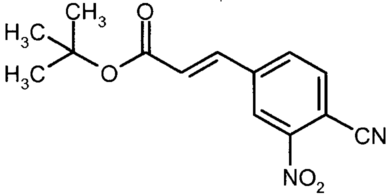

Zu einer Lösung von 500 mg (1.87 mmol) *tert*.-Butyl-(2*E*)-3-(4-fluor-3-nitrophenyl)acrylat in 5.4 ml DMF wurden 134 mg (2.06 mmol) Kaliumcyanid gegeben. Nach Rühren über Nacht bei RT wurde das Reaktionsgemisch direkt durch Flash-Chromatographie an Kieselgel gereinigt (Laufmittel Cyclohexan/Ethylacetat-Gemisch). Es wurden 57 mg (11% d. Th.) der Titelverbindung erhalten.

LC-MS (Methode 5): Rₜ = 2.40 min; m/z = 292 (M+NH₄)⁺.

¹H-NMR (400 MHz, DMSO-d₆): δ [ppm] = 1.39-1.58 (m, 9H), 6.91 (d, 1H), 7.73 (d, 1H), 8.19 (d, 1H), 8.26-8.38 (m, 1H), 8.72 (d, 1H).

### Beispiel 51A

### tert.-Butyl-3-(3-amino-4-cyanophenyl)propanoat

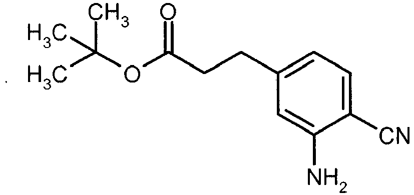

Zu einer Lösung von 48.9 mg (0.18 mmol) *tert*.-Butyl-(2*E*)-3-(4-cyano-3-nitrophenyl)acrylat in 4.4 ml Ethanol wurden 4.9 mg Palladium auf Kohle (10%) gegeben. Das Reaktionsgemisch wurde über Nacht bei RT mit 1 bar Wasserstoffdruck hydriert. Anschließend wurde über Celite filtriert und das Filtrat eingeengt. Es wurden 43.5 mg (99% d. Th.) der Zielverbindung mit einer Reinheit von 85% erhalten.

LC-MS (Methode 6): Rₜ = 1.06 min; m/z = 247 (M+H)⁺.

### Beispiel 52A

### Ethyl-(3R)-4,4,4-trifluor-3-methylbutanoat

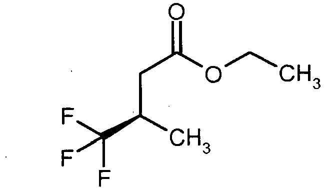

287 g (1.65 mol) (3*R*)-4,4,4-Trifluor-3-methylbutansäure [A. Gerlach und U. Schulz, Speciality Chemicals Magazine 24 (4), 37-38 (2004); CAS Acc.-Nr. 142:179196] in 580 ml Ethanol wurden bei Raumtemperatur langsam mit 133 ml (1.82 mol) Thionylchlorid versetzt. Die Reaktionslösung wurde anschließend auf 80°C erwärmt und 2 h bei dieser Temperatur gerührt. Danach wurde auf Raumtemperatur abgekühlt, langsam mit 250 ml Wasser versetzt und dreimal mit je 150 ml *tert.-*Butylmethylether extrahiert. Die vereinigten organischen Phasen wurden über Natriumsulfat getrocknet. Nach Filtration wurde das Lösungsmittel im Vakuum bei 30°C und einem Druck von 300 mbar entfernt. Das Rohprodukt wurde anschließend bei 100 mbar und einer Kopftemperatur von 65°C destilliert. Es wurden 225.8 g (113 mol, 74% d. Th.) der Titelverbindung als farblose Flüssigkeit isoliert.

¹H-NMR (400 MHz, DMSO-d₆, δ/ppm): 4.10 (2H, q), 2.88-2.72 (1H, m), 2.66-2.57 (1H, m), 2.46-2.36 (1H, m), 1.19 (3H, t), 1.11 (3H, d).

GC-MS (Methode 1): Rₜ = 1.19 min; m/z = 184 (M)⁺.

[α]_{D}²⁰=16.1°, c = 0.41, Methanol.

### Beispiel 53A

### Ethyl-4,4,4-trifluor-3-methyl-2-(4-methylphenyl)butanoat (Diastereomerengemisch)

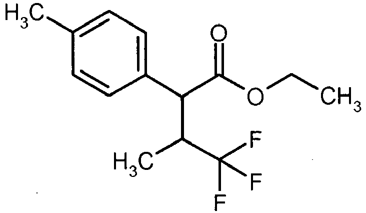

Unter Argon wurden 196.9 mg (0.88 mmol) Palladium(II)acetat und 724.8 mg (1.84 mmol) 2-Di-cyclohexylphosphino-2'-(*N,N*-dimethylamino)biphenyl in 50 ml wasserfreiem Toluol vorgelegt. Die Reaktionslösung wurde anschließend langsam mit 43.8 ml (43.8 mmol) einer 1 M Lösung von Lithiumhexamethyldisilazid in THF versetzt und 10 min bei RT gerührt. Nachfolgend wurde die Reaktionslösung auf -10°C abgekühlt, langsam mit 7 g (38.0 mmol) (+/-)-Ethyl-4,4,4-trifluor-3-methylbutanoat versetzt und 10 min bei -10°C nachgerührt. Danach wurden 5 g (29.2 mmol) 4-Bromtoluol, gelöst in 50 ml Toluol, zugetropft und die Reaktionslösung erst auf RT und dann auf 80°C erwärmt. Das Gemisch wurde 2 h bei dieser Temperatur gerührt, dann auf RT abgekühlt und über Nacht nachgerührt. Nach erfolgter Umsetzung (DC-Kontrolle; Laufmittel Cyclohexan/ Dichlormethan 2:1) wurde die Reaktionsmischung über Kieselgur filtriert, der Rückstand mehrfach mit Essigsäureethylester und Dichlormethan gewaschen und die vereinigten Filtrate im Vakuum eingeengt. Das erhaltene Rohprodukt wurde über Kieselgel chromatographisch gereinigt (Laufmittel Petrolether/Dichlormethan 4:1 → 3:1). Es wurden 3.91 g (14.3 mmol, 48.8% d. Th.) der Titelverbindung als farblose Flüssigkeit isoliert.

¹H-NMR (400 MHz, DMSO-d₆, δ/ppm): 7.26 (2H, d), 7.20-7.12 (2H, m), 4.17-3.95 (2H, m), 3.74 (0.25H, d), 3.66 (0.75H, d), 3.35-3.07 (1H, m), 2.29 (2.25H, s), 2.28 (0.75H, s), 1.17 (0.75H, d), 1.11 (3H, t), 0.76 (2.25H, d).

GC-MS (Methode 1): Rₜ = 4.20 min; m/z = 275 (M+H)⁺ (Diastereomer 1), Rₜ = 4.23 min; m/z = 275 (M+H)⁺ (Diastereomer 2).

### Beispiel 54A

### Ethyl-(3R)-2-(4-chlorphenyl)-4,4,4-trifluor-3-methylbutanoat

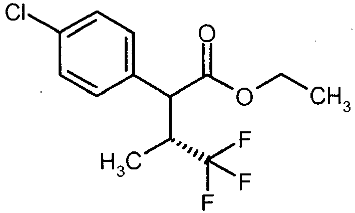

Herstellung von Lösung A: 163.9 ml einer 1 M Lösung von Lithiumhexamethyldisilazid in Toluol wurden unter Argon auf -10°C bis -20°C gekühlt (Kühlung mittels Aceton/Trockeneis) und langsam mit 20 g (108.6 mmol) Ethyl-(3*R*)-4,4,4-trifluor-3-methylbutanoat, gelöst in 150 ml Toluol, versetzt, wobei darauf geachtet wurde, dass eine Temperatur von -10°C nicht überschritten wurde. Die Lösung wurde anschließend 10 min bei maximal -10°C nachgerührt.

Herstellung von Lösung B: 27.03 g (141.2 mmol) 1-Brom-4-chlorbenzol wurden unter Argon bei RT in 100 ml Toluol gelöst und mit 731 mg (3.26 mmol) Palladium(II)acetat und 2.693 g (6.84 mmol) 2'-(Dicyclohexylphosphanyl)-*N,N-*dimethyl-biphenyl-2-amin versetzt. Die Lösung wurde 10 min bei RT nachgerührt.

Zunächst wurde das Kühlbad von Lösung A entfernt. Anschließend wurde zu der noch kalten Lösung A langsam die Lösung B hinzugetropft. Die nun vereinigten Lösungen wurden langsam auf RT erwärmt und 1 h bei dieser Temperatur gerührt. Anschließend wurde die Reaktionslösung auf 80°C (Innentemperatur) erwärmt und 3 h bei dieser Temperatur gerührt. Danach wurde die Reaktionslösung langsam auf RT abgekühlt und noch 12 h nachgerührt. Das Reaktionsgemisch wurde dann über Kieselgur filtriert, der Rückstand mehrfach mit Toluol nachgewaschen und die vereinigten Filtrate im Vakuum eingeengt. Das erhaltene Rohprodukt wurde über Kieselgel chromatographisch gereinigt (Laufmittel Cyclohexan/Dichlormethan 4:1). Es wurden 27.4 g (92.98 mmol, 86% d. Th.) der Titelverbindung als gelbes Öl in einem Diastereomerenverhältnis von 3:1 isoliert.

GC-MS (Methode 1): Rₜ = 4.45 min; m/z = 294 (M)⁺ (Diastereomer 1), Rₜ = 4.48 min; m/z = 294 (M)⁺ (Diastereomer 2).

Analog Synthesebeispiel 53A und 54A wurden die folgenden Verbindungen erhalten:

| **Beispiel** | **Name / Struktur / Edukte** | **Analytische Daten** |
|---|---|---|
| **55A** | Ethyl-(3*R*)-4,4,4-trifluor-2-(4-isopropylphenyl)-3-methylbutanoat | GC-MS (Methode 1): Rₜ = 4.61 min; m/z = 302 (M)⁺ (*Diastereomer 1*); Rₜ = 4.64 min; m/z = 302 (M)⁺ (*Diastereomer 2).* |
| | 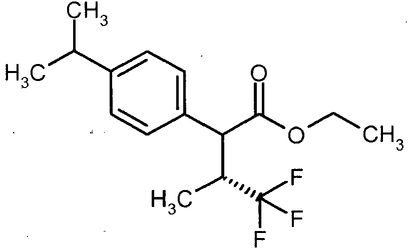 | |
| | (aus 1-Brom-4-isopropylbenzol und Ethyl-(3R)-4,4,4-trifluor-3-methylbutanoat) | |
| **56A** | Ethyl-(3*R*)-2-(4-*tert*.-butylphenyl)-4,4,4-trifluor-3-methylbutanoat | GC-MS (Methode 1): Rₜ = 4.83 min; m/z = 317 (M+H)⁺ (*Diastereomer 1*); Rₜ = 4.85 min; m/z = 317 (M+H)⁺ (*Diastereomer 2).* |
| | 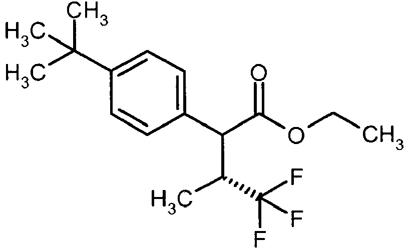 | |
| | | |
| | | MS (DCI): m/z = 334 (M+NH₄)⁺. |
| | (aus 1-Brom-4-*tert*.-butylbenzol und Ethyl-(3*R*)-4,4,4-trifluor-3-methylbutanoat) | |
| **57A** | Ethyl-(3*R*)-4,4,4-trifluor-3-methyl-2-[4-(trifluonnethyl)phenyl]butanoat | GC-MS (Methode 1): Rₜ = 3.38 min; m/z = 328 (M)⁺ (*Diastereomer 1*); Rₜ = 3.42 min; m/z = 328 (M)⁺ (*Diastereomer 2).* |
| | 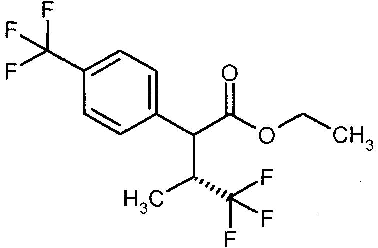 | |
| | (aus 1-Brom-4-(trifluormethyl)benzol und Ethyl-(3*R*)-4,4,4-trifluor-3-methylbutanoat) | |
| **58A** | Ethyl-(3*R*)-4,4,4-trifluor-3-methyl-2-[4-(1,1,1-trifluor-2-methylpropan-2-yl)phenyl]butanoat | GC-MS (Methode 1): Rₜ = 4.68 min; m/z = 370 (M)⁺. |
| | 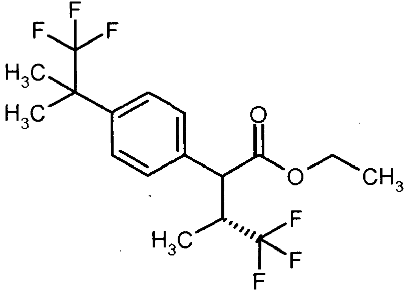 | |
| | (aus 1-Brom-4-(1,1,1-trifluor-2-methylpropan-2-yl)benzol und Ethyl-(3*R*)-4,4,4-trifluor-3-methylbutanoat) | |

### Beispiel 59A

### Ethyl-2-[4-(brommethyl)phenyl]-4,4,4-trifluor-3-methylbutanoat

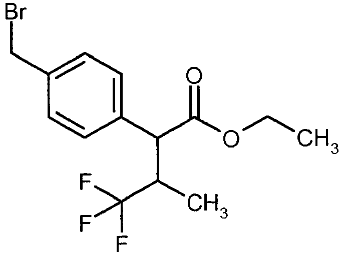

2.25 g (8.2 mmol) Ethyl-4,4,4-trifluor-3-methyl-2-(4-methylphenyl)butanoat, 1.53 g (8.6 mmol) *N-*Bromsuccinimid und 67 mg (0.41 mmol) 2,2'-Azobis-2-methylpropannitril in 36 ml Trichlormethan wurden über Nacht unter Rückfluss gerührt. Nach vollständiger Umsetzung wurde das Succinimid abfiltriert, der Filterrückstand mit Dichlormethan nachgewaschen und das Filtrat im Vakuum eingeengt. Das Rohprodukt wurde über Kieselgel chromatographisch gereinigt (Laufmittel Cyclohexan/Essigsäureethylester 40:1). Es wurden 2.667 g (7.5 mmol, 92% d. Th.) eines gelblichen Öls isoliert.

GC-MS (Methode 1): Rₜ = 5.72 min; m/z = 373 (M-Br)⁺ (Diastereomer 1), Rₜ = 5.74 min; m/z = 373 (M-Br)⁺ (Diastereomer 2).

### Beispiel 60A

### Ethyl-4,4,4-trifluor-3-methyl-2-[4-(2,2,2-trifluorethyl)phenyl]butanoat

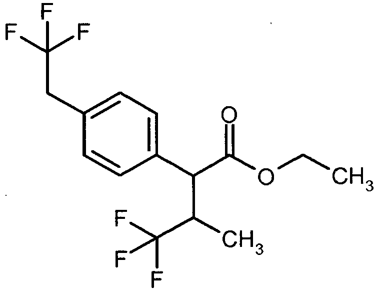

3.77 g (10.67 mmol) Ethyl-2-[4-(brommethyl)phenyl]-4,4,4-trifluor-3-methylbutanoat in 40 ml 1-Methylpyrrolidin-2-on wurden mit 529 mg (2.78 mmol) Kupfer(I)iodid und 4 g (20.82 mmol) Methyl-2,2-difluor-2-(fluorsulfonyl)acetat versetzt und über Nacht bei 80°C gerührt. Nach erfolgter Umsetzung wurde die Reaktionslösung langsam auf 100 ml Eiswasser gegossen. Das erhaltene Gemisch wurde anschließend dreimal mit Diethylether extrahiert. Die vereinigten organischen Phasen wurden über Magnesiumsulfat getrocknet. Nach Filtration wurde das Lösungsmittel im Vakuum entfernt. Das erhaltene Rohprodukt wurde über Kieselgel chromatographisch gereinigt (Laufmittel Cyctohexan/Dichlormethan 4:1). Es wurden 1.48 g (4.32 mmol, 41% d. Th.) der Titelverbindung als gelbliches Öl isoliert.

GC-MS (Methode 1): Rₜ = 4.06 min; m/z = 342 (M)⁺ (Diastereomer 1), Rₜ = 4.09 min; m/z = 342 (M)⁺ (Diastereomer 2).

MS (DCI): m/z = 360 (M+NH₄)⁺.

### Beispiel 61A

### 1-Brom-4-(2-brom-1-fluorethyl)benzol

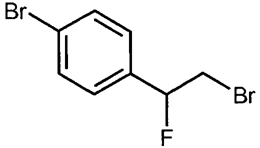

5.0 g (27.31 mmol) 4-Bromstyrol wurden in 40 ml Dichlormethan gelöst, auf 0°C gekühlt und mit 13.21 g (81.94 mmol) Triethylamin-Trihydrofluorid versetzt. Anschließend wurde in drei Portionen 5.83 g (32.78 mmol) *N*-Bromsuccinimid zugegeben. Die Mischung wurde über Nacht bei RT gerührt. Nach Verdünnen mit Dichlormethan wurde die Reaktionsmischung auf Eiswasser gegeben. Die organische Phase wurde nacheinander mit 1 N Salzsäure, Wasser und gesättigter Natriumhydrogencarbonat-Lösung gewaschen, über Magnesiumsulfat getrocknet und im Vakuum eingeengt. Der Rückstand wurde durch Chromatographie an Silicagel gereinigt (Laufmittel Pentan). Es wurden 4.14 g (53.8% d. Th.) der Titelverbindung isoliert.

GC-MS (Methode 1): Rₜ = 4.94 min; m/z = 277/281/283 (M+H)⁺

¹H-NMR (400 MHz, DMSO-*d₆*): δ [ppm] = 3.75-4.04 (m, 2H), 5.84 (dt, 1H), 7.31-7.51 (m, 2H), 7.55-7.78 (m, 2H).

### Beispiel 62A

### 1-Brom-4-(1-fluorvinyl)benzol

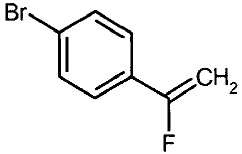

Zu einer auf 0°C gekühlten Lösung von 1.0 g (3.55 mmol) 1-Brom-4-(2-brom-1-fluorethyl)benzol in 10 ml Pentan wurden 796 mg (7.09 mmol) Kalium-*tert*.-butylat in mehreren Portionen gegeben. Die resultierende Suspension wurde 30 min bei 0°C und dann 1 h bei RT gerührt. Der Feststoff wurde abfiltriert, und das Filtrat wurde mit gesättigter Ammoniumchlorid-Lösung gewaschen, über Magnesiumsulfat getrocknet und vorsichtig im Vakuum eingeengt. Es wurden 0.61 g (85.6% d. Th.) der Titelverbindung erhalten.

GC-MS (Methode 1): Rₜ = 3.14min; m/z = 200/202 (M+H)⁺

¹H-NMR (400 MHz, DMSO-*d₆*): δ [ppm] = 5.10 (dd, 1H), 5.47 (dd, 1H), 7.48-7.61 (m, 2H), 7.62-7.72 (m, 2H).

### Beispiel 63A

### (3R)-2-(4-Ethylphenyl)-4,4,4-trifluor-3-methylbutansäureethylester (Diastereomeregemisch)

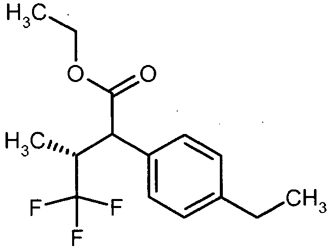

24.4 ml (24.4 mmol) einer 1 M Lösung von Lithiumhexamethyldisilazid in Toluol wurden auf -10°C abgekühlt und tropfenweise mit einer Lösung von 3.0 g (16.29 mmol) Ethyl-(3*R*)-4,4,4-trifluor-3-methylbutanoat in 15 ml abs. Toluol versetzt. Die Mischung wurde 10 min nachgerührt. Anschließend wurde bei -10°C eine zuvor hergestellte Lösung von 3.92 g (21.18 mmol) 1-Brom-4-ethylbenzol, 110 mg (0.49 mmol) Palladium(II)acetat und 404 mg (1.03 mmol) 2'-Dicyclohexyl-phosphino-2-(*N,N*-dimethylamino)biphenyl in 20 ml abs. Toluol zugetropft. Die resultierende Reaktionsmischung wurde zunächst 1 h bei RT, dann 3 h bei 80°C gerührt. Danach wurde die Mischung im Vakuum eingeengt und der Rückstand in Ethylacetat aufgenommen und auf Wasser gegeben. Die wässrige Phase wurde mit Ethylacetat rückextrahiert, und die vereinigten organischen Phasen wurden mit gesättigter Ammoniumchlorid-Lösung und gesättigter Natriumchlorid-Lösung gewaschen, über Magnesiumsulfat getrocknet und im Vakuum eingeengt. Aus dem Rückstand wurden nach Chromatographie an Silicagel (Laufmittel: zunächst Cyclohexan, dann Gradient Cyclohexan/Ethylacetat 200:1 → 50:1) 3.051 g der Titelverbindung erhalten (64.9% d. Th., Diastereomerenverhältnis ca. 3: 1).

LC-MS (Methode 4): Rₜ = 1.52 min; m/z = 289 (M+H)⁺ (Neben-Diastereomer); Rₜ = 1.54 min; m/z = 289 (M+H)⁺ (Haupt-Diastereomer).

¹H-NMR (400 MHz, DMSO-*d₆*): Haupt-Diastereomer: δ [ppm] = 0.76 (d, 3H), 1.13 (t, 3H), 1.17 (t, 3H), 2.55-2.63 (m, 2H), 3.21-3.31 (m, 1H), 3.67 (d, 1H), 3.95-4.16 (m, 2H), 7.15-7.23 (m, 2H), 7.25-7.31 (m, 2H).

Auf analoge Weise wurden ausgehend von Ethyl-(3*R*)-4,4,4-trifluor-3-methylbutanoat und dem entsprechenden Phenylbromid die beiden folgenden Verbindungen hergestellt:

### Beispiel 64A

### (3R)-4,4,4-Trifluor-3-methyl-2-(4-vinylphenyl)butansäureethylester (Diastereomerengemisch)

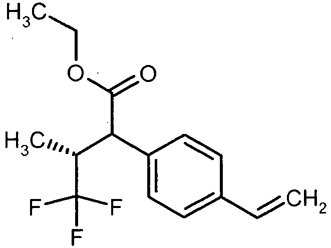

GC-MS (Methode 1): Rₜ = 4.64 min und 4.66 min; m/z = 286 (M)⁺.

¹H-NMR (400 MHz, DMSO-*d₆*): Haupt-Diastereomer: δ [ppm] = 0.79 (d, 3H), 1.12 (t, 3H), 3.22-3.32 (m, 1H), 3.73 (d, 1H), 3.99-4.17 (m, 2H), 5.28 (d, 1H), 5.84 (d, 1H), 6.72 (dd, 1H), 7.34-7.40 (m, 2H), 7.45-7.51 (m, 2H).

### Beispiel 65A

### (3R)-4,4,4-Trifluor-2-[4-(1-fluorvinyl)phenyl]-3-methylbutansäureethylester

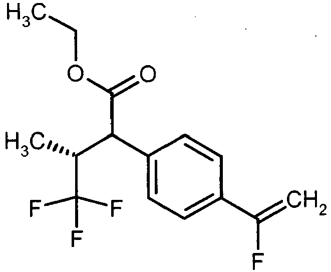

GC-MS (Methode 1): Rₜ = 4.60 min und 4.63 min; m/z = 304 (M)⁺.

LC-MS (Methode 6): Rₜ = 1.29 min und 1.30 min; m/z = 279.

¹H-NMR (400 MHz, DMSO-*d₆*): Haupt-Diastereomer: δ [ppm] = 0.79 (d, 3H), 1.12 (t, 3H), 3.34-3.38 (m, 1H), 3.81 (d, 1H), 3.99-4.17 (m, 2H), 4.97 (dd, 1H), 5.42 (dd, 1H), 7.46-7.49 (m, 2H), 7.63 (d, 2H).

### Beispiel 66A

### Methyl-(4-chlorphenyl)(3-oxocyclopentyl)acetat

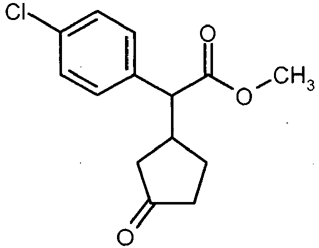

Unter Argon wurden 14.8 ml (105.6 mmol) Diisopropylamin in 150 ml THF vorgelegt, auf -30°C abgekühlt und langsam mit 42.3 ml (105.75 mmol) einer 2.5 M Lösung von n-Butyllithium in Hexan versetzt. Anschließend wurde die Reaktionslösung auf -20°C erwärmt, langsam mit 15 g (81.25 mmol) Methyl-(4-chlorphenyl)acetat, gelöst in 90 ml THF, versetzt und 2 h bei dieser Temperatur nachgerührt. Die Reaktionslösung wurde dann auf -78°C abgekühlt und langsam mit 7.2 ml (86.1 mmol) 2-Cyclopenten-1-on, gelöst in 60 ml THF, versetzt. Nach beendeter Zugabe wurde die Lösung 1 h bei dieser Temperatur nachgerührt. Nach DC-Kontrolle (Laufmittel Cyclohexan/Essigsäureethylester 9:1) wurde der Ansatz mit gesättigter Ammoniumchlorid-Lösung versetzt und in Essigsäureethylester aufgenommen. Die wässrige Phase wurde zweimal mit Essigsäureethylester extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 4:1). Es wurden 15.65 g (58.67 mmol, 72% d. Th.) der Titelverbindung als gelbliches Öl isoliert.

GC-MS (Methode 1): Rₜ = 7.02 min; m/z = 266 (M)⁺ (Diastereomer 1), Rₜ = 7.04 min; m/z = 266 (M)⁺ (Diastereomer 2).

MS (DCI): m/z = 284 (M+NH₄)⁺.

### Beispiel 67A

### Methyl-(4-chlorphenyl)(3,3-difluorcyclopentyl)acetat

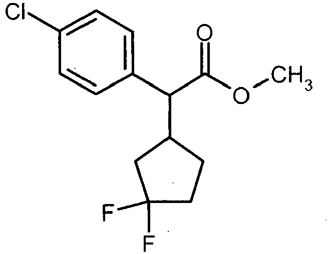

Unter Argon wurden 82.5 ml (82.14 mmol) einer 50%-igen Lösung von 1,1'-[(Trifluor-λ⁴-sulfanyl)-imino]bis(2-methoxyethan) (Desoxofluor) in THF, verdünnt mit 200 ml Toluol, vorgelegt, auf 5°C abgekühlt und langsam mit 744 µl (5.87 mmol) einer 1 M Bortrifluorid-Diethylether-Komplex-Lösung versetzt. Die Mischung wurde 2 h bei 5°C nachgerührt. Anschließend wurde die Reaktionslösung langsam mit 15.65 g (58.67 mmol) Methyl-(4-chlorphenyl)(3-oxocyclopentyl)-acetat, gelöst in 200 ml Toluol, versetzt, dann auf 55°C erwärmt und 60 h bei dieser Temperatur nachgerührt. Die Reaktionsmischung wurde danach in ein auf 0°C gekühltes Gemisch bestehend aus 100 ml Toluol und 100 ml 2 M Natronlauge gegeben. Die organische Phase wurde abgetrennt, und die wässrige Phase wurde noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 7:1). Es wurden 13.24 g (45.86 mmol, 78% d. Th.) der Titelverbindung als farbloses Öl isoliert.

MS (DCI): m/z = 306 (M+NH₄)⁺.

GC-MS (Methode 1): Rₜ = 5.83 min; m/z = 288 (M)⁺ (Diastereomer 1); Rₜ = 5.86 min; m/z = 288 (M)⁺ (Diastereomer 2).

### Beispiel 68A

### (+/-)-Ethyl-(2,2-difluorcyclopentyl)acetat

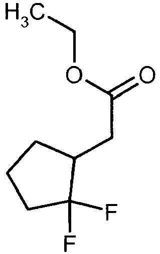

Zu einer Lösung von 52.8 ml (399.5 mmol) Diethylaminoschwefeltrifluorid (DAST) in 150 ml abs. Dichlormethan wurden bei RT 17.0 g (99.88 mmol) (+/-)-Ethyl-2-oxocyclopentylacetat getropft. Die Mischung wurde über Nacht unter Rückfluss erhitzt. Nach Abkühlen wurden weitere 13.2 ml (99.88 mmol) Diethylaminoschwefeltrifluorid (DAST) hinzugefügt, und die Mischung wurde erneut für 36 h unter Rückfluss gerührt. Nach Abkühlen wurde mit Dichlormethan verdünnt, vorsichtig mit gesättigter Natriumhydrogencarbonat-Lösung versetzt und dann kräftig gerührt. Die organische Phase wurde nacheinander mit gesättigter Natriumhydrogencarbonat-Lösung, zweimal mit 1 N Salzsäure und mit gesättigter Natriumchlorid-Lösung gewaschen, über Magnesiumsulfat getrocknet und im Vakuum eingeengt. Aus dem dunkel-braunen Rückstand wurde das Produkt durch Säulenchromatographie an Silicagel isoliert (Laufmittel Pentan/Dichlormethan 10:1 → 1: 1). Es wurden 7.52 g (39% d. Th.) der Titelverbindung erhalten.

GC-MS (Methode 1): Rₜ = 2.88 min; m/z = 172.

¹H-NMR (400 MHz, DMSO-*d₆*): δ [ppm] = 1.18 (t, 3H), 1.33-1.48 (m, 1H), 1.61-1.77 (m, 2H), 1.92-2.20 (m, 3H), 2.24-2.38 (m, 1H), 2.43-2.60 (m, 2H), 4.07 (q, 2H).

### Beispiel 69A

### (4-Chlorphenyl)(2,2-difluorcyclopentyl)essigsäureethylester (Diastereomererigemisch)

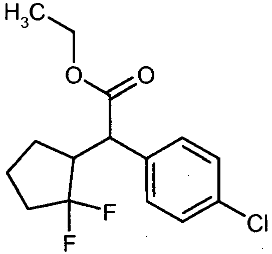

22.6 ml (22.6 mmol) einer 1 M Lösung von Lithiumhexamethyldisilazid in Toluol wurden auf -20°C abgekühlt und tropfenweise mit einer Lösung von 2.90 g (15.09 mmol) (+/-)-Ethyl-(2,2-difluorcyclopentyl)acetat in 20 ml abs. Toluol versetzt. Die Mischung wurde 10 min bei -20°C gerührt. Nach Entfernen der Kühlung wurde eine zuvor hergestellte Lösung von 3.75 g (19.61 mmol) 4-Bromchlorbenzol, 110 mg (0.49 mmol) Palladium(II)acetat und 374 mg (0.95 mmol) 2'-Dicyclohexylphosphino-2-(*N,N*-dimethylamino)biphenyl in 20 ml abs. Toluol zugetropft. Die resultierende Reaktionsmischung wurde zunächst 1 h bei RT und dann 2 h bei 90°C gerührt. Nach Abkühlen wurde die Reaktionsmischung auf Wasser gegeben. Die wässrige Phase wurde dreimal mit Ethylacetat extrahiert, und die vereinigten organischen Phasen wurden über Magnesiumsulfat getrocknet und im Vakuum eingeengt. Aus dem Rückstand wurde nach Chromatographie an Silicagel (Laufmittel Cyclohexan/Ethylacetat 50:1) 2.70 g der Titelverbindung erhalten (59.1 % d. Th., Diastereomerenverhältnis ca. 1:4.3).

GC-MS (Methode 1): Rₜ = 6.09 min und 6.20 min.

¹H-NMR (400 MHz, DMSO-*d₆*): δ [ppm] = 1.01-1.27 (m, 3H), 1.37-1.50 (m, 1H), 1.51-1.75 (m, 3H), 1.94-2.23 (m, 3H), 2.84-3.07 (m, 1H), 3.55-3.79 (m, 1H), 3.93-4.20 (m, 2H), 7.29-7.53 (m, 4H).

### Beispiel 70A

### (+)-(2S,3R)-2-(4-Chlorphenyl)-4,4,4-trifluor-3-methylbutansäure

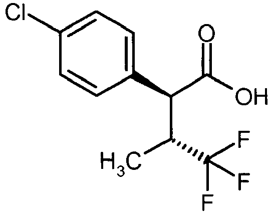

5.086 g (17.26 mmol) Ethyl-(3*R*)-2-(4-chlorphenyl)-4,4,4-trifluor-3-methylbutanoat wurden in 68 ml Dioxan gelöst und mit 34 ml 1 N Natronlauge versetzt. Der Ansatz wurde 2 h bei 50°C gerührt. Das Reaktionsgemisch wurde dann mit 1 N Salzsäure auf pH 1 angesäuert und mehrmals mit Dichlormethan extrahiert. Die vereinigten organischen Phasen wurden mit gesättigter Natriumchlorid-Lösung gewaschen, über Natriumsulfat getrocknet und im Vakuum eingeengt. Es wurden 3.9 g (14.63 mmol, 85% d. Th., 83% de) der Zielverbindung erhalten.

¹H-NMR (400 MHz, DMSO-d₆, δ/ppm): 12.95-12.73 (1H, br. s), 7.49-7.34 (4H, m), 3.68 (1H, d), 3.31-3.18 (1H, m), 1.20 (0.25H, d), 0.78 (2.75H, d).

GC-MS (Methode 1): Rₜ = 4.85 min; m/z = 266 (M)⁺.

[α]_{D}²⁰ = +57.2°, c = 0.41, Methanol.

Analog Synthesebeispiel 70A wurden die in der folgenden Tabelle aufgeführten Verbindungen hergestellt:

| **Beispiel** | **Name / Struktur / Edukt** | **Analytische Daten** |
|---|---|---|
| **71A** | 4,4,4-Trifluor-3-methyl-2-(4-methylphenyl)-butansäure | GC-MS (Methode 1): Rₜ = 4.48 min; m/z = 246 (M)⁺. |
| | 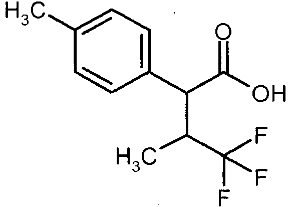 | |
| | (aus Ethyl-4,4,4-trifluor-3-methyl-2-(4-methylphenyl)butanoat) | |
| **72A** | (2*S*,3*R*)-4,4,4-Trifluor-2-(4-isopropylphenyl)3-methylbutansäure | ¹H-NMR (400 MHz, DMSO-d₆, δ/ppm): 12.56 (1H, br. s), 7.25 (4H, q), 3.56 (1H, d), 3.28-3.16 (1H, m), 2.94-2.81 (1H, m), 1.19 (6H, d), 0.75 (3H, d). |
| | 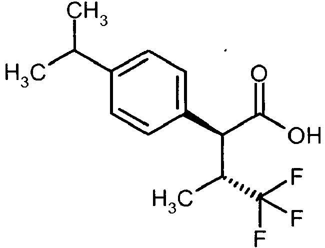 | |
| | | |
| | | GC-MS (Methode 1): Rₜ = 4.93 min; m/z = 274 (M)⁺. |
| | (aus Ethyl-(3*R*)-4,4,4-trifluor-2-(4-isopropylphenyl)-3-methylbutanoat) | |
| **73A** | (2*S*,3*R*)-2-(4-*tert*.-Butylphenyl)-4,4,4-trifluor-3-methylbutansäure | GC-MS (Methode 1): Rₜ = 5.15 min; m/z = 288 (M)⁺. |
| | 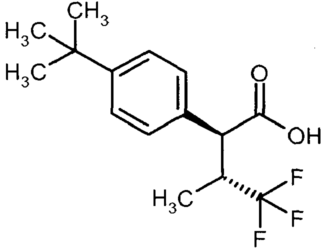 | |
| | (aus Ethyl-(2*S*,3*R*)-2-(4-*tert*.-butylphenyl)-4,4,4-trifluor-3-methylbutanoat) | |
| **74A** | (2*S*,3*R*)-4,4,4-Trifluor-3-methyl-2-[4-(trifluormethyl)phenyl]butansäure | GC-MS (Methode 1): Rₜ = 3.85 min; m/z = 300 (M)⁺. |
| | 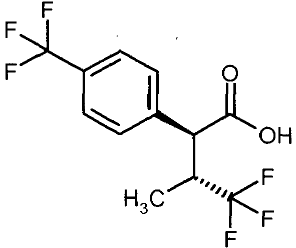 | |
| | (aus Ethyl-(2*S*,3*R*)-4,4,4-trifluor-3-methyl-2-[4-(trifluormethyl)phenyl]butanoat) | |
| **75A** | (2*S*,3*R*)-4,4,4-Trifluor-3-methyl-2-[4-(1,1,1-trifluor-2-methylpropan-2-yl)phenyl]butansäure | ¹H-NMR (400 MHz, DMSO-d₆, δ/ppm): 12.90-12.40 (1H, br. s), 7.53 (2H, d), 7.40 (2H, d), 3.69 (0.11 H, d), 3.64 (0.89H, d), 3.30-3.20 (1H, m), 1.55 (6H, s), 1.21 (0.33H, d), 0.76 (2.67H, d). |
| | 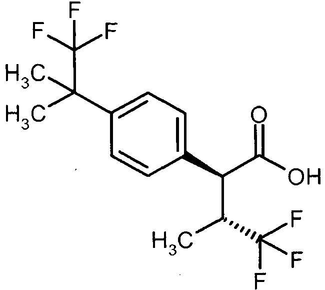 | |
| | | LC-MS (Methode 6): Rₜ = 1.19 min; m/z = 341 (M-H)⁻. |
| | (aus Ethyl-(2*S*,3*R*)-4,4,4-trifluor-3-methyl-2-[4-(1,1,1-trifluor-2-methylpropan-2-yl)phenyl]-butanoat) | |
| **76A** | 4,4,4-Trifluor-3-methyl-2-[4-(2,2,2-trifluorethyl)-phenyl]butansäure | ¹H-NMR (400 MHz, DMSO-d₆, δ/ppm): 12.95-12.59 (1H, br. s), 7.37 (4H, q), 3.70-3.57 (3H, m), 3.30-3.18 (1H, m), 0.76 (3H, d). |
| | 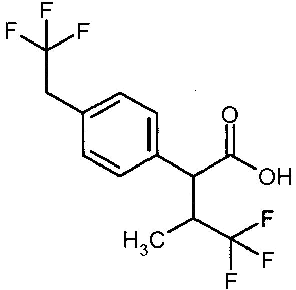 | |
| | | |
| | | GC-MS (Methode 8): Rₜ = 4.45 min; m/z = 315 (M+H)⁺. |
| | (aus Ethyl-4,4,4-trifluor-3-methyl-2-[4-(2,2,2-trifluorethyl)phenyl]butanoat) | |
| **77A** | (4-Chlorphenyl)(3,3-difluorcyclopentyl)essigsäure | ¹H-NMR (400 MHz, DMSO-d₆, δ/ppm): 12.59 (1H, br. s), 7.38 (4H, q), 3.51 (0.5H, d), 3.48 (0.5H, d), 2.77-2.60 (1H, m), 2.42-2.27 (0.5H, m), 2.26-1.20 (5.5H, m). |
| | 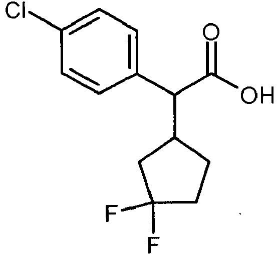 | |
| | (aus Methyl-(4-chlophenyl)(3,3-difluor-cyclopentyl)acetat) | GC-MS (Methode 1): Rₜ = 6.33 min; m/z = 274 (M)⁺ (*Diastereomer 1*); Rₜ = 6.38 min; m/z = 274 (M)⁺ (*Diastereomer 2*). |

### Beispiel 78A

### (3R)-2-(4-Ethylphenyl)-4,4,4-trifluor-3-methylbutansäure (Dinstereomenengemisch)

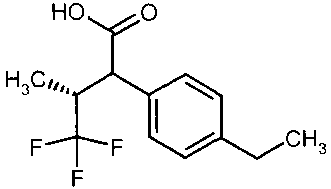

3.0 g (3*R*)-2-(4-Ethylphenyl)-4,4,4-trifluor-3-methylbutansäureethylester (ca. 88% Reinheit, ca. 9.16 mmol; Diastereomerengemisch) wurden in einem Gemisch aus je 12.4 ml Methanol, THF und Wasser gelöst und portionsweise mit 5.49 g (137.35 mmol) Natriumhydroxid versetzt. Die Reaktionsmischung wurde 9 h bei 40°C gerührt. Nach Abkühlen wurden die flüchtigen Lösungsmittel im Vakuum weitgehend entfernt und der Rückstand mit Wasser verdünnt. Durch Zugabe von Salzsäure wurde angesäuert, und die wässrige Phase wurde dreimal mit Ethylacetat extrahiert. Die Vereinigten organischen Phasen wurden über Natriumsulfat getrocknet, im Vakuum eingeengt und der Rückstand im Hochvakuum getrocknet. Erhalten wurden 2.61 g der Titelverbindung als Rohprodukt, welches nicht weiter aufgereinigt wurde (Diastereomerenverhältnis ca. 9:1).

LC-MS (Methode 6): Rₜ = 1.08 min; m/z = 259 (M-H)⁻ (Neben-Diastereomer); Rₜ = 1.11 min; m/z = 259 (M-H)⁻ (Haupt-Diastereomer).

¹H-NMR (400 MHz, DMSO-*d₆*): Haupt-Diastereomer: δ [ppm] = 0.76 (d, 3H), 1.17 (t, 3H), 2.54-2.66 (m, 4H), 3.10-3.29 (m, 1H), 3.56 (d, 1H), 7.14-7.22 (m, 2H), 7.22-7.32 (m, 2H), 12.58 (br. s, 1 H).

Auf vergleichbare Weise (Reaktionstemperatur: RT bis 40°C; Reaktionszeit: 9-12 h) wurden aus den entsprechenden Estern die beiden folgenden Carbonsäure-Derivate hergestellt:

### Beispiel 79A

### (3R)-4,4,4-Trifluor-3-methyl-2-(4-vinylphenyl)butansäure (Diastereomerengemisch)

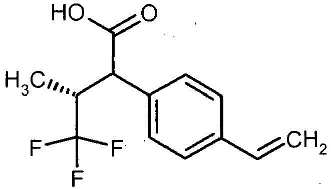

Diastereomerenverhältnis ca. 10:1.

LC-MS (Methode 6): Rₜ = 1.04 min; m/z = 257 (M-H)⁻ (Neben-Diastereomer); Rₜ = 1.06 min; m/z = 257 (M-H)⁻ (Haupt-Diastereomer).

¹H-NMR (400 MHz, DMSO-*d₆*): Haupt-Diastereomer: δ [ppm] = 0.78 (d, 3H), 3.18-3.31 (m, 1H), 3.62 (d, 1H), 5.28 (d, 1H), 5.84 (d, 1H), 6.73 (dd, 1H), 7.31-7.39 (m, 2H), 7.40-7.54 (m, 2H), 12.74 (br. s, 1H).

### Beispiel 80A

### (3R)-4,4,4-Trifluor-2-[4-(1-fluorvinyl)phenyl]-3-methylbutansäure (Diastereomerengemisch)

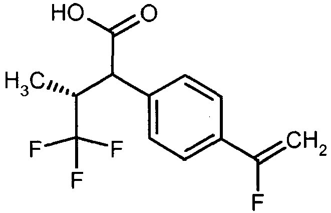

Diastereomerenverhältnis ca. 9:1.

GC-MS (Methode 1): Rₜ = 4.97 min; m/z = 276.

¹H-NMR (400 MHz, DMSO-*d₆*): Haupt-Diastereomer: δ [ppm] = 0.78 (d, 3H), 3.16-3.29 (m, 1H), 3.70 (d, 1H), 4.96 (dd, 1H), 5.34 (d, 1H), 5.47 (d, 1H), 7.39-7.51 (m, 2H), 7.58-7.69 (m, 2H), 12.83 (br. s, 1H).

### Beispiel 81A

### (4-Chlorphenyl)(,2,2-difluorcyclopentyl)essigsäure (Diastereomerengemisch)

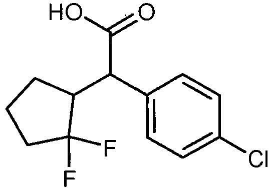

2.70 g (8.92 mmol) (4-Chlorphenyl)(2,2-difluorcyclopentyl)essigsäureethylester (Diastereomerengemisch) wurden in 10 ml Methanol, 10 ml THF und 5 ml Wasser gelöst und bei RT mit 7.13 g (89.18 mmol) 50%-iger Natronlauge versetzt. Die Reaktionsmischung wurde über Nacht bei RT gerührt. Danach wurde mit Wasser verdünnt und mit Salzsäure sauer gestellt. Die wässrige Phase wurde dreimal mit Ethylacetat extrahiert, und die vereinigten organischen Phasen wurden über Magnesiumsulfat getrocknet, im Vakuum eingeengt und der Rückstand im Hochvakuum getrocknet. Erhalten wurden 2.39 g der Zielverbindung (97.6% d. Th., Diastereomerenverhältnis ca. 1:1).

LC-MS (Methode 6): Rₜ = 1.05 min und 1.07 min; m/z = 273 (M-H)⁻.

### Beispiel 82A

### (2S,3R)-2-(4-Chlorphenyl)-4,4,4-trifluor-3-methylbutanoylchlorid

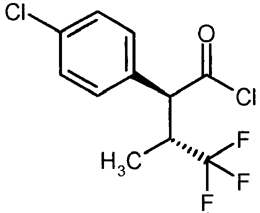

19.5 g (73.13 mmol) (2*S*,3*R*)-2-(4-Chlorphenyl)-4,4,4-trifluor-3-methylbutansäure wurden in 860 ml Dichlormethan gelöst und mit 0.5 ml DMF versetzt. Anschließend wurden bei -5°C bis -10°C (Eis/Aceton-Kühlbad) 73 ml (146.26 mmol) eine 2 M Lösung von Oxalylchlorid in Dichlormethan langsam zugetropft und die Mischung 1 h bei dieser Temperatur nachgerührt. Nach vollständiger Umsetzung wurde die Reaktionslösung im Vakuum eingedampft und der erhaltene Rückstand in 200 ml Dichlormethan aufgenommen und anschließend wieder bis zur Trockene eingeengt. Es wurden 20.1 g (70.5 mmol, 96% d. Th.) der Titelverbindung als farbloses Öl erhalten. Das so erhaltene Produkt wurde ohne weitere Aufreinigung und ohne weitere spektroskopische Charakterisierung in Folgereaktionen eingesetzt.

Auf analoge Weise wurden die in der folgenden Tabelle aufgeführten Verbindungen hergestellt:

| **Beispiel** | **Name / Struktur** | **Ausgangsmaterial** |
|---|---|---|
| **83A** | (2*S*,3*R*)-4,4,4-Trifluor-2-(4-isopropylphenyl)-3-methylbutanoylchlorid | (2*S*,3*R*)-4,4,4-Trifluor-2-(4-isopropylphenyl)-3-methylbutansäure |
| | 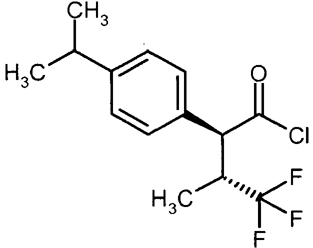 | |
| **84A** | (2*S*,3*R*)-2-(4-*tert*.-Butylphenyl)-4,4,4-trifluor-3-methylbutanoylchlorid | (2*S,*3*R*)*-*2*-*(4*-tert.*-Butylphenyl)-4,4,4-trifluor-3-methylbutansäure |
| | 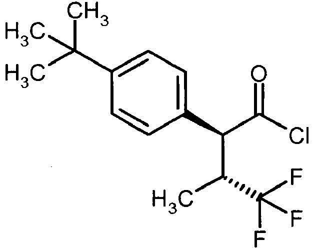 | |
| **85A** | (2*S*,3*R*)-4,4,4-Trifluor-3-methyl-2-[4-(trifluormethyl)phenyl]butanoylchlorid | (2*S*,3*R*)-4,4,4-Trifluor-3-methyl-2-[4-(trifluor-methyl)phenyl]butansäure |
| | 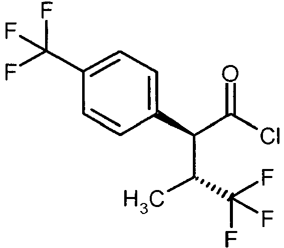 | |
| **86A** | (2*S*,3*R*)-4,4,4-Trifluor-3-methyl-2-[4-(1,1,1-trifluor-2-methylpropan-2-yl)phenyl]butanoylchlorid | (2S,3R)-4,4,4-Trifluor-3-methyl-2-[4-(1,1,1-trifluor-2-methylpropan-2-yl)phenyl]butansäure |
| | 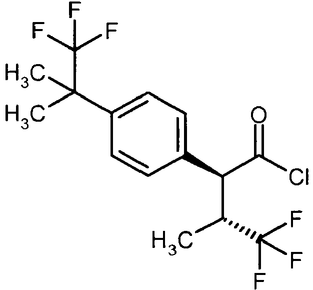 | |
| **87A** | 4,4,4-Trifluor-3-methyl-2-[4-(2,2,2-trifluorethyl)phenyl]butanoylchlorid | 4,4,4-Trifluor-3-methyl-2-[4-(2,2,2-trifluorethyl)-phenyl]butansäure |
| | 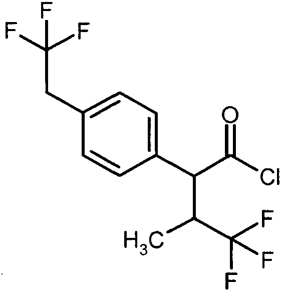 | |
| **88A** | (4-Chlorphenyl)(3,3-difluorcyclopentyl)acetylchlorid | (4-Chlorphenyl)-(3,3-difluorcyclopentyl)-essigsäure |
| | 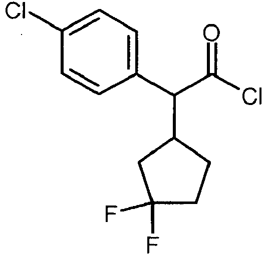 | |

### Beispiel 89A

### tert.-Butyl-3-(4-chlor-3-{[(2S,3R)-2-(4-chlorphenyl)-4,4,4-trifluor-3-methylbutanoyl]amino}-phenyl)propanoat

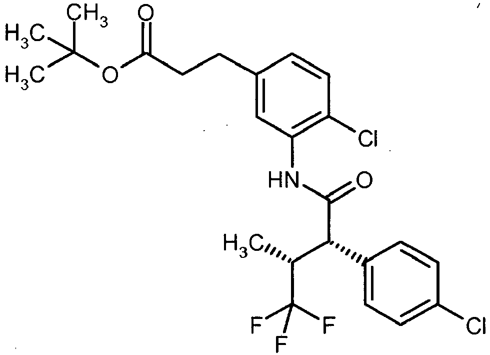

18 g (70.38 mmol) (2*S*,3*R*)-2-(4-Chlorphenyl)-4,4,4-trifluor-3-methylbutanoylchlorid wurden in 500 ml THF gelöst, mit 18.4 ml (105.57 mmol) *N,N*-Diisopropylethylamin versetzt und auf -10°C abgekühlt. Anschließend wurden 20.07 g (70.38 mmol) *tert*.-Butyl-3-(3-amino-4-chlophenyl)-propanoat, gelöst in 500 ml THF, langsam zugegeben, wobei darauf geachtet wurde, dass während der Zugabe eine Reaktionstemperatur von 0°C nicht überschritten wurde. Die Mischung wurde anschließend noch 1 h nachgerührt. Die Reaktionslösung wurde dann mit Wasser und mit Essigsäureethylester versetzt, die organische Phase abgetrennt und die wässrige Phase noch dreimal mit Essigsäureethylester extrahiert. Die vereinigten organischen Phasen wurden über Natriumsulfat getrocknet und am Rotationsverdampfer eingeengt. Der Rückstand wurde durch Chromatographie an Kieselgel gereinigt (Laufmittel Cyclohexan/Essigsäureethylester 20:1). Es wurden 30.13 g (59.74 mmol, 85% d. Th.) der Titelverbindung erhalten.

¹H-NMR (400 MHz, DMSO-d₆, δ/ppm): 9.82 (1H, s), 7.50-7.42 (4H, m), 7.39-7.32 (2H, m), 7.07-7.01 (1H, m), 4.12 (1H, d), 3.42-3.29 (1H, m), 2.75 (2H, t), 2.46 (2H, t), 1.31 (9H, s), 0.80 (3H, d).

LC-MS (Methode 7): Rₜ = 3.03 min; m/z = 502/504 (M-H)⁻.

Auf analoge Weise wurden die in der folgenden Tabelle aufgeführten Verbindungen erhalten:

| **Beispiel** | **Name / Struktur / Edukte** | **Analytische Daten** |
|---|---|---|
| **90A** | *tert*.-Butyl-3-(4-chlor-3-{[(2*S*,3*R*)-4,4,4-trifluor-2-(4-isopropylphenyl)-3-methylbutanoyl]-amino}phenyl)propanoat | ¹H-NMR (400 MHz, DMSO-d₆, δ/ppm): 9.70 (1H, s), 7.47-7.42 (1H, m), 7.34 (3H, t), 7.23 (2H, d), 7.04-6.99 (1H, m), 4.07 (1H, d), 3.40-3.26 (1H, m), 2.94-2.81 (1H, m), 2.75 (2H, t), 2.45 (2H, t), 1.31 (9H, s), 1.19 (6H, d), 0.78 (3H, d). |
| | 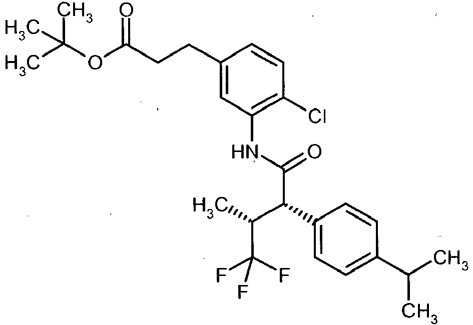 | |
| | | |
| | | LC-MS (Methode 4): Rₜ = 1.72 min; m/z = 510/512 (M-H)⁻. |
| | (aus (2*S*,3*R*)-4,4,4-Trifluor-2-(4-isopropylphenyl)-3-methylbutanoylchlorid und *tert*.-Butyl-3-(3-amino-4-chlorphenyl)propanoat) | |
| **91A** | *tert*.-Butyl-3-(3-{[(2*S*,3*R*)-2-(4-*tert*.-butylphenyl)-4,4,4-trifluor-3-methylbutanoyl]amino}-4-chlorphenyl)propanoat | ¹H-NMR (400 MHz, DMSO-d₆, δ/ppm): 9.71 (1H, s), 7.49-7.43 (1H, m), 7.41-7.35 (4H, m), 7.34 (1H, d), 7.04-6.98 (1H, m), 4.08 (1H, d), 3.39-3.25 (1H, m), 2.75 (2H, t), 2.45 (2H, t), 1.31 (9H, s), 1.27 (9H, s), 0.78 (3H, d). |
| | 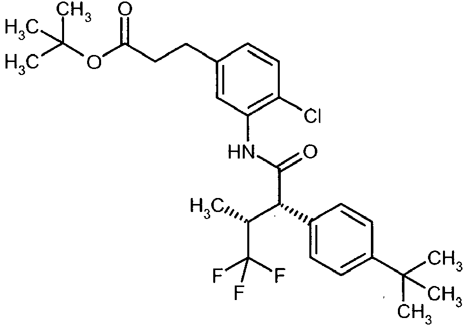 | |
| | | |
| | | LC-MS (Methode 6): Rₜ = 1.52 min; m/z = 524/526 (M-H)⁻. |
| | (aus (2*S*,3*R*)-2-(4-*tert*.-Butylphenyl)-4,4,4-trifluor-3-methylbutanoylchlorid und *tert*.-Butyl-3-(3-amino-4-chlorphenyl)propanoat) | |
| **92A** | *tert*.-Butyl-3-[4-chlor-3-({(2*S*,3*R*)-4,4,4-trifluor-3-methyl-2-[4-(trifluormethyl)phenyl]butanoyl}-amino)phenyl]propanoat | ¹H-NMR (400 MHz, DMSO-d₆, δ/ppm): 9.89 (1H, s), 7.76 (2H, d), 7.69 (2H, d), 7.37 (1H, d), 7.35 (1H, d), 7.04 (1H, dd), 4.24 (1H, d), 3.48-3.36 (1H, m), 2.75 (2H, t), 2.45 (2H, t), 1.29 (9H, s), 0.80 (3H, d). |
| | 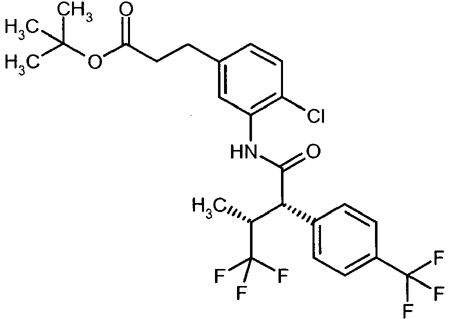 | |
| | | |
| | | LC-MS (Methode 6): Rₜ = 1.43 min; m/z = 536 (M-H)⁻. |
| | (aus (2*S*,3*R*)-4,4,4-Trifluor-3-methyl-2-[4-(trifluonnethyl)phenyl]butanoylchlorid und *tert*.-Butyl-3-(3-amino-4-chlorphenyl)propanoat) | |
| **93A** | *tert*.-Butyl-3-[4-chlor-3-({(2*S*,3*R*)-4,4,4-trifluor-3-methyl-2-[4-(1,1,1-trifluor-2-methylpropan-2-yl)-phenyl]butanoyl} amino)phenyl]propanoat | LC-MS (Methode 6): Rₜ = 1.48 min; m/z = 579 (M-H)⁻. |
| | 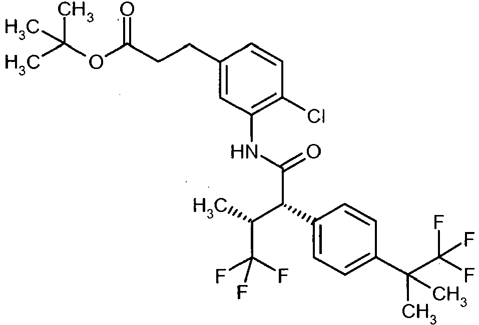 | |
| | (aus (2*S*,3*R*)-4,4,4-Trifluor-3-methyl-2-[4-(1,1,1-trifluor-2-methylpropan-2-yl)phenyl]butanoylchlorid und *tert*.-Butyl-3-(3-amino-4-chlorphenyl)propanoat) | |
| **94A** | *tert*.-Butyl-3-[4-chlor-3-({4,4,4-trifluor-3-methyl-2-[4-(2,2,2-trifluorethyl)phenyl]butanoyl} amino)-phenyl]propanoat | ¹H-NMR (400 MHz, DMSO-d₆, δ/ppm): 9.78 (1H, s), 7.46 (2H, d), 7.41 (1H, d), 7.35 (3H, t), 7.02 (1H, dd), 4.11 (1H, d), 3.63 (2H, q), 3.42-3.28 (1H, m), 2.75 (2H, t), 2.45 (2H, t), 1.30 (9H, s), 0.79 (3H, d). |
| | 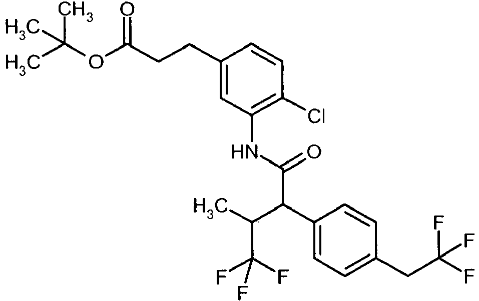 | |
| | | LC-MS (Methode 6): Rₜ = 1.41 min; m/z = 550 (M-H)⁻. |
| | (aus 4,4,4-Trifluor-3-methyl-2-[4-(2,2,2-trifluorethyl)phenyl]butanoylchlorid und *tert*.-Butyl-3-(3-amino-4-chlophenyl)propanoat) | |
| **95A** | *tert*.-Butyl-3-(4-chlor-3-{[(4-chlorphenyl)(3,3-difluorcyclopentyl)acetyl]amino}phenyl)propanoat | LC-MS (Methode 5): Rₜ = 3.01 min; m/z = 510/512 (M-H)⁻. |
| | 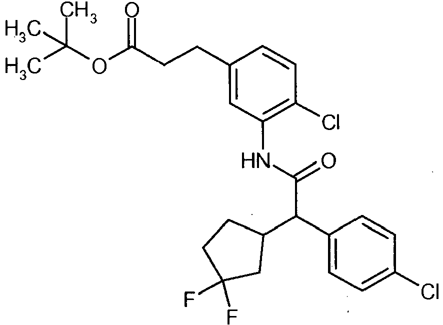 | |
| | (aus (4-Chlophenyl)(3,3-difluorcyclopentyl)-acetylchlorid und *tert*.-Butyl-3-(3-amino-4-chlorphenyl)propanoat) | |
| **96A** | Ethyl-(2*S*)-3-(4-chlor-3-{[(4-chlorphenyl)-(3,3-difluorcyclopentyl)acetyl]amino} phenyl)-2-methylpropanoat | LC-MS (Methode 7): Rₜ =2.94 min; m/z = 498 (M)⁺. |
| | 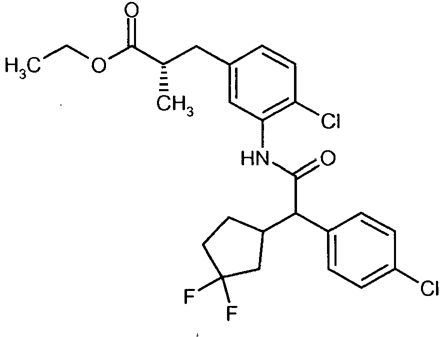 | |
| | (aus (4-Chlorphenyl)(3,3-difluorcyclopentyl)-acetylchlorid und Ethyl-(2*S*)-3-(3-amino-4-chlor-phenyl)-2-methylpropanoat) | |
| **97A** | Ethyl-(2*R*)-3-(4-chlor-3-{[(4-chlorphenyl)-(3,3-difluorcyclopentyl)acetyl]amino}phenyl)-2-methylpropanoat | LC-MS (Methode 7): Rₜ = 2.94 min; m/z = 498 (M)⁺. |
| | 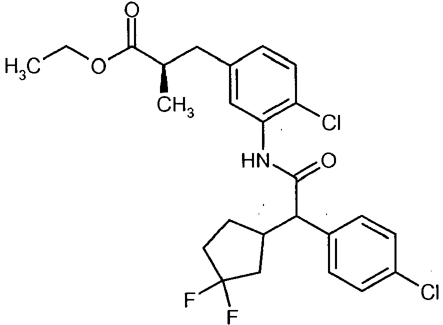 | |
| | (aus (4-Chlorphenyl)(3,3-difluorcyclopentyl)-acetylchlorid und Ethyl-(2R)-3-(3-amino-4-chlorphenyl)-2-methylpropanoat) | |
| **98A** | Methyl-[1-(4-chlor-3-{[(3*R*)-2-(4-chlorphenyl)-4,4,4-trifluor-3-methylbutanoyl]amino}phenyl)-cyclobutyl]acetat | ¹H-NMR (400 MHz, DMSO-d₆, δ/ppm): 9.95 (0.33H, s), 9.81 (0.66H, s), 7.54-7.30 (6H, m), 7.02-6.93 (1H, m), 4.14 (1H, d), 3.41-3.28 (1H, m), 3.37 (3H, s), 2.80-2.74 (2H, m), 2.35-2.19 (4H, m), 2.11-1.97 (1H, m), 1.82-1.69 (1H, m), 1.25 (1H, d), 0.80 (2H, d). |
| | 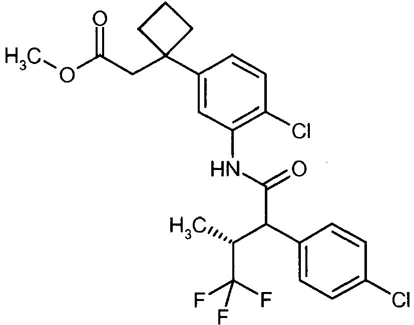 | |
| | | |
| | | LC-MS (Methode 7): Rₜ = 2.96 min; m/z = 500/502 (M-H)⁻. |
| | (aus (2*S*,3*R*)-2-(4-Chlorphenyl)-4,4,4-trifluor-3-methylbutanoylchlorid und Methyl-[1-(3-amino-4-chlorphenyl)cyclobutyl]acetat) | |
| **99A** | Benzyl-[3-(4-chlor-3-{[(2*S*,3*R*)-2-(4-chlorphenyl)-4,4,4-trifluor-3-methylbutanoyl]amino}phenyl)-oxetan-3-yl]acetat | ¹H-NMR (400 MHz, DMSO-d₆, δ/ppm): 9.88 (1H, s), 7.51 (1H, d), 7.45 (4H, q), 7.38 (1H, d), 7.33-7.23 (3H, m), 7.17-7.10 (2H, m), 7.03 (1H, dd), 4.92 (2H, s), 4.78-4.67 (4H, m), 4.17 (1H, d), 3.42-3.28 (1H, m), 3.18 (2H, s), 0.80 (3H, d). |
| | 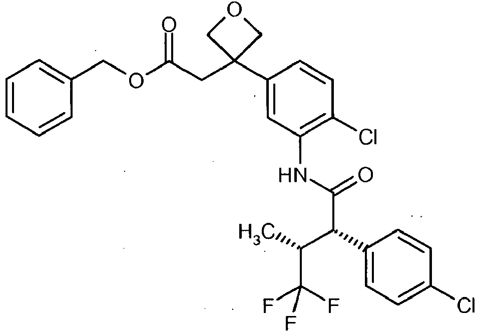 | |
| | | |
| | | LC-MS (Methode 7): Rₜ = 2.87 min; m/z = 578 (M-H)⁻. |
| | (aus (2*S*,3*R*)-2-(4-Chlorphenyl)-4,4,4-trifluor-3-methylbutanoylchlorid und Benzyl-[3-(3-amino-4-chlorphenyl)oxetan-3-yl]acetat) | |

### Beispiel 100A

### Methyl-[1-(3-{[(2S,3R)-2-(4-chlorphenyl)-4,4,4-trifluor-3-methylbutanoyl]amino}-4-fluorophenyl)-cyclopropyl]acetat

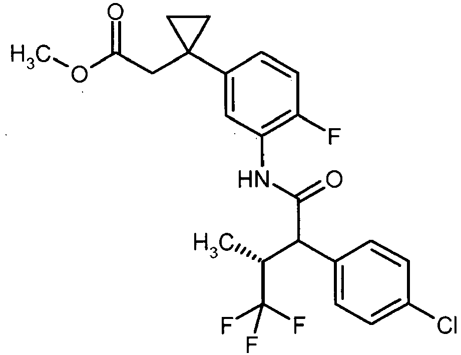

Eine Lösung von 70 mg (0.31 mmol) (2*S*,3*R*)-2-(4-Chlorphenyl)-4,4,4-trifluor-3-methylbutansäure, 84 mg (0.31 mmol) Methyl-[1-(3-amino-4-fluorphenyl)cyclopropyl]acetat, 179 mg (0.47 mmol) 2-(1*H*-7-Azabenzotriazol-1-yl)-1,1,3,3-tetramethyluronium-hexafluorophosphat (HATU) und 0.6 ml Pyridin wurden in 2.4 ml DMF über Nacht bei Raumtemperatur gerührt. Nach Beendigung der Reaktion wurde der Ansatz direkt ohne weitere Aufarbeitung mittels präparativer HPLC aufgetrennt. Es wurden 106 mg (0.22 mmol, 72% d. Th.) der Titelverbindung als ein farbloses Öl erhalten.

¹H-NMR (400 MHz, DMSO-d₆, δ/ppm): 10.02 (1H, s), 7.71 (1H, dd), 7.52-7.38 (4H, m), 7.15-7.06 (1H, m), 7.05-6.98 (1H, m), 4.11 (1H, d), 3.48 (3H, s), 3.42-3.25 (1H, m), 2.57 (2H, s), 0.90-0.84 (2H, m), 0.81-0.74 (5H, m).

LC-MS (Methode 6): Rₜ = 1.33 min; m/z = 472 (M+H)⁺.

Auf analoge Weise wurde die folgende Verbindung erhalten:

| **Beispiel** | **Name / Struktur / Edukte** | **Analytische Daten** |
|---|---|---|
| **101A** | Methyl-[1-(3-{[(2*S*,3*R*)-2-(4-ethylphenyl)-4,4,4-trifluor-3-methylbutanoyl]amino}-4-fluorphenyl)cyclopropyl]acetat | ¹H-NMR (400 MHz, DMSO-d₆, δ/ppm): 9.96 (1H, s), 7.74 (1H, dd), 7.34 (2H, d), 7.20 (2H, d), 7.12-7.05 (1H, m), 7.03-6.96 (1H, m), 4.04 (1H, d), 3.47 (3H, s), 3.41-3.25 (1H, m), 2.63-2.52 (4H, m), 1.17 (3H, t), 0.89-0.84 (2H, m), 0.81-0.73 (5H, m). |
| | 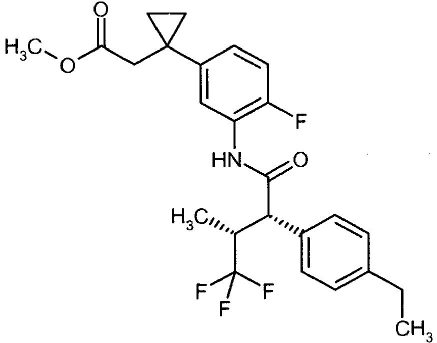 | |
| | | |
| | | LC-MS (Methode 4): Rₜ = 1.53 min; m/z = 466 (M+H)⁺. |
| | (aus (2*S*,3*R*)-2-(4-Ethylphenyl)-4,4,4-trifluor-3-methylbutansäure und Methyl-[1-(3-amino-4-fluorphenyl)cyclopropyl]acetat) | |

### Allgemeine Vorschrift 1: HATU-vermittelte Amidkupplung von 4,4,4-Trifluor-3-methyl-2-phenyl-butansäure-Derivaten mit Anilinen

Zu einer Lösung des betreffenden 4,4,4-Trifluor-3-methyl-2-phenylbutansäure-Derivats (ca. 0.8 bis 1.5 eq., 0.15 bis 1.5 mol/l) und eines Anilins (ca. 0.8 bis 1.5 eq., 0.15 bis 1.5 mol/l) in einem Gemisch aus DMF und Pyridin (Mischungsverhältnis ca. 3:1 bis 1.5:1) wird bei RT HATU (1.0 bis 2.0 eq.) gegeben. Alternativ kann statt Pyridin auch *N*,*N*-Diisopropylethylamin (2.0 bis 5.0 eq.) verwendet werden. Die resultierende Mischung wird für 4 h bis 48 h bei einer Temperatur von RT bis 60°C gerührt. Gegebenenfalls wird nach 24 h ein weiterer Anteil an Anilin oder an Carbonsäure und HATU nachgesetzt. Nach Ende der Reaktion kann das Rohprodukt nach Entfernen des Lösungsmittels im Vakuum durch präparative RP-HPLC (Eluent: Acetonitril/Wasser-Gradient) oder alternativ, nach wässriger Aufarbeitung der Reaktionsmischung, durch Chromatographie an Silicagel (Eluent: Cyclohexan/Ethylacetat- oder Dichlormethan/Methanol-Gemische) gereinigt werden.

Die folgenden Beispiele wurden gemäß der Allgemeinen Vorschrift 1 hergestellt:

| **Beispiel** | **Name / Struktur** | **Analytische Daten** |
|---|---|---|
| **102A** | (+/-)-3-(3-{[2-(4-Chlorphenyl)-4,4,4-trifluor-3-methylbutanoyl]amino}-2-fluorphenyl)-propansäure-*tert*.-butylester (*Diastereomer 1*) | LC-MS (Methode 4): Rₜ = 1.64 min; m/z = 487 (M-H)⁻. |
| | | |
| | 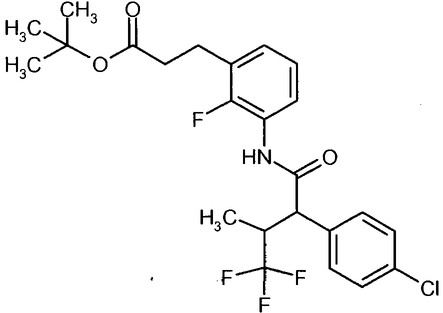 | ¹H-NMR (400 MHz, DMSO-*d*₆): δ [ppm] = 0.79 (d, 3H), 1.32 (s, 9H), 2.48 (t, 2H), 2.81 (t, 2H), 3.34-3.45 (m, 1H), 4.12 (d, 1H), 6.88-7.12 (m, 2H), 7.36-7.52 (m, 4H), 7.63 (td, 1H), 10.03 (s, 1H). |
| **103A** | (+/-)-3-(3-{[2-(4-Chlorphenyl)-4,4,4-trifluor-3-methylbutanoyl]amino}-2-fluorphenyl)-propansäure-*tert*.-butylester (*Diastereomer* 2) | ¹H-NMR (400 MHz, DMSO-*d*₆): δ [ppm] = 1.22 (d, 3H), 1.32 (s, 9H), 2.49 (t, 2H), 2.82 (t, 2H), 3.21 (dd, 1H), 4.15 (d, 1H), 6.93-7.12 (m, 2H), 7.35-7.43 (m, 2H), 7.43-7.52 (m, 2H), 7.54-7.74 (m, 1H), 10.12 (s, 1H). |
| | 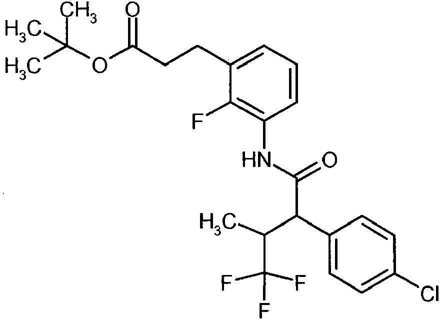 | |
| **104A** | (+/-)-3-(3-{[2-(4-Chlorphenyl)-4,4,4-trifluor-3-methylbutanoyl]amino}-4-fluorphenyl)-propansäure-*tert*.-butylester (*Diastereomer 1*) | LC-MS (Methode 4): Rₜ = 1.63 min; m/z = 486 (M-H)⁻. |
| | | |
| | 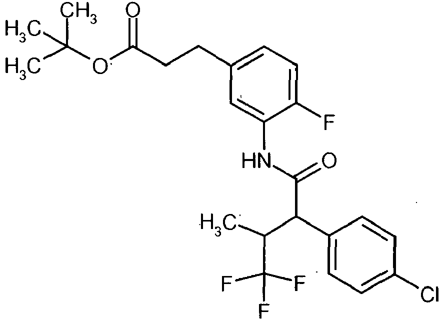 | ¹H-NMR (400 MHz, DMSO-*d*₆): δ [ppm] = 0.78 (d, 3H), 1.31 (m, 9H), 2.44 (t, 2H), 2.74 (t, 2H), 3.33-3.48 (m, 1H), 4.11 (d, 1H), 6.92-7.04 (m, 1H), 7.12 (dd, 1H), 7.35-7.52 (m, 4H), 7.65 (dd, 1H), 10.02 (s, 1H). |
| **105A** | (+/-)-3-(3-{[2-(4-Chlorphenyl)-4,4,4-trifluor-3-methylbutanoyl]amino}-4-fluorphenyl)-propansäure-*tert*-butylester (*Diastereomer 2)* | LC-MS (Methode 4): Rₜ = 1.63 min; m/z = 486 (M-H)⁻. |
| | | |
| | 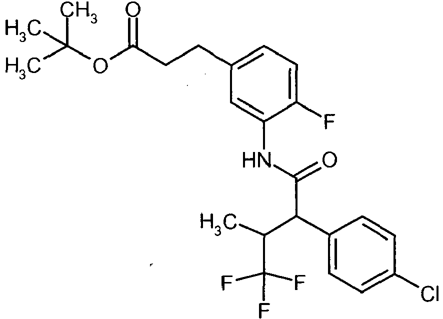 | ¹H-NMR (400 MHz, DMSO-*d*₆): δ [ppm] = 1.21 (d, 3H), 1.31 (s, 9H), 2.45 (t, 2H), 2.74 (t, 2H), 3.21 (dd, 1H), 4.13 (d, 1H), 6.89-7.06 (m, 1H), 7.14 (dd, 1H), 7.36-7.44 (m, 2H), 7.45-7.55 (m, 2H), 7.62 (dd, 1H), 10.12 (s, 1H). |
| **106A** | (+)-3-(3-{[(2*S*,3*R*)-2-(4-Chlorphenyl)-4,4,4-trifluor-3-methylbutanoyl]amino}-4-fluorphenyl)-propansäure-*tert*.-butylester | LC-MS (Methode 6): Rₜ = 1.43 min; m/z = 486 (M-H)⁻. |
| | | |
| | 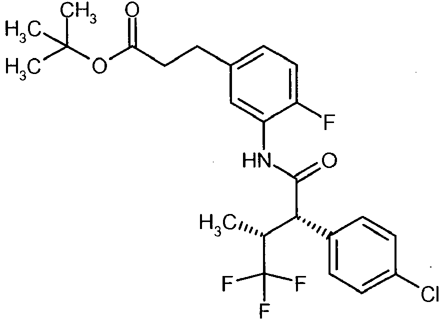 | ¹H-NMR (400 MHz, DMSO-*d*₆): δ [ppm] = 0.78 (d, 3H), 1.31 (s, 9H), 2.44 (t, 2H), 2.74 (t, 2H), 3.34-3.43 (m, 1H), 4.11 (d, 1H), 6.87-7.02 (m, 1H), 7.12 (dd, 1H), 7.36-7.51 (m, 4H), 7.65 (dd, 1H), 10.03 (s, 1H). |
| | | |
| | | [α]_{D}²⁰ = +127°, c = 0.52, Chloroform. |
| **107A** | (+)-3-(4-Fluor-3-{[(2*S*,3*R*)-4,4,4-trifluor-3-methyl-2-(4-vinylphenyl)butanoyl]amino}-phenyl)propansäure-*tert*.-butylester | LC-MS (Methode 6): Rₜ = 1.39 min; m/z = 478 (M-H)⁻. |
| | | |
| | 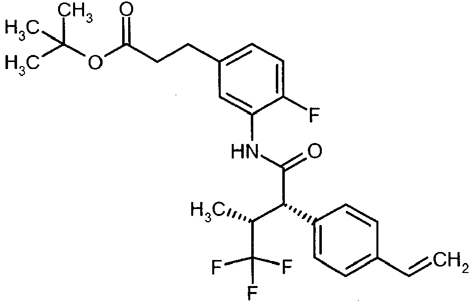 | ¹H-NMR (400 MHz, DMSO-*d*₆): δ [ppm] = 0.79 (d, 3H), 1.31 (s, 9H), 2.44 (t, 2H), 2.74 (t, 2H), 3.35-3.43 (m, 1H), 4.08 (d, 1H), 5.26 (d, 1H), 5.83 (d, 1H), 6.72 (dd, 1H), 6.97 (td, 1H), 7.11 (dd, 1H), 7.32-7.51 (m, 4H), 7.66 (dd, 1H), 9.99 (s, 1H). |
| | | |
| | | [α]_{D}²⁰ = +119.4°, c = 0.455, Chloroform. |
| **108A** | Ethyl-3-(3-{[(2*S*,3*R*)-2-(4-chlorphenyl)-4,4,4-trifluor-3-methylbutanoyl]amino}-4-fluorphenyl)-2-methylpropanoat (*Diastereomerengemisch*) | LC-MS (Methode 6): Rₜ = 1.35 min; m/z = 474 (M+H)⁺. |
| | | |
| | 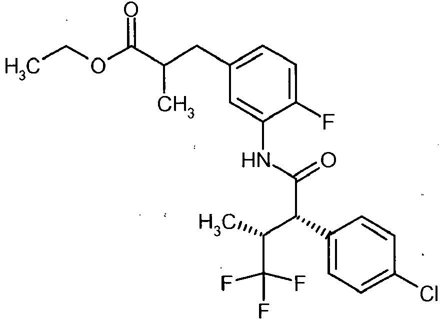 | ¹H-NMR (400 MHz, DMSO-*d*₆): δ [ppm] = 0.78 (d, 3H), 1.01-1.11 (m, 6H), 2.56-2.69 (m, 2H), 2.69-2.83 (m, 1H), 3.34-3.44 (m, 1H), 3.87-3.99 (m, 2H), 4.11 (d, 1H), 6.88-7.00 (m, 1H), 7.12 (dd, 1H), 7.39-7.48 (m, 4H), 7.55-7.66 (m, 1H), 10.03 (s, 1H). |
| **109A** | (+)-3-(4-Chlor-3-{[(2*S*,3*R*)-4,4,4-trifluor-3-methyl-2-(4-vinylphenyl)butanoyl]amino}-phenyl)propansäure-*tert*.-butylester | LC-MS (Methode 6): Rₜ = 1.43 min; m/z = 496 (M+H)⁺. |
| | | |
| | 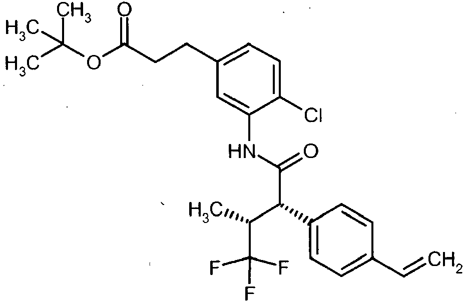 | ¹H-NMR (400 MHz, DMSO-*d*₆): δ [ppm] = 0.80 (d, 3H), 1.31 (s, 9H), 2.45 (t, 2H), 2.75 (t, 2H), 3.34-3.43 (m, 1H), 4.09 (d, 1H), 5.27 (d, 1H), 5.84 (d, 1H), 6.72 (dd, 1H), 7.03 (dd, 1H), 7.29-7.53 (m, 6H), 9.78 (s, 1H). |
| | | |
| | | [α]_{D}²⁰ = +105.2°, c = 0.315, Chloroform. |
| **110A** | (+)-3-(3-{[(2*S*,3*R*)-2-(4-Ethylphenyl)-4,4,4-trifluor-3-methylbutanoyl]amino}-4-fluorphenyl)propansäure-*tert*.-butylester | LC-MS (Methode 6): Rₜ = 1.46 min; m/z = 480 (M-H)⁻. |
| | | |
| | 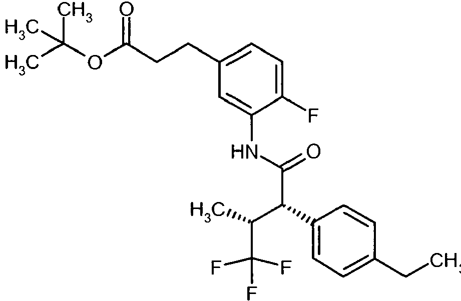 | ¹H-NMR (400 MHz, DMSO-*d*₆): δ [ppm] = 0.77 (d, 3H), 1.17 (t, 3H), 1.31 (s, 9H), 2.44 (t, 2H), 2.60 (q, 2H), 2.74 (t, 2H), 3.29-3.34 (m, 1H), 4.05 (d, 1H), 6.86-7.00 (m, 1H), 7.11 (dd, 1H), 7.16-7.26 (m, 2H), 7.28-7.41 (m, 2H), 7.69 (dd, 1H), 9.96 (s, 1H). |
| | | |
| | | [α]_{D}²⁰ = +108.7°, c = 0.500, Chloroform. |
| **111A** | (+)-3-(4-Chlor-3-{[(2*S*,3*R*)-2-(4-ethylphenyl)-4,4,4-trifluor-3-methylbutanoyl]amino}phenyl)-propansäure-*test*-butylester | LC-MS (Methode 6): Rₜ = 1.51 min; m/z = 496 (M-H)⁻. |
| | | |
| | 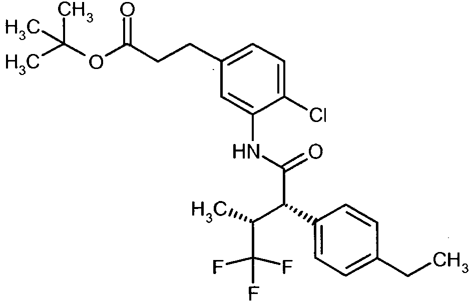 | ¹H-NMR (400 MHz, DMSO-*d*₆): δ [ppm] = 0.78 (d, 3H), 1.17 (t, 3H), 1.31 (s, 9H), 2.45 (t, 2H), 2.59 (q, 2H), 2.75 (t, 2H), 3.34-3.40 (m, 1H), 4.06 (d, 1H), 7.02 (dd, 1H), 7.20 (d, 2H), 7.34 (dd, 3H), 7.42 (d, 1H), 9.73 (s, 1H). |
| | | |
| | | [α]_{D}²⁰ = +62.7°, c = 0.475, Chloroform. |
| **112A** | 3-(4-Chlor-3-{[(2*S*,3*R*)-2-(4-ethylphenyl)-4,4,4-trifluor-3-methylbutanoyl]amino}phenyl)-2-methylpropansäureethylester (*Diastereomerengemish*) | LC-MS (Methode 4): Rₜ = 1.61 min; m/z = 484 (M+H)⁺. |
| | | |
| | 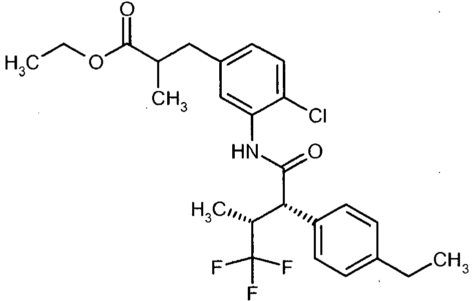 | ¹H-NMR (400 MHz, DMSO-*d*₆): δ [ppm] = 0.78 (d, 3H), 1.03-1.07 (m, 5H), 1.17 (t, 3H), 2.55-2.69 (m, 4H), 2.74-2.83 (m, 1H), 3.27-3.40 (m, 2H), 3.96 (qd, 2H), 4.03-4.12 (m, 1H), 6.97 (dd, 1H), 7.20 (d, 2H), 7.33-7.41 (d, 4H), 9.73 (s, 1H). |
| **113A** | 3-(3-{[(2*S*,3*R*)-2-(4-Ethylphenyl)-4,4,4-trifluor-3-methylbutanoyl]amino}-4-fluorphenyl)-2-methyl-propansäureethylester (*Diastereomerengemisch*) | LC-MS (Methode 4): Rₜ = 1.57 min; m/z = 468 (M+H)⁺. |
| | | |
| | 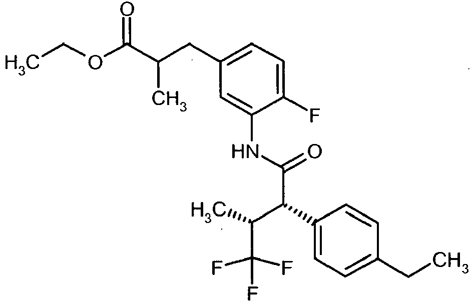 | ¹H-NMR (400 MHz, DMSO-*d*₆): δ [ppm] = 0.77 (d, 3H), 0.99-1.11 (m, 6H), 1.11-1.23 (m, 3H), 2.52-2.68 (m, ca. 5H), 2.70-2.85 (m, 1H), 3.28-3.32 (m, ca. 1H), 3.90-4.00 (m, 2H), 4.00-4.08 (m, 1H), 6.82-6.96 (m, 1H), 7.11 (dd, 1H), 7.16-7.25 (m, 2H), 7.27-7.41 (m, 2H), 7.65 (dd, 1H), 9.96 (s, 1H). |
| **114A** | 3-(4-Chlor-3-{[(2*S*,3*R*)-2-(4-chlorphenyl)-4,4,4-trifluor-3-methylbutanoyl]amino}phenyl)-2-methylpropansäureethylester (*Diastereomerengemisch*) | LC-MS (Methode 5): Rₜ = 2.95 min; m/z = 490/492 (M+H)⁺. |
| | | |
| | 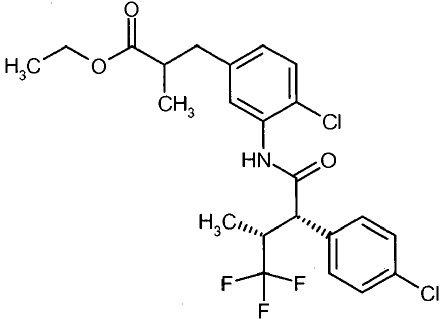 | ¹H-NMR (400 MHz, DMSO-*d*₆): δ [ppm] = 0.80 (d, 3H), 1.00-1.12 (m, 6H), 2.59-2.72 (m, 2H), 2.74-2.86 (m, 1H), 3.34-3.42 (m, 1H), 3.96 (qd, 2H), 4.12 (d, 1H), 6.99 (dd, 1H), 7.26-7.39 (m, 2H), 7.39-7.54 (m, 4H), 9.81 (s, 1H). |
| **115A** | 3-(4-Chlor-3-{[(2*S*,3*R*)-2-(4-ethylphenyl)-4,4,4-trifluor-3-methylbutanoyl]amino}phenyl)-butansäure-*tert*.-butylester (*Diastereomerengemisch*) | LC-MS (Methode 6): Rₜ = 1.55 min; m/z = 510 (M-H)⁻. |
| | | |
| | | ¹H-NMR (400 MHz, DMSO-*d*₆): δ [ppm] = 0.79 (d, 3H), 1.10-1.19 (m, 6H), 1.24/1.26 (2s, zus. 9H), 2.32-2.46 (m, 2H), 2.59 (q, 2H), 2.97-3.11 (m, 1H), 3.33-3.40 (m, 1H), 4.02-4.14 (m, 1H), 7.06 (d, 1H), 7.20 (d, 2H), 7.35 (d, 3H), 7.48 (dd, 1H), 9.72 (s, 1H). |
| | 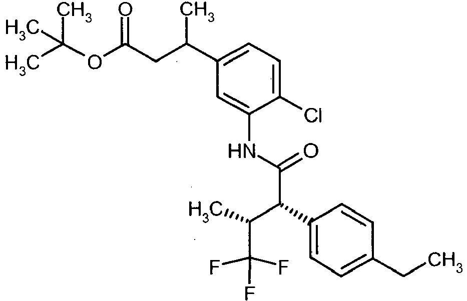 | |
| **116A** | (+)-3-[4-Chlor-3-({(2*S*,3*R*)-4,4,4-trifluor-2-[4-(1-fluorvinyl)phenyl]-3-methylbutanoyl}amino)-phenyl]propansäuremethylester | LC-MS (Methode 6): Rₜ = 1.30 min; m/z = 472/474 (M+H)⁺. |
| | | |
| | 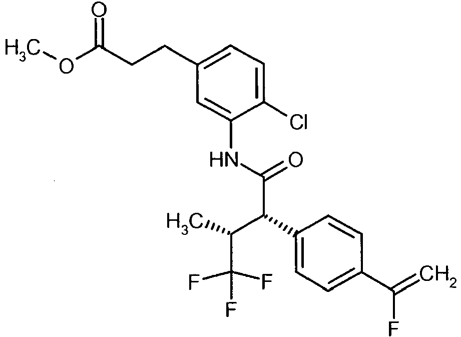 | ¹H-NMR (400 MHz, DMSO-*d*₆): δ [ppm] = 0.80 (d, 3H), 2.54-2.60 (m, 2H), 2.73-2.88 (m, 2H), 3.35-3.45 (m, 1H), 4.15 (d, 1H), 4.96 (dd, 1H), 5.40 (dd, 1H), 7.04 (dd, 1H), 7.28-7.40 (m, 2H), 7.45-7.55 (m, 2H), 7.59-7.71 (m, 2H), 9.84 (s, 1H). |
| | | |
| | | [α]_{D}²⁰ = +66.3°, c = 0.455, Chloroform. |
| **117A** | 3-(3-{[(2*S*,3*R*)-2-(4-Chlorphenyl)-4,4,4-trifluor-3-methylbutanoyl]amino}-4-chlorphenyl)butan-säure-*tert*.-butylester (*Diastereomerengemisch*) | LC-MS (Methode 4): Rₜ = 1.66 min; m/z = 516/517 (M+H)⁺. |
| | | |
| | 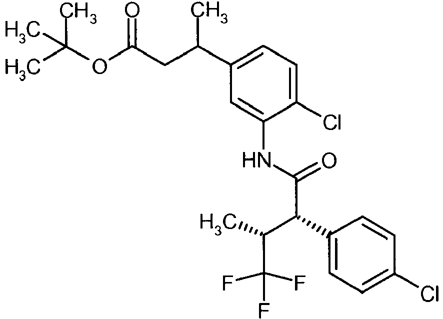 | ¹H-NMR (400 MHz, DMSO-*d*₆): δ [ppm] = 0.80 (d, 3H), 1.16 (d, 3H), 1.24/1.26 (2s, zus. 9H), 2.34-2.47 (m, 2H), 3.01-3.14 (m, 1H), 3.33-3.42 (m, 1H), 4.10-4.18 (m, 1H), 7.08 (d, 1H), 7.36 (d, 1H), 7.40-7.51 (m, 5H), 9.80 (s, 1H). |
| **118A** | (3*S*)-3-(4-Chlor-3-{[(4-chlorphenyl)(2,2-difluor-cyclopentyl)acetyl]amino}phenyl)butansäure-*tert*.-butylester (*Diastereomerengemisch*) | LC-MS (Methode 6): Rₜ = 1.45 min; m/z = 524/526 (M-H)⁻ und Rₜ = 1.46 min; m/z = 524/526 (M-H)⁻. |
| | 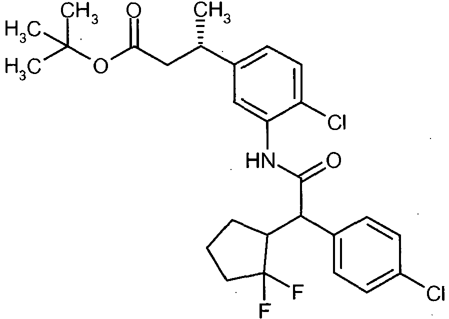 | |
| | | ¹H-NMR (400 MHz, DMSO-*d*₆): δ [ppm] = 1.16 (d, 3H), 1.24/1.26 (2s, zus. 9H), 1.48-1.78 (m, 3H), 1.96-2.25 (m, 3H), 2.33-2.47 (m, 2H), 2.89-3.18 (m, 2H), 4.06 (ddd, 1H), 7.07 (ddd, 1H), 7.30-7.50 (m, 6H), 9.60/9.81 (2s, zus. 1H). |
| **119A** | 3-(3-{[(2*S*,3*R*)-2-(4-Chlorphenyl)-4,4,4-trifluor-3-methylbutanoyl]amino}-4-fluorphenyl)butansäure-*tert.*-butylester (*Diastereomerengemisch*) | LC-MS (Methode 6): Rₜ = 1.45 min; m/z = 500 (M-H)⁻. |
| | | |
| | 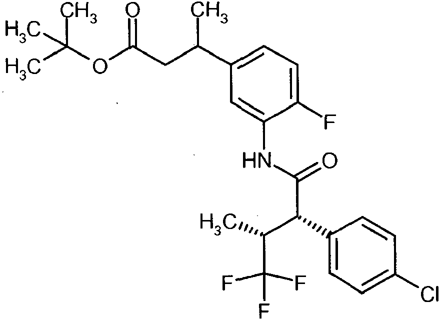 | ¹H-NMR (400 MHz, DMSO-*d*₆): δ [ppm] = 0.79 (d, 3H), 1.16 (d, 3H), 1.23/1.24 (2s, zus. 9H), 1.58-1.72 (m, 1H), 2.30-2.47 (m, 2H), 2.99-3.10 (m, 1H), 3.32-3.43 (m, 1H), 4.12 (d, 1H), 6.97-7.06 (m, 1H), 7.13 (dd, 1H), 7.38-7.54 (m, 4H), 7.62-7.79 (m, 1H), 10.02 (s, 1H). |
| **120A** | 3-(4-Chlor-3-{[(4-chlorphenyl)(2,2-difluor-cyclopentyl)acetyl]amino}phenyl)propansäure-*tert*.-butylester (*Diastereomerengemisch*) | LC-MS (Methode 6): Rₜ = 1.44 min; m/z = 510/512 (M-H)⁻ und Rₜ = 1.45 min; m/z = 510/512 (M-H)⁻. |
| | 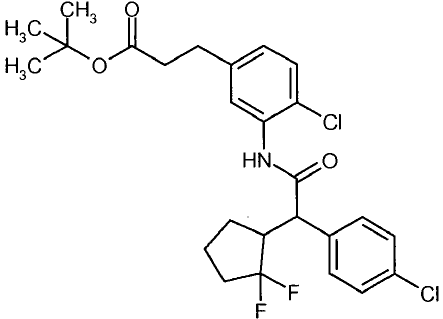 | |
| | | ¹H-NMR (400 MHz, DMSO-*d₆*): δ [ppm] = 1.07-1.25 (m, 1H), 1.31 (s, 9H), 1.46-1.75 (m, 3H), 1.95-2.25 (m, 2H), 2.42-2.47 (m, 2H), 2.70-2.81 (m, 2H), 2.87-3.20 (m, 1H), 4.03/4.06 (2d, zus. 1H), 6.97-7.12 (m, 1H), 7.29-7.54 (m, 6H), 9.63/9.84 (2s, zus. 1H). |
| **121A** | (2*S*)-3-(4-Chlor-3-{[(4-chlorphenyl)(2,2-difluor-cyclopentyl)acetyl]amino}phenyl)-2-methyl-propansäureethylester (*Diastereomerengemisch*) | LC-MS (Methode 6): Rₜ = 1.40 min; m/z = 498/500 (M+H)⁺ und Rₜ = 1.41 min; m/z = 498/500 (M+H)⁺. |
| | 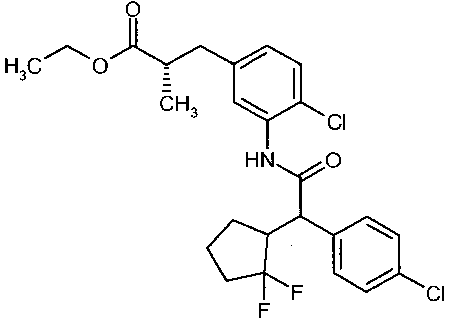 | |
| | | ¹H-NMR (400 MHz, DMSO-*d*₆): δ [ppm] = 0.98-1.08 (m, 6H), 1.10-1.24 (m, 1H), 1.48-1.80 (m, 3H), 1.96-2.26 (m, 2H), 2.57-2.70 (m, 2H), 2.70-2.86 (m, 1H), 2.90-3.22 (m, 1H), 3.90-4.10 (m, 3H), 6.98 (ddd, 1H), 7.30-7.51 (m, 6H), 9.63/9.83 (2s, zus. 1H). |
| **122A** | 2-(4-Chlor-3-{[(2*S*,3*R*)-2-(4-chlorphenyl)-4,4,4-trifluor-3-methylbutanoyl]amino}benzyl)-butansäureethylester (*Diastereomerengemisch*) | LC-MS (Methode 6): Rₜ = 1.45 min; m/z = 504 (M+H)⁺. |
| | | |
| | 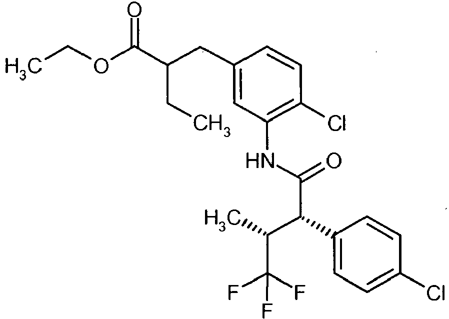 | ¹H-NMR (400 MHz, DMSO-*d*₆): δ [ppm] = 0.77-0.88 (m, ca. 6H), 0.98-1.07 (m, ca. 3H), 1.44-1.56 (m, 2H), 2.42-2.48 (m, 1H), 2.72 (d, 2H), 3.33-3.43 (m, 1H), 3.88-4.01 (m, 2H), 4.12 (d, 1H), 6.98 (dd, 1H), 7.30-7.37 (m, 2H), 7.40-7.51 (m, 4H), 9.81 (s, 1H). |

### Beispiel 123A

### Ethyl-(2R)-3-(4-chlor-3-{[(2S,3R)-2-(4-chlorphenyl)-4,4,4-trifluor-3-methylbutanoyl]amino}-phenyl)-2-methylpropanoat

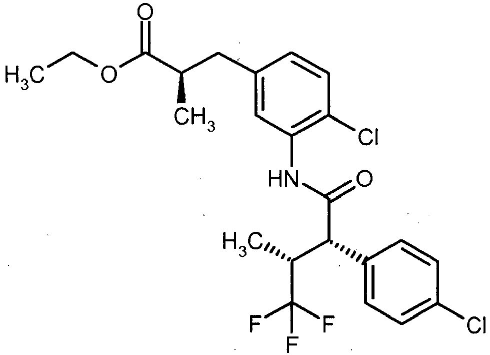

500 mg (2.07 mmol) (-)-(2*R*)-3-(3-Amino-4-chlorphenyl)-2-methylpropansäureethylester und 607 mg (2.28 mmol) (2*S*,3*R*)-2-(4-Chlorphenyl)-4,4,4-trifluor-3-methylbutansäure wurden in einer Mischung aus 2.0 mg DMF und 1.0 ml Pyridin gelöst und bei RT mit 1022 mg (2.69 mmol) HATU versetzt. Die Reaktionsmischung wurde über Nacht bei RT gerührt. Danach wurde mit Ethylacetat verdünnt und die Lösung nacheinander mit 1 N Salzsäure, Wasser, gesättigter Natriumhydrogencarbonat-Lösung und gesättigter Natriumchlorid-Lösung gewaschen. Die organische Phase wurde über Magnesiumsulfat getrocknet und im Vakuum eingeengt. Der Rückstand wurde durch Chromatographie an Silicagel gereinigt (Laufmittel Cyclohexan/Ethylacetat 40:1). Es wurden so 998 mg (98.4% d. Th.) der Zielverbindung erhalten.

LC-MS (Methode 6): Rₜ = 1.41 min; m/z = 490/492 (M+H)⁺.

¹H-NMR (400 MHz, DMSO-d₆): δ [ppm] = 0.80 (d, 3H), 0.99-1.09 (m, 6H), 1.54-1.74 (m, 1H), 2.59-2.73 (m, 2H), 2.74-2.88 (m, 1H), 3.97 (q, 2H), 4.12 (d, 1H), 6.99 (dd, 1H), 7.25-7.37 (m, 2H), 7.40-7.55 (m, 4H), 9.81 (s, 1H).

### Beispiel 124A

### (+)-Ethyl-(2S)-3-(4-chlor-3-{[(2S,3R)-2-(4-chlorphenyl)-4,4,4-trifluor-3-methylbutanoyl]amino}-phenyl)-2-methylpropanoat

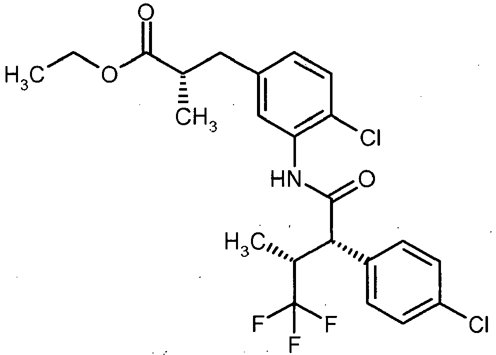

### Methode A:

1.50 g (6.21 mmol) (+)-(2*S*)-3-(3-Amino-4-chlorphenyl)-2-methylpropansäureethylester und 1.82 g (6.83 mmol) (3*R*)-2-(4-Chlorphenyl)-4,4,4-trifluor-3-methylbutansäure (als ca. 9:1-Diastereomeren-gemisch) wurden in einer Mischung aus 6.3 ml DMF und 3.2 ml Pyridin gelöst und bei RT mit 2.83 g (7.45 mmol) HATU versetzt. Die Reaktionsmischung wurde über Nacht bei RT gerührt. Danach wurde mit Methylacetat verdünnt und die Lösung nacheinander mit gesättigter Ammoniumchlorid-Lösung und gesättigter Natriumchlorid-Lösung gewaschen. Die organische Phase wurde über Magnesiumsulfat getrocknet und im Vakuum eingeengt. Der Rückstand wurde durch Chromatographie an Silicagel gereinigt (Laufmittel Cyclohexan/Ethylacetat 50:1 → 40:1). Eine dabei erhaltene Mischfraktion (welche das Neben-Diastereomer enthielt) wurde durch erneute Chromatographie an Silicagel aufgetrennt (Laufmittel Cyclohexan/Ethylacetat 40:1). Insgesamt wurden 2.46 g (80.8% d. Th.) der Zielverbindung erhalten.

### Methode B:

Zu 7.60 g (28.50 mmol) (3*R*)-2-(4-Chlorphenyl)-4,4,4-trifluor-3-methylbutansäure (als ca. 9:1-Diastereomerengemisch) wurden 30 ml Dichlormethan und 1 Tropfen DMF gegeben. Die auf -10°C abgekühlte Lösung wurde tropfenweise mit 4.48 ml (51.3 mmol) Oxalylchlorid versetzt, so dass die Temperatur -5°C nicht überschritt. Die Reaktionsmischung wurde 1 h bei -5°C bis 0°C gerührt und anschließend ca. 30 min ohne Kühlung unter Erwärmung auf RT, bevor im Vakuum eingeengt wurde. Der Rückstand wurde in Dichlormethan aufgenommen und die Lösung erneut im Vakuum eingeengt. Nach nochmaliger Wiederholung dieses Vorgangs wurde das erhaltene Säurechlorid kurz im Hochvakuum getrocknet und ohne weitere Reinigung direkt weiter umgesetzt.

Zu einer Lösung von 6.05 g (25.03 mmol) (+)-(2*S*)-3-(3-Amino-4-chlorphenyl)-2-methylpropansäureethylester in 25 ml abs. THF wurden 6.1 ml (35.04 mmol) *N*,*N*-Diisopropylethylamin gegeben. Die resultierende Lösung wurde auf -10°C gekühlt und tropfenweise mit einer Lösung des oben hergestellten Säurechlorids (7.85 g, 27.5 mmol) in ca. 10 ml abs. THF versetzt, wobei die Temperatur unter 0°C gehalten wurde. Die Reaktionsmischung wurde anschließend 1 h bei -10°C bis 0°C gerührt und dann mit Ethylacetat und drei Tropfen Wasser versetzt. Nach 10 min wurde die mit Ethylacetat weiter verdünnte Mischung nacheinander mit 1 N Salzsäure, gesättigter Natriumhydrogencarbonat-Lösung und gesättigter Natriumchlorid-Lösung gewaschen, über Magnesiumsulfat getrocknet und im Vakuum eingeengt. Das Rohprokt wurde 4 h lang in 50 ml Diisopropylether verrührt. Nach Filtration wurde der erhaltene Feststoff nochmals mit 40 ml Diisopropylether verrührt. Der erhaltene Feststoff wurde im Hochvakuum gründlich getrocknet. Es wurden so 8.32 g der Zielverbindung erhalten. Die oben erhaltenen Filtrate wurden vereinigt und im Vakuum eingeengt. Aus dem Rückstand wurden durch Chromatographie an Silicagel (Laufmittel Cyclo-hexan/Ethylacetat 50:1) weitere 1.75 g Produkt isoliert. Insgesamt wurden auf diese Weise 10.07 g (82.1% d. Th.) der Titelverbindung erhalten.

LC-MS (Methode 7): Rₜ = 2.95 min; m/z = 490 (M+H)⁺.

¹H-NMR (400 MHz, DMSO-d₆): δ [ppm] = 0.80 (d, 3H), 1.00-1.10 (m, 6H), 2.58-2.72 (m, 2H), 2.72-2.83 (m, 1H), 3.34-3.44 (m, 1H), 3.96 (q, 2H), 4.12 (d, 1H), 6.99 (dd, 1H), 7.27-7.38 (m, 2H), 7.42-7.51 (m, 4H), 9.82 (s, 1H).

[α]_{D}²⁰ = +94°, c = 0.58, Chloroform.

### Beispiel 125A

### (+)-(2S)-2-(4-Chlor-3-{[(2S,3R)-2-(4-chlorphenyl)-4,4,4-trifluor-3-methylbutanoyl]amino}benzyl)-butansäureethylester

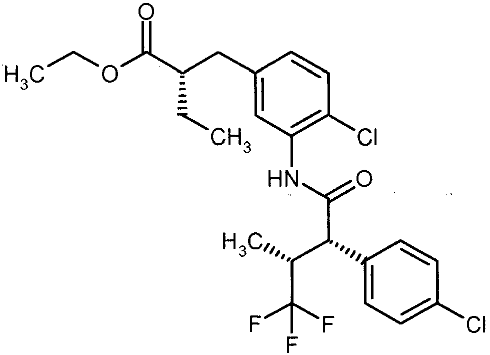

Zu 3.3 g (12.38 mmol) (3*R*)-2-(4-Chlorphenyl)-4,4,4-trifluor-3-methylbutansäure (als ca. 9:1-Diastereomerengemisch) wurden 13 ml Dichlormethan und 1 Tropfen DMF gegeben. Die auf -10°C abgekühlte Lösung wurde tropfenweise mit 1.94 ml (22.28 mmol) Oxalylchlorid versetzt, so dass die Temperatur -5°C nicht überschritt. Die Reaktionsmischung wurde anschließend 1 h bei -5°C bis 0°C gerührt, bevor im Vakuum eingeengt wurde. Der Rückstand wurde in Dichlormethan aufgenommen und die Lösung erneut im Vakuum eingeengt. Nach nochmaliger Wiederholung dieses Vorgangs wurde das erhaltene Säurechlorid kurz im Hochvakuum getrocknet und ohne weitere Reinigung direkt weiter umgesetzt.

Zu einer Lösung von 1.22 g (4.77 mmol) (+)-Ethyl-(2*S*)-2-(3-amino-4-chlorbenzyl)butanoat in 4.8 ml abs. THF wurden 1.2 ml (6.68 mmol) *N,N*-Diisopropylethylamin gegeben. Die resultierende Lösung wurde auf -10°C gekühlt und tropfenweise mit einer Lösung des oben hergestellten Säurechlorids (1.5 g, 5.25 mmol) in 2 ml abs. THF versetzt, wobei die Temperatur unter 0°C gehalten wurde. Die Reaktionsmischung wurde anschließend 1 h bei -10°C bis 0°C gerührt und dann mit Ethylacetat und drei Tropfen Wasser versetzt. Nach 10 min wurde die mit Ethylacetat weiter verdünnte Mischung nacheinander mit 1 N Salzsäure, gesättigter Natriumhydrogencarbonat-Lösung und gesättigter Natriumchlorid-Lösung gewaschen, über Magnesiumsulfat getrocknet und im Vakuum eingeengt. Das gewünschte Produkt wurde durch Chromatographie des Rückstands an Silicagel isoliert (Laufmittel Cyclohexan/Ethylacetat 40:1). Es wurden 2.13 g (88.5% d. Th.) der Titelverbindung erhalten.

LC-MS (Methode 6): Rₜ = 1.46 min; m/z = 504 (M+H)⁺.

¹H-NMR (400 MHz, DMSO-d₆): δ [ppm] = 0.76-0.88 (m, 6H), 1.02 (t, 3H), 1.45-1.56 (m, 2H), 2.47 (d, 1H), 2.72 (d, 2H), 3.34-3.43 (m, 1H), 3.93 (qd, 2H), 4.12 (d, 1H), 6.98 (dd, 1H), 7.29-7.38 (m, 2H), 7.40-7.52 (m, 4H), 9.81 (s, 1H).

[α]_{D}²⁰ = +62.6°, c = 0.515, Chloroform.

Nach analoger Vorgehensweise wurde die folgende Verbindung hergestellt:

### Beispiel 126A

### (+)-(2R)-2-(4-Chlor-3-{[(2S,3R)-2-(4-chlorphenyl)-4,4,4-trifluor-3-methylbutanoyl]amino}benzyl)-butansäureethylester

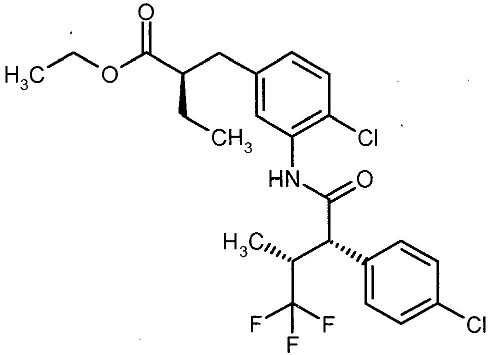

LC-MS (Methode 6): Rₜ = 1.46 min; m/z = 504/506 (M+H)⁺.

¹H-NMR (400 MHz, DMSO-d₆): δ [ppm] = 0.79 (d, 3H), 0.84 (t, 3H), 1.04 (t, 3H), 1.46-1.56 (m, 2H), 2.45-2.49 (m, 1H), 2.70-2.74 (m, 2H), 3.34-3.42 (m, 1H), 3.95 (q, 2H), 4.12 (d, 1H), 6.98 (dd, 1H), 7.30-7.37 (m, 2H), 7.42-7.50 (m, 4H), 9.81 (s, 1H).

[α]_{D}²⁰ = +52.3°, c = 0.485, Chloroform.

### Beispiel 127A

### Ethyl-2-(3-{[(2S,3R)-2-(4-chlorphenyl)-4,4,4-trifluor-3-methylbutanoyl]amino}-4-fluorphenyl)-trans-cyclopropancarboxylat

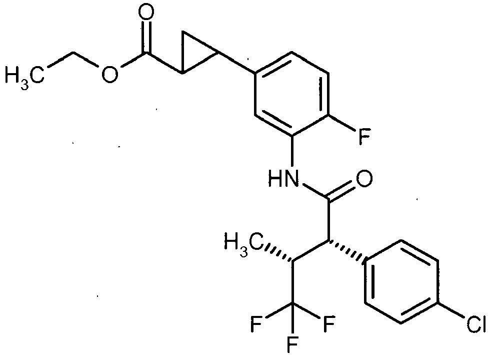

Zu einer Lösung von 90 mg (0.34 mmol) (2*S*,3*R*)-2-(4-Chlorphenyl)-4,4,4-trifluor-3-methylbutan-säure in 1.5 ml eines 4:1-Gemisches von DMF und Pyridin wurden 167 mg (0.44 mmol) HATU gegeben. Nach 30 min Rühren bei RT wurden 83 mg (0.37 mmol) *rac*-Ethyl-2-[3-amino-4-fluorphenyl]-*trans*-cyclopropancarboxylat hinzugefügt. Das Reaktionsgemisch wurde über Nacht bei RT gerührt, dann mit Ethylacetat (ca. 50 ml) verdünnt und mit gesättigter Kochsalz-Lösung gewaschen. Die organische Phase wurde über Magnesiumsulfat getrocknet und eingeengt und der Rückstand durch präparative HPLC gereinigt. Es wurden 123 mg (77% d. Th.) der Titelverbindung erhalten.

LC-MS (Methode 6): Rₜ = 1.35 min; m/z = 472 (M+H)⁺.

¹H-NMR (400 MHz, DMSO-d₆): δ [ppm] = 0.79 (d, 3H), 1.19 (t, 3H), 1.25-1.34 (m, 1H), 1.42 (dt, 1H), 1.73-1.91 (m, 1H), 2.31-2.45 (m, 1H), 3.96-4.20 (m, 3H), 6.83-7.00 (m, 1H), 7.13 (dd, 1H), 7.45 (s, 4H), 7.57-7.68 (m, 1H), 10.06 (s, 1H).

### Beispiel 128A

### Ethyl-threo-3-(3-{[(2S,3R)-2-(4-chlorphenyl)-4,4,4-trifluor-3-methylbutanoyl]amino}-4-fluor-phenyl)-2-methylbutanoat

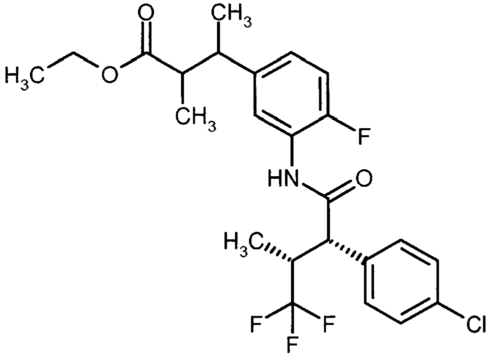

Zu einer Lösung von 35.5 mg (0.13 mmol) (2*S*,3*R*)-2-(4-Chlorphenyl)-4,4,4-trifluor-3-methylbutan-säure in 0.98 ml DMF wurden 15 µl (0.19 mmol) Pyridin und 75.8 mg (0.2 mmol) HATU gegeben. Das Reaktionsgemisch wurde 30 min bei RT gerührt und anschließend mit 35 mg (0.15 mmol) *rac-threo*-Ethyl-3-(3-amino-4-fluorphenyl)-2-methylbutanoat versetzt. Nach Rühren über Nacht wurde das Reaktionsgemisch direkt mittels präparativer HPLC aufgereinigt. Es wurden 34 mg (52% d. Th.) der Titelverbindung erhalten.

LC-MS (Methode 5): Rₜ = 2.89 min; m/z = 488 (M+H)⁺.

### Beispiel 129A

### Ethyl-erythro-3-(3-{[(2S,3R)-2-(4-chlorphenyl)-4,4,4-trifluor-3-methylbutanoyl]amino}-4-fluorphenyl)-2-methylbutanoat

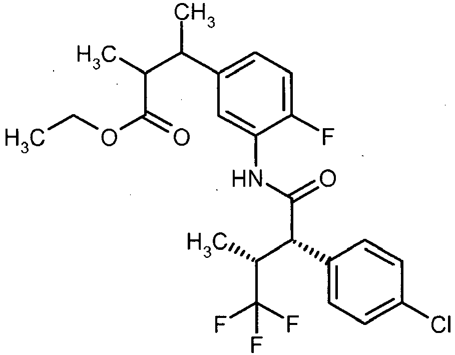

Zu einer Lösung von 22.3 mg (84 µmol) (2*S*,3*R*)-2-(4-Chlorphenyl)-4,4,4-trifluor-3-methylbutan-säure in 0.62 ml DMF wurden 9.5 µl (117 µmol) Pyridin und 47.7 mg (125 µmol) HATU gegeben. Das Reaktionsgemisch wurde 30 min bei RT gerührt und anschließend mit 22 mg (92 µmol) *rac-erythro*-Ethyl-3-(3-amino-4-fluorphenyl)-2-methylbutanoat versetzt. Nach Rühren über Nacht wurde das Reaktionsgemisch direkt mittels präparativer HPLC aufgereinigt. Es wurden 10.7 mg (24% d. Th.) der Titelverbindung erhalten.

LC-MS (Methode 4): Rₜ = 1.57 min; m/z = 488 (M+H)⁺.

### Beispiel 130A

### tert.-Butyl-3-(3-{[(2S,3R)-2-(4-chlorphenyl)-4,4,4-trifluor-3-methylbutanoyl]amino}-4-cyano-phenyl)propanoat

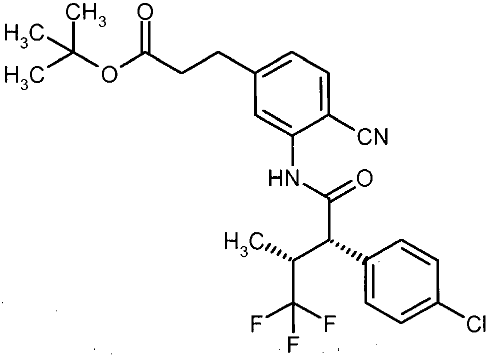

Zu einer Lösung von 41.3 mg (0.16 mmol) (2*S*,3*R*)-2-(4-Chlorphenyl)-4,4,4-trifluor-3-methylbutan-säure in 1.16 ml Dichlormethan wurden bei 0°C 155 µl (0.31 mmol) einer 2 M Lösung von Oxalylchlorid in Dichlormethan und ein Tropfen DMF gegeben. Nach 1 h Rühren bei 0°C wurde der Ansatz eingeengt, der verbliebene Rückstand in 1 ml THF gelöst, mit 32 µl (0.19 mmol) *N,N-*Diisopropylethylamin versetzt, auf 0°C gekühlt und mit einer Lösung von 42 mg (0.17 mmol) *tert.-*Butyl-3-(3-amino-4-cyanophenyl)propanoat in 2 ml THF versetzt. Das Reaktionsgemisch wurde über Nacht bei RT gerührt. Anschließend wurde das Gemisch auf 10 ml Wasser gegossen und mit Ethylacetat extrahiert. Die vereinigten organischen Phasen wurden über Magnesiumsulfat getrocknet und eingeengt. Der Rückstand wurde mittels präparativer HPLC gereinigt. Es wurden 16.5 mg (22% d. Th.) der Titelverbindung erhalten.

LC-MS (Methode 7): Rₜ = 2.86 min; m/z = 439 (M-^{t}Bu)⁺.

¹H-NMR (400 MHz, DMSO-d₆): δ [ppm] = 0.81 (d, 3H), 1.27-1.35 (m, 9H), 2.83 (t, 2H), 4.01 (d, 1H), 7.21 (dd, 1H), 7.30 (s, 1H), 7.40-7.52 (m, 4H), 7.69 (d, 1H), 10.47 (s, 1H).

### Beispiel 131A

### Methyl-(2E)-3-(2-methyl-3-nitrophenyl)acrylat

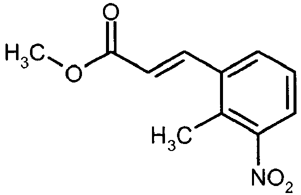

Unter Argon wurden 119.5 g (1.39 mol) Acrylsäuremethylester zu einer Mischung aus 100 g (0.463 mol) 2-Brom-6-nitrotoluol, 323 ml (2.31 mol) Triethylamin, 28.2 g (92.6 mmol) Tri-2-tolylphosphin und 10.4 g (46.3 mmol) Palladium(II)acetat in 2.0 Liter DMF getropft und die Mischung anschließend 36 h bei 125°C gerührt. Nach Abkühlen auf Raumtemperatur wurde das Reaktionsgemisch mit 4 Liter gesättigter wässriger Ammoniumchlorid-Lösung verrührt und dreimal mit insgesamt 5 Liter Diethylether extrahiert. Die vereinigten organischen Phasen wurden mit Wasser und gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 6:1). Das Produkt wurde mit Heptan verrührt, und der angefallene Feststoff wurde abgesaugt und im Hochvakuum getrocknet. Erhalten wurden 48.7 g der Zielverbindung (46.6% d. Th.).

MS: m/z = 162 (M-C₂H₃O₂)⁺.

¹H-NMR (400 MHz, DMSO-*d*₆): δ [ppm] = 2.41 (s, 3H), 3.76 (s, 3H), 6.63 (d, 1H), 7.48 (t, 1H), 7.84-7.95 (m, 2H), 8.00 (d, 1H).

### Beispiel 132A

### Methyl-3-(3-amino-2-methylphenyl)propanoat

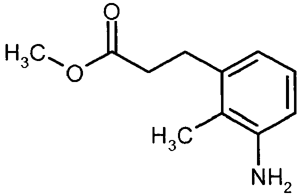

48.7 g (220.1 mmol) Methyl-(2*E*)-3-(2-methyl-3-nitrophenyl)acrylat wurden in 2.2 Liter Methanol gelöst, und die Lösung wurde in einer Durchfluss-Hydrierapparatur ("H-Cube midi" der Firma Thales Nano, Budapest) bei einem Fluss von 6-10 ml/min und bei einer Reaktionstemperatur von 35-40°C sowie bei maximalem Wasserstoffdruck hydriert. Nach beendeter Umsetzung wurde die produkthaltige Lösung im Vakuum eingeengt. Erhalten wurden 40.0 g des Zielprodukts als Feststoff (92.1% d. Th.).

MS: m/z = 194 (M+H)⁺.

¹H-NMR (400 MHz, DMSO-*d*₆): δ [ppm] = 1.97 (s, 3H), 2.45 (t, 2H), 2.78 (t, 2H), 3.58 (s, 3H), 4.75 (s, 2H), 6.37 (d, 1H), 6.50 (d, 1H), 6.79 (t, 1H).

### Beispiel 133A

### Methyl-3-(3-amino-4-chlor-2-methylphenyl)propanoat

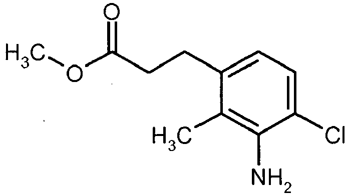

Zu einer Lösung von 2.0 g (10.3 mmol) Methyl-3-(3-amino-2-methylphenyl)propanoat in 10 ml Acetonitril wurden bei RT 1.38 g (10.3 mmol) *N*-Chlorsuccinimid gegeben. Die Reaktionsmischung wurde 30 min gerührt und dann mit Ethylacetat verdünnt. Das Gemisch wurde nacheinander mit ges. Natriumhydrogencarbonat-Lösung und ges. Natriumchlorid-Lösung gewaschen, über Magnesiumsulfat getrocknet und im Vakuum eingeengt. Aus dem Rohprodukt wurden nach Chromatographie an Silicagel (Laufmittel Cyclohexan/Ethylacetat 10:1) 279 mg des Zielprodukts isoliert (11.8% d. Th.).

LC-MS (Methode 4): Rₜ = 1.11 min; m/z = 228 (M+H)⁺.

¹H-NMR (500 MHz, DMSO-*d*₆): δ [ppm] = 2.06 (s, 3H), 2.46 (t, 2H), 2.78 (t, 2H), 3.58 (s, 3H), 4.94 (s, 2H), 6.42 (d, 1H), 6.98 (d, 1H).

### Beispiele 134A

### 3-Brom-6-chlor-2-methylanilin

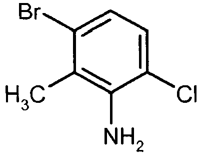

Zu einer Lösung von 12.0 g (64.5 mmol) 3-Brom-2-methylanilin in 150 ml Acetonitril wurden bei RT 8.61 g (64.5 mmol) *N*-Chlorsuccinimid gegeben. Die Reaktionsmischung wurde 7 h bei 60°C gerührt und nach Abkühlen im Vakuum eingeengt. Der Rückstand wurde in Dichlormethan aufgenommen, und das Gemisch wurde nacheinander mit ges. Natriumhydrogencarbonat-Lösung und ges. Natriumchlorid-Lösung gewaschen, über Natriumsulfat getrocknet und im Vakuum eingeengt. Aus dem Rohprodukt wurden nach Chromatographie an Silicagel (Laufmittel Cyclohexan/Ethylacetat 40:1 bis 20:1) 3.78 g des Zielprodukts isoliert (26.6% d. Th.).

GC-MS (Methode 1): Rₜ = 5.07 min; m/z = 218/220 (M+H)⁺.

¹H-NMR (400 MHz, DMSO-*d*₆): δ [ppm] = 2.24 (s, 3H), 5.39 (s, 2H), 6.80 (d, 1H), 7.03 (d, 1H).

### Beispiel 135A

### tert.-Butyl-(2E)-3-(3-amino-4-chlor-2-methylphenyl)-2-methylacrylat und tert.-Butyl-2-(3-amino-4-chlor-2-methylbenzyl)acrylat

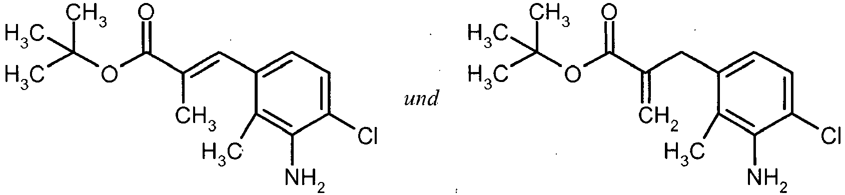

1.50 g (6.80 mmol) 3-Brom-6-chlor-2-methylanilin, 2.90 g (20.4 mmol) *tert*.-Butylmethacrylat und 4.74 ml (34.0 mmol) Triethylamin wurden in 10.0 ml DMF gelöst. Die Reaktionslösung wurde dreimal evakuiert und jeweils mit Argon wieder belüftet. Nach Zugabe von 152.7 mg (0.68 mmol) Palladium(II)acetat und 414.1 mg (1.36 mmol) Tri-2-tolylphosphin wurde erneut zweimal evakuiert und jeweils mit Argon belüftet. Die Reaktionsmischung wurde dann 2 h bei 150°C gerührt. Nach Abkühlen wurde die Mischung über Celite abfiltriert und der Rückstand mit DMF nachgewaschen. Das Filtrat wurde im Vakuum eingeengt und der Rückstand durch Chromatographie an Silicagel gereinigt (Laufmittel Cyclohexan/Ethylacetat 100: 1). Es wurden 1.59 g eines Gemisches der beiden Titelverbindungen erhalten (Verhältnis ca. 2:1, 83% d. Th.).

LC-MS (Methode 4): Rₜ = 1.45 min; m/z = 226 (M-C₄H₈)⁺ und Rₜ = 1.49 min; m/z = 282 (M+H)⁺.

¹H-NMR (400 MHz, DMSO-*d*₆): *tert*.-Butyl-(2*E*)-3-(3-amino-4-chlor-2-methylphenyl)-2-methylacrylat: δ [ppm] = 1.49 (s, 9H), 1.75 (d, 3H), 2.02 (s, 3H), 5.12 (s, 2H), 6.44 (d, 1H), 7.11 (d, 1H), 7.51 (s, 1H).

¹H-NMR (400 MHz, DMSO-*d*₆): *tert*.-Butyl-2-(3-amino-4-chlor-2-methylbenzyl)acrylat: δ [ppm] = 1.42 (s, 9H), 1.98 (s, 3H), 3.45 (s, 2H), 4.97 (s, 2H), 5.15 (d, 1H), 6.01 (d, 1H), 6.38 (d, 1H), 7.02 (d, 1H).

### Beispiel 136A

### (+/-)-tert.-Butyl-3-(3-amino-4-chlor-2-methylphenyl)-2-methylpropanoat

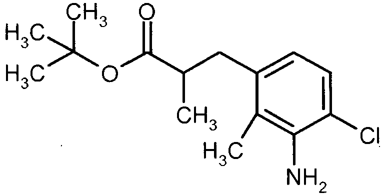

Zu 354 mg (14.6 mmol) Magnesiumspänen und einigen Körnchen Iod wurde eine Lösung von 1.58 g (5.61 mmol) des Gemisches von *tert*.-Butyl-(2*E*)-3-(3-amino-4-chlor-2-methylphenyl)-2-methylacrylat und *tert*.-Butyl-2-(3-amino-4-chlor-2-methylbenzyl)acrylat (Beispiel 135A) in 5.0 ml Methanol gegeben. Die Mischung wurde bei RT (zunächst unter Kühlung) über Nacht gerührt. Anschließend wurden unter Eiskühlung 50 ml 1 N Salzsäure zugesetzt. Durch Zugabe von 10%-iger Natronlauge wurde der pH-Wert der Mischung dann auf ca. 10 eingestellt. Die Mischung wurde dreimal mit Ethylacetat extrahiert. Die vereinigten organischen Phasen wurden über Magnesiumsulfat getrocknet und im Vakuum eingeengt. Aus dem Rohprodukt wurden nach Chromatographie an Silicagel (Laufmittel CyclohexanEthylacetat 50:1 bis 40:1) 962 mg des Zielprodukts isoliert (60.5% d. Th.).

LC-MS (Methode 9): Rₜ = 2.30 min; m/z = 284 (M+H)⁺.

¹H-NMR (400 MHz, DMSO-*d*₆): δ [ppm] = 1.02 (d, 3H), 1.32 (s, 9H), 2.06 (s, 3H), 2.46 (dd, 1H), 2.80 (dd, 1H), 4.94 (br. s, 2H), 6.38 (d, 1H), 6.97 (d, 1H).

Das oben erhaltene Racemat wurde durch präparative HPLC an chiraler Phase in die Enantiomere getrennt [Säule: Daicel Chiralpak OJ-H, 5 µm, 250 mm x 20 mm; Fluss: 20 ml/min; Detektion: 220 nm; Injektionsvolumen: 0.28 ml; Temperatur: 224°C; Eluent: 93% Isohexan / 7% Isopropanol]. Ausgehend von 962 mg Racemat wurden 434 mg Enantiomer 1 (*Beispiel 137A)* und 325 mg Enantiomer *2* (*Beispiel 138A*) erhalten:

### Beispiel 137A

### (-)-tert.-Butyl-(2R)-3-(3-amino-4-chlor-2-methylphenyl)-2-methylpropanoat

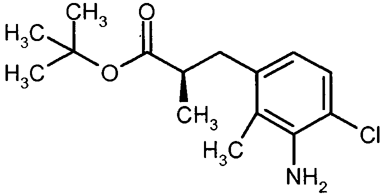

Ausbeute: 434 mg

LC-MS (Methode 4): Rₜ = 1.44 min; m/z = 284 (M+H)⁺.

¹H-NMR (400 MHz, DMSO-*d*₆): δ [ppm] = 1.03 (d, 3H), 1.32 (s, 9H), 2.06 (s, 3H), 2.46 (dd, 1H), 2.80 (dd, 1H), 4.93 (s, 2H), 6.38 (d, 1H), 6.97 (d, 1H).

[α]_{D}²⁰ = -37.3°, c = 0.455, Chloroform.

### Beispiel 138A

### (+)-tert.-Butyl-(2S)-3-(3-amino-4-chlor-2-methylphenyl)-2-methylpropanoat

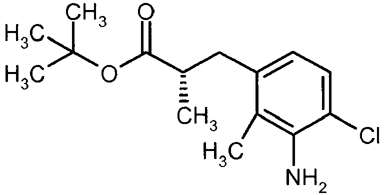

Ausbeute: 325 mg

LC-MS (Methode 4): Rₜ = 1.44 min; m/z = 284 (M+H)⁺.

¹H-NMR (400 MHz, DMSO-*d*₆): δ [ppm] = 1.03 (d, 3H), 1.32 (s, 9H), 2.06 (s, 3H), 2.46 (dd, 1H), 2.80 (dd, 1H), 4.93 (s, 2H), 6.38 (d, 1H), 6.97 (d, 1H).

[α]_{D}²⁰= +35.0°, c = 0.455, Chloroform.

### Beispiel 139A

### Ethyl-4,4,4-trifluor-3-(4-fluor-3-nitrophenyl)but-2-enoat

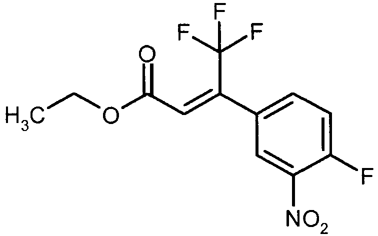

Zu einer eisgekühlten Suspension von 1.86 g (60% in Mineralöl, 46.4 mmol) Natriumhydrid in einer Mischung aus 70 ml THF und 20 ml DMF wurden langsam 10.9 g (48.5 mmol) Diethylphosphonoessigsäureethylester getropft. Nach Ende der Zugabe wurde die Mischung noch 30 min bei 0°C gerührt, bevor 10.0 g (42.2 mmol) 2,2,2-Trifluor-1-(4-fluor-3-nitrophenyl)ethanon zugegeben wurden. Die Reaktionsmischung wurde über Nacht bei RT gerührt und dann auf Wasser gegeben. Es wurde dreimal mit Ethylacetat extrahiert, und die vereinigten organischen Phasen wurden im Vakuum eingeengt. Aus dem Rohprodukt wurden nach Chromatographie an Silicagel (Laufmittel Cyclohexan/Ethylacetat 20:1 bis 10:1) 9.23 g des Zielprodukts isoliert (71.2% d. Th.).

GC-MS (Methode 1): Rₜ = 4.51 min; m/z = 262 (M-C₂H₅O)⁺.

¹H-NMR (400 MHz, DMSO-*d*₆): δ [ppm] = 1.04 (t, 3H), 4.03 (q, 2H), 6.96 (d, 1H), 7.67-7.76 (m, 1H), 7.78-7.86 (m, 1H), 8.16 (dd, 1H).

### Beispiel 140A

### (+/-)-Ethyl-3-(3-amino-4-fluorphenyl)-4,4,4-trifluorbutanoat

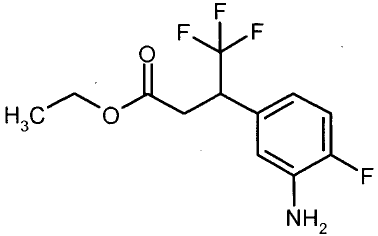

5.0 g (16.3 mmol) Ethyl-4,4,4-trifluor-3-(4-fluor-3-nitrophenyl)but-2-enoat wurden in 133 ml Ethanol gelöst und unter Argon mit 866 mg Palladium auf Kohle (10%) versetzt. Die Reaktionsmischung wurde über Nacht bei RT unter einer Wasserstoffatmosphäre (Normaldruck) kräftig gerührt. Danach wurde über Celite abfiltriert und der Rückstand mit Ethylacetat nachgewaschen. Das Filtrat wurde im Vakuum eingeengt und der erhaltene Rückstand durch Chromatographie an Silicagel gereinigt (Laufmittel Cyclohexan/Ethylacetat 50:1). Erhalten wurden 3.91 g des Zielprodukts (85.9% d. Th.).

LC-MS (Methode 6): Rₜ = 0.97 min; m/z = 280 (M+H)⁺.

¹H-NMR (400 MHz, DMSO-*d*₆): δ [ppm] = 1.08 (t, 3H), 2.85 (dd, 1H), 2.99 (dd, 1H), 3.81-3.92 (m, 1H), 3.94-4.07 (m, 2H), 5.21 (s, 2H), 6.40-6.58 (m, 1H), 6.77 (dd, 1H), 6.96 (dd, 1H).

### Beispiel 141A

### tert.-Butyl-2-methylbutanoat

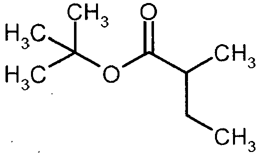

15.0 g (124.4 mmol) 2-Methylbuttersäurechlorid wurden in 150 ml abs. THF gelöst, auf 0°C abgekühlt und tropfenweise mit 114 ml (114 mmol) einer 1 M Lösung von Kalium-*tert*.-butylat in THF versetzt. Nach Ende der Zugabe wurde 1 h bei 0°C, dann 5 h bei RT gerührt, bevor im Vakuum ca. die Hälfte des Lösungsmittels entfernt wurde. Nach Zugabe von Diethylether wurde unter kräftigem Rühren ges. Natriumhydrogencarbonat-Lösung tropfenweise zugesetzt. Nach Phasentrennung wurde die wässrige Phase mit Diethylether extrahiert, und die vereinigten organischen Phasen wurden mit ges. Natriumcarbonat-Lösung gewaschen, über Natriumsulfat getrocknet und im Vakuum eingeengt. Das Rohprodukt wurde durch Vakuumdestillation (19 nun Hg, 40-45°C) gereinigt. Erhalten wurden insgesamt 6.35 g des Zielprodukts (32.3% d. Th.).

GC-MS (Methode 1): Rₜ = 1.53 min; m/z = 85.

¹H-NMR (400 MHz, DMSO-*d*₆): δ [ppm] = 0.84 (m, 3H), 1.01 (d, 3H), 1.33-1.41 (m, 1H), 1.39 (s, 9H), 1.48-1.55 (m, 1H), 2.13-2.26 (m, 1H).

### Beispiel 142A

### 2-Brom-4-(brommethyl)-1-chlorbenzol

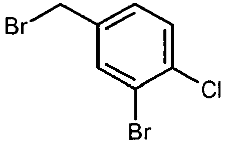

### Stufe 1:

199.0 g (0.845 mol) 3-Brom-4-chlorbenzoesäure wurden in 2.5 Liter THF gelöst, auf -10°C gekühlt und bei dieser Temperatur mit 1.69 Liter (1.69 mol) einer 1 M Boran-Lösung in THF versetzt. Die Reaktionsmischung wurde über Nacht auf RT erwärmt, bevor mit gesättigter wässriger Ammoniumchlorid-Lösung versetzt wurde. Nach Zugabe von Wasser wurde zweimal mit Ethylacetat extrahiert, die vereinigten organischen Phasen über Magnesiumsulfat getrocknet und im Vakuum eingeengt. Erhalten wurden als Rohprodukt 206 g (3-Brom-4-chlorphenyl)methanol, die ohne weitere Reinigung in der Folgestufe eingesetzt wurden.

### Stufe 2:

260 g (ca. 1.05 mol) rohes (3-Brom-4-chlorphenyl)methanol wurden in 2.86 Liter Dichlormethan gelöst, auf -5°C gekühlt und langsam mit 127.1 g (44.6 ml, 460 mmol) Phosphortribromid versetzt. Nach Ende der Zugabe wurde noch 1 h bei -5°C gerührt, bevor mit Dichlormethan und Wasser verdünnt wurde. Die organische Phase wurde abgetrennt, über Magnesiumsulfat getrocknet und im Vakuum eingeengt. Erhalten wurden als Rohprodukt 280.5 g (ca. 84% d. Th.) 2-Brom-4-(brom-methyl)-1-chlorbenzol.

GC-MS (Methode 1): Rₜ = 5.36 min; m/z = 281/283/285 (M+H)⁺.

¹H-NMR (400 MHz, DMSO-*d*₆): δ [ppm] = 4.71 (s, 2H), 7.49 (dd, 1H), 7.63 (d, 1H), 7.89 (d, 1H).

### Beispiel 143A

### (+/-)-tert.-Butyl-2-(3-brom-4-chlorbenzyl)-2-methylbutanoat

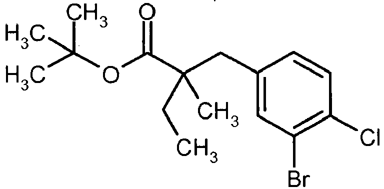

5.8 ml (41.6 mmol) Diisopropylamin wurden unter Argon in 50 ml trockenem THF gelöst und auf -30°C abgekühlt. Es wurden 16.6 ml (41.6 mmol) *n*-Butyllithium-Lösung (2.5 M in Hexan) zugetropft, und das resultierende Gemisch wurde auf 0°C erwärmt, bevor auf -70°C abgekühlt wurde. Es wurde mit einer Lösung von 5.06 g (32.0 mmol) *tert*.-Butyl-2-methylbutanoat in 20 ml THF versetzt, wobei die Reaktionstemperatur unter -60°C gehalten wurde. Nach 4 h Rühren bei -60°C wurde eine Lösung von 10.0 g (35.2 mmol) 2-Brom-4-(brommethyl)-1-chlorbenzol in 30 ml THF zugegeben, wobei die Temperatur wiederum unter -60°C gehalten wurde. Das Reaktionsgemisch wurde dann über Nacht langsam auf RT erwärmt, bevor mit gesättigter wässriger Ammoniumchlorid-Lösung und Ethylacetat versetzt wurde. Nach Phasentrennung wurde die wässrige Phase zweimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 100:1). Es wurden 7.62 g (65.9% d. Th.) der Titelverbindung erhalten.

GC-MS (Methode 1): Rₜ = 6.52 min; m/z = 306 (M-C₄H₇)⁺.

¹H-NMR (400 MHz, DMSO-*d*₆): δ [ppm] = 0.83 (t, 3H), 0.93 (s, 3H), 1.32-1.45 (m, 10H), 1.60-1.73 (m, 1H), 2.62 (d, 1H), 2.91 (d, 1H), 7.18 (dd, 1H), 7.47-7.56 (m, 2H).

### Beispiel 144A

### (+/-)-tert.-Butyl-2-[3-(benzylamino)-4-chlorbenzyl]-2-methylbutanoat

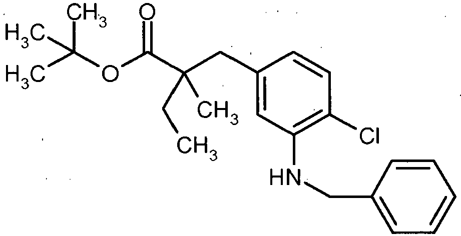

Unter Argon wurden in einem trockenen Kolben 1.59 g (16.6 mmol) Natrium-*tert*.-butylat eingewogen und mit 34.6 ml abs. Toluol versetzt. Nacheinander wurden 5.0 g (13.8 mmol) (+/-)-*tert*.-Butyl-2-(3-brom-4-chlorbenzyl)-2-methylbutanoat, 1.8 ml (16.6 mmol) Benzylamin, 633 mg (0.69 mmol) Tris(dibenzylidenaceton)dipalladium und 344 mg (0.55 mmol) (+/-)-2,2'-Bis(diphenylphos-phino)-1,1'-binaphthyl hinzugefügt. Die Reaktionsmischung wurde dann für 2.0 h bei 110°C gerührt. Nach Abkühlen wurde das Reaktionsgemisch mit gesättigter wässriger Ammoniumchlorid-Lösung und Ethylacetat versetzt und über Kieselgur abgesaugt. Nach Phasentrennung wurde die organische Phase mit ges. Ammoniumchlorid-Lösung und ges. Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 80:1). Es wurden 4.44 g der Titelverbindung in noch etwas verunreinigter Form erhalten (ca. 83% d. Th.).

LC-MS (Methode 6): Rₜ = 1.57 min; m/z = 388 (M+H)⁺.

¹H-NMR (400 MHz, DMSO-*d*₆): δ [ppm] = 0.70 (t, 3H), 1.13-1.22 (m, 1H), 1.35 (s, 9H), 1.39 (s, 3H), 1.39-1.50 (m, 1H), 2.42 (d, 1H), 2.66 (d, 1H), 4.26-4.46 (m, 2H), 6.00 (t, 1H), 6.26-6.35 (m, 1H), 7.11 (d, 1H), 7.16-7.23 (m, 1H), 7.28-7.34 (m, 4H), 7.45-7.55 (m, 1H).

### Beispiel 145A

### (+/-)-tert.-Butyl-2-(3-amino-4-chlorbenzyl)-2-methylbutanoat

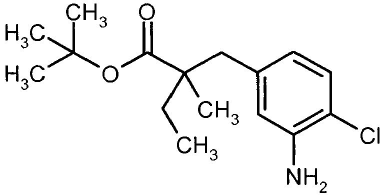

2.20 g (ca. 5.67 mmol) (+/-)-*tert*.-Butyl-2-[3-(benzylamino)-4-chlorbenzyl]-2-methylbutanoat wurden in 130 ml Ethylacetat gelöst und mit 100 mg Palladium auf Kohle (10%) versetzt. Die Reaktionsmischung wurde bei RT über Nacht unter einer Wasserstoffatmosphäre bei Normaldruck gerührt. Das Reaktionsgemisch wurde dann über Kieselgur abgesaugt, der Rückstand gründlich mit Ethylacetat gewaschen und das vereinigte Filtrat eingeengt. Das Rohprodukt wurde durch Chromatographie an Silicagel gereinigt (Laufmittel Cyclohexan/Ethylacetat 50:1 bis 30:1). Es wurden 924 mg (54.7% d. Th.) der Zielverbindung erhalten.

LC-MS (Methode 6): Rₜ = 1.34 min; m/z = 298 (M+H)⁺.

Das oben erhaltene Racemat wurde durch präparative HPLC an chiraler Phase in die Enantiomere getrennt [Säule: Daicel Chiralpak OJ-H, 5 µm, 250 mm x 20 mm; Fluss: 20 ml/min; Detektion: 220 mm; Injektionsvolumen: 0.30 ml; Temperatur: 35°C; Eluent: 70% Isohexan / 30% Ethanol]. Ausgehend von 924 mg Racemat wurden 405 mg Enantiomer 1 *(Beispiel 146A)* und 403 mg Enantiomer 2 (*Beispiel 147A)* erhalten:

### Beispiel 146A

### (-)-tert.-Butyl-2-(3-amino-4-chlorbenzyl)-2-methylbutanoat (Enantiomer 1)

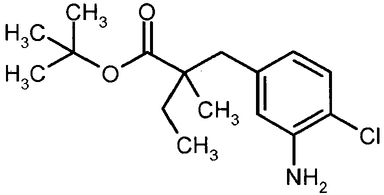

Ausbeute: 405 mg

LC-MS (Methode 6): Rₜ = 1.32 min; m/z = 298 (M+H)⁺.

¹H-NMR (400 MHz, DMSO-*d*₆): δ [ppm] = 0.81 (t, 3H), 0.93 (s, 3H), 1.28-1.37 (m, 1H), 1.38 (s, 9H), 1.59-1.71 (m, 1H), 2.45 (d, 1H), 2.74 (d, 1H), 5.14-5.22 (m, 2H), 6.31 (dd, 1H), 6.57 (d, 1H), 7.04 (d, 1H).

[α]_{D}²⁰ = -11.8°, c = 0.50, Chloroform.

### Beispiel 147A

### (+)-tert.-Butyl-2-(3-amino-4-chlorbenzyl)-2-methylbutanoat (Enantiomer 2)

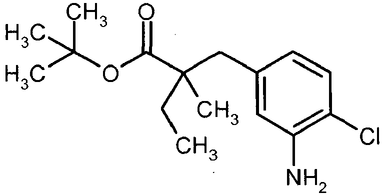

Ausbeute: 403 mg

LC-MS (Methode 6): Rₜ = 1.32 min; m/z = 298 (M+H)⁺.

¹H-NMR (400 MHz, DMSO-*d*₆): δ [ppm] = 0.75-0.85 (m, 3H), 0.93 (s, 3H), 1.30-1.37 (m, 1H), 1.39 (s, 9H), 1.58-1.70 (m, 1H), 2.45 (d, 1H), 2.74 (d, 1H), 5.09-5.23 (m, 2H), 6.31 (dd, 1H), 6.57 (d, 1H), 7.04 (d, 1H).

[α]_{D}²⁰ = +12.0°, c = 0.420, Chloroform.

### Beispiel 148A

### 2,2,2-Trifluor-1-(3-nitrophenyl)ethanon

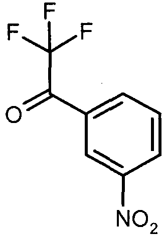

20.0 g (114.9 mmol) 2,2,2-Trifluoracetophenon wurden in 80 ml konz. Schwefelsäure vorgelegt und auf -10°C gekühlt. Zu dieser Mischung wurde eine zuvor bei -10°C bereitete Lösung von 4.8 ml (114.8 mmol) Salpetersäure in 20 ml konz. Schwefelsäure getropft, so dass die Reaktionstemperatur -5°C nicht überschritt. Nach Ende der Zugabe wurde die Reaktionsmischung 1 h zwischen -10°C und 0°C gerührt und dann vorsichtig auf Eiswasser gegeben. Durch Zugabe von 50%-iger Natronlauge wurde der pH-Wert der Mischung auf ca. 9-10 eingestellt. Es wurde dreimal mit Ethylacetat extrahiert, die vereinigten organischen Phasen über Magnesiumsulfat getrocknet und im Vakuum eingeengt. Der Rückstand wurde durch Chromatographie an Silicagel gereinigt (Laufmittel zunächst Cyclohexan/Dichlormethan 2:1 bis 1:1, zuletzt reines Dichlormethan). Es wurden 19.2 g des Zielprodukts erhalten (76.2% d. Th.).

LC-MS (Methode 6): Rₜ = 0.81 min; m/z = 236.

GC-MS (Methode 1): Rₜ = 3.19 min; m/z = 150 (M-CF₃)⁺.

### Beispiel 149A

### tert.-Butyl-(2E/Z)-4,4,4-trifluor-3-(3-nitrophenyl)but-2-enoat

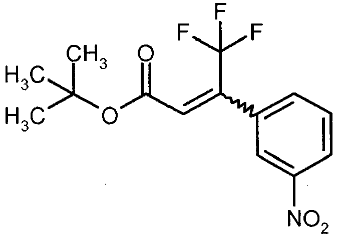

Zu einer auf 0°C gekühlten Suspension von 4.41 g (60% in Mineralöl, 110.4 mmol) Natriumhydrid in einer Mischung aus 37.2 ml THF und 37.2 ml DMF wurden 25.9 ml (110.4 mmol) *tert.*-Butyl-(diethoxyphosphoryl)acetat getropft. Nach 30 min wurden 18.6 g (84.9 mmol) 2,2,2-Trifluor-1-(3-nitrophenyl)ethanon hinzugefügt, das Kältebad entfernt und die Reaktionsmischung 2 h bei RT gerührt. Die Reaktionsmischung wurde dann auf Wasser gegeben und nach Sättigung mit Natriumchlorid dreimal mit Ethylacetat extrahiert. Die vereinigten organischen Phasen wurden über Magnesiumsulfat getrocknet und im Vakuum eingeengt. Der Rückstand wurde durch Chromatographie an Silicagel gereinigt (Laufmittel Cyclohexan/Ethylacetat 100:1 1 bis 20:1). Erhalten wurden 18.0 g des Zielprodukts als *E*/*Z*-Isomerengemisch (66.8% d. Th.).

LC-MS (Methode 6): Rₜ = 1.25 min; keine Ionisierung.

MS (DCI): m/z = 335 (M+H₂O)⁺.

¹H-NMR (400 MHz, DMSO-*d*₆): δ [ppm] = 1.17/1.50 (2s, zus. 9H), 6.93/7.14 (2d, zus. 1H), 7.74-7.94 (m, 2H), 8.16/8.23 (2s, zus. 1H), 8.30-8.42 (m, 1H).

### Beispiel 150A

### (+/-)-tert.-Butyl-3-(3-aminophenyl)-4,4,4-trifluorbutanoat

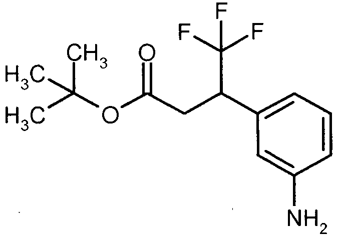

18.0 g (56.7 mmol) *tert*.-Butyl-(2*E*/*Z*)-4,4,4-trifluor-3-(3-nitrophenyl)but-2-enoat wurden in 100 ml Ethanol gelöst und mit Argon deoxygeniert. Nach Zugabe von 1.21 g Palladium auf Kohle (10%) wurde die Mischung bei RT über Nacht unter einer Wasserstoffatmosphäre bei Nonnaldruck kräftig gerührt. Die Reaktionsmischung wurde dann über Celite filtriert, der Rückstand gründlich mit Ethanol gewaschen, das Filtrat im Vakuum eingeengt und das anfallende Produkt über Nacht im Hochvakuum getrocknet. Erhalten wurden 13.7 g des Zielprodukts (83.7% d. Th.).

LC-MS (Methode 6): Rₜ = 1.02 min; m/z = 290 (M+H)⁺.

¹H-NMR (400 MHz, DMSO-*d*₆): δ [ppm] = 1.27 (s, 9H), 2.70 (dd, 1H), 2.89 (dd, 1H), 3.62-3.79 (m, 1H), 5.11-5.17 (m, 2H), 6.43-6.56 (m, 3H), 6.99 (t, 1H).

### Beispiel 151A

### (+/-)-tert.-Butyl-3-(3-amino-4-chlorphenyl)-4,4,4-trifluorbutanoat

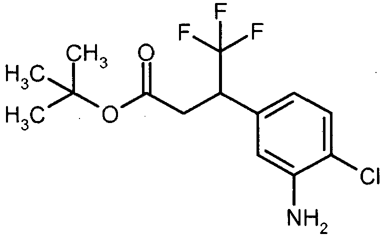

13.6 g (47.0 mmol) (+/-)-*tert*.-Butyl-3-(3-aminophenyl)-4,4,4-trifluorbutanoat wurden in 100 ml Acetonitril vorgelegt und bei RT mit 6.28 g (47.0 mmol) *N*-Chlorsuccinimid versetzt. Die Reaktionsmischung wurde zunächst 12 h bei 60°C gerührt und dann 3 Tage bei RT stehen gelassen. Nach Einengen im Vakuum wurde der Rückstand wurde durch Chromatographie an Silicagel (Laufmittel Cyclohexan/Ethylacetat 100: 1) aufgetrennt und das gewünschte Zielprodukt isoliert. Es wurden 4.49 g der Titelverbindung erhalten (29.5% d. Th.).

LC-MS (Methode 6): Rₜ = 1.17 min; m/z = 324 (M+H)⁺.

¹H-NMR (400 MHz, DMSO-*d*₆): δ [ppm] = 1.27 (s, 9H), 2.72 (dd, 1H), 2.91 (dd, 1H), 3.74-3.86 (m, 1H), 5.43 (s, 2H), 6.55 (dd, 1H), 6.79 (d, 1H), 7.17 (d, 1H).

Das oben erhaltene Racemat wurde durch präparative HPLC an chiraler Phase in die Enantiomere getrennt [Säule: Daicel Chiralpak OJ-H, 5 µm, 250 mm x 20 mm; Fluss: 15 ml/min; Detektion: 220 nm; Injektionsvolumen: 0.20 ml; Temperatur: 35°C; Eluent: 70% Isohexan / 30% Isopropanol]. Ausgehend von 4.49 g Racemat wurden 2.02 g Enantiomer 1 (*Beispiel 152A*) und 2.04 g Enantiomer 2 (*Beispiel 153A)* erhalten:

### Beispiel 152A

### (-)-tert.-Butyl-(3R)-3-(3-amino-4-chlorphenyl)-4,4,4-trifluorbutanoat

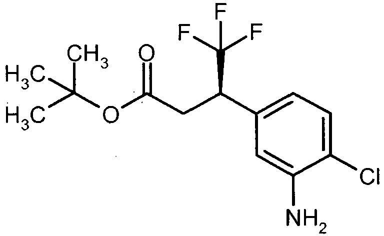

Ausbeute: 2.02 g

LC-MS (Methode 6): Rₜ = 1.17 min; m/z = 324 (M+H)⁺.

¹H-NMR (400 MHz, DMSO-*d*₆): δ [ppm] = 1.27 (s, 9H), 2.72 (dd, 1H), 2.91 (dd, 1H), 3.75-3.85 (m, 1H), 5.40-5.46 (m, 2H), 6.55 (dd, 1H), 6.79 (d, 1H), 7.17 (d, 1H).

[α]_{D}²⁰ = -69.4°, c = 0.520, Chloroform.

### Beispiel 153A

### (+)-tert.-Butyl-(3S)-3-(3-amino-4-chlorphenyl)-4,4,4-trifluorbutanoat

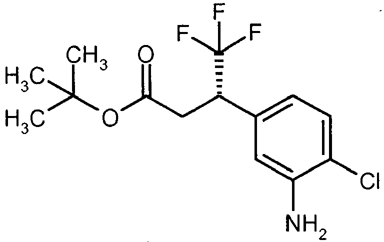

Ausbeute: 2.04 g

LC-MS (Methode 6): Rₜ = 1.17 min; m/z = 324 (M+H)⁺.

¹H-NMR (400 MHz, DMSO-*d*₆): δ [ppm] = 1.27 (s, 9H), 2.71 (dd, 1H), 2.91 (dd, 1H), 3.74-3.86 (m, 1H), 5.38-5.46 (m, 2H), 6.55 (dd, 1H), 6.73-6.80 (m, 1H), 7.17 (d, 1H).

[α]_{D}²⁰ = +66.3°, c = 0.495, Chloroform.

### Beispiel 154A

### tert.-Butyl-cyclobutylacetat

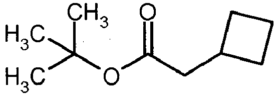

4.0 g (35.0 mmol) Cyclobutylessigsäure wurden in 20 ml Dichlormethan gelöst, mit einem Tropfen DMF versetzt und nach Abkühlen auf 0°C tropfenweise mit 4.0 ml (45.6 mmol) Oxalsäuredichlorid versetzt. Die Reaktionsmischung wurde 2 h zwischen 0°C und 10°C gerührt und dann im Vakuum in der Kälte eingeengt. Der Rückstand wurde in abs. Dichlormethan aufgenommen und erneut in der Kälte im Vakuum eingeengt. Der Vorgang wurde noch einmal wiederholt, bevor das erhaltene Säurechlorid kurz für 5 min im Hochvakuum getrocknet wurde. Der Rückstand wurde dann in 20 ml abs. THF aufgenommen, auf 0°C abgekühlt und tropfenweise mit 28 ml (28 mmol) einer 1 M Lösung von Kalium-*tert*.-butylat in THF versetzt. Nach Ende der Zugabe wurde die Kühlung entfernt und 1 h bei RT nachgerührt, bevor auf Wasser gegeben wurde. Es wurde dreimal mit Dichlormethan extrahiert, und die vereinigten organischen Phasen wurden über Magnesiumsulfat getrocknet und im Vakuum eingeengt. Es wurden 3.88 g des rohen Zielprodukts erhalten (ca. 65% d. Th.).

GC-MS (Methode 1): Rₜ = 2.29 min; m/z = 97 (M-C₃H₅O₂)⁺.

¹H-NMR (400 MHz, DMSO-*d*₆): δ [ppm] = 1.38 (s, 9H), 1.60-1.89 (m, 5H), 1.95-2.11 (m, 2H), 2.28 (d, 2H).

### Beispiel 155A

### tert.-Butyl-cyclopropylacetat

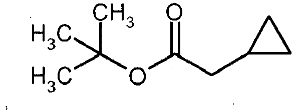

10.0 g (99.9 mmol) Cyclopropylessigsäure wurden in 50 ml Dichlormethan gelöst, mit einem Tropfen DMF versetzt und nach Abkühlen auf 0°C tropfenweise mit 9.6 ml (109.9 mmol) Oxalsäuredichlorid versetzt. Die Reaktionsmischung wurde 2 h zwischen 0°C und 10°C gerührt und dann im Vakuum in der Kälte eingeengt. Der Rückstand wurde kurz (ca. 5 min) im Hochvakuum getrocknet, anschließend in 20 ml abs. THF aufgenommen, auf 0°C abgekühlt und tropfenweise mit 89.9 ml (89.9 mmol) einer 1 M Lösung von Kalium-*tert*.-butylat in THF versetzt. Nach Ende der Zugabe wurde die Kühlung entfernt und 2 h bei RT nachgerührt, bevor das THF im Vakuum (bis zu 150 mm Hg, Wasserbad ca. 30°C) weitgehend entfernt wurde. Zum Rückstand wurde Diethylether und 0.5 N Natronlauge gegeben. Nach Phasentrennung wurde die organische Phase über Magnesiumsulfat getrocknet, im Vakuum eingeengt und der Rückstand kurz im Hochvakuum getrocknet. Es wurden 8.38 g des rohen Zielprodukts erhalten (ca. 53% d. Th.).

GC-MS (Methode 1): Rₜ = 1.80 min; m/z = 100 (M-C₄H₈)⁺.

¹H-NMR (400 MHz, DMSO-*d*₆): δ [ppm] = 0.05-0.14 (m, 2H), 0.38-0.51 (m, 2H), 0.81-0.99 (m, 1H), 1.40 (s, 9H), 2.10 (d, 2H).

### Beispiel 156A

### (+/-)-tert.-Butyl-3-(3-brom-4-chlorphenyl)-2-cyclobutylpropanoat

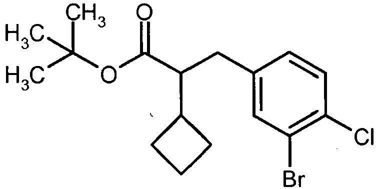

2.9 ml (20.8 mmol) Diisopropylamin wurden unter Argon in 30 ml trockenem THF gelöst und auf -20°C abgekühlt. Es wurden 8.3 ml (20.8 mmol) *n*-Butyllithium-Lösung (2.5 M in Hexan) zugetropft, und das resultierende Gemisch wurde 30 min bei -20°C gerührt, bevor auf -78°C abgekühlt wurde. Bei dieser Temperatur wurde mit einer Lösung von 2.60 g (ca. 15.3 mmol, roh) *tert*.-Butyl-cyclobutylacetat in 10 ml THF versetzt. Nach 4 h Rühren bei -78°C wurde eine Lösung von 3.95 g (13.9 mmol) 2-Brom-4-(brommethyl)-1-chlorbenzol in 10 ml THF zugegeben. Das Reaktionsgemisch wurde über Nacht langsam auf RT erwärmt, bevor mit gesättigter wässriger Ammonium-chlorid-Lösung versetzt wurde. Es wurde dreimal mit Ethylacetat extrahiert. Die vereinigten organischen Phasen wurden über Magnesiumsulfat getrocknet und im Vakuum eingeengt. Der zurückbleibende Feststoff wurde mit 30 ml Cyclohexan/Dichlormethan (1:1) verrührt und abfiltriert. Der Feststoff wurde erneut mit 10 ml Cyclohexan/Dichlormethan (1:1) verrührt und abermals abfiltriert; dieser Vorgang wurde noch ein weiteres Mal wiederholt. Alle Filtrate wurden aufgefangen, vereinigt und im Vakuum eingeengt. Dieser Rückstand wurde dann durch Chromatographie an Silicagel weiter gereinigt (Laufmittel von reinem Cyclohexan bis Cyclohexän/Dichlormethan 20:1 bis 10: 1). Es wurden so 2.24 g der Titelverbindung erhalten (43.2% d. Th.).

GC-MS (Methode 1): Rₜ = 6.92 min; m/z = 318 (M-C₄H₇)⁺.

¹H-NMR (400 MHz, DMSO-*d*₆): δ [ppm] = 1.27 (s, 9H), 1.71-1.84 (m, 4H), 1.90-1.94 (m, 1H), 1.96-2.04 (m, 1H), 2.33-2.44 (m, 1H), 2.53-2.60 (m, 1H), 2.61-2.71 (m, 1H), 7.22 (dd, 1H), 7.52 (d, 1H), 7.57 (d, 1H).

Auf analoge Weise wurde das folgende Beispiel erhalten:

### Beispiel 157A

### tert.-Butyl-3-(3-brom-4-chlorphenyl)-2-cyclopropylpropanoat

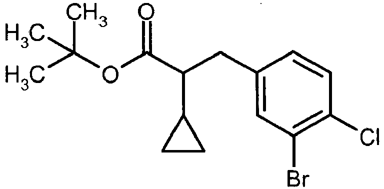

Ausgehend von *tert*.-Butyl-cyclopropylacetat und 2-Brom-4-(brommethyl)-1-chlorbenzol wurden 3.13 g der Titelverbindung erhalten (45% d. Th.).

GC-MS (Methode 1): Rₜ = 6.41 min; m/z = 301/304 (M-C₄H₈)⁺.

¹H-NMR (400 MHz, DMSO-*d*₆): δ [ppm] = 0.22 (tt, 2H), 0.40-0.50 (m, 2H), 0.82-0.93 (m, 1H), 1.28 (s, 9H), 1.82-1.88 (m, 1H), 2.81-2.89 (m, 2H), 7.24 (dd, 1H), 7.52 (d, 1H), 7.60 (d, 1H).

### Beispiel 158A

### (+/-)-tert.-Butyl-3-[3-(benzylamino)-4-chlorphenyl]-2-cyclobutylpropanoat

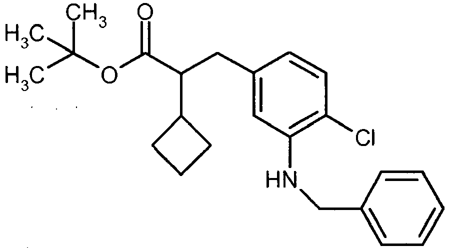

In einem trockenem Kolben wurden unter Argon 848.6 mg (8.83 mmol) Natrium-*tert*.-butylat, 337 mg (0.39 mmol) Tris(dibenzylidenaceton)dipalladium und 183 mg (0.29 mmol) *rac*-2,2'-Bis-(diphenylphosphino)-1,1'-binaphthyl eingewogen, für 10 min unter Hochvakuum gehalten und dann mit Argon belüftet. Nacheinander wurden 5 ml abs. Toluol, 0.96 ml (8.83 mmol) Benzylamin und eine Lösung von 2.75 g (7.36 mmol) (+/-)-*tert*.-Butyl-3-(3-brom-4-chlorphenyl)-2-cyclobutyl-propanoat in 5 ml abs. Toluol hinzugefügt. Die Reaktionsmischung wurde noch dreimal evakuiert und jeweils wieder mit Argon belüftet und dann für 3 h bei 110°C gerührt. Nach Abkühlen wurde das Reaktionsgemisch auf gesättigte wässrige Ammoniumchlorid-Lösung gegeben. Es wurde dreimal mit Ethylacetat extrahiert. Die organischen Phasen wurden vereinigt, über Magnesiumsulfat getrocknet und im Vakuum eingeengt. Das Rohprodukt wurde durch Chromatographie an Silicagel gereinigt (Laufmittel Cyclohexan/Dichlormethan 4:1 bis 2:1, dann reines Dichlormethan). Es wurden 1.95 g der Titelverbindung erhalten (65.1% d. Th.).

LC-MS (Methode 4): Rₜ = 1.90 min; m/z = 400 (M+H)⁺.

¹H-NMR (400 MHz, DMSO-*d*₆): δ [ppm] = 1.26 (s, 9H), 1.54-1.76 (m, 4H), 1.78-1.86 (m, 2H), 2.19-2.38 (m, 2H), 2.38-2.45 (m, 2H), 4.33-4.44 (m, 2H), 5.95 (t, 1H), 6.25-6.40 (m, 2H), 7.11 (d, 1H), 7.23 (td, 1H), 7.27-7.37 (m, 4H).

Auf analoge Weise wurde das folgende Beispiel erhalten:

### Beispiel 159A

### tert.-Butyl-3-[3-(benzylamino)-4-chlorphenyl]-2-cyclopropylpropanoat

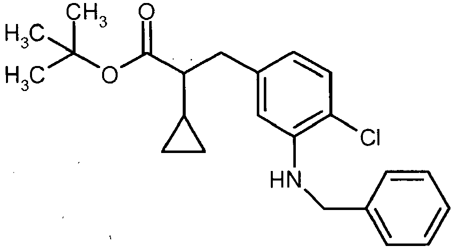

Ausgehend von *tert*.-Butyl-3-(3-brom-4-chlorphenyl)-2-cyclopropylpropanoat und Benzylamin wurden 2.51 g der Titelverbindung erhalten (74.7% d. Th.).

LC-MS (Methode 6): Rₜ = 1.55 min; m/z = 386 (M+H)⁺.

### Beispiel 160A

### (+/-)-tert.-Butyl-3-(3-amino-4-chlorphenyl)-2-cyclobutylpropanoat

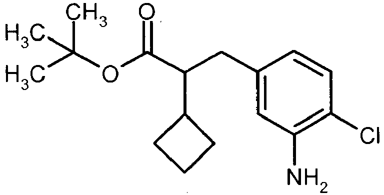

1.85 g (4.63 mmol) (+/-)-*tert*.-Butyl-3-[3-(benzylamino)-4-chlorphenyl]-2-cyclobutylpropanoat wurden in 10 ml Ethylacetat gelöst, mit Argon deoxygeniert und mit 98 mg (0.093 mmol) Palladium auf Kohle (10%) versetzt. Die Reaktionsmischung wurde 4 h bei RT unter einer Wasserstoffatmosphäre bei Normaldruck gerührt und dann über das Wochenende stehen gelassen. Die Mischung wurde über Celite filtriert, der Rückstand mit Ethylacetat nachgewaschen, das Filtrat im Vakuum eingeengt und der Filtrat-Rückstand im Hochvakuum getrocknet. Dieser Rückstand (ca. 1:1-Mischung aus Startmaterial und Zielprodukt) wurde erneut in ca. 10 ml Ethylacetat gelöst, mit Argon deoxygeniert und wieder mit 98 mg (0.093 mmol) Palladium auf Kohle (10%) versetzt. Die Reaktionsmischung wurde erneut 4 h bei RT unter einer Wasserstoffatmosphäre bei Normaldruck gerührt. Es wurde dann über Celite filtriert, mit Ethylacetat nachgewaschen, das Filtrat im Vakuum eingeengt und der Rückstand im Hochvakuum getrocknet. Nach chromatographischer Reinigung an Silicagel (Laufmittel Cyclohexan/Ethylacetat 20:1) wurden 1.12 g des Zielprodukts (78.2% d. Th.) erhalten.

LC-MS (Methode 6): Rₜ = 1.34 min; m/z = 310 (M+H)⁺, 254 (M-C₄H₇)⁺.

¹H-NMR (400 MHz, DMSO-*d*₆): δ [ppm] = 1.29 (s, 9H), 1.68-1.86 (m, 4H), 1.87-1.95 (m, 1H), 1.96-2.07 (m, 1H), 2.32-2.48 (m, 4H), 5.21 (s, 2H), 6.33 (dd, 1H), 6.57 (d, 1H), 7.04 (d, 1H).

Das oben erhaltene Racemat wurde durch präparative HPLC an chiraler Phase in die Enantiomere getrennt [Säule: Daicel Chiralpak AD-H, 5 µm, 250 mm x 20 mm; Fluss: 20 ml/min; Detektion: 230 nm; Injektionsvolumen: 0.80 ml; Temperatur: 25°C; Eluent: 95% Isohexan / 5% Ethanol]. Ausgehend von 790 mg Racemat wurden 318 mg Enantiomer 1 (*Beispiel 161A*) und 339 mg Enantiomer *2* (*Beispiel 162A)* erhalten:

### Beispiel 161A

### tert.-Butyl-3-(3-amino-4-chlorphenyl)-2-cyclobutylpropanoat (Enantiomer 1)

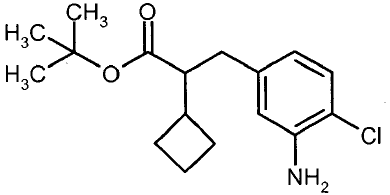

Ausbeute: 318 mg

LC-MS (Methode 4): Rₜ = 1.70 min; m/z = 254 (M-C₄H₇)⁺.

¹H-NMR (400 MHz, DMSO-*d*₆): δ [ppm] = 1.29 (s, 9H), 1.68-1.85 (m, 4H), 1.87-1.94 (m, 1H), 1.96-2.06 (m, 1H), 2.31-2.48 (m, 4H), 5.21 (s, 2H), 6.33 (dd, 1H), 6.57 (d, 1H), 7.04 (d, 1H).

### Beispiel 162A

### tert.-Butyl-3-(3-amino-4-chlorphenyl)-2-cyclobutylpropanoat (Enantiomer 2)

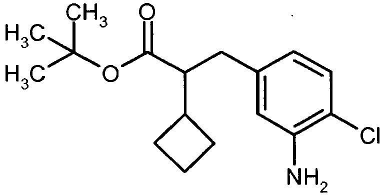

Ausbeute: 339 mg

LC-MS (Methode 4): Rₜ = 1.71 min; m/z = 254 (M-C₄H₇)⁺.

¹H-NMR (400 MHz, DMSO-*d*₆): δ [ppm] = 1.29 (s, 9H), 1.67-1.84 (m, 4H), 1.87-1.94 (m, 1H), 1.95-2.05 (m, 1H), 2.32-2.48 (m, 4H), 5.22 (s, 2H), 6.33 (dd, 1H), 6.57 (d, 1H), 7.04 (d, 1H).

### Beispiel 163A

### (+/-)-tert.-Butyl-3-(3-amino-4-chlorphenyl)-2-cyclopropylpropanoat

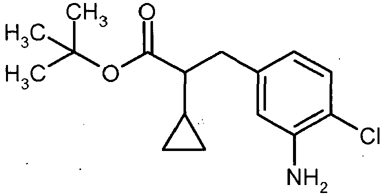

2.50 g (4.63 mmol) (+/-)-*tert*.-Butyl-3-[3-(benzylamino)-4-chlorphenyl]-2-cyclopropylpropanoat wurden in 160 ml Ethylacetat gelöst, mit Argon deoxygeniert und mit 150 mg Palladium auf Kohle (10%) versetzt. Die Reaktionsmischung wurde 8 h bei RT unter einer Wasserstoffatmosphäre bei Normaldruck gerührt. Anschließend wurde das Gemisch über Celite filtriert, mit Ethylacetat nachgewaschen, das Filtrat im Vakuum eingeengt und der Rückstand im Hochvakuum getrocknet. Der Rückstand wurde durch Chromatographie an Silicagel gereinigt (Laufmittel reines Cyclohexan bis Cyclohexan/Ethylacetat 20:1). Erhalten wurden 1.41 g des Zielprodukts (73.6% d. Th.).

LC-MS (Methode 6): Rₜ = 1.28 min; m/z = 240 (M-C₄H₇)⁺.

¹H-NMR (400 MHz, DMSO-*d*₆): δ [ppm] = 0.10-0.18 (m, 1H), 0.19-0.26 (m, 1H), 0.37-0.52 (m, 2H), 0.79-0.92 (m, 1H), 1.30 (s, 9H), 1.73 (td, 1H), 2.65-2.74 (m, 2H), 5.10-5.25 (m, 2H), 6.35 (dd, 1H), 6.59 (d, 1H), 6.99-7.06 (m, 1H).

### Beispiel 164A

### Methyl-3-(3-amino-4-chlorphenyl)hex-2-enoat und Methyl-3-(3-amino-4-chlorphenyl)hex-3-enoat

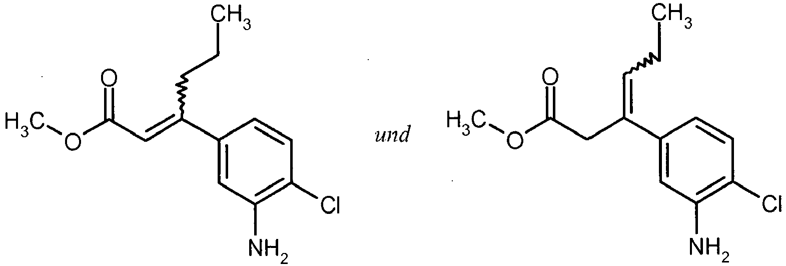

Zu einer Mischung von 10.0 g (48.4 mmol) 5-Brom-2-chloranilin und 8.69 g (67.8 mmol) Methyl-(2*E*)-hex-2-enoat in 100 ml DMF wurden 33.8 ml (242.2 mmol) Triethylamin gegeben. Die Mischung wurde dreimal evakuiert und jeweils wieder mit Argon belüftet. Nach Zugabe von 1.09 g (4.84 mmol) Palladium(II)acetat und 2.95 g (9.69 mmol) Tri-2-tolylphosphin wurde erneut zweimal evakuiert und jeweils mit Argon belüftet, bevor die Reaktionsmischung dann 4 h bei 150°C gerührt wurde. Nach Abkühlen wurde das Gemisch auf Wasser gegeben, mit Natriumchlorid gesättigt und dreimal mit Ethylacetat extrahiert. Die vereinigten organischen Phasen wurden über Magnesiumsulfat getrocknet und im Vakuum, zuletzt im Hochvakuum eingeengt. Der Rückstand wurde durch Chromatographie an Silicagel aufgereinigt (Laufmittel Cyclohexan/Ethylacetat 50:1). Es wurden 7.70 g eines Gemisches der beiden Titelverbindungen erhalten (62.7% d. Th., Verhältnis ca. 1.5:1 zugunsten des α,β-ungesättigten Isomers).

LC-MS (Methode 6): Methyl-3-(3-amino-4-chlorphenyl)hex-2-enoat: Rₜ = 1.04 min, m/z = 254 (M+H)⁺; Methyl-3-(3-amino-4-chlorphenyl)hex-3-enoat: Rₜ = 1.12 min, m/z = 254 (M+H)⁺.

¹H-NMR (400 MHz, DMSO-*d*₆): Methyl-3-(3-amino-4-chlorphenyl)hex-2-enoat: δ [ppm] = 0.85 (t, 3H), 1.29-1.41 (m, 2H), 2.92-3.00 (m, 2H), 3.46 (s, 3H), 5.45 (s, 2H), 5.98 (s, 1H), 6.69 (dd, 1H), 6.94 (d, 1H), 7.20 (d, 1H).

¹H-NMR (400 MHz, DMSO-*d*₆): Methyl-3-(3-amino-4-chlorphenyl)hex-3-enoat: δ [ppm] = 1.00 (t, 3H), 2.15 (quin, 2H), 3.56 (s, 3H), 3.66 (s, 2H), 5.26-5.31 (m, 2H), 5.84 (t, 1H), 6.54 (dd, 1H), 6.79 (d, 1H), 7.09 (d, 1H).

### Beispiel 165A

### (+/-)-Methyl-3-(3-amino-4-chlorphenyl)hexanoat

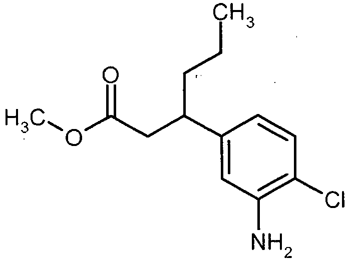

7.70 g (30.3 mmol) des Gemisches aus Methyl-3-(3-amino-4-chlorphenyl)hex-2-enoat und Methyl-3-(3-amino-4-chlorphenyl)hex-3-enoat (ca. 1.5:1, Beispiel 164A) wurden in 45 ml Ethylacetat gelöst, mit 646 mg (0.303 mmol) Palladium auf Kohle (5%) versetzt und bei RT unter einer Wasserstoffatmosphäre bei Normaldruck gerührt. Nach 10 h wurde die Reaktionsmischung über Celite abfiltriert, mit Ethylacetat nachgewaschen und das Filtrat eingeengt. Der Rückstand wurde in ca. 50 ml Ethylacetat aufgenommen, erneut mit ca. 650 mg Palladium auf Kohle (5%) versetzt und bei RT unter einer Wasserstoffatmosphäre bei Normaldruck gerührt. Nach weiteren 36 h wurde die Reak-tionsmischung wieder über Celite abfiltriert, mit Ethylacetat nachgewaschen und das Filtrat eingeengt. Der Rückstand wurde in 800 ml Ethylacetat aufgenommen, abermals mit ca. 650 mg Palladium auf Kohle (5%) versetzt und 24 h bei RT unter einer Wasserstoffatmosphäre bei Normaldruck gerührt. Die Reaktionsmischung wurde erneut über Celite abfiltriert, mit Ethylacetat nachgewaschen und das Filtrat eingeengt. Der Rückstand wurde durch Chromatographie an Silicagel gereinigt (Laufmittel Cyclohexan/Ethylacetat 50:1 bis 10:1). Es wurden insgesamt 4.79 g eines Gemisches aus Zielprodukt und Ausgangsmaterial erhalten. Dieses Gemisch wurde in 180 ml Ethylacetat gelöst, erneut mit 603 mg (0.566 mmol) Palladium auf Kohle (10%) versetzt und über Nacht bei RT unter einer Wasserstoffatmosphäre bei Normaldruck gerührt. Das Reaktionsgemisch wurde über Celite abfiltriert, mit Ethylacetat nachgewaschen, das Filtrat eingeengt und der Rückstand im Hochvakuum getrocknet. Es wurden so 4.45 g (ca. 57% d. Th.) des Zielprodukts erhalten.

LC-MS (Methode 4): Rₜ = 1.50 min; m/z = 256 (M+H)⁺.

¹H-NMR (400 MHz, DMSO-*d*₆): δ [ppm] = 0.80 (t, 3H), 1.03-1.15 (m, 2H), 1.39-1.56 (m, 2H), 2.46 (dd, 1H), 2.59 (dd, 1H), 2.78-2.89 (m, 1H), 3.50 (s, 3H), 5.22 (br. s, 2H), 6.39 (dd, 1H), 6.61 (d, 1H), 7.06 (d, 1H).

### Beispiel 166A und Beispiel 167A

### Methyl-3-(3-amino-4-chlorphenyl)pent-2-enoat und Methyl-3-(3-amino-4-chlorphenyl)pent-3-enoat

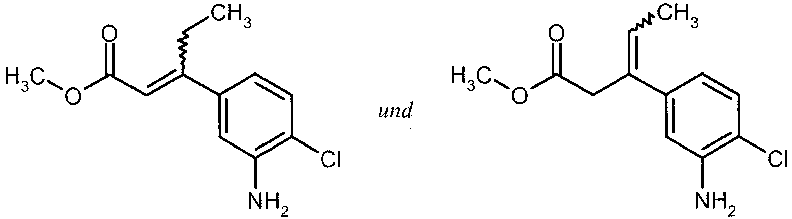

Zu einer Mischung von 5.0 g (24.2 mmol) 5-Brom-2-chloranilin und 5.53 g (48.4 mmol) Methyl-2-pentenoat in 50 ml DMF wurden 16.9 ml (121 mmol) Triethylamin gegeben. Die Mischung wurde dreimal evakuiert und jeweils wieder mit Argon belüftet. Nach Zugabe von 544 mg (2.42 mmol) Palladium(II)acetat und 1.47 g (4.84 mmol) Tri-2-tolylphosphin wurde erneut zweimal evakuiert und jeweils mit Argon belüftet und die Reaktionsmischung dann 6 h bei 150°C gerührt. Nach Abkühlen wurde das Gemisch über Nacht bei RT aufbewahrt und danach auf Wasser gegeben. Es wurde dreimal mit Ethylacetat extrahiert. Die organischen Phasen wurden vereinigt, über Magnesiumsulfat getrocknet, im Vakuum eingeengt und der Rückstand im Hochvakuum getrocknet. Aus dem Rückstand wurden durch Chromatographie an Silicagel (Laufmittel Cyclohexan/Ethylacetat 50:1 bis 10:1) die beiden isomeren Zielprodukte in getrennter Form isoliert. Es wurden 0.85 g Methyl-3-(3-amino-4-chlorphenyl)pent-2-enoat (14.6% d. Th.) sowie 3.05 g Methyl-3-(3-amino-4-chlorphenyl)pent-3-enoat (52.5% d. Th.) erhalten.

### Methyl-3-(3-amino-4-chlorphenyl)pent-2-enoat (Beispiel 166A):

LC-MS (Methode 6): Rₜ = 1.09 min; m/z = 240 (M+H)⁺.

¹H-NMR (400 MHz, DMSO-*d*₆): δ [ppm] = 0.97 (t, 3H), 2.98 (q, 2H), 3.66 (s, 3H), 5.45 (s, 2H), 5.96 (s, 1H), 6.70 (dd, 1H), 6.95 (d, 1H), 7.21 (d, 1H).

### Methyl-3-(3-amino-4-chlorphenyl)pent-3-enoat (Beispiel 167A):

LC-MS (Methode 6): Rₜ = 1.00 min; m/z = 240 (M+H)⁺.

¹H-NMR (400 MHz, DMSO-*d*₆): δ [ppm] = 1.75 (d, 3H), 3.47 (s, 2H), 3.56 (s, 3H), 5.28 (s, 2H), 5.94 (q, 1H), 6.54 (dd, 1H), 6.77 (d, 1H), 7.09 (d, 1H).

### Beispiel 168A

### (+/-)-Methyl-3-(3-amino-4-chlorphenyl)pentanoat

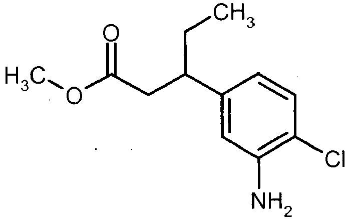

3.05 g (12.7 mmol) Methyl-3-(3-amino-4-chlorphenyl)pent-3-enoat und 0.85 g (3.55 mmol) Methyl-3-(3-amino-4-chlorphenyl)pent-2-enoat wurden zusammen in 500 ml Ethylacetat gelöst, mit 346 mg (0.325 mmol) Palladium auf Kohle (10%) versetzt und über Nacht bei RT unter einer Wasserstoffatmosphäre bei Normaldruck gerührt. Die Reaktionsmischung wurde danach über Celite abfiltriert, mit Ethylacetat nachgewaschen und das Filtrat eingeengt. Nach Trocknen des Rückstands im Hochvakuum wurden 3.73 g des Zielprodukts erhalten (94.8% d. Th.).

GC-MS (Methode 1): Rₜ = 6.07 min; m/z = 242 (M)⁺.

¹H-NMR (400 MHz, DMSO-*d*₆): δ [ppm] = 0.71 (t, 3H), 1.42-1.49 (m, 1H), 1.55-1.61 (m, 1H), 2.42-2.48 (m, 1H), 2.60 (dd, 1H), 2.68-2.78 (m, 1H), 3.50 (s, 3H), 5.22 (s, 2H), 6.39 (dd, 1H), 6.61 (d, 1H), 7.05-7.08 (m, 1H).

### Beispiel 169A

### (3R)-2-(4-Chlor-2-fluorphenyl)-4,4,4-trifluor-3-methylbutansäureethylester (Diastereomerengemisch)

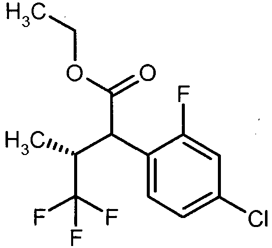

81.5 ml (81.5 mmol) einer 1 M Lösung von Lithiumhexamethyldisilazid in Toluol wurden auf -20°C abgekühlt und tropfenweise mit einer Lösung von 10.0 g (50.3 mmol) Ethyl-(3*R*)-4,4,4-trifluor-3-methylbutanoat in 50 ml abs. Toluol versetzt. Die Mischung wurde 10 min nachgerührt. Anschließend wurde bei -20°C eine zuvor hergestellte Lösung von 14.8 g (70.6 mmol) 1-Brom-4-chlor-2-fluorbenzol, 366 mg (1.63 mmol) Palladium(II)acetat und 1.35 g (3.42 mmol) 2-Dicyclohexyl-phosphino-2'-(*N*,*N*-dimethylamino)biphenyl in 50 ml abs. Toluol zugetropft. Nach Ende der Zugabe wurde die Kühlung entfernt, und die resultierende Reaktionsmischung wurde zunächst 1 h bei RT, dann über Nacht bei 80°C gerührt. Nach Abkühlen wurde das Gemisch über Celite filtriert, mehrfach mit Toluol nachgewaschen und das erhaltene Filtrat im Vakuum eingeengt. Aus dem Rückstand wurden nach Chromatographie an Silicagel (Laufmittel Cyclohexan/Ethylacetat 100:1 → 100:4) 4.26 g der Titelverbindung erhalten (25.1 % d. Th.).

GC-MS (Methode 1): Rₜ = 4.21 min; m/z = 312 (M)⁺.

Auf analoge Weise wurde das folgende Beispiel erhalten:

### Beispiel 170A

### (3R)-2-(4-Chlor-3-fluorphenyl)-4,4,4-trifluor-3-methylbutansäureethylester (Diastereomerengemisch)

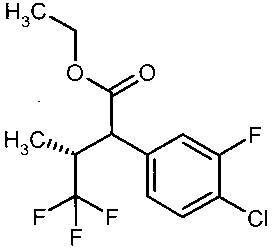

Ausgehend von 2.0 g Ethyl-(3*R*)-4,4,4-trifluor-3-methylbutanoat und 2.96 g 1-Brom-4-chlor-3-fluor-benzol wurden 2.47 g der Zielverbindung erhalten.

GC-MS (Methode 1): Rₜ = 4.33 min + 4.36 min; beide m/z = 312 (M)⁺.

¹H-NMR (400 MHz, DMSO-*d*₆): *Haupt-Diastereomer* δ [ppm] = 0.80 (d, 3H), 1.08-1.19 (m, 3H), 3.34-3.41 (m, 1H), 3.88 (d, 1H), 4.01-4.18 (m, 2H), 7.28-7.34 (m, 1H), 7.51-7.64 (m, 2H).

### Beispiel 171A

### Ethyl-(3R)-2-(4-chlor-2-methylphenyl)-4,4,4-trifluor-3-methylbutanoat (Diastereomerengemisch)

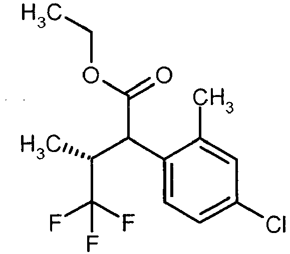

22.5 ml (22.5 mmol) einer 1 M Lösung von Lithiumhexamethyldisilazid in Toluol wurden auf -20°C abgekühlt und tropfenweise mit einer Lösung von 2.76 g (50.3 mmol) Ethyl-(3*R*)-4,4,4-trifluor-3-methylbutanoat in 15 ml abs. Toluol versetzt. Die Mischung wurde 10 min nachgerührt. Anschließend wurde bei -20°C eine zuvor hergestellte Lösung von 4.0 g (19.5 mmol) 2-Brom-5-chlortoluol, 101 mg (0.45 mmol) Palladium(II)acetat und 371 mg (0.94 mmol) 2-Dicyclohexyl-phosphino-2'-(*N*,*N*-dimethylamino)biphenyl in 15 ml abs. Toluol zugetropft. Nach Ende der Zugabe wurde die Kühlung entfernt, und die resultierende Reaktionsmischung wurde zunächst 1 h bei RT, dann über Nacht bei 100°C gerührt. Nach Abkühlen wurde das Gemisch über Celite filtriert, mehrfach mit Toluol nachgewaschen und das erhaltene Filtrat im Vakuum eingeengt. Es wurden 3.10 g der Titelverbindung als Rohprodukt erhalten, welches direkt weiter umgesetzt wurde.

GC-MS (Methode 1): Rₜ = 4.72 min; m/z = 308 (M)⁺.

### Beispiel 172A

### Ethyl-(3R)-2-(4-chlor-3-methylphenyl)-4,4,4-trifluor-3-methylbutanoat (Diastereomerengemisch)

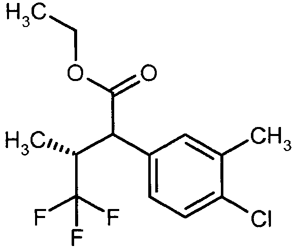

29.2 ml (29.2 mmol) einer 1 M Lösung von Lithiumhexamethyldisilazid in Toluol wurden auf -10°C abgekühlt und tropfenweise mit einer Lösung von 4.30 g (23.4 mmol) Ethyl-(3R)-4,4,4-trifluor-3-methylbutanoat in 26 ml abs. Toluol versetzt. Die Mischung wurde 10 min nachgerührt. Anschließend wurde bei -10°C eine zuvor hergestellte Lösung von 5.0 g (19.5 mmol, 80%-ig) 5-Brom-2-chlortoluol, 131 mg (0.58 mmol) Palladium(II)acetat und 483 mg (1.23 mmol) 2-Dicyclohexyl-phosphino-2'-(*N*,*N*-dimethylamino)biphenyl in 26 ml abs. Toluol zugetropft. Die resultierende Reak-tionsmischung wurde zunächst 1 h bei RT, dann 4 h bei 80°C gerührt. Nach Abkühlen wurde das Gemisch mit Ethylacetat verdünnt, zweimal mit ges. wässriger Natriumhydrogencarbonat-Lösung und einmal mit ges. Natriumchlorid-Lösung gewaschen, über Natriumsulfat getrocknet und im Vakuum eingeengt. Es wurden 7.80 g der Titelverbindung als Rohprodukt erhalten, welches direkt weiter umgesetzt wurde.

LC-MS (Methode 4): Rₜ = 1.55 min; m/z = 309 (M+H)⁺.

Auf analoge Weise wurde das folgende Beispiel erhalten:

### Beispiel 173A

### Ethyl-(3R)-2-(3,4-dichlophenyl)-4,4,4-trifluor-3-methylbutanoat (Diastereomerengemisch)

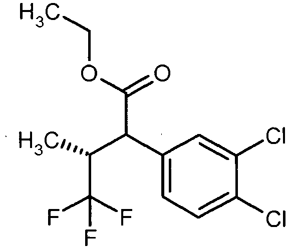

Ausgehend von 3.91 g Ethyl-(3*R*)-4,4,4-trifluor-3-methylbutanoat und 5.0 g 4-Brom-1,2-dichlor-benzol wurden 7.54 g der Zielverbindung als Rohprodukt erhalten.

LC-MS (Methode 4): Rₜ = 1.54 min; m/z = 329 (M+H)⁺.

¹H-NMR (400 MHz, DMSO-*d₆*): beide Diastereomere δ [ppm] = 0.80/1.17 (2d, zus. 3H), 1.10-1.15 (m, 3H), 3.30-3.41 (m, 1H), 3.89/3.94 (2d, zus. 1H), 4.01-4.18 (m, 2H), 7.38-7.48 (m, ca. 1H), 7.59-7.68 (m, ca. 1H), 7.74/7.75 (2d, zus. 1H).

### Beispiel 174A

### (3R)-2-(4-Chlor-2-fluorphenyl)-4,4,4-trifluor-3-methylbutansäure (Diastereomerengemisch)

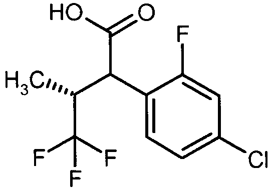

4.26 g (13.6 mmol) (3*R*)-2-(4-Chlor-2-fluophenyl)-4,4,4-trifluor-3-methylbutansäureethylester (Diastereomerengemisch) wurden in einem Gemisch aus 22 ml Methanol, 22 ml THF und 11 ml Wasser gelöst und bei 0°C mit 10.9 g 50%-iger Natronlauge versetzt. Die Reaktionsmischung wurde über Nacht bei RT gerührt. Anschließend wurden im Vakuum die organischen Lösungsmittel weitgehend entfernt. Die verbliebene Mischung wurde mit Wasser verdünnt und mit Diethylether extrahiert. Nach Phasentrennung wurde organische Phase verworfen, und die wässrige Phase wurde mit halbkonzentrierter Salzsäure angesäuert (pH ca. 2) und mehrfach mit Ethylacetat extrahiert. Die vereinigten Ethylacetat-Phasen wurden über Natriumsulfat getrocknet und im Vakuum eingeengt. Es wurden 3.38 g (76.7% d. Th.) des Zielprodukts als Diastereomerengemisch erhalten, das ohne weitere Reinigung in Folgereaktionen eingesetzt werden konnte.

LC-MS (Methode 4): Rₜ = 1.25 min; m/z = 283 (M-H)⁻.

¹H-NMR (400 MHz, DMSO-*d₆*): *Haupt-Diastereomer* δ [ppm] = 0.87 (d, 3H), 3.27-3.37 (m, 1H), 4.02 (d, 1H), 7.35 (dd, 1H), 7.45-7.52 (m, 2H), 13.02 (br. s, 1H).

Auf analoge Weise wurden die beiden folgenden Carbonsäuren erhalten:

### Beispiel 175A

### (3R)-2-(4-Chlor-3-fluorphenyl)-4,4,4-trifluor-3-methylbutansäure (Diastereomenengemisch)

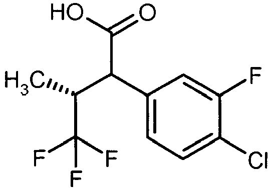

Diastereomerenverhältnis ca. 1:1.

GC-MS (Methode 1): Rₜ = 4.79 min; m/z = 284 (M)⁺.

¹H-NMR (400 MHz, DMSO-*d₆*): beide Diastereomere δ [ppm] = 0.80/1.19 (2d, zus. 3H), 3.18-3.29 (m, 1H), 3.74/3.77 (2dd, zus. 1H), 7.28 (d, 1H), 7.43-7.65 (m, 2H), 12.91/13.24 (2 br. s, zus. 1H).

### Beispiel 176A

### (3R)-2-(4-Chlor-3-methylphenyl)-4,4,4-trifluor-3-methylbutansäure (Diastereomerengemisch)

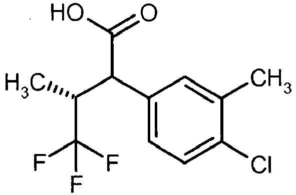

Diastereomerenverhältnis ca. 5:1.

¹H-NMR (400 MHz, DMSO-*d₆*): beide Diastereomere δ [ppm] = 0.78/1.11 (2d, zus. 3H), 2.31/2.32 (2s, zus. 3H), 3.24-3.30 (m, 1H), 3.61/3.64 (2d, zus. 1H), 7.20-7.50 (m, 5H), 12.80 (br. s, 1H).

### Beispiel 177A

### (3R)-2-(4-Chlor-2-methylphenyl)-4,4,4-trifluor-3-methylbutansäure (Diastereomerengemisch)

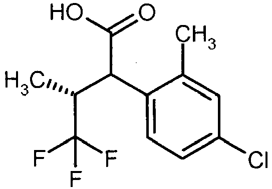

3.10 g (roh, ca. 10.04 mmol) Ethyl-(3*R*)-2-(4-chlor-2-methylphenyl)-4,4,4-trifluor-3-methylbutanoat (Diastereomerengemisch) wurden in einem Gemisch aus 10 ml Methanol, 10 ml THF und 5 ml Wasser gelöst und bei 0°C mit 8.03 g 50%-iger Natronlauge versetzt. Die Reaktionsmischung wurde über Nacht bei RT gerührt. Danach wurde mit 1 N Salzsäure angesäuert (pH ca. 2) und dreimal mit Ethylacetat extrahiert. Die vereinigten organischen Phasen wurden mit ges. Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan/Ethylacetat 50:1 bis 4:1). Es wurden 1.46 g (51.8% d. Th.) des Zielprodukts als Diastereomerengemisch (ca. 5:1) erhalten.

GC-MS (Methode 1): Rₜ = 5.14 min; m/z = 280 (M)⁺.

¹H-NMR (400 MHz, DMSO-*d₆*): beide Diastereomere δ [ppm] = 0.76/1.11 (2d, zus. 3H), 2.34/2.36 (2s, zus. 3H), 3.33-3.38 (m, ca. 1H, verdeckt), 3.81/3.88 (2d, zus. 1H), 7.27-7.41 (m, 3H), 12.81 (br. s, 1H).

### Beispiel 178A

### (3R)-2-(3,4-Dichlorphenyl)-4,4,4-trifluor-3-methylbutansäure (Diastereomerengemisch)

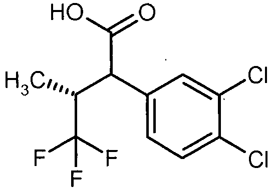

3.77 g (roh, ca. 11.5 mmol) Ethyl-(3*R*)-2-(3,4-dichlorphenyl)-4,4,4-trifluor-3-methylbutanoat (Diastereomerengemisch) wurden in einem Gemisch aus 14 ml Methanol, 14 ml THF und 5 ml Wasser gelöst und bei 0°C mit 9.16 g 50%-iger Natronlauge versetzt. Die Reaktionsmischung wurde ca. 6 h bei 40°C gerührt. Anschließend wurde mit 1 N Salzsäure angesäuert (pH ca. 2) und dreimal mit Ethylacetat extrahiert. Die vereinigten organischen Phasen wurden mit ges. Natriumchlorid-Lösung gewaschen, über Natriumsulfat getrocknet und im Vakuum eingeengt. Es wurden 3.94 g der Zielverbindung als Rohprodukt erhalten, das ohne weitere Reinigung in Folgereaktionen eingesetzt werden konnte.

¹H-NMR (400 MHz, DMSO-*d₆*): beide Diastereomere δ [ppm] = 0.80/1.19 (2d, zus. 3H), 3.21-3.30 (m, 1H), 3.69-3.82 (m, 1H), 7.42 (dd, 1H), 7.63-7.67 (m, 1H), 7.70-7.73 (m, 1H), 12.97 (br. s, 1H).

### Beispiel 179A

### (3R)-2-(4-Chlor-2-fluorphenyl)-4,4,4-trifluor-3-methylbutanoylchlorid (Diastereomerengemisch)

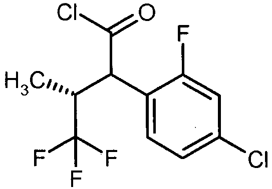

660 mg (2.32 mmol) (3*R*)-2-(4-Chlor-2-fluorphenyl)-4,4,4-trifluor-3-methylbutansäure (Diastereomerengemisch) wurden in 2 ml Dichlormethan gelöst. Nach Zugabe von einem kleinen Tropfen DMF wurde die Reaktionslösung auf -5°C bis 0°C gekühlt und tropfenweise mit 0.4 ml (4.64 mmol) Oxalylchlorid versetzt. Die Kühlung wurde entfernt, und die Reaktionsmischung wurde 1 h bei RT bis zum Ende der Gasentwicklung gerührt. Danach wurde der Ansatz im Vakuum eingeengt. Der Rückstand wurde zweimal in abs. Dichlormethan aufgenommen, jeweils wieder im Vakuum eingeengt und der Rückstand schließlich im Hochvakuum getrocknet. Es wurden 640 mg des Zielprodukts erhalten, das ohne weitere Reinigung direkt weiter umgesetzt wurde.

Die folgenden Beispiele wurden gemäß der *Allgemeinen Vorschrift 1* hergestellt (HATU-vermittelte Amidkupplung von 4,4,4-Trifluor-3-methyl-2-phenylbutansäure-Derivaten mit Anilinen in DMF unter Verwendung von Pyridin oder *N*,*N* Diisopropylethylamin als Base):

| **Beispiel** | **Name / Struktur / Edukte** | **Analytische Daten** |
|---|---|---|
| **180A** | (+)-*tert*.-Butyl-3-(4-chlor-3-{[(2*S*,3*R*)-2-(4-chlor-3-fluorphenyl)-4,4,4-trifluor-3-methylbutanoyl]-amino}phenyl)propanoat | LC-MS (Methode 6): Rₜ = 1.44 min; m/z = 520 (M-H)⁻. |
| | | |
| | 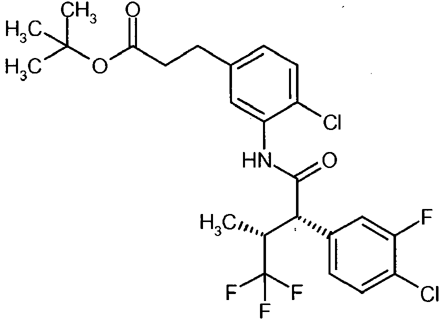 | ¹H-NMR (400 MHz, DMSO-*d₆*): δ [ppm] = 0.83 (d, 3H), 1.30 (s, 9H), 2.42-2.48 (m, 2H), 2.76 (t, 2H), 3.35-3.46 (m, 1H), 4.09-4.19 (m, 1H), 7.05 (dd, 1H), 7.26-7.41 (m, 3H), 7.49 (dd, 1H), 7.62 (t, 1H), 9.86 (s, 1H). |
| | | |
| | (aus *tert*.-Butyl-3-(3-amino-4-chlorphenyl)propanoat und (3*R*)-2-(4-Chlor-3-fluorphenyl)-4,4,4-trifluor-3-methylbutansäure (*Diastereomerengemisch*)) | [α]_{D}²⁰ = +66.9°, c = 0.46, Chloroform. |
| **181A** | (+)-Ethyl-(2*S*)-3-(4-chlor-3-{[(2*S*,3*R*)-2-(4-chlor-3-fluorphenyl)-4,4,4-trifluor-3-methylbutanoyl]-amino}phenyl)-2-methylpropanoat | LC-MS (Methode 6): Rₜ = 1.40 min; m/z = 508 (M+H)⁺. |
| | | |
| | 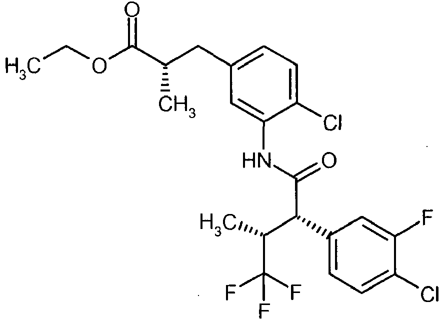 | ¹H-NMR (400 MHz, DMSO-*d₆*): δ [ppm] = 0.83 (d, 3H), 1.01-1.10 (m, 6H), 2.60-2.71 (m, 2H), 2.74-2.84 (m, 1H), 3.35-3.49 (m, 1H), 3.96 (q, 2H), 4.15 (d, 1H), 7.00 (dd, 1H), 7.24-7.39 (m, 3H), 7.49 (dd, 1H), 7.62 (t, 1H), 9.87 (s, 1H). |
| | | |
| | (aus (+)-(2*S*)-3-(3-Amino-4-chlorphenyl)-2-methyl-propansäureethylester und (3R)-2-(4-Chlor-3-fluor-phenyl)-4,4,4-trifluor-3-methylbutansäure (*Diastereomerengemisch*)) | [α]_{D}²⁰ = +98.6°, c = 0.45, Chloroform. |
| **182A** | (+)-Ethyl-(2*S*)-3-(4-chlor-3-{[(2*S*,3*R*)-2-(4-chlor-2-methylphenyl)-4,4,4-trifluor-3-methylbutanoyl]-amino}phenyl)-2-methylpropanoat | LC-MS (Methode 6): Rₜ = 1.46 min; m/z = 504 (M+H)⁺. |
| | | |
| | 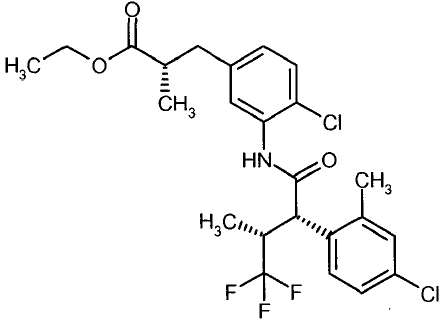 | ¹H-NMR (400 MHz, DMSO-*d₆*): δ [ppm] = 0.75 (d, 3H), 1.02-1.12 (m, 6H), 2.61-2.72 (m, 2H), 2.77-2.84 (m, 1H), 3.33-3.42 (m, 1H), 3.98 (q, 2H), 4.15 (d, 1H), 7.02 (dd, 1H), 7.24-7.30 (m, 2H), 7.32-7.38 (m, 2H), 7.52 (d, 1H), 9.88 (s, 1H). |
| | | |
| | (aus (+)-(2*S*)-3-(3-Amino-4-chlorphenyl)-2-methyl-propansäureethylester und (3*R*)-2-(4-Chlor-2-methyl-phenyl)-4,4,4-trifluor-3-methylbutansäure (*Diastereomerengemisch*)) | [α]_{D}²⁰ = +112.3°, c = 0.40, Chloroform. |
| **183A** | *tert*.-Butyl-3-(4-chlor-3-{[(2*S*,3*R*)-2-(4-chlor-3-methylphenyl)-4,4,4-trifluor-3-methylbutanoyl]-amino}phenyl)propanoat | LC-MS (Methode 4): Rₜ = 1.69 min; m/z = 516/518 (M-H)⁻. |
| | | |
| | 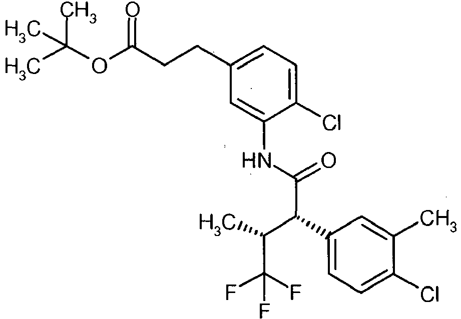 | ¹H-NMR (400 MHz, DMSO-*d₆*): δ [ppm] = 0.80 (d, 3H), 1.31 (s, 9H), 2.33 (s, 3H), 2.46 (t, 2H), 2.75 (t, 2H), 3.34-3.41 (m, 1H), 4.07 (d, 1H), 7.03 (dd, 1H), 7.27-7.45 (m, 5H), 9.80 (s, 1H). |
| | (aus *tert*.-Butyl-3-(3-amino-4-chlorphenyl)propanoat und (3*R*)-2-(4-Chlor-3-methylphenyl)-4,4,4-trifluor-3-methylbutansäure) | |
| **184A** | Ethyl-(2*S*)-3-(4-chlor-3-{[(2*S*,3*R*)-2-(4-chlor-3-methylphenyl)-4,4,4-trifluor-3-methylbutanoyl]-amino}phenyl)-2-methylpropanoat | LC-MS (Methode 4): Rₜ = 1.64 min; m/z = 502/504 (M-H)⁻. |
| | | |
| | 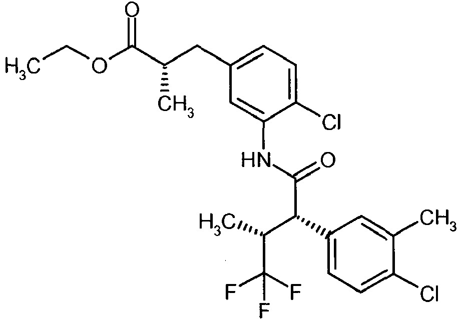 | ¹H-NMR (400 MHz, DMSO-*d₆*): δ [ppm] = 0.80 (d, 3H), 1.02-1.09 (m, 6H), 2.33 (s, 3H), 2.59-2.72 (m, 2H), 2.74-2.85 (m, 1H), 3.34-3.44 (m, 1H), 3.96 (q, 2H), 4.04-4.11 (m, 1H), 6.99 (dd, 1H), 7.26-7.38 (m, 3H), 7.39-7.44 (m, 2H), 9.80 (s, 1H). |
| | (aus (+)-(2*S*)-3-(3-Amino-4-chlorphenyl)-2-methyl-propansäureethylester und (3*R*)-2-(4-Chlor-3-methyl-phenyl)-4,4,4-trifluor-3-methylbutansäure) | |
| **185A** | Ethyl-(2*S*)-3-(4-chlor-3-{[(2*S*,3*R*)-2-(3,4-dichlophenyl)-4,4,4-trifluor-3-methylbutanoyl]-amino} phenyl)-2-methylpropanoat | LC-MS (Methode 6): Rₜ = 1.44 min; m/z = 524/526 (M+H)⁺. |
| | | |
| | 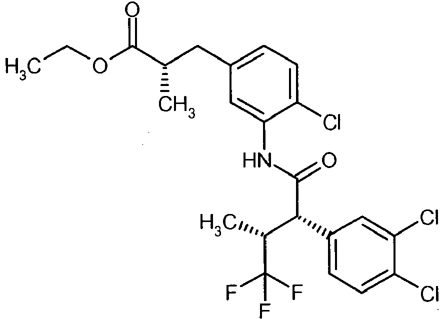 | ¹H-NMR (400 MHz, DMSO-*d₆*): δ [ppm] = 0.83 (d, 3H), 1.01-1.08 (m, 6H), 2.60-2.70 (m, 2H), 2.75-2.83 (m, 1H), 3.35-3.48 (m, 1H), 3.96 (q, 2H), 4.09-4.16 (m, 1H), 7.01 (dd, 1H), 7.30-7.38 (m, 2H), 7.45 (dd, 1H), 7.67 (d, 1H), 7.72 (d, 1H), 9.87 (s, 1H). |
| | (aus (+)-(2*S*)-3-(3-Amino-4-chlorphenyl)-2-methyl-propansäureethylester und (3*R*)-2-(3,4-Dichlor-phenyl)-4,4,4-trifluor-3-methylbutansäure) | |
| **186A** | *tert*.-Butyl-3-(4-chlor-3-{[(2*S*,3*R*)-2-(3,4-dichlorphenyl)-4,4,4-trifluor-3-methylbutanoyl]-amino}phenyl)propanoat | LC-MS (Methode 6): Rₜ = 1.48 min; m/z = 536/538 (M-H)⁻. |
| | 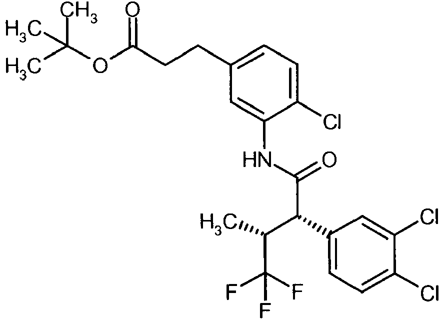 | |
| | | ¹H-NMR (400 MHz, DMSO-*d₆*): δ [ppm] = 0.83 (d, 5H), 1.30 (s, 9H), 2.42-2.48 (m, 2H), 2.72-2.80 (m, 2H), 3.34-3.48 (m, 1H), 4.07-4.17 (m, 1H), 7.05 (dd, 1H), 7.31-7.39 (m, 2H), 7.45 (dd, 1H), 7.67 (d, 1H), 7.72 (d, 1H), 9.87 (s, 1H). |
| | (aus *tert*.-Butyl-3-(3-amino-4-chlorphenyl)propanoat und (3*R*)-2-(3,4-Dichlorphenyl)-4,4,4-trifluor-3-methylbutansäure) | |
| **187A** | *tert*.-Butyl-3-(4-chlor-3-{[(2*S*,3*R*)-2-(4-chlorphenyl)-4,4,4-trifluor-3-methylbutanoyl]amino}-2-methyl-phenyl)-2-methylpropanoat (*Diastereomerengemisch*) | LC-MS (Methode 6): Rₜ = 1.45 min; m/z = 530/532 (M-H)⁻. |
| | | |
| | 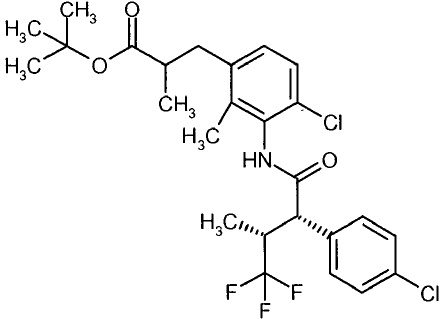 | ¹H-NMR (400 MHz, DMSO-*d₆*): δ [ppm] = 0.80 (d, 3H), 1.03 (br. s, ca. 3H), 1.29 (s, ca. 9H), 1.51 (br. s, ca. 1H), 1.56 (br. s, ca. 1H), 2.15 (br. s, 1H), 2.77 (br. s, 1H), 3.34-3.43 (m, 1H), 3.86-4.02 (m, 1H), 6.97-7.08 (m, 1H), 7.15 (br. s, 1H), 7.23 (br. s, 1H), 7.38-7.53 (m, 5H), 9.87 (br. s, 1H) *[Signale sind infolge Rotamere stark verbreitert*]*.* |
| | (aus (+/-)-*tert*.-Butyl-3-(3-amino-4-chlor-2-methylphenyl)-2-methylpropanoat und (+)-(2S,3R)-2-(4-Chlorphenyl)-4,4,4-trifluor-3-methylbutansäure) | |
| **188A** | Ethyl-3-(3-{[(2*S*,3*R*)-2-(4-chlorphenyl)-4,4,4-trifluor-3-methylbutanoyl]amino}-4-fluorphenyl)-4,4,4-trifluorbutanoat | LC-MS (Methode 4): Rₜ = 1.65 min; m/z = 526 (M-H)⁻. |
| | | |
| | 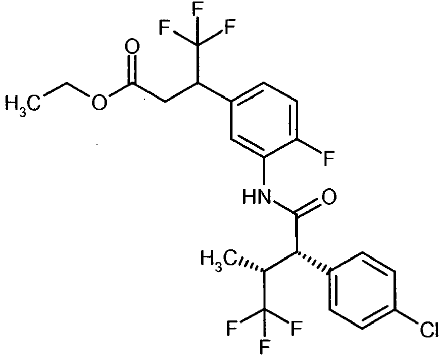 | ¹H-NMR (400 MHz, DMSO-*d₆*): beide Diastereomere δ [ppm] = 0.79 (d, 3H), 1.03 (t, 3H), 2.91 (dd, 1H), 3.03 (dd, 1H), 3.34-3.46 (m, 1H), 3.89-4.00 (m, 2H), 4.04-4.18 (m, 2H), 7.15-7.32 (m, 2H), 7.42-7.55 (m, 4H), 7.85-8.06 (m, 1H), 10.17 (s, 1H). |
| | (aus (+/-)-Ethyl-3-(3-amino-4-fluorphenyl)-4,4,4-trifluorbutanoat und (+)-(2*S*,3*R*)-2-(4-Chlorphenyl)-4,4,4-trifluor-3-methylbutansäure) | |
| **189A** | *tert*.-Butyl-2-(4-chlor-3-{[(2*S*,3*R*)-2-(4-chlorphenyl)-4,4,4-trifluor-3-methylbutanoyl]amino}benzyl)-2-methylbutanoat (*Diastereomerengemisch*) | LC-MS (Methode 6): Rₜ = 1.64 min; m/z = 544/546 (M-H)⁻. |
| | | |
| | 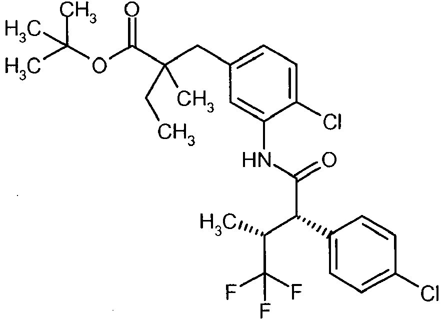 | ¹H-NMR (400 MHz, DMSO-*d₆*): beide Diastereomere δ [ppm] = 0.74-0.84 (m, 6H), 0.88/0.91 (2d, zus. 3H), 1.22/ 1.32 (2s, zus. 9H), 1.32-1.40 (m, 1H), 1.58-1.68 (m, 1H), 2.57 (d, 1H), 2.84/2.85 (2d, zus. 1H), 3.35-3.43 (m, 1H), 4.03-4.08/4.10 (2d, zus. 1H), 6.95 (dd, 1H), 7.26-7.38 (m, 2H), 7.39-7.52 (m, 4H), 9.81/9.83 (2s, zus. 1H). |
| | (aus (+/-)-*tert*.-Butyl-2-(3-amino-4-chlorbenzyl)-2-methylbutanoat und (+)-(2*S*,3*R*)-2-(4-Chlorphenyl)-4,4,4-trifluor-3-methylbutansäure) | |
| **190A** | *tert*.-Butyl-2-(4-chlor-3-{[(2*S*,3*R*)-2-(4-chlorphenyl)-4,4,4-trifluor-3-methylbutanoyl]amino} benzyl)-2-methylbutanoat (*Diastereomer B)* | LC-MS (Methode 6): Rₜ = 1.57 min; m/z = 544/546 (M-H)⁻. |
| | | |
| | 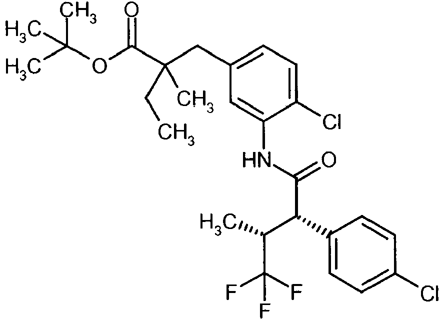 | ¹H-NMR (400 MHz, DMSO-*d₆*): δ [ppm] = 0.75-0.82 (m, 6H), 0.88 (s, 3H), 1.22 (s, 9H), 1.27-1.38 (m, 1H), 1.56-1.70 (m, 1H), 2.54 (d, ca. 1H, verdeckt), 2.84 (d, 1H), 3.35-3.43 (m, 1H), 4.01-4.14 (m, 1H), 6.95 (d, 1H), 7.17-7.32 (m, 1H), 7.35 (d, 1H), 7.41-7.57 (m, 4H), 9.83 (s, 1H). |
| | (aus (+)-*tert*.-Butyl-2-(3-amino-4-chlorbenzyl)-2-methylbutanoat (*Enantiomer* 2) und (+)-(2S,3R)-2-(4-Chlorphenyl)-4,4,4-trifluor-3-methylbutansäure) | |
| | | |
| | | [a]_{D}²⁰ = +68.0°, c = 0.280, Chloroform. |
| **191A** | *tert.*-Butyl-3-(4-chlor-3-{[(2*S*,3*R*)-2-(4-chlorphenyl)-4,4,4-trifluor-3-methylbutanoyl]amino}phenyl)-4,4,4-trifluorbutanoat (*Diastereomerengemisch*) | LC-MS (Methode 6): Rₜ = 1.50 min; m/z = 570/572 (M-H)⁻. |
| | | |
| | 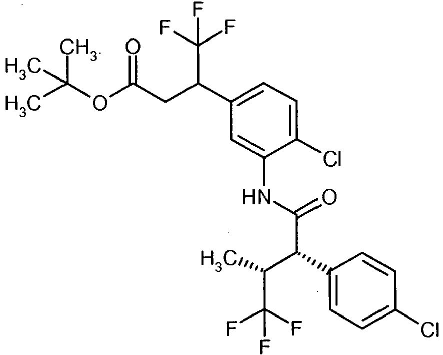 | ¹H-NMR (400 MHz, DMSO-*d₆*): beide Diastereomere δ [ppm] = 0.80 (d, 3H), 1.21 (s, 9H), 2.74-2.81 (m, 1H), 2.88-2.99 (m, 1H), 3.34-3.46 (m, 1H), 3.95-4.10 (m, 1H), 4.11-4.19 (m, 1H), 7.25 (dd, 1H), 7.40-7.54 (m, 5H), 7.58-7.72 (m, 1H), 9.93/9.94 (2s, zus. 1H). |
| | (aus (+/-)-*tert*.-Butyl-3-(3-amino-4-chlorphenyl)-4,4,4-trifluorbutanoat und (+)-(2*S*,3*R*)-2-(4-Chlorphenyl)-4,4,4-trifluor-3-methylbutansäure) | |
| **192A** | *tert*.-Butyl-3-(4-chlor-3-{[(2*S*,3*R*)-2-(4-chlorphenyl)-. 4,4,4-trifluor-3-methylbutanoyl]amino} phenyl)-2-cyclobutylpropanoat (*Diastereomerengemisch*) | LG-MS (Methode 4): Rₜ = 1.97 min; m/z = 556 (M-H)⁻. |
| | | |
| | 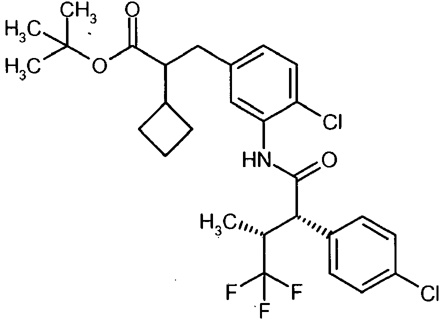 | ¹H-NMR (400 MHz, DMSO-*d₆*): beide Diastereomere δ [ppm] = 0.79 (d, 3H), 1.18/1.22 (2s, zus. 9H), 1.66-1.85 (m, 4H), 1.86-2.02 (m, 2H), 2.28-2.45 (m, 2H), 2.55-2.64 (m, 1H), 3.34-3.42 (m, 1H), 4.11/4.12 (2d, zus. 1H), 6.97/6.99 (2d, zus. 1H), 7.30-7.37 (m, 2H), 7.40-7.51 (m, 4H), 9.80/9.81 (2d, zus. 1H). |
| | (aus (+/-)-*tert*.-Butyl-3-(3-amino-4-chlorphenyl)-2-cyclobutylpropanoat und (+)-(2*S*,3*R*)-2-(4-Chlorphenyl)-4,4,4-trifluor-3-methylbutansäure) | |
| **193A** | (+)-*tert*.-Butyl-3-(4-chlor-3-{[(2*S*,3*R*)-2-(4-chlorphenyl)-4,4,4-trifluor-3-methylbutanoyl]amino}-phenyl)-2-cyclobutylpropanoat (*Diastereomer A)* | LC-MS (Methode 6): Rₜ = 1.67 min; m/z = 556 (M-H)⁻. |
| | | |
| | 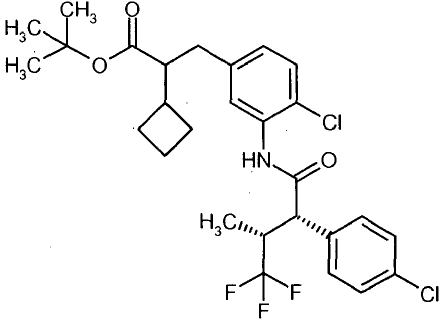 | ¹H-NMR (400 MHz, DMSO-*d₆*): δ [ppm] = 0.79 (d, 3H), 1.22 (s, 9H), 1.68-1.82 (m, 4H), 1.86-1.93 (m, 1H), 1.94-2.03 (m, 1H), 2.31-2.47 (m, 2H), 2.56-2.63 (m, 2H), 3.36-3.43 (m, 1H), 4.12 (d, 1H), 6.98 (dd, 1H), 7.31-7.37 (m, 2H), 7.41-7.51 (m, 4H), 9.81 (s, 1H). |
| | (aus *tert*.-Butyl-3-(3-amino-4-chlorphenyl)-2-cyclo-butylpropanoat (*Enantiomer 1*) und (+)-(2*S*,3*R*)-2-(4-Chlorphenyl)-4,4,4-trifluor-3-methylbutansäure) | |
| | | [a]_{D}²⁰ = +51.3°, c = 0.445, Chloroform. |
| **194A** | (+)-*tert*.-Butyl-3-(4-chlor-3-{[(2*S*,3*R*)-2-(4-chlor-phenyl)-4,4,4-trifluor-3-methylbutanoyl]amino}-phenyl)-2-cyclobutylpropanoat (*Diastereomer B*) | LC-MS (Methode 6): Rₜ = 1.58 min; m/z = 556 (M-H)⁻. |
| | | |
| | 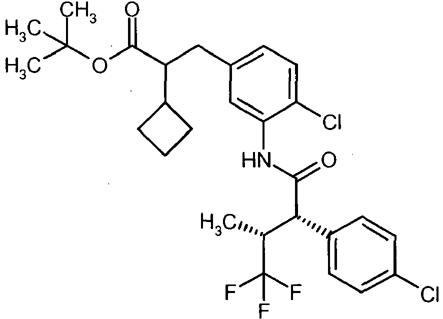 | ¹H-NMR (400 MHz, DMSO-*d₆*): δ [ppm] = 0.79 (d, 3H), 1.18 (s, 9H), 1.67-1.83 (m, 4H), 1.84-1.93 (m, 1H), 1.94-2.02 (m, 1H), 2.31-2.44 (m, 2H), 2.57-2.64 (m, 1H), 3.35-3.42 (m, 1H), 4.08-4.14 (m, 1H), 6.98 (dd, 1H), 7.29-7.37 (m, 2H), 7.42-7.49 (m, 4H), 9.81 (s, 1H). |
| | (aus *tert*.-Butyl-3-(3-amino-4-chlorphenyl)-2-cyclo-butylpropanoat (*Enantiomer 2*) und (+)-(2*S*,3*R*)-2-(4-Chlorphenyl)-4,4,4-trifluor-3-methylbutansäure) | |
| | | [α]_{D}²⁰ = +81.8°, c = 0.475, Chloroform. |
| **195A** | *tert*.-Butyl-3-(4-chlor-3-{[(2*S*,3*R*)-2-(4-chlorphenyl)-4,4,4-trifluor-3-methylbutanoyl]amino}phenyl)-2-cyclopropylpropanoat (*Diastereomerengemisch*) | LC-MS (Methode 4): Rₜ = 1.80 min; m/z = 542 (M-H)⁻. |
| | | |
| | 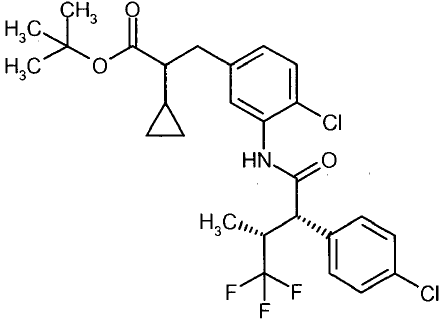 | ¹H-NMR (400 MHz, DMSO-*d₆*): beide Diastereomere δ [ppm] = 0.12-0.26 (m, 2H), 0.43 (q, 2H), 0.79 (d, 3H), 0.81-0.90 (m, 1H), 1.20/1.24 (2s, zus. 9H), 1.67-1.81 (m, 1H), 2.76-2.83 (m, 2H), 3.36-3.43 (m, 1H), 4.11/4.12 (2d, zus. 1H), 7.01 (dd, 1H), 7.30-7.39 (m, 2H), 7.41-7.51 (m, 4H), 9.78-9.85 (m, 1H). |
| | (aus (+/-)-*tert*.-Butyl-3-(3-amino-4-chlorphenyl)-2-cyclopropylpropanoat und (+)-(2*S*,3*R*)-2-(4-Chlorphenyl)-4,4,4-trifluor-3-methylbutansäure) | |

### Beispiel 196A

### (+)-Ethyl-(2S)-3-(4-chlor-3-{[(2S,3R)-2-(4-chlor-2-fluorphenyl)-4,4,4-trifluor-3-methylbutanoyl]-amino}phenyl)-2-methylpropanoat

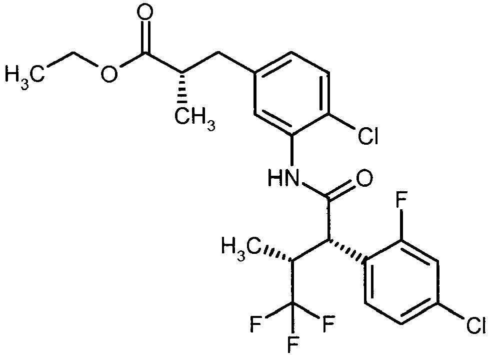

280.7 mg (1.16 mmol) (+)-(2*S*)-3-(3-Amino-4-chlorphenyl)-2-methylpropansäureethylester wurden in 1.5 ml abs. THF gelöst, mit 0.26 ml (1.48 mmol) *N*,*N-*Diisopropylethylamin versetzt und nach Abkühlen auf -10°C tropfenweise mit einer Lösung von 320 mg (roh, ca. 1.06 mmol) *in situ* hergestelltem (3*R*)-2-(4-Chlor-2-fluorphenyl)-4,4,4-trifluor-3-methylbutanoylchlorid in 0.5 ml abs. THF versetzt. Die Reaktionsmischung wurde nach Ende der Zugabe 30 min lang zwischen -10°C und 0°C gerührt. Dann wurde nach Zugabe von einigen Tropfen Wasser mit Dichlormethan verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde zunächst durch präparative RP-HPLC (Eluent Methanol/Wasser), dann durch Chromatographie an Silicagel (Laufmittel Cyxlohexan/Ethylacetat 40:1) gereinigt. Erhalten wurden 144 mg der Zielverbindung (26.9% d. Th.).

LC-MS (Methode 6): Rₜ = 1.42 min; m/z = 508 (M+H)⁺.

¹H-NMR (400 MHz, DMSO-*d₆*): δ [ppm] = 0.86 (d, 3H), 1.02-1.12 (m, 6H), 2.63-2.72 (m, 2H), 2.76-2.86 (m, 1H), 3.34-3.44 (m, 1H), 3.93-4.02 (m, 2H), 4.36 (d, 1H), 7.03 (dd, 1H), 7.25-7.29 (m, 1H), 7.32-7.38 (m, 2H), 7.51 (dd, 1H), 7.61 (t, 1H), 10.02 (s, 1H).

[α]_{D}²⁰ = +90°, c = 0.30, Chloroform.

### Beispiel 197A

### Methyl-3-(4-chlor-3-{[(2S,3R)-2-(4-chlorphenyl)-4,4,4-trifluor-3-methylbutanoyl]amino} -2-methyl-phenyl)propanoat

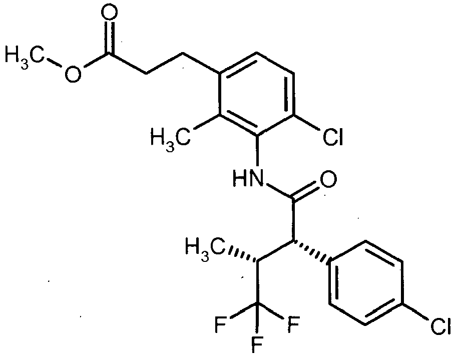

265 mg (1.16 mmol) Methyl-3-(3-amino-4-chlor-2-methylphenyl)propanoat wurden in 1.5 ml abs. THF gelöst, mit 0.28 ml (1.63 mmol) *N*,*N-*Diisopropylethylamin versetzt und nach Abkühlen auf -10°C tropfenweise mit einer Lösung von 398 mg (roh, ca. 1.40 mmol) *in situ* hergestelltem (2*S*,3*R*)-2-(4-Chlorphenyl)-4,4,4-trifluor-3-methylbutanoylchlorid in 0.5 ml abs. THF versetzt. Die Reaktionsmischung wurde nach Ende der Zugabe über 1 h von -10°C auf RT erwärmt und dann mit Ethylacetat verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde durch präparative RP-HPLC gereinigt (Eluent Methanol/Wasser). Erhalten wurden 485 mg der Zielverbindung (87.5% d. Th.).

LC-MS (Methode 6): Rₜ = 1.25 min; m/z = 476 (M+H)⁺.

### Beispiel 198A

### (+)-tert.-Butyl-(2R)-3-(4-chlor-3-{[(2S,3R)-2-(4-chlorphenyl)-4,4,4-trifluor-3-methylbutanoyl]-amino}-2-methylphenyl)-2-methylpropanoat

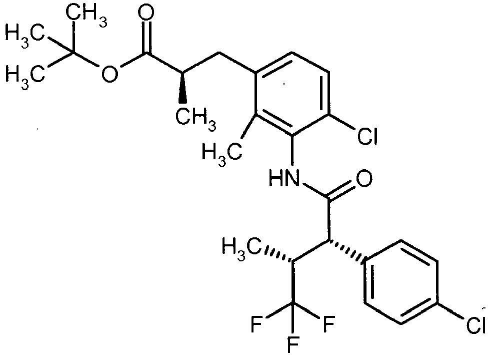

200 mg (0.705 mmol) (-)-*tert*.-Butyl-(2*R*)-3-(3-amino-4-chlor-2-methylphenyl)-2-methylpropanoat wurden in 1 ml abs. THF gelöst, mit 0.17 ml (0.987 mmol) *N*,*N*-Diisopropylethylamin versetzt und nach Abkühlen auf -10°C tropfenweise mit einer Lösung von 241 mg (roh, ca. 0.846 mmol) *in situ* hergestelltem (2*S*,3*R*)-2-(4-Chlorphenyl)-4,4,4-trifluor-3-methylbutanoylchlorid in 0.2 ml abs. THF versetzt. Die Reaktionsmischung wurde nach Ende der Zugabe über 2 h von - -10°C auf RT erwärmt und dann auf Wasser gegeben. Die wässrige Phase wurde dreimal mit Ethylacetat extrahiert, und die vereinigten organischen Phasen wurden mit 1 N Salzsäure und ges. Natriumchlorid-Lösung gewaschen, über Magnesiumsulfat getrocknet, im Vakuum eingeengt und der Rückstand im Hochvakuum getrocknet. Erhalten wurden 282 mg der Zielverbindung (75.2% d. Th.).

LC-MS (Methode 6): Rₜ = 1.45 min; m/z = 530 (M-H)⁻.

¹H-NMR (400 MHz, DMSO-*d₆*): δ [ppm] = 0.80 (d, 3H), 1.03 (br. s, 3H), 1.30 (s, 9H), 1.50 (br. s, 1H), 2.15 (br. s, 1H), 2.42 (br. s, 1H), 2.69-2.92 (m, 1H), 3.34-3.45 (m, 1H), 3.94 (d, 1H), 7.03 (d, 1H), 7.23 (br. s, 1H), 7.45 (s, 4H), 9.83/9.91 (2 br. s, zus. 1H) [*signale sind infolge Rotamere stark verbreitert*]*.*

[α]_{D}²⁰ = +68.9°, c = 0.50, Chloroform.

Auf analoge Weise wurde das folgende Beispiel erhalten:

### Beispiel 199A

### (+)-tert.-Butyl-(2S)-3-(4-chlor-3-{[(2S,3R)-2-(4-chlorphenyl)-4,4,4-trifluor-3-methylbutanoyl]-amino}-2-methylphenyl)-2-methylpropanoat

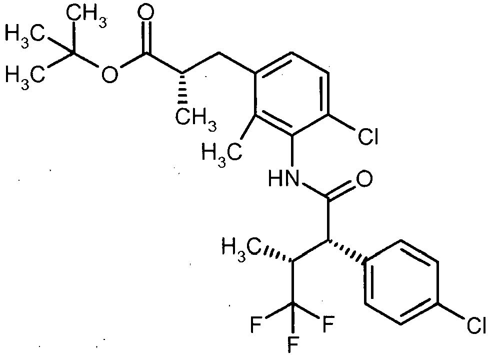

Ausgehend von 200 mg (+)-*tert*.-Butyl-(2*S*)-3-(3-amino-4-chlor-2-methylphenyl)-2-methylpropanoat und 241 mg frisch hergestelltem (2*S*,3*R*)-2-(4-Chlorphenyl)-4,4,4-trifluor-3-methylbutanoylchlorid wurden 287 mg des Zielprodukts erhalten (75.2% d. Th.).

LC-MS (Methode 6): Rₜ = 1.51 min; m/z = 530 (M-H)⁻.

¹H-NMR (400 MHz, DMSO-*d₆*): δ [ppm] = 0.80 (d, 3H), 1.04 (br. s, 3H), 1.29 (s, 9H), 1.51 (br. s, 1H), 2.15 (br. s, 1H), 2.56-2.68 (m, 1H), 2.79 (br. s, 1H), 3.34-3.45 (m, 1H), 3.94 (br. d, 1H), 7.03 (d, 1H), 7.15 (br. s, 1H), 7.23 (br. s, 1H), 7.45 (s, 4H), 9.87 (br. s, 1H) [*signale sind infolge Rotamere stark verbreitert*]*.*

[a]_{D}²⁰ = +116.1°, c = 0.520, Chloroform.

### Beispiel 200A

### (+)-tert.-Butyl-2-(4-chlor-3-{[(2S,3R)-2-(4-chlorphenyl)-4,4,4-trifluor-3-methylbutanoyl]amino}-benzyl)-2-methylbutanoat (Diastereomer A)

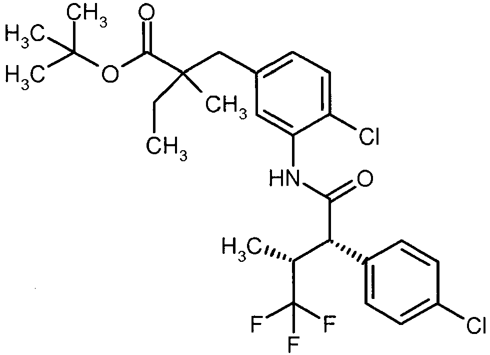

225 mg (0.756 mmol) (-)-*tert*.-Butyl-2-(3-amino-4-chlorbenzyl)-2-methylbutanoat (*Enantiomer 1)* und 231 mg (0.907 mmol) (+)-(2*S*,3*R*)-2-(4-Chlorphenyl)-4,4,4-trifluor-3-methylbutansäure wurden in 0.9 ml Pyridin und 2.7 ml DMF gelöst und bei RT mit 345 mg (0.907 mmol) HATU versetzt. Die Reaktionsmischung wurde über Nacht bei 45°C gerührt, bevor weitere 0.5 eq. (+)-(2*S*,3*R*)-2-(4-Chlorphenyl)-4,4,4-trifluor-3-methylbutansäure und 0.6 eq. HATU zugesetzt wurden. Die Reaktionsmischung wurde nochmals 3 h bei 45°C gerührt und dann nach Abkühlen mit Ethylacetat verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde durch präparative RP-HPLC (Eluent Acetonitril/Wasser) und nachfolgende Chromatographie an Silicagel (Lauf mittel Cyclohexan/Ethylacetat 40:1) gereinigt. Es wurden 177 mg des Zielprodukts erhalten (35.6% d. Th.).

LC-MS (Methode 4): Rₜ = 1.97 min; m/z = 544 (M-H)⁻.

¹H-NMR (400 MHz, DMSO-*d₆*): δ [ppm] = 0.76-0.83 (m, 6H), 0.91 (s, 3H), 1.31 (s, 9H), 1.33-1.40 (m, 1H), 1.57-1.67 (m, 1H), 2.57 (d, 1H), 2.85 (d, 1H), 3.35-3.43 (m, 1H), 4.07-4.13 (m, 1H), 6.95 (dd, 1H), 7.30-7.36 (m, 2H), 7.41-7.48 (m, 4H), 9.82 (s, 1H).

[a]_{D}20 = +63.2°, c = 0.365, Chloroform.

### Beispiel 201A

### Methyl-3-(4-chlor-3-{[(2S,3R)-2-(4-chlorphenyl)-4,4,4-trifluor-3-methylbutanoyl]amino}phenyl)-hexanoat (Diastereomerengemisch)

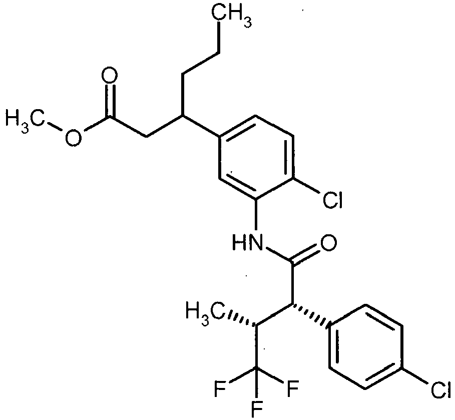

1.45 g (5.67 mmol) (+/-)-Methyl-3-(3-amino-4-chlorphenyl)hexanoat und 1.81 g (6.80 mmol) (+)-(2*S*,3*R*)-2-(4-Chlorphenyl)-4,4,4-trifluor-3-methylbutansäure wurden in 5.0 ml Pyridin und 10.0 ml DMF gelöst und bei RT mit 2.80 g (7.37 mmol) HATU versetzt. Die Reaktionsmischung wurde über Nacht bei RT gerührt und dann mit Ethylacetat verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde durch Chromatographie an Silicagel gereinigt (Laufmittel zunächst Cyclohexan, dann Cyclohexan/Ethylacetat 50: 1). Es wurden in zwei Fraktionen insgesamt 2.02 g des Zielprodukts erhalten (70.6% d. Th.).

LC-MS (Methode 6): Rₜ = 1.43 min; m/z = 504 (M+H)⁺.

¹H-NMR (400 MHz, DMSO-*d₆*): beide Diastereomere δ [ppm] = 0.74-0.85 (m, 6H), 0.98-1.16 (m, 2H), 1.42-1.61 (m, 2H), 2.49 (dd, ca. 1H, verdeckt), 2.64 (dd, 1H), 2.84-3.02 (m, 1H), 3.37-3.42 (m, 1H), 3.47/3.48 (2s, zus. 3H), 4.12 (d, 1H), 7.05 (dd, 1H), 7.31-7.38 (m, 2H), 7.41-7.55 (m, 4H), 9.83 (s, 1H).

### Beispiel 202A

### Methyl-3-(4-chlor-3-{[(2S,3R)-2-(4-chlorphenyl)-4,4,4-trifluor-3-methylbutanoyl]amino}phenyl)-pentanoat (Diastereomerengemisch)

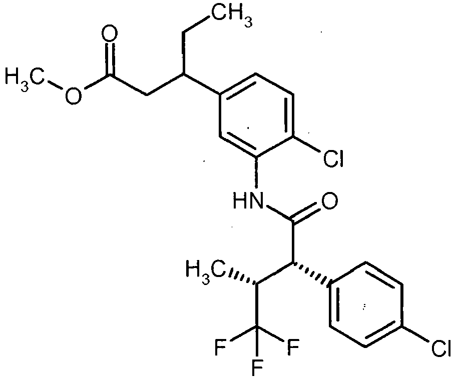

500 mg (2.07 mmol) (+/-)-Methyl-3-(3-amino-4-chlorphenyl)pentanoat und 668.9 mg (2.48 mmol) (+)-(2*S*,3*R*)-2-(4-Chlorphenyl)-4,4,4-trifluor-3-methylbutansäure wurden in 1.7 ml Pyridin und 3.3 ml DMF gelöst und bei RT mit 1.02 g (2.69 mmol) HATU versetzt. Die Reaktionsmischung wurde über Nacht bei RT gerührt und dann mit Ethylacetat verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde durch Chromatographie an Silicagel gereinigt (Laufmittel zunächst Cyclohexan, dann Cyclohexan/Ethylacetat 50:1). Es wurden 675 mg des Zielprodukts erhalten (66.6% d. Th.).

LC-MS (Methode 6): Rₜ = 1.39 min; m/z = 490 (M-H)⁻.

¹H-NMR (400 MHz, DMSO-*d₆*): beide Diastereomere δ [ppm] = 0.65-0.74 (m, 3H), 0.80 (d, 3H), 1.43-1.67 (m, 2H), 2.49 (dd, ca. 1H, verdeckt), 2.65 (dd, 1H), 2.80-2.92 (m, 1H), 3.35-3.43 (m, 1H), 3.47/3.48 (2s, zus. 3H), 4.13 (d, 1H), 7.05 (dd, 1H), 7.36 (dd, 2H), 7.43-7.51 (m, 4H), 9.84 (s, 1H).

### Beispiel 203A

### (+)-tert.-Butyl-(3S)-3-(4-chlor-3-{[(2S,3R)-2-(4-chlorphenyl)-4,4,4-trifluor-3-methylbutanoyl]-amino}phenyl)-4,4,4-trifluorbutanoat

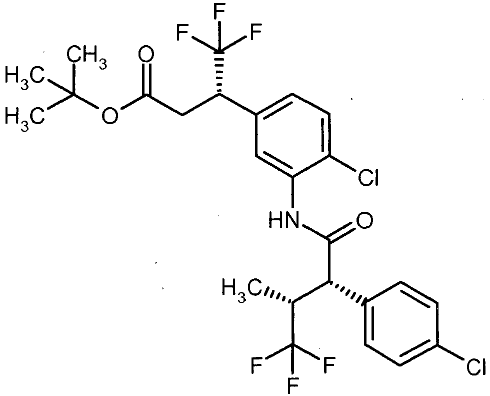

2.0 g (6.18 mmol) (+)-*tert*.-Butyl-(3*S*)-3-(3-amino-4-chlorphenyl)-4,4,4-trifluorbutanoat und 1.98 g (7.41 mmol) (+)-(2*S*,3*R*)-2-(4-Chlorphenyl)-4,4,4-trifluor-3-methylbutansäure wurden in 5.0 ml Pyridin und 10.0 ml DMF gelöst und bei RT mit 3.05 g (8.03 mmol) HATU versetzt. Die Reaktionsmischung wurde über Nacht bei RT gerührt, bevor weitere 1.98 g (7.41 mmol) (+)-(2*S*,3*R*)-2-(4-Chlorphenyl)-4,4,4-trifluor-3-methylbutansäure und 3.05 g (8.03 mmol) HATU zugesetzt wurden. Die Reaktionsmischung wurde erneut für 8 h bei 40°C gerührt und dann nach Abkühlen mit Ethylacetat verdünnt. Das Gemisch wurde mit 1 N Salzsäure und ges. Natriumchlorid-Lösung gewaschen, über Magnesiumsulfat getrocknet und im Vakuum eingeengt. Das Rohprodukt wurde durch Chromatographie an Silicagel aufgereinigt (Laufmittel zunächst Cyclohexan, dann Cyclohexan/Ethylacetat 50:1). Das so erhaltene Produkt (2.7 g) wurde durch erneute Chromatographie an Silicagel (Laufmittel Cyclohexan/Ethylacetat 100:1) nochmals nachgereinigt. Es wurden 1.80 g des Zielprodukts erhalten (50.9% d. Th.).

LC-MS (Methode 4): Rₜ = 1.74 min; m/z = 570 (M-H)⁻.

¹H-NMR (400 MHz, DMSO-*d₆*): δ [ppm] = 0.80 (d, 3H), 1.21 (s, 9H), 2.77 (dd, 1H), 2.94 (dd, 1H), 3.36-3.46 (m, 1H), 3.99-4.09 (m, 1H), 4.15 (d, 1H), 7.17-7.29 (m, 1H), 7.42-7.52 (m, 5H), 7.59-7.65 (m, 1H), 9.94 (s, 1H).

[a]_{D}²⁰ = +84.0°, c = 0.48, Chloroform.

Auf analoge Weise wurde das folgende Beispiel hergestellt:

### Beispiel 204A

### (+)-tert.-Butyl-(3R)-3-(4-chlor-3-{[(2S,3R)-2-(4-chlorphenyl)-4,4,4-trifluor-3-methylbutanoyl]-amino}phenyl)-4,4,4-trifluorbutanoat

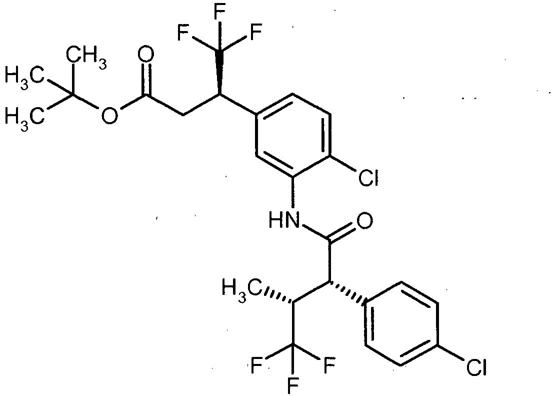

Ausgehend von 1.0 g (3.09 mmol) (-)-*tert*.-Butyl-(3*R*)-3-(3-amino-4-chlorphenyl)-4,4,4-trifluorbutanoat und 988 mg (3.71 mmol) (+)-(2*S*,3*R*)-2-(4-Chlorphenyl)-4,4,4-trifluor-3-methylbutansäure wurden 1.2 g (68% d. Th.) des Zielprodukts erhalten.

LC-MS (Methode 4): Rₜ = 1.75 min; m/z = 570 (M-H)⁻.

¹H-NMR (400 MHz, DMSO-*d₆*): δ [ppm] = 0.80 (d, 3H), 1.21 (s, 9H), 2.78 (dd, 1H), 2.93 (dd, 1H), 3.35-3.47 (m, 1H), 3.99-4.10 (m, 1H), 4.15 (d, 1H), 7.19-7.28 (m, 1H), 7.40-7.52 (m, 5H), 7.60-7.66 (m, 1H), 9.93 (s, 1H).

[a]_{D}²⁰ = +42.7°, c = 0.48, Chloroform.

### Beispiel 205A

### tert.-Butyl-3-(3-amino-2-methylphenyl)propanoat

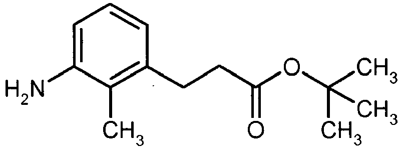

Unter Argon wurden 201 ml (1.39 mol) *tert*.-Butyl-prop-2-enoat zu einer Lösung von 100 g (463 mmol) 1-Brom-2-methyl-3-nitrobenzol, 322 ml (2.31 mol) Triethylamin, 28.18 g (92.58 mmol) Tri-2-tolylphosphin und 10.39 g (46.29 mmol) Palladium(II)acetat in 2 Liter DMF getropft und die Mischung anschließend 36 h bei 125°C gerührt. Nach Abkühlen auf Raumtemperatur wurde das Reaktionsgemisch mit gesättigter wässriger Ammoniumchlorid-Lösung verrührt und die organische Phase abgetrennt. Die wässrige Phase wurde dreimal mit *tert*.-Butylmethylether extrahiert, und die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 9:1). Es wurden 89 g (338 mmol, 73% d. Th.) des Zwischenprodukts *tert*.-Butyl-(2*E*)-3-(2-methyl-3-nitrophenyl)prop-2-enoat als farbloser Feststoff erhalten.

88 g (334 mmol) dieses Feststoffs wurden in 2 Liter Ethanol gelöst, bei Raumtemperatur mit 7 g Palladium auf Kohle (10%) versetzt und 18 h unter Normaldruck hydriert. Nach vollständiger Umsetzung wurde die Reaktionslösung über Kieselgur filtriert und das erhaltene Filtrat im Vakuum eingeengt. Es wurden 61.3 g (260.5 mmol, 78% d. Th.) der Titelverbindung als farbloser Feststoff erhalten.

LC-MS (Methode 2): Rₜ = 1.84 min; m/z = 236 (M+H)⁺.

¹H-NMR (400 MHz, DMSO-d₆, δ/ppm): 6.77 (1H, t), 6.47 (1H, d), 6.36 (1H, d), 4.72 (2H, s), 2.14 (2H, t), 2.36 (2H, t), 1.95 (3H, s), 1.39 (9H, s).

### Beispiel 206A

### tert.-Butyl-3-(3-brom-4-chlorphenyl)-2,2-dimethylpropanoat

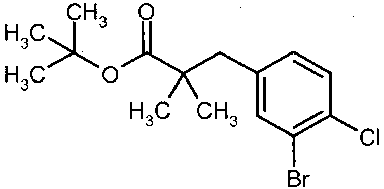

4.0 ml (28.8 mmol) Diisopropylamin wurden unter Argon in 50 ml trockenem THF gelöst und auf -30°C abgekühlt. Es wurden 11.5 ml (28.8 mmol) *n*-Butyllithium-Lösung (2.5 M in Hexan) zugetropft. Das resultierende Gemisch wurde auf 0°C erwärmt und dann auf -70°C abgekühlt. Anschließend wurde mit einer Lösung von 2.77 g (19.2 mmol) *tert*.-Butyl-2-methylpropanoat in 20 ml THF versetzt, wobei die Temperatur unterhalb von -60°C gehalten wurde. Nach 4 h Rühren bei -60°C wurde eine Lösung von 6.0 g (21.1 mmol) 2-Brom-4-(brommethyl)-1-chlorbenzol in 30 ml THF zugegeben, wobei die Reaktionstemperatur wiederum unterhalb von -60°C gehalten wurde. Das Reaktionsgemisch wurde über Nacht unter langsamer Erwärmung auf RT gerührt und dann mit gesättigter wässriger Ammoniumchlorid-Lösung und Ethylacetat versetzt. Nach Phasentrennung wurde die wässrige Phase zweimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 10:1 → 4:1). Es wurden 5.6 g (84% d. Th.) der Titelverbindung erhalten.

GC-MS (Methode 1): Rₜ = 6.16 min; m/z = 290/292 (M-C₄H₈)⁺.

### Beispiel 207A

### tert.-Butyl-3-[3-(benzylamino)-4-chlorphenyl]-2,2-dimethylpropanoat

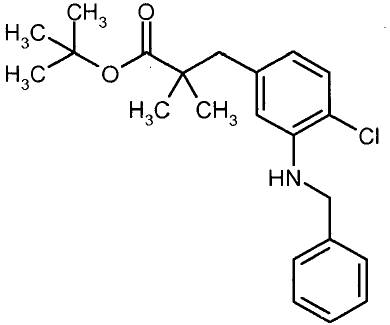

Unter Argon wurden in einem trockenen Kolben 1.73 g (17.95 mmol) Natrium-*tert*.-butylat eingewogen und mit 40 ml abs. Toluol versetzt. Nacheinander wurden 5.2 g (14.96 mmol) *tert*.-Butyl-3-(3-brom-4-chlorphenyl)-2,2-dimethylpropanoat, 1.96 ml (17.95 mmol) Benzylamin, 685 mg (0.75 mmol) Tris(dibenzylidenaceton)dipalladium sowie 373 mg (0.60 mmol) (+/-)-2,2'-Bis(diphenyl-phosphino)-1,1'-binaphthyl hinzugefügt. Die Reaktionsmischung wurde für 2.0 h bei 110°C gerührt, anschließend auf RT abgekühlt und über Nacht bei dieser Temperatur nachgerührt. Das Reaktionsgemisch wurde dann mit gesättigter wässriger Ammoniumchlorid-Lösung und Ethylacetat versetzt und über Kieselgur abgesaugt. Nach Phasentrennung wurde die organische Phase mit gesättigter Ammoniumchlorid-Lösung und gesättigter Natriumchlorid-Lösung gewaschen, über Magnesiumsulfat getrocknet und im Vakuum eingeengt. Das Rohprodukt wurde durch präparative HPLC gereinigt (Eluent Acetonitril/Wasser). Es wurden 2.78 g der Titelverbindung erhalten (50% d. Th.).

LC-MS (Methode 6): Rₜ = 1.53 min; m/z = 374/376 (M+H)⁺.

### Beispiel 208A

### tert.-Butyl-3-(3-amino-4-chlorphenyl)-2,2-dimethylpropanoat

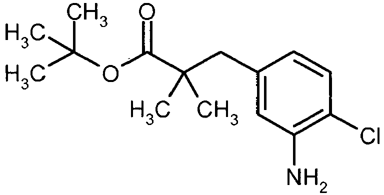

2.7 g (ca. 7.22 mmol) *tert*.-Butyl-3-[3-(benzylamino)-4-chlorphenyl]-2,2-dimethylpropanoat wurden in 150 ml Ethylacetat gelöst und mit 100 mg Palladium auf Kohle (10%) versetzt. Die Reaktionsmischung wurde bei RT über Nacht unter einer Wasserstoffatmosphäre bei Normaldruck gerührt. Das Gemisch wurde dann über Kieselgur abgesaugt, der Rückstand gründlich mit Ethylacetat gewaschen und das vereinigte Filtrat eingeengt. Das Rohprodukt wurde durch Chromatographie an Silicagel gereinigt (Laufmittel Cyclohexan/Ethylacetat 10:1 → 7:1). Es wurden 1.49 g (72.7% d. Th.) der Zielverbindung erhalten.

LC-MS (Methode 4): Rₜ = 1.46 min; m/z = 284/286 (M+H)⁺.

¹H-NMR (400 MHz, DMSO-d₆, δ/ppm): 7.05 (1H, d), 6.57 (1H, d), 6.32 (1H, dd), 5.20 (2H, s), 2.60 (2H, s), 1.38 (9H, s), 1.05 (6H, s).

### Beispiel 209A

### N,N-Dibenzyl-5-brom-2-chloranilin

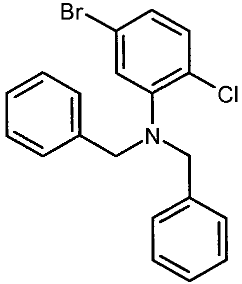

Unter Argon wurden 9.69 g (242.16 mmol, 60% in Mineralöl) Natriumhydrid in 100 ml THF suspendiert und auf 0°C abgekühlt. Anschließend wurden 20.0 g (96.86 mmol) 5-Brom-2-chloranilin, gelöst in 50 ml THF, langsam zugetropft und das Gemisch 30 min bei 0°C gerührt. Danach wurden 39.76 g (232.47 mmol) Benzylbromid, gelöst in 150 ml THF, langsam zur Reaktionsmischung gegeben und der Ansatz dann auf Raumtemperatur erwärmt. Das Gemisch wurde über Nacht bei RT gerührt und danach vorsichtig auf 150 ml Eiswasser gegossen. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das erhaltene Rohprodukt wurde mit Isopropanol versetzt, und die entstandenen Kristalle wurden abgesaugt und im Hochvakuum bei 40°C getrocknet. Es wurden 14 g der Titelverbindung erhalten. Das Filtrat wurde eingedampft und der erhaltene Rückstand durch Chromatographie an Silicagel gereinigt (Laufmittel Cyclohexan/Ethylacetat 20: 1). Es wurden so weitere 7.57 g der Titelverbindung erhalten (Gesamtausbeute: 21.57 g, 58% d. Th.).

LC-MS (Methode 6): Rₜ = 1.53 min; m/z = 386/388 (M+H)⁺.

Analog zu Beispiel 209A wurde die folgende Verbindung erhalten:

| **Beispiel** | **Name / Struktur / Edukte** | **Analytische Daten** |
|---|---|---|
| **210A** | *N,N*-Dibenzyl-2-chlor-5-iodanilin | LC-MS (Methode 4): Rₜ = 1.86 min; m/z = 433/435 (M+H)⁺. |
| | 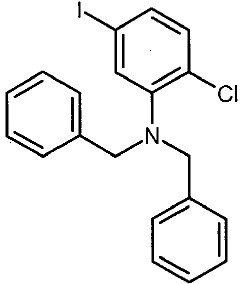 | |
| | (aus 2-Chlor-5-iodanilin und Benzylbromid) | |

### Beispiel 211A

### [4-Chlor-3-(dibenzylamino)phenyl]boronsäure

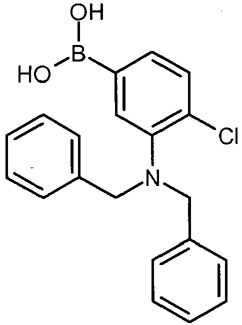

Unter Argon bei -78°C wurden zu einer Lösung von 15 g (38.79 mmol) N,N-Dibenzyl-5-brom-2-chloranilin in 350 ml THF/Diethylether (1:1) langsam 20.2 ml (50.42 mmol) einer 2.5 M Lösung von *n*-Butyllithium in Hexan getropft. Nachdem die Reaktionslösung 60 min bei -78°C nachgerührt worden war, wurden langsam 14.3 ml (62.1 mmol) Triisopropylborat hinzugefügt. Die Reaktionslösung wurde anschließend noch 15 min bei -78°C nachgerührt, dann langsam auf Raumtemperatur erwärmt und über Nacht bei dieser Temperatur weiter gerührt. Danach wurden 150 ml Eiswasser zudosiert. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 10:1 → 4:1). Es wurden 9 g (66% d. Th.) der Titelverbindung erhalten.

LC-MS (Methode 6): Rₜ = 1.21 min; m/z = 352 (M+H)⁺.

### Beispiel 212A

### tert.-Butylcyclobutylidenacetat

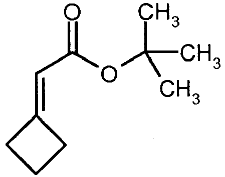

Unter Argon wurden bei Raumtemperatur 3.0 g (42.8 mmol) Cyclobutanon in 160 ml Dichlonnethan gelöst und anschließend mit 20.95 g (55.64 mmol) *tert*.-Butyl(triphenyl-A5-phosphanyliden)acetat und 0.68 g (5.56 mmol) Benzoesäure versetzt. Das Reaktionsgemisch wurde über Nacht bei Raumtemperatur gerührt und dann bis zur Trockene eingeengt. Der Rückstand wurde in 25 ml Diethylether verrührt und das Gemisch 12 h bei 4°C gelagert. Das ausgefallene Triphenyl-phosphanoxid wurde abfiltriert und das Filtrat bis zur Trockene eingeengt. Das erhaltene Rohprodukt wurde über Kieselgel chromatographisch gereinigt (Laufmittel Cyclohexan/Essigsäureethylester 20:1). Es wurden 9.3 g (99% d. Th.) der Titelverbindung erhalten.

¹H-NMR (400 MHz, DMSO-d₆, δ/ppm): 5.47-5.41 (1H, m), 3.05-2.95 (2H, m), 2.82-2.74 (2H, m), 2.06-1.95 (2H, m), 1.50 (9H, s).

GC-MS (Methode 1): Rₜ = 3.01 min; m/z = 112 (M-C₄H₈)⁺.

### Beispiel 213A

### tert.-Butylcyclopropylidenacetat

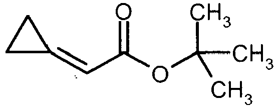

Zu einer Lösung von 9.65 g (55.34 mmol) [(1-Ethoxycyclopropyl)oxy](trimethyl)silan, 25 g (66.41 mmol) *tert*.-Butyl(triphenyl-λ⁵-phosphanyliden)acetat und 8.11 g (66.41 mmol) Benzoesäure in 240 ml THF wurden 55 ml (55 mmol) einer 1 M Lösung von Tetra-*n*-butylammoniumfluorid in THF bei Raumtemperatur zugetropft. Nach 1 h Rühren wurde das Reaktionsgemisch auf 80°C erhitzt und 2 h bei dieser Temperatur nachgerührt. Anschließend wurde mittels Rotationsverdampfer das Lösungsmittel abdestilliert (200 mbar, 40°C Badtemperatur). Der erhaltene Rückstand wurde in Diethylether aufgenommen, auf 4°C abgekühlt und 1 h bei dieser Temperatur stehen gelassen. Der angefallene Niederschlag (Triphenylphosphanoxid) wurde abfiltriert. Anschließend wurde das Filtrat mittels Rotationsverdampfer vom Lösungsmittel befreit. Das erhaltene Rohprodukt wurde über Kieselgel chromatographisch gereinigt (Laufmittel Cyclohexan/ Essigsäureethylester 20:1). Es wurden 3.58 g (42% d. Th.) der Titelverbindung erhalten.

GC-MS (Methode 1): Rₜ = 2.45 min; m/z = 98 (M-C₄H₈)⁺.

¹H-NMR (400 MHz, DMSO-d₆, δ/ppm): 1.18-1.26 (m, 2H), 1.34-1.41 (m, 3H), 1.44 (s, 9H), 6.06-6.13 (m, 1 H).

### Beispiel 214A

### Ethyl-(3,3-dimethoxycyclobutyliden)acetat

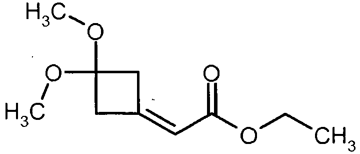

Eine Lösung von 3.93 g (44.59 mmol) 1,1-Dimethoxyethen und 5 g (44.59 mmol) Ethyl-buta-2,3-dienoat in 50 ml Toluol wurde auf Rückfluss erhitzt und 24 h gerührt. Nach Abkühlen auf Raumtemperatur wurde das Reaktionsgemisch vom Lösungsmittel befreit, und das erhaltene Rohprodukt wurde über Kieselgel chromatographisch gereinigt (Laufmittel Cyclohexan/Essigsäureethylester 20:1). Es wurden 1.9 g (21% d. Th.) der Titelverbindung als farblose Flüssigkeit erhalten, die ohne weitere Charakterisierung in Folgereaktionen eingesetzt wurde.

### Beispiel 215A

### tert.-Butyl-{1-[4-chlor-3-(dibenzylamino)phenyl]cyclopropyl}acetat

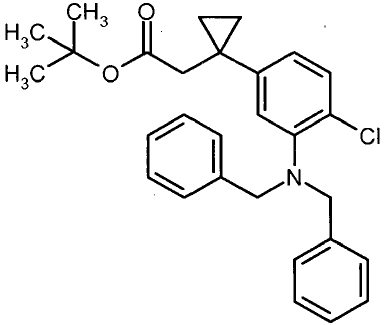

Herstellung von Lösung A: 300 mg (0.69 mmol) *N,N*-Dibenzyl-2-chlor-5-iodanilin wurden unter Argon in 3 ml THF gelöst und auf -78°C abgekühlt. Anschließend wurden 0.4 ml (0.80 mmol) einer 2 M Lösung von Isopropylmagnesiumchlorid in THF langsam zugetropft. Die Reaktionslösung wurde danach langsam auf -40°C erwärmt und 30 min bei dieser Temperatur nachgerührt.

Herstellung von Lösung B: 6 mg (0.14 mmol) Lithiumchlorid und 13 mg (0.07 mmol) Kupfer(I)-chlorid wurden unter Argon bei Raumtemperatur in 12 ml THF suspendiert und anschließend mit 84 µl (0.66 mmol) Chlor(trimethyl)silan sowie 102 mg (0.66 mmol) *tert*.-Butyl-cyclopropylidenacetat versetzt. Die Lösung wurde danach noch 1 h bei RT nachgerührt.

Lösung B wurde auf -40°C abgekühlt und langsam zur Lösung A hinzugetropft. Das erhaltene Reaktionsgemisch wurde anschließend noch 1 h bei -40°C nachgerührt. Dann wurden 20 ml einer eiskalten, halbgesättigten wässrigen Ammoniumchlorid-Lösung zur Reaktionsmischung gegeben. Nach Trennung der Phasen wurde die wässrige Phase noch dreimal mit Essigsäureethylester extrahiert, und die vereinigten organischen Phasen wurden über Magnesiumsulfat getrocknet, filtriert und bis zur Trockene eingeengt. Das erhaltene Rohprodukt wurde über Kieselgel chromatographisch gereinigt (Laufmittel Cyclohexan/Essigsäureethylester 20:1). Es wurden 135 mg (42% d. Th.) der Titelverbindung erhalten.

LC-MS (Methode 6): Rₜ = 1.73 min; m/z = 462/464 (M+H)⁺.

Analog zu Beispiel 13A wurden die folgenden Verbindungen erhalten:

| **Beispiel** | **Name / Struktur / Edukte** | **Analytische Daten** |
|---|---|---|
| **216A** | *tert.*-Butyl- {1-[4-chlor-3-(dibenzylamino)-phenyl]cyclobutyl}acetat | LC-MS (Methode 4): Rₜ = 1.96 min; m/z = 476/478 (M+H)⁺. |
| | 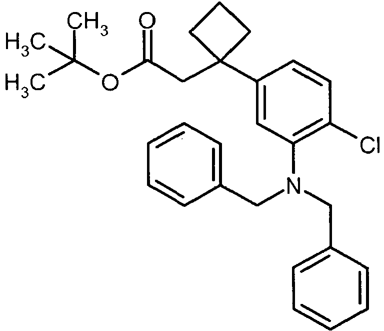 | |
| | (aus [4-Chlor-3-(dibenzylamino)phenyl]boronsäure und *tert.*-Butylcyclobutylidenacetat) | |
| **217A** | Ethyl-{1-[4-chlor-3-(dibenzylamino)phenyl]-3,3-dimethoxycyclobutyl}acetat | LC-MS (Methode 6): Rₜ = 1.53 min; m/z = 508/510 (M+H)⁺. |
| | 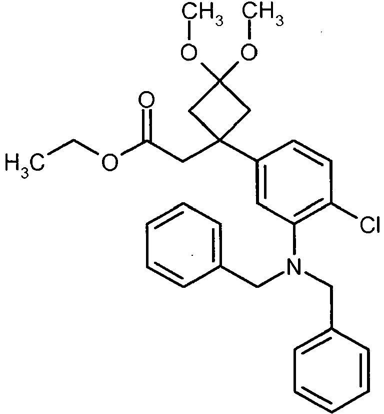 | |
| | (aus [4-Chlor-3-(dibenzylamino)phenyl]boronsäure und Ethyl-(3,3-dimethoxycyclobutyliden)acetat) | |

### Beispiel 218A

### Ethyl-{1-[4-chlor-3-(dibenzylamino)phenyl]-3-oxocyclobutyl}acetat

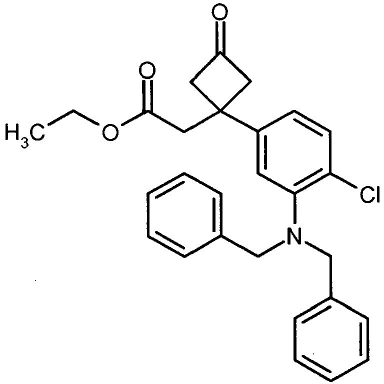

770 mg (1.52 mmol) Ethyl-{1-[4-chlor-3-(dibenzylamino)phenyl]-3,3-dimethoxycyclobutyl}acetat wurden in 10 ml THF gelöst, mit 2 ml 1 M Salzsäure versetzt und 1 h bei 50°C gerührt. Anschließend wurde die Reaktionslösung mit 10 ml Wasser und 10 ml Essigsäureethylester verdünnt. Nach Trennung der Phasen wurde die organische Phase über Magnesiumsulfat getrocknet, filtriert und mittels Rotationsverdampfer bis zur Trockene eingeengt. Es wurden 607 mg der Titelverbindung erhalten (87% d. Th.).

LC-MS (Methode 6): Rₜ = 1.44 min; m/z = 462/464 (M+H)⁺.

### Beispiel 219A

### Ethyl-{1-[4-chlor-3-(dibenzylamino)phenyl]-3,3-difluorcyclobutyl} acetat

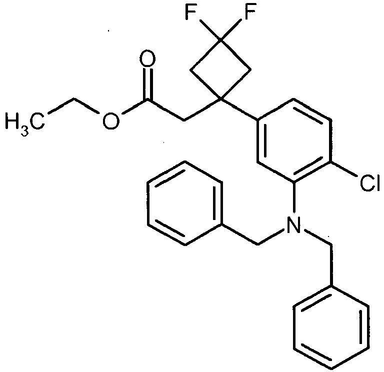

Unter Argon wurden zu 2 ml Dichlormethan 0.3 ml (2.27 mmol) [Ethyl(trifluor-λ⁴-sulfanyl)amino]-ethan gegeben. Die Reaktionslösung wurde auf 0°C. abgekühlt und langsam mit 175 mg (0.38 mmol) Ethyl-{1-[4-chlor-3-(dibenzylamino)phenyl]-3-oxocyclobutyl}acetat in 3 ml Dichlormethan versetzt. Anschließend wurde die Lösung langsam auf Raumtemperatur erwärmt und über Nacht bei dieser Temperatur nachgerührt. Die Reaktionsmischung wurde danach auf 50 ml Eiswasser gegossen und die organische Phase abgetrennt. Die wässrige Phase wurde noch dreimal mit Dichlormethan extrahiert. Die vereinigten organischen Phasen wurden über Magnesiumsulfat getrocknet. Nach Filtration wurde das Lösungsmittel im Vakuum entfernt und das erhaltene Rohprodukt durch präparative HPLC gereinigt (Eluent Methanol/Wasser 8:2). Es wurden 59 mg der Titelverbindung erhalten (32% d. Th.).

LC-MS (Methode 6): Rₜ = 1.53 min; m/z = 484/486 (M+H)⁺.

### Beispiel 220A

### tert.-Butyl-[1-(3-amino-4-chlorphenyl)cyclopropyl]acetat

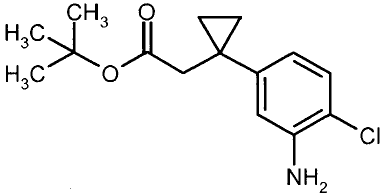

135 mg (0.29 mmol) *tert*.-Butyl-{1-[4-chlor-3-(dibenzylamino)phenyl]cyclopropyl}acetat wurden in 10 ml Ethylacetat gelöst, mit 15 mg Palladium auf Kohle (10%) versetzt und 2 h bei RT unter einer Wasserstoffatmosphäre bei Normaldruck gerührt. Die Reaktionsmischung wurde danach über Celite abfiltriert, der Rückstand mit Ethylacetat nachgewaschen und das Filtrat eingeengt. Es wurden 73 mg der Titelverbindung erhalten (89% d. Th.).

LC-MS (Methode 6): Rₜ = 1.15 min; m/z = 282/284 (M+H)⁺.

Analog zu Beispiel 220A wurden die folgenden Verbindungen erhalten:

| **Beispiel** | **Name / Struktur / Edukt** | **Analytische Daten** |
|---|---|---|
| **221A** | *tert*.-Butyl-[1-(3-amino-4-chlorphenyl)-cyclobutyl]acetat | LC-MS (Methode 6): Rₜ = 1.24 min; m/z = 296 (M+H)⁺. |
| | 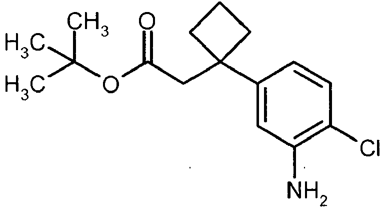 | |
| | (aus *tert*.-Butyl-{1-[4-chlor-3-(dibenzylamino)-phenyl]cyclobutyl} acetat) | |
| **222A** | Ethyl-[1-(3-amino-4-chlorphenyl)-3,3-difluorcyclobutyl]acetat | LC-MS (Methode 4): Rₜ = 1.36 min; m/z = 304/306 (M+H)⁺. |
| | 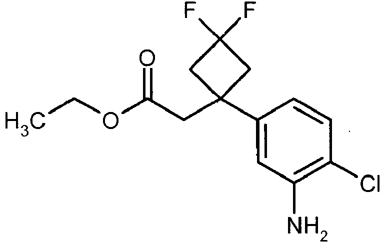 | |
| | (aus Ethyl-{1-[4-chlor-3-(dibenzylamino)phenyl]-3,3-difluorcyclobutyl}acetat) | |

### Beispiel 223A

### tert.-Butyl-(2E)-3-(3-amino-4-cyanophenyl)-2-methylacrylat

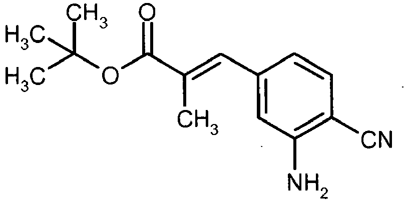

Unter Argon wurde eine Mischung aus 2.0 g (10.15 mmol) 2-Amino-4-brombenzonitril, 2.165 g (2.5 ml, 15.23 mmol) *tert*.-Butyl-2-methylacrylat, 93 mg (0.10 mmol) Tris(dibenzylidenaceton)-dipalladium, 41 mg (0.20 mmol) Tri-*tert*.-butylphosphin und 2.4 ml (11.17 mmol) *N,N*-Dicyclo-hexylmethylamin in 20 ml Dioxan auf 120°C erhitzt und über Nacht bei dieser Temperatur gerührt. Nach Reaktionskontrolle (DC, Laufmittel Cyclohexan/Ethylacetat 9:1) wurden nochmals 10 mg Tris(dibenzylidenaceton)dipalladium, 10 mg Tri-*tert*.-butylphosphin und 500 µl *tert*.-Butyl-2-methylacrylat hinzugefügt und die Mischung weitere 4 h bei 120°C gerührt. Anschließend wurde das Reaktionsgemisch über Celite filtriert und das Filtrat im Vakuum eingeengt. Der Rückstand wurde durch Chromatographie an Silicagel gereinigt (Laufmittel Cyclohexan/Ethylacetat 9:1 → 4:1). Es wurden 1.375 g der Titelverbindung erhalten (52% d. Th.).

LC-MS (Methode 4): Rₜ = 1.33 min; m/z = 259 (M+H)⁺.

### Beispiel 224A

### tert.-Butyl-3-(3-amino-4-cyanophenyl)-2-methylpropanoat

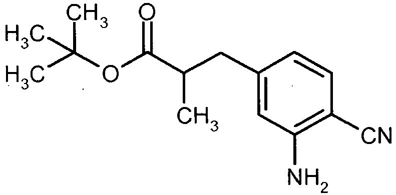

1370 mg (5.3 mmol) *tert*.-Butyl-(2E)-3-(3-amino-4-cyanophenyl)-2-methylacrylat wurden in 30 ml Ethylacetat gelöst, mit 282 mg Palladium auf Kohle (10%) versetzt und bei RT drei Tage lang unter einer Wasserstoffatmosphäre bei Normaldruck gerührt. Die Reaktionsmischung wurde anschließend über Celite abfiltriert, der Filter-Rückstand mit Ethylacetat nachgewaschen und das vereinigte Filtrat eingeengt. Das Rohprodukt wurde durch präparative HPLC gereinigt (Eluent Acetonitril/Wasser). Es wurden 870 mg der Titelverbindung erhalten (63% d. Th.).

LC-MS (Methode 6): Rₜ = 1.04 min; m/z = 261 (M+H)⁺.

Analog zu Beispiel 54A wurde die folgende Verbindung erhalten:

| **Beispiel** | **Name / Struktur / Edukte** | **Analytische Daten** |
|---|---|---|
| **225A** | Ethyl-(3*R*)-2-(4-chlor-3-methoxyphenyl)-4,4,4-trifluor-3-methylbutanoat | GC-MS (Methode 1): Rₜ = 5.34 min; m/z = 324/326 (M)⁺. |
| | 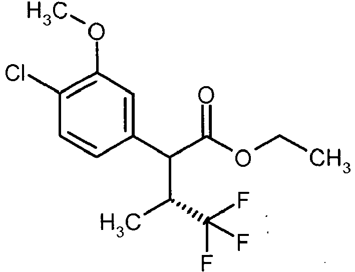 | |
| | (aus 4-Brom-1-chlor-2-methoxybenzol und Ethyl-(3*R*)-4,4,4-trifluor-3-methylbutanoat) | |

### Beispiel 226A

### Ethyl-(3R)-2-[4-(2,2-dichlor-3-oxocyclobutyl)phenyl]-4,4,4-trifluor-3-methylbutanoat

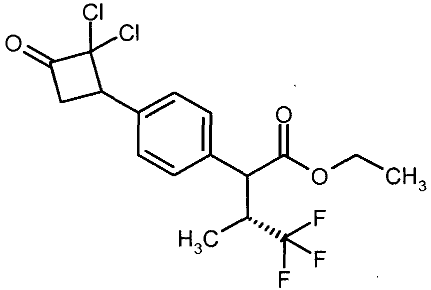

3.83 g (13.38 mmol) Ethyl-(3*R*)-4,4,4-trifluor-3-methyl-2-(4-vinylphenyl)butanoat wurden in 50 ml Diethylether gelöst und nacheinander mit 2.67 g (20.74 mmol) Zink-Kupfer-Paar und 6.5 ml 1,2-Dimethoxyethan versetzt. Zu der erhaltenen Suspension wurden anschließend 4 ml (36.1 mmol) Trichloracetylchlorid langsam zugetropft. Die Reaktionslösung wurde dann unter Rückfluss erhitzt und über Nacht gerührt. Nach Zugabe von Dichlormethan wurde das Reaktionsgemisch nacheinander mit Wasser und gesättigter Natriumchlorid-Lösung gewaschen. Die organische Phase wurde über Magnesiumsulfat getrocknet, filtriert und im Vakuum eingeengt. Das erhaltene Rohprodukt wurde über Kieselgel chromatographisch gereinigt (Laufmittel: Cyclohexan/Essigsäureethylester 4:1). Es wurden 4.57 g (86% d. Th.) der Titelverbindung in Form eines gelblichen Öls erhalten, das ohne weitere Charakterisierung in Folgereaktionen eingesetzt wurde.

### Beispiel 227A

### Ethyl-(3R)-4,4,4-trifluor-3-methyl-2-[4-(3-oxocyclobutyl)phenyl]butanoat

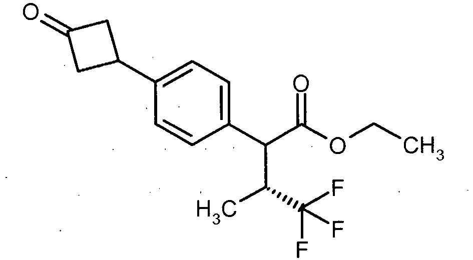

4.57 g (11.51 mmol) Ethyl-(3*R*)-2-[4-(2,2-dichlor-3-oxocyclobutyl)phenyl]-4,4,4-trifluor-3-methyl-butanoat und 3.76 g (57.5 mmol) Zinkstaub in 100 ml THF wurden mit 100 ml gesättigter wässriger Ammoniumchlorid-Lösung versetzt und anschließend 5 h bei 75°C gerührt. Nach Abkühlen auf Raumtemperatur und Zugabe von Dichlormethan wurde die Reaktionsmischung mit Wasser ge-waschen. Nach Trennung der Phasen wurde die wässrige Phase dreimal mit Dichlormethan rückextrahiert. Anschließend wurden die vereinigten organischen Phasen über Magnesiumsulfat getrocknet, filtriert und im Vakuum eingeengt. Es wurden 1.21 g der Titelverbindung erhalten (32% d. Th.).

GC-MS (Methode 1): Rₜ = 6.52 min, m/z = 286 (M-C₂H₂O)⁺ (Diastereomer 1); Rₜ = 6.55 min, m/z = 286 (M-C₂H₂O)⁺ (Diastereomer 2).

MS (DCI): m/z = 346 (M+NH₄)⁺.

### Beispiel 228A

### Ethyl-(3R)-2-[4-(3,3-difluorcyclobutyl)phenyl]-4,4,4-trifluor-3-methylbutanoat

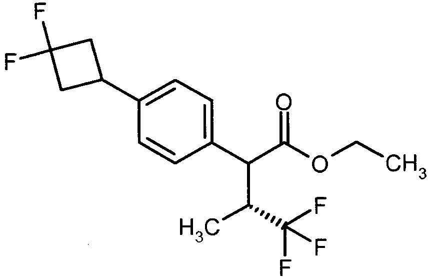

Unter Argon wurden 7.3 ml (5.16 mmol) einer 50%-igen Lösung von 1,1'-[(Trifluor-λ⁴-sulfanyl)-imino]bis(2-methoxyethan) (Desoxofluor) in THF, verdünnt mit 20 ml Toluol, vorgelegt, auf 5°C abgekühlt und langsam mit 47 µl (0.37 mmol) einer 1 M Bortrifluorid-Diethylether-Komplex-Lösung versetzt. Die Mischung wurde 2 h bei 5°C nachgerührt. Anschließend wurde die Reaktionslösung langsam mit 1.21 g (3.69 mmol) Ethyl-(3*R*)-4,4,4-trifluor-3-methyl-2-[4-(3-oxocyclo-butyl)phenyl]butanoat, gelöst in 20 ml Toluol, versetzt, dann auf 55°C erwärmt und 48 h bei dieser Temperatur nachgerührt. Die Reaktionsmischung wurde danach in ein auf 0°C gekühltes Gemisch bestehend aus 20 ml Toluol und 20 ml 2 M Natronlauge gegeben. Die organische Phase wurde abgetrennt, und die wässrige Phase wurde noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 10:1). Es wurden 558 mg (43% d. Th.) der Titelverbindung als gelbliche Flüssigkeit isoliert.

GC-MS (Methode 1): Rₜ = 5.40 min, m/z = 350 (M)⁺ (Diastereomer 1); Rₜ = 5.44 min, m/z = 350 (M)⁺ (Diastereomer 2).

MS (DCI): m/z = 368 (M+NH₄)⁺.

### Beispiel 229A

### Ethyl-(3R)-2-[4-(2,2-difluorcyclopropyl)phenyl]-4,4,4-trifluor-3-methylbutanoat

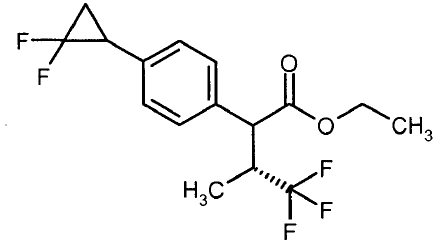

1.58 g (5.52 mmol) Ethyl-(3*R*)-4,4,4-trifluor-3-methyl-2-(4-vinylphenyl)butanoat, 23 mg (0.55 mmol) Natriumfluorid und 24 mg (0.11 mmol) 2,6-Di-*tert*.-butyl-4-methylphenol wurden auf 110°C erhitzt und 5 min gerührt. Anschließend wurden langsam 1.9 ml (9.38 mmol) Trimethylsilyl-difluor(fluorsulfonyl)acetat zugetropft und das Gemisch 60 min bei 110°C nachgerührt (Vorsicht: Gasentwicklung nach ca. 30 min). Nach Abkühlen auf Raumtemperatur und Zugabe von Essigsäureethylester und gesättigter wässriger Natriumhydrogencarbonat-Lösung wurde die organische Phase abgetrennt, über Magnesiumsulfat getrocknet, filtriert und bis zur Trockene eingeengt. Das Rohprodukt wurde über Kieselgel chromatographisch gereinigt (Laufmittel Cyclohexan/ Dichlormethan 4:1). Es wurden 1. 5 g der Titelverbindung erhalten (81% d. Th.).

GC-MS (Methode 1): Rₜ = 4.99 min, m/z = 336 (M)⁺ (Diastereomer 1); Rₜ = 5.01 min, m/z = 336 (M)⁺ (Diastereomer 2).

MS (DCI): m/z = 354 (M+NH₄)⁺.

Analog zu Beispiel 70A wurden die in der folgenden Tabelle aufgeführten Verbindungen hergestellt:

| **Beispiel** | **Name / Struktur / Edukt** | **Analytische Daten** |
|---|---|---|
| **230A** | (2*S*,3*R*)-2-[4-(3,3-Difluorcyclobutyl)phenyl]-4,4,4-trifluor-3-methylbutansäure | GC-MS (Methode 1): Rₜ = 5.76 min; m/z = 322 (M)⁺. |
| | 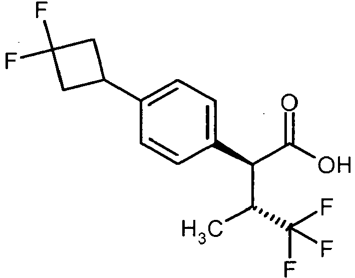 | |
| | | MS (EI): m/z = 322 (M)⁺. |
| | | |
| | | ¹H-NMR (400 MHz, DMSO-*d*₆): δ [ppm] = 0.76 (d, 3H), 2.58-2.76 (m, 2H), 2.91-3.05 (m, 2H), 3.17-3.28 (m, 1H), 3.34-3.45 (m, 1H), 3.60 (d, 1H), 7.27-7.36 (m, 4H), 12.63-12.81 (br. s, 1H). |
| | (aus Ethyl-(3*R*)-2-[4-(3,3-difluorcyclobutyl)-phenyl]-4,4,4-trifluor-3-methylbutanoat) | |
| **231A** | (2*S*,3*R*)-2-(4-Chlor-3-methoxyphenyl)-4,4,4-trifluor-3-methylbutansäure | ¹H-NMR (400 MHz, DMSO-*d₆*, δ/ppm): 12.91-12.71 (1H, br. s), 7.41 (1H, d), 7.18 (1H, d), 6.98 (1H, dd), 3.86 (3H, s), 3.66 (1H, d), 3.40-3.19 (1H, m, teilweise verdeckt durch H₂O-Signal), 0.79 (3H, d). |
| | 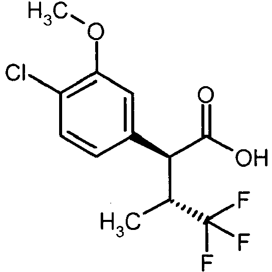 | |
| | | |
| | | LC-MS (Methode 5): Rₜ = 2.20 min; m/z = 295/297 (M-H)⁻. |
| | (aus Ethyl-(3*R*)-2-(4-chlor-3-methoxyphenyl)-4,4,4-trifluor-3-methylbutanoat) | |
| **232A** | (2*S*,3*R*)-2-[4-(2,2-Difluorcyclopropyl)phenyl]-4,4,4-trifluor-3-methylbutansäure | LC-MS (Methode 6): Rₜ = 1.09 min; m/z = 307 (M-H)⁻. |
| | | |
| | 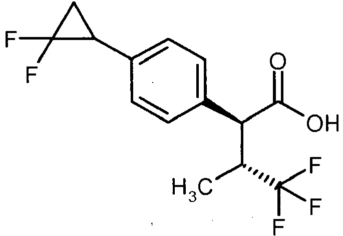 | ¹H-NMR (400 MHz, DMSO-*d*₆): δ [ppm] = 0.76 (d, 3H), 1.86-2.04 (m, 2H), 2.92-3.06 (m, 1H), 3.18-3.29 (m, 1H), 3.61 (d, 1H), 7.27 (d, 2H), 7.34 (d, 2H), 12.72 (br. s, 1H). |
| | (aus Ethyl-(2*S*,3*R*)-2-[4-(2,2-difluorcyclopropyl)-phenyl]-4,4,4-trifluor-3-methylbutanoat) | |

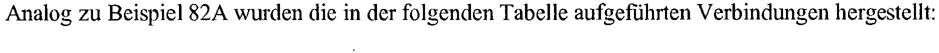

| **Beispiel** | **Name / Struktur** | **Ausgangsmaterial** |
|---|---|---|
| **233A** | (2*S*,3*R*)-2-[4-(3,3-Difluorcyclobutyl)phenyl]-4,4,4-trifluor-3-methylbutanoylchlorid | (2*S*,3*R*)-2-[4-(3,3-Difluor-cyclobutyl)phenyl]-4,4,4-trifluor-3-methylbutansäure |
| | 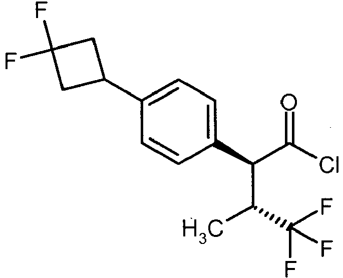 | |
| **234A** | (2*S*,3*R*)-2-[4-(2,2-Difluorcyclopropyl)phenyl]-4,4,4-trifluor-3-methylbutanoylchlorid | (2*S*,3*R*)-2-[4-(2,2-Difluor-cyclopropyl)phenyl]-4,4,4-trifluor-3-methylbutansäure |
| | 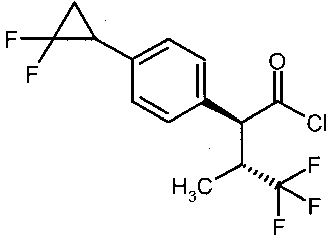 | |

### Beispiel 235A

### tert.-Butyl-3-(4-chlor-3-{[(2S,3R)-2-(4-chlorphenyl)-4,4,4-trifluor-3-methylbutanoyl]amino}-phenyl)-2,2-dimethylpropanoat

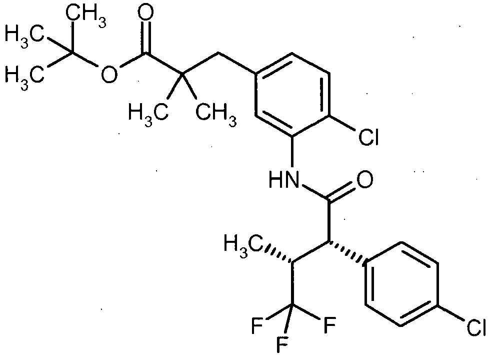

400 mg (1.50 mmol) (2*S*,3*R*)-2-(4-Chlorphenyl)-4,4,4-trifluor-3-methylbutansäure wurden in 24 ml Dichlormethan gelöst, mit 320 mg (2.40 mmol) 1-Chlor-*N,N*,2-trimethylprop-1-en-1-amin versetzt und 30 min bei Raumtemperatur gerührt. Anschließend wurden 364 µl (4.5 mmol) Pyridin sowie 510 mg (1.80 mmol) *tert*.-Butyl-3-(3-amino-4-chlorphenyl)-2,2-dimethylpropanoat hinzugefügt und das Gemisch 2 h bei Raumtemperatur nachgerührt. Die Reaktionsmischung wurde danach im Vakuum eingeengt und das erhaltene Rohprodukt durch Chromatographie an Silicagel gereinigt (Laufmittel Cyclohexan/Ethylacetat 20:1). Es wurden 462 mg der Zielverbindung erhalten (58% d. Th.).

LC-MS (Methode 6): Rₜ = 1.53 min; m/z = 530/532 (M-H)⁻.

¹H-NMR (400 MHz, DMSO-d₆): δ [ppm] = 0.79 (d, 3H), 1.02 (s, 3H), 1.05 (s, 3H), 1.26 (s, 9H), 2.65-2.78 (m, 2H), 3.27-3.44 (m, 1H, teilweise verdeckt durch H₂O-Signal), 4.10 (d, 1H), 6.96 (dd, 1H), 7.31 (d, 1H), 7.35 (d, 1H), 7.41-7.51 (m, 4H), 9.83 (s, 1H).

Auf analoge Weise wurden die in der folgenden Tabelle aufgeführten Verbindungen hergestellt:

| **Beispiel** | **Name / Struktur / Edukte** | **Analytische Daten** |
|---|---|---|
| **236A** | *tert*.-Butyl-[1-(4-chlor-3-{[(2*S*,3*R*)-2-(4-chlorphenyl)-4,4,4-trifluor-3-methylbutanoyl]amino}phenyl)-cyclobutyl]acetat | LC-MS (Methode 6): Rₜ = 1.52 min; m/z = 542/544 (M-H)⁻. |
| | 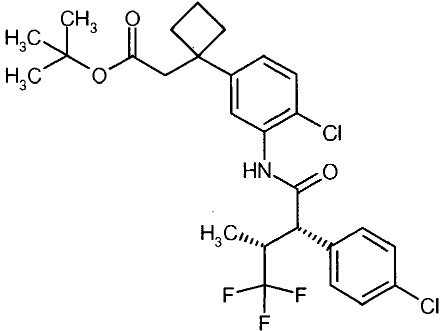 | |
| | (aus *tert*.-Butyl-[1-(3-amino-4-chlorphenyl)-cyclobutyl]acetat und (2*S*,3*R*)-2-(4-Chlorphenyl)-4,4,4-trifluor-3-methylbutansäure) | |
| **237A** | Ethyl-(2*S*)-3-[4-chlor-3-({(2*S*,3*R*)-4,4,4-trifluor-3-methyl-2-[4-(1,1,1-trifluor-2-methylpropan-2-yl)-phenyl]butanoyl}amino)phenyl]-2-methylpropanoat | LC-MS (Methode 6): Rₜ = 1.46 min; m/z = 564 (M-H)⁻. |
| | 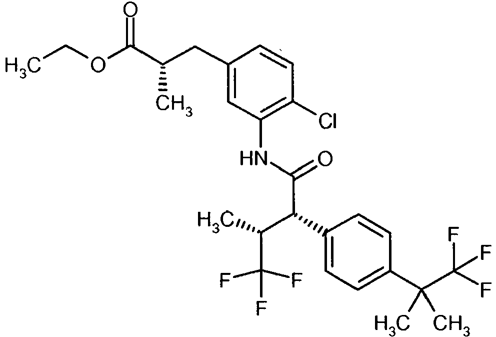 | |
| | (aus Ethyl-(2*S*)-3-(3-amino-4-chlorphenyl)-2-methyl-propanoat und (2*S*,3*R*)-4,4,4-Trifluor-3-methyl-2-[4-(1,1,1-trifluor-2-methylpropan-2-yl)phenyl]-butansäure) | |
| **238A** | Ethyl-(2*S*)-3-[4-chlor-3-({4,4,4-trifluor-3-methyl-2-[4-(2,2,2-trifluorethyl)phenyl]butanoyl}amino)phenyl]-2-methylpropanoat | LC-MS (Methode 6): Rₜ = 1.37 min; m/z = 536/538 (M-H)⁻. |
| | 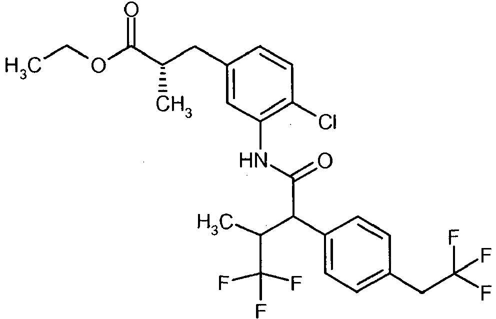 | |
| | (aus Ethyl-(2*S*)-3-(3-amino-4-chlorphenyl)-2-methylpropanoat und 4,4,4-Trifluor-3-methyl-2-[4-(2,2,2-trifluorethyl)phenyl]butansäure) | |
| **239A** | Ethyl-(2*S*)-3-(4-chlor-3-{[(2*S*,3*R*)-2-(4-chlor-3-methoxyphenyl)-4,4,4-trifluor-3-methylbutanoyl]-amino}phenyl)-2-methylpropanoat | LC-MS (Methode 6): Rₜ = 1.37 min; m/z = 520/522 (M+H)⁺. |
| | | |
| | 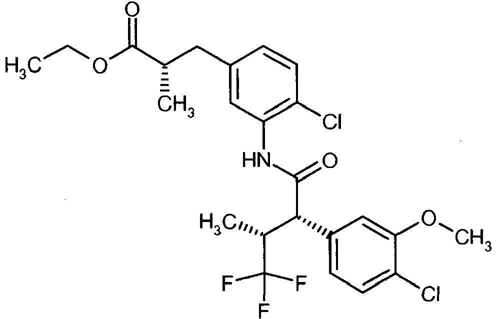 | ¹H-NMR (400 MHz, DMSO-d₆, δ/ppm): 9.80 (1H, s), 7.42 (1H, d), 7.35 (2H, d), 7.25-7.20 (1H, m), 7.06-6.96 (2H, m), 4.10 (1H, d), 3.95 (2H, q), 3.87 (3H, s), 3.49-3.34 (1H, m), 2.84-2.74 (1H, m), 2.72-2.58 (2H, m), 1.11-1.00 (6H, m), 0.83 (3H, d). |
| | (aus Ethyl-(2*S*)-3-(3-amino-4-chlorphenyl)-2-methyl-propanoat und (2*S*,3*R*)-2-(4-Chlor-3-methoxyphenyl)-4,4,4-trifluor-3-methylbutansäure) | |
| **240A** | *tert*.-Butyl-3-(4-chlor-3-{[(2S,3R)-2-(4-chlor-3-methoxyphenyl)-4,4,4-trifluor-3-methyl-butanoyl]amino}phenyl)propanoat | LC-MS (Methode 6): Rₜ = 1.43 min; m/z = 532/534 (M+H)⁺. |
| | | |
| | 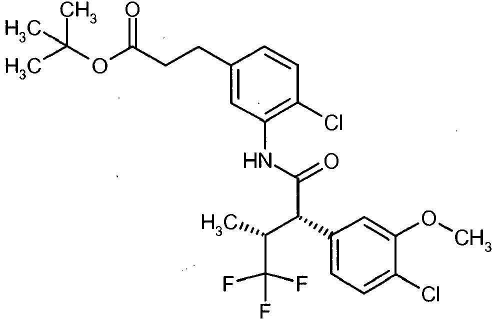 | ¹H-NMR (400 MHz, DMSO-d₆, δ/ppm): 9.80 (1H, s), 7.42 (1H, d), 7.38 (1H, d), 7.36 (1H, d), 7.23 (1H, d), 7.07-6.99 (2H, m), 4.09 (1H, d), 3.87 (3H, s), 3.50-3.34 (1H, m), 2.76 (2H, t), 2.46 (2H, t), 1.30 (9H, s), 0.83 (3H, d). |
| | (aus *tert*.-Butyl-3-(3-amino-4-chlorphenyl)propanoat und (2*S*,3*R*)-2-(4-Chlor-3-methoxyphenyl)-4,4,4-trifluor-3-methylbutansäure) | |
| **241A** | *tert*.-Butyl-[1-(4-chlor-3-{[(2*S*,3*R*)-2-(4-chlorphenyl)-4,4,4-trifluor-3-methylbutanoyl]amino}phenyl)-cyclopropyl]acetat | LC-MS (Methode 6): Rₜ = 1.48 min; m/z = 528/530 (M-H)⁻. |
| | 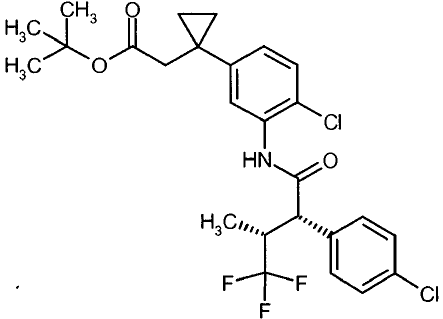 | |
| | (aus *tert*.-Butyl-[1-(3-amino-4-chlorphenyl)-cyclopropyl]acetat und (2*S*,3*R*)-2-(4-Chlorphenyl)-4,4,4-trifluor-3-methylbutansäure) | |
| **242A** | Ethyl-[1-(4-chlor-3-{[(2*S*,3*R*)-2-(4-chlorphenyl)-4,4,4-trifluor-3-methylbutanoyl]amino}phenyl)-3,3-difluorcyclobutyl]acetat | LC-MS (Methode 6): Rₜ = 1.39 min; m/z = 550/552 (M-H)⁻. |
| | 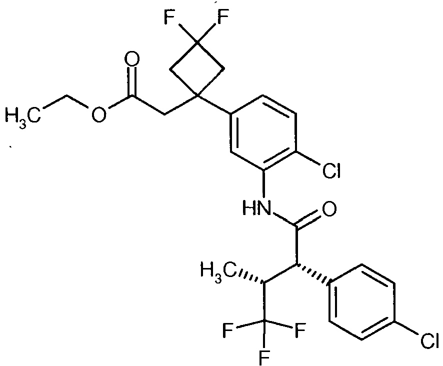 | |
| | (aus Ethyl-[1-(3-amino-4-chlorphenyl)-3,3-difluor-cyclobutyl]acetat und (2*S*,3*R*)-2-(4-Chlorphenyl)-4,4,4-trifluor-3-methylbutansäure) | |
| **243A** | *tert*.-Butyl-3-(3-{[(2*S*,3*R*)-2-(4-chlorphenyl)-4,4,4-trifluor-3-methylbutanoyl]amino}-4-cyanophenyl)-2-methylpropanoat | LC-MS (Methode 6): Rₜ = 1.39 min; m/z = 507/509 (M-H)⁻. |
| | 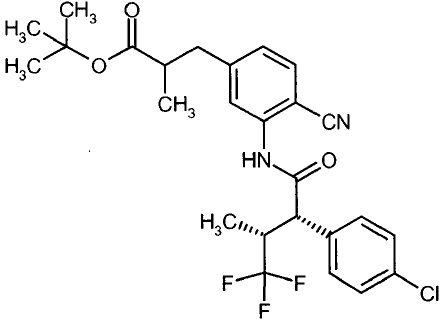 | |
| | (aus *tert*.-Butyl-3-(3-amino-4-cyanophenyl)-2-methyl-propanoat und (2*S*,3*R*)-2-(4-Chlorphenyl)-4,4,4-trifluor-3-methylbutansäure) | |

Analog zu Beispiel 89A wurden die in der folgenden Tabelle aufgeführten Verbindungen hergestellt:

| **Beispiel** | **Name / Struktur / Edukte** | **Analytische Daten** |
|---|---|---|
| **244A** | *tert*.-Butyl-3-[4-chlor-3-({(2*S*,3*R*)-2-[4-(3,3-difluor-cyclobutyl)phenyl]-4,4,4-trifluor-3-methyl-butanoyl}amino)phenyl]propanoat | ¹H-NMR (400 MHz, DMSO-d₆, δ/ppm): 9.75 (1H, s), 7.44-7.37 (3H, m), 7.36-7.27 (3H, m), 7.02 (1H, dd), 4.10 (1H, d), 3.46-3.27 (2H, m, teilweise verdeckt durch H₂O-Signal), 3.06-2.91 (2H, m), 2.75 (2H, t), 2.71-2.59 (2H, m), 2.45 (2H, t), 1.31 (9H, s); 0.79 (3H, d). |
| | 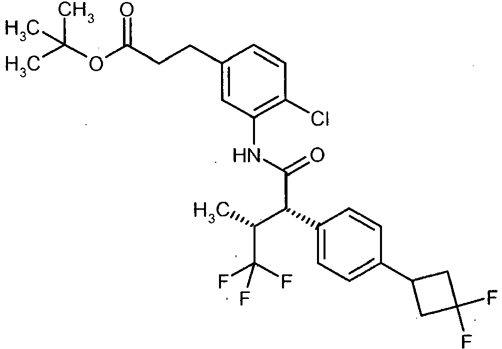 | |
| | | |
| | | LC-MS (Methode 4): Rₜ = 1.64 min; m/z = 558/560 (M-H)⁻. |
| | (aus (2*S*,3*R*)-2-[4-(3,3-Difluorcyclobutyl)phenyl]-4,4,4-trifluor-3-methylbutanoylchlorid und *tert.*-Butyl-3-(3-amino-4-chlorphenyl)propanoat) | |
| **245A** | Ethyl-(2*S*)-3-[4-chlor-3-({(2*S*,3*R*)-2-[4-(3,3-difluor-cyclobutyl)phenyl]-4,4,4-trifluor-3-methyl-butanoyl}amino)phenyl]-2-methylpropanoat | ¹H-NMR (400 MHz, DMSO-d₆, δ/ppm): 9.75 (1H, s), 7.40 (3H, t), 7.32 (3H, t), 6.97 (1H, dd), 4.10 (1H, d), 3.96 (2H, q), 3.46-3.28 (2H, m, teilweise verdeckt durch H₂O-Signal), 3.06-2.91 (2H, m), 2.84-2.58 (5H, m), 1.10-1.01 (6H, m), 0.79 (3H, d). |
| | 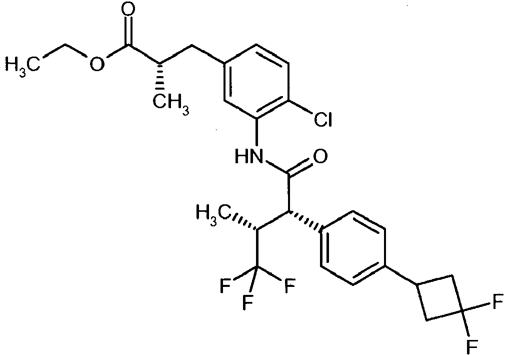 | |
| | | |
| | | LC-MS (Methode 6): Rₜ = 1.43 min; m/z = 544/546 (M-H)⁻. |
| | (aus (2*S*,3*R*)-2-[4-(3,3-Difluorcyclobutyl)phenyl]-4,4,4-trifluor-3-methylbutanoylchlorid und Ethyl-(2S)-3-(3-amino-4-chlorphenyl)-2-methylpropanoat) | |
| **246A** | *tert*.-Butyl-3-[4-chlor-3-({(2*S*,3*R*)-2-[4-(2,2-difluor-cyclopropyl)phenyl]-4,4,4-trifluor-3-methyl-butanoyl}amino)phenyl]propanoat | ¹H-NMR (400 MHz, DMSO-d₆, δ/ppm): 9.77 (1H, s), 7.42 (3H, d), 7.34 (1H, d), 7.27 (2H, d), 7.02 (1H, dd), 4.09 (1H, d), 3.43-3.28 (1H, m, teilweise verdeckt durch H₂O-Signal), 3.05-2.94 (1H, m), 2.75 (2H, t), 2.45 (2H, t), 2.04-1.86 (1H, m), 1.31 (9H, s), 0.78 (3H, d). |
| | 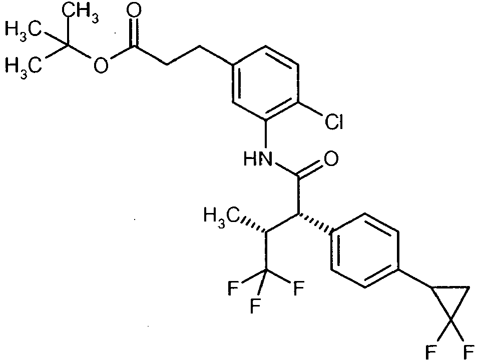 | |
| | | |
| | | LC-MS (Methode 7): Rₜ = 2.99 min; m/z = 544/546 (M-H)⁻. |
| | (aus (2*S*,3*R*)-2-[4-(2,2-Difluorcyclopropyl)phenyl]-4,4,4-trifluor-3-methylbutanoylchlorid und *tert*.-Butyl-3-(3-amino-4-chlorphenyl)propanoat) | |

### Beispiel 247A

### tert.-Butyl-3-(3-{[(2S,3R)-2-(4-chlorphenyl)-4,4,4-trifluor-3-methylbutanoyl]amino}-2-methyl-phenyl)propanoat

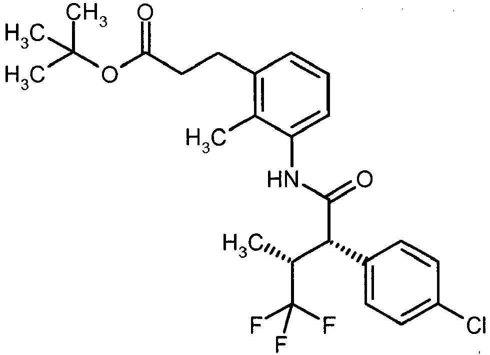

Eine Mischung von 100 mg (0.38 mmol) (2*S*,3*R*)-2-(4-Chlorphenyl)-4,4,4-trifluor-3-methylbutansäure, 88 mg (0.38 mmol) *tert*.-Butyl-3-(3-amino-2-methylphenyl)propanoat, 213 mg (0.56 mmol) 2-(1*H*-7-Azabenzotriazol-1-yl)-1,1,3,3-tetramethyluronium-hexafluorophosphat (HATU) und 1 ml Pyridin in 4 ml DMF wurde über Nacht bei Raumtemperatur gerührt. Nach Beendigung der Umsetzung wurde das Reaktionsgemisch direkt ohne weitere Aufarbeitung mittels präparativer HPLC (Eluent Acetonitril/Wasser) in seine Komponenten aufgetrennt. Es wurden 151 mg (83% d. Th.) der Titelverbindung als farbloses Öl erhalten.

¹H-NMR (400 MHz, DMSO-d₆, δ/ppm): 9.68 (1H, s), 7.46 (4H, s), 7.09-6.93 (3H, m), 3.94 (1H, d), 3.43-3.28 (1H, m, teilweise verdeckt durch H₂O-Signal) 2.78 (2H, t), 2.41 (2H, t), 1.91 (3H, s), 1.35 (9H, s), 0.80 (3H, d).

LC-MS (Methode 4): Rₜ = 1.57 min; m/z = 482 (M-H)⁻.

Auf analoge Weise wurde die folgende Verbindung erhalten:

| **Beispiel** | **Name / Struktur / Edukte** | **Analytische Daten** |
|---|---|---|
| **248A** | *tert*.-Butyl-3-(3-{[(2*S*,3*R*)-2-(4-chlor-3-methoxyphenyl)-4,4,4-trifluor-3-methylbutanoyl]amino}-4-fluorphenyl)propanoat | ¹H-NMR (400 MHz, DMSO-d₆, δ/ppm): 10.00 (1H, s), 7.64 (1H, d), 7.41 (1H, d), 7.20 (1H, s), 7.13 (1H, t), 7.03-6.94 (2H, m), 4.07 (1H, d), 3.87 (3H, s), 3.48-3.34 (1H, m), 2.74 (2H, t), 2.45 (2H, t), 1.30 (9H, s), 0.82 (3H, d). |
| | 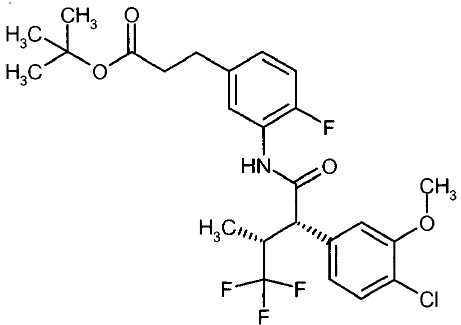 | |
| | | |
| | | LC-MS (Methode 6): Rₜ = 1.38 min; m/z = 516/518 (M-H)⁻. |
| | (aus (2*S*,3*R*)-2-(4-Chlor-3-methoxyphenyl)-4,4,4-trifluor-3-methylbutansäure und *tert*.-Butyl-3-(3-amino-4-fluorphenyl)propanoat) | |

### Beispiel 249A

### Diethyl-[2-(4-chlorphenyl)propan-2-yl]malonat

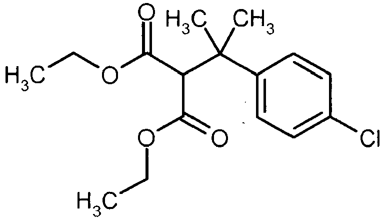

Unter Argon wurden 254 mg (10.45 mmol) Magnesiumspäne in 5 ml Diethylether langsam mit 1 g (5.23 mmol) 1-Brom-4-chlorbenzol in 2.5 ml Diethylether versetzt. Nach Einsetzen der Reaktion wurde dem Reaktionsgemisch weitere 1 g (5.23 mmol) 1-Brom-4-chlorbenzol in 2.5 ml Diethylether zudosiert. Die Reaktionsmischung wurde 30 min bei Raumtemperatur nachgerührt, mit 103 mg (1.05 mmol) Kupfer(I)chlorid versetzt und dann auf -10°C abgekühlt. Anschließend wurden langsam 2.09 g (10.45 mmol) Diethyl-propan-2-ylidenmalonat hinzugetropft. Die Reaktionsmischung wurde nachfolgend auf Rückfluss erhitzt und 3 h bei dieser Temperatur nachgerührt. Danach wurden sehr langsam 20 ml eiskalte 1 M Salzsäure zugesetzt. Nach Trennung der Phasen wurde die wässrige Phase noch dreimal mit Diethylether extrahiert. Die vereinigten organischen Phasen wurden über Magnesiumsulfat getrocknet und dann bis zur Trockene eingeengt. Das Rohprodukt wurde durch präparative HPLC gereinigt (Eluent Methanol/Wasser 70:30). Es wurden so 800 mg der Titelverbindung erhalten (25% d. Th.).

MS (DCI): m/z = 330 (M+NH₄)⁺.

GC-MS (Methode 1): Rₜ = 6.19 min; m/z = 312 (M)⁺.

### Beispiel 250A

### Ethyl-3-(4-chlorphenyl)-3-methylbutanoat

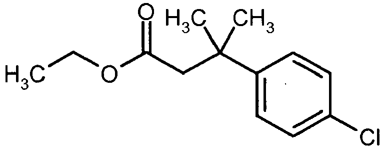

Eine Lösung von 796 mg (2.55 mmol) Diethyl-[2-(4-chlorphenyl)propan-2-yl]malonat, 216 mg (5.10 mmol) Lithiumchlorid und 46 µl (2.55 mmol) Wasser in 5 ml DMSO wurden auf Rückfluss erhitzt und 4 h bei dieser Temperatur gerührt. Nach Abkühlung auf Raumtemperatur wurden 20 ml Diethylether und 20 ml Wasser zur Reaktionsmischung gegeben. Nach Trennung der Phasen wurde die organische Phase noch dreimal mit Wasser gewaschen, und die organische Phase wurde über Magnesiumsulfat getrocknet und anschließend bis zur Trockene eingeengt. Das Rohprodukt wurde durch präparative HPLC gereinigt (Eluent Methanol/Wasser 70:30). Es wurden 276 mg der Titelverbindung erhalten (45% d. Th.).

MS (DCI): m/z = 258 (M+NHₐ)⁺.

GC-MS (Methode 1): Rₜ = 4.99 min; m/z = 240/242 (M)⁺.

### Beispiel 251A

### Ethyl-3-(4-chlor-3-nitrophenyl)-3-methylbutanoat

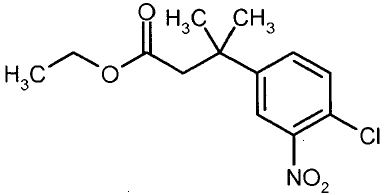

276 mg (1.45 mmol) Ethyl-3-(4-chlorphenyl)-3-methylbutanoat wurden in 10 ml Dichlormethan gelöst und auf 0°C gekühlt. Anschließend wurden portionsweise 278 mg (1.38 mmol) Nitroniumtetrafluoroborat zugegeben und die Mischung 4 h bei einer Temperatur zwischen 0°C und 10°C gerührt. Danach wurden 10 ml Wasser sowie 10 ml Dichlormethan zugesetzt und die Phasen getrennt. Die organische Phase wurde über Magnesiumsulfat getrocknet und bis zur Trockene eingeengt. Der Rückstand wurde durch Chromatographie an Silicagel gereinigt (Laufmittel Cyclohexan/ Ethylacetat 10:1). Es wurden 223 mg der Titelverbindung erhalten (68% d. Th.).

MS (DCI): m/z = 303 (M+NH₄)⁺.

GC-MS (Methode 1): Rₜ = 6.39 min; m/z = 285 (M)⁺.

¹H-NMR (400 MHz, DMSO-*d*₆): δ [ppm] = 1.00 (t, 3H), 1.38 (s, 6H), 2.74 (s, 2H), 3.89 (q, 2H), 7.66-7.76 (m, 2H), 8.03 (d, 1H).

### Beispiel 252A

### Ethyl-3-(3-amino-4-chlorphenyl)-3-methylbutanoat

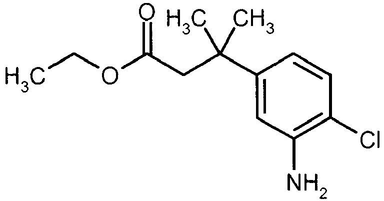

Zu einer Lösung von 213 mg (0.75 mmol) Ethyl-3-(4-chlor-3-nitrophenyl)-3-methylbutanoat in 10 ml Essigsäureethylester wurden 40 mg Palladium auf Kohle (10%) gegeben. Das Reaktionsgemisch wurde über Nacht bei RT mit 1 bar Wasserstoffdruck hydriert. Anschließend wurde über Celite filtriert und das Filtrat eingeengt. Es wurden 166 mg (87% d. Th.) der Zielverbindung als gelbliches Öl erhalten.

LC-MS (Methode 6): Rₜ = 1.05 min; m/z = 256/258 (M+H)⁺.

Analog zu Beispiel 235A wurde die folgende Verbindung erhalten:

| **Beispiel** | **Name / Struktur / Edukte** | **Analytische Daten** |
|---|---|---|
| **253A** | Ethyl-3-(4-chlor-3-{[(2*S*,3*R*)-2-(4-chlorphenyl)-4,4,4-trifluor-3-methylbutanoyl]amino}phenyl)-3-methylbutanoat | LC-MS (Methode 6): Rₜ = 1.42 min; m/z = 504/506 (M+H)⁺. |
| | 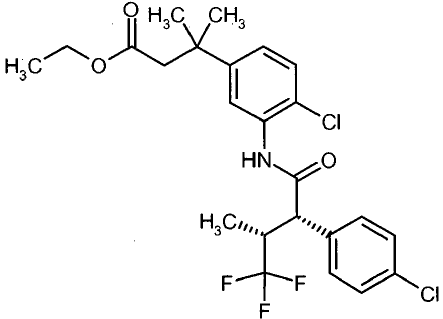 | ¹H-NMR (400 MHz, DMSO-*d*₆): δ [ppm] = 0.80 (d, 3H), 0.98 (t, 3H), 1.32 (s, 6H), 2.58 (s, 2H), 3.30-3.43 (m, 1H, teilweise verdeckt durch H₂O-Signal), 3.86 (q, 2H), 4.14 (d, 1H), 7.20 (dd, 1H), 7.35 (d, 1H), 7.43-7.50 (m, 4H), 7.55 (d, 1H), 9.82 (s, 1H). |
| | (aus Ethyl-3-(3-amino-4-chlorphenyl)- 3-methylbutanoat und (2*S*,3*R*)-2-(4-Chlorphenyl)-4,4,4-trifluor-3-methylbutansäure) | |

### Ausführungsbeispiele:

### Allgemeine Vorschrift 2: Spaltung von tert.-Butylestern zu den entsprechenden Carbonsäuren mit Trifluoressigsäure

Zu einer Lösung des betreffenden *tert*.-Butylesters in Dichlormethan (Konzentration ca. 0.1 bis 2.0 mol/l; zusätzlich optional ein Tropfen Wasser) wird bei 0°C bis RT tropfenweise Trifluoressigsäure (TFA) hinzugefügt, bis ein Dichlormethan/TFA-Verhältnis von ca. 2:1 bis 1:2 (v/v) erreicht ist. Die Mischung wird 1-24 h bei RT gerührt; gegebenenfalls wird die Mischung bis auf 40°C erwärmt, bis vollständiger Umsatz erreicht ist. Danach wird das Reaktionsgemisch im Vakuum eingeengt. Das Rohprodukt kann durch Chromatographie an Silicagel (Elution mit Dichlormethan/ Ethylacetat- oder Cyclohexan/Ethylacetat-Gemischen, gegebenenfalls unter Zusatz geringer Mengen Essigsäure, oder mit Dichlormethan/Methanol-Gemischen), durch Kristallisation aus Acetonitril oder Wasser/Acetonitril-Gemischen oder durch präparative RP-HPLC (Eluent: Acetonitril/ Wasser-Gradient) gereinigt werden.

Die folgenden Beispiele wurden gemäß der Allgemeinen Vorschrift 2 hergestellt:

| **Beispiel** | **Name / Struktur / Edukt** | **Analytische Daten** |
|---|---|---|
| **1** | (+)-3-(4-Chlor-3-{[(2*S*,3*R*)-4,4,4-trifluor-2-(4-isopropylphenyl)-3-methylbutanoyl]amino}-phenyl)propansäure | ¹H-NMR (400 MHz, DMSO-d₆, δ/ppm): 12.65-11.44 (1H, br. s), 9.72 (1H, s), 7.45 (1H, d), 7.35 (3H, t), 7.24 (2H, d), 7.03 (1H, dd), 4.07 (1H, d), 3.39-3.24 (1H, m), 2.94-2.81 (1H, m), 2.76 (2H, t), 2.48 (2H, t), 1.19 (6H, d), 0.79 (3H, d). |
| | 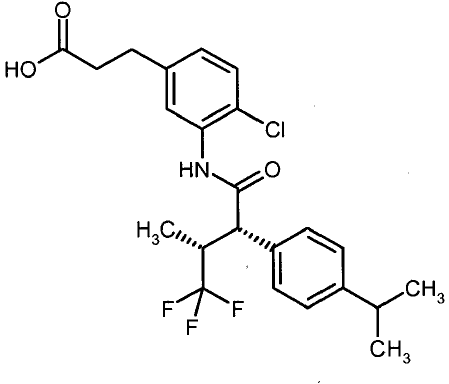 | |
| | | |
| | | LC-MS (Methode 5): Rₜ = 2.69 min; m/z = 456 (M+H)⁺. |
| | | |
| | (aus *tert*.-Butyl-3-(4-chlor-3-{[(2*S*,3*R*)-4,4,4-trifluor-2-(4-isopropylphenyl)-3-methylbutanoyl]amino}-phenyl)propanoat) | [α]_{D}²⁰ = +102.5 °, c = 0.44, Methanol. |
| **2** | (+)-3-(3-{[(2*S*,3*R*)-2-(4-*tert*.-Butylphenyl)-4,4,4-trifluor-3-methylbutanoyl]amino}-4-chlorphenyl)-propansäure | ¹H-NMR (400 MHz, DMSO-d₆, δ/ppm): 12.11 (1H, s), 9.72 (1H, s), 7.47 (1H, d), 7.41-7.35 (4H, m), 7.33 (1H, d), 7.03 (1H, dd), 4.08 (1H, d), 3.39-3.24 (1H, m), 2.76 (2H, t), 2.48 (2H, t), 1.27 (9H, s), 0.79 (3H, d). |
| | 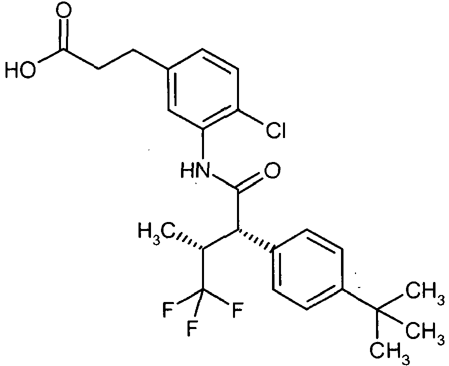 | |
| | | |
| | | LC-MS (Methode 4): Rₜ = 1.47 min; m/z = 470 (M+H)⁺. |
| | | |
| | | [α]_{D}²⁰ = +94.9°, c = 0.42, Methanol. |
| | (aus *tert*.-Butyl-3-(3-{[(2*S*,3*R*)-2-(4-*tert*.-butyl-phenyl)-4,4,4-trifluor-3-methylbutanoyl]amino}-4-chlorphenyl)propanoat) | |
| **3** | 3-[4-Chlor-3-({(2*S*,3*R*)-4,4,4-trifluor-3-methyl-2-[4-(trifluormethyl)phenyl]butanoyl}amino)-phenyl]propansäure | ¹H-NMR (400 MHz, DMSO-d₆, δ/ppm): 12.49-11.83 (1H, br. s), 9.89 (1H, s), 7.77 (2H, d), 7.69 (2H, d), 7.39 (1H, d), 7.35 (1H, d), 7.05 (1H, dd), 4.24 (1H, d), 3.59-3.26 (1H, m), 2.76 (2H, t), 2.48 (2H, t), 0.80 (3H, d). |
| | 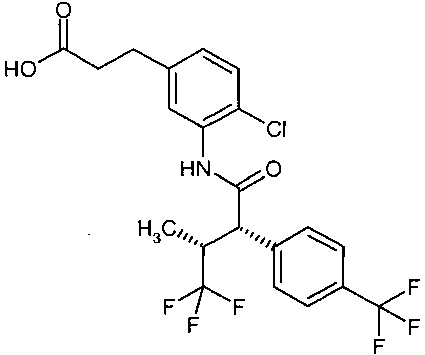 | |
| | | |
| | | LC-MS (Methode 4): Rₜ = 1.34 min; m/z = 482 (M+H)⁺. |
| | (aus *tert*.-Butyl-3-[4-chlor-3-({(2*S*,3*R*)-4,4,4-trifluor-3-methyl-2-[4-(trifluormethyl)phenyl]butanoyl}-amino)phenyl] propanoat) | |
| **4** | (+)-3-[4-Chlor-3-({(2*S*,3*R*)-4,4,4-trifluor-3-methyl-2-[4-(1,1,1-trifluor-2-methylpropan-2-yl)phenyl]-butanoyl}amino)phenyl]propansäure | ¹H-NMR (400 MHz, DMSO-d₆, δ/ppm): 12.11 (1H, s), 9.78 (1H, s), 7.54 (2H, d), 7.51-7.42 (3H, m), 7.34 (1H, d), 7.03 (1H, dd), 4.14 (1H, d), 3.42-3.26 (1H, m), 2.76 (2H, t), 2.48 (2H, t), 1.55 (6H, s), 0.79 (3H, d). |
| | 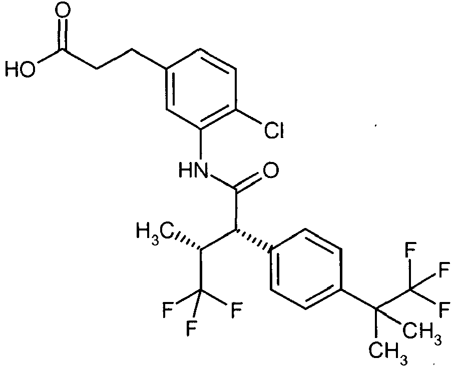 | |
| | | |
| | | LC-MS (Methode 6): Rₜ = 1.24 min; m/z = 524 (M+H)⁺. |
| | | |
| | | [α]_{D}²⁰ = +72.1 °, c = 0.43, Methanol. |
| | (aus *tert*.-Butyl-3-[4-chlor-3-({(2*S*,3*R*)-4,4,4-trifluor-3-methyl-2-[4-(1,1,1-trifluor-2-methylpropan-2-yl)-phenyl]butanoyl}amino)phenyl]propanoat) | |
| **5** | 3-[4-Chlor-3-({4,4,4-trifluor-3-methyl-2-[4-(2,2,2-trifluorethyl)phenyl]butanoyl}amino)phenyl]-propansäure | ¹H-NMR (400 MHz, DMSO-d₆, δ/ppm): 12.11 (1H, s), 9.79 (1H, s), 7.46 (2H, d), 7.41 (1H, d), 7.35 (3H, t), 7.04 (1H, dd), 4.11 (1H, d), 3.64 (2H, q), 3.44-3.26 (1H, m), 2.76 (2H, t), 2.48 (2H, t), 0.79 (3H, d). |
| | 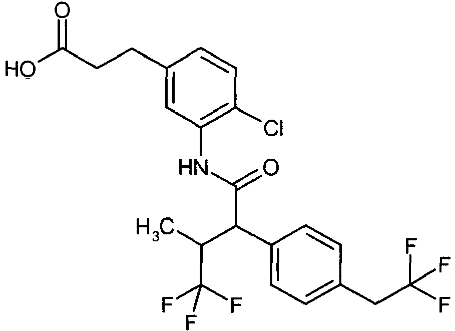 | |
| | | |
| | | LC-MS (Methode 4): Rₜ = 1.33 min; m/z = 496 (M+H)⁺. |
| | (aus *tert*.-Butyl-3-[4-chlor-3-({4,4,4-trifluor-3-methyl-2-[4-(2,2,2-trifluorethyl)phenyl]butanoyl}-amino)phenyl]propanoat) | |
| **6** | 3-(4-Chlor-3-{[(4-chlorphenyl)(3,3-difluor-cyclopentyl)acetyl]amino}phenyl)propansäure | ¹H-NMR (400 MHz, DMSO-d₆, δ/ppm): 12.13 (1H, s), 9.79 (0.5H, s), 9.75 (0.5H, s), 7.48-7.34 (6H, m), 7.07 (1H, d), 3.78 (0.5H, d), 3.75 (0.5H, d), 3.58-3.45 (0.5H, m), 3.43-3.26 (0.5H, m), 2.92-2.81 (1H, m), 2.77 (2H, t), 2.57-2.45 (2H, t), 2.44-1.80 (3H, m), 1.71-1.45 (1.5H, m), 1.35-1.20 (0.5H, m). |
| | 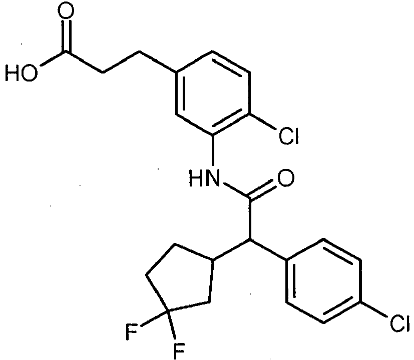 | |
| | | |
| | (aus *tert*.-Butyl-3-(4-chlor-3-{[(4-chlorphenyl)(3,3-difluorcyclopentyl)acetyl]amino}phenyl)propanoat) | LC-MS (Methode 6): Rₜ = 1.15 min; m/z = 456/458 (M+H)⁺. |
| **7** | 3-(4-Chlor-3- {[(2*S*,3*R*)-2-(4-ethylphenyl)-4,4,4-trifluor-3-methylbutanoyl]-amino}phenyl)butansäure (*Diastereomerengemisch*) | LC-MS (Methode 6): Rₜ = 1.24 min; m/z = 456 (M+H)⁺. |
| | | |
| | 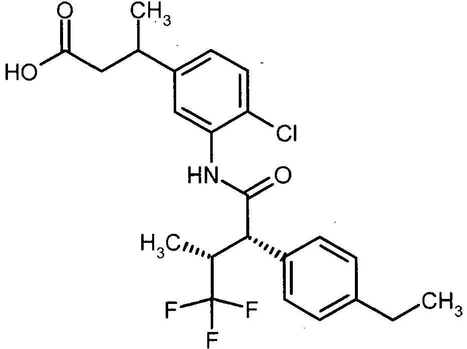 | ¹H-NMR (400 MHz, DMSO-*d*₆): δ [ppm] = 0.79 (d, 3H), 1.12-1.21 (m, 6H), 2.45 (d, 2H), 2.59 (q, 2H), 3.03-3.13 (m, 1H), 3.32 (d, 1H), 4.07 (d, 1H), 7.06 (d, 1H), 7.20 (d, 2H), 7.31-7.38 (m, 3H), 7.47 (d, 1H), 9.69 (s, 1H), 12.03 (br. s, 1H). |
| **8** | 3-(3-{[(2*S*,3*R*)-2-(4-Chlorphenyl)-4,4,4-trifluor-3-methylbutanoyl]amino}-4-chlorphenyl)butansäure *(Diastereomerengemisch)* | LC-MS (Methode 4): Rₜ = 1.45 min; m/z = 462 (M+H)⁺. |
| | | |
| | 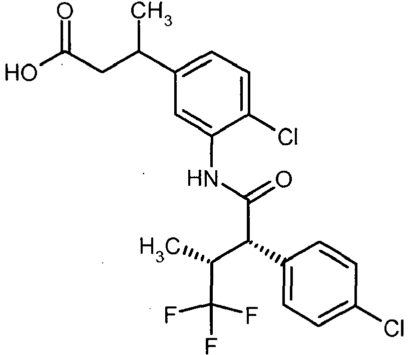 | ¹H-NMR (400 MHz, DMSO-*d*₆): δ [ppm] = 0.80 (d, 3H), 1.16 (d, 3H), 2.45 (d, 2H), 3.01-3.14 (m, 1H), 3.34-3.42 (m, 1H), 4.13 (d, 1H), 7.08 (dd, 1H), 7.36 (d, 1H), 7.40-7.50 (m, 5H), 9.81 (s, 1H), 12.06 (s, 1H). |
| **9** | 3-(3- {[(2*S*,3*R*)-2-(4-Chlorphenyl)-4,4,4-trifluor-3-methylbutanoyl]amino}-4-fluorphenyl)butansäure *(Diastereomerengemisch)* | LC-MS (Methode 5): Rₜ = 2.47 min; m/z = 446 (M+H)⁺. |
| | | |
| | 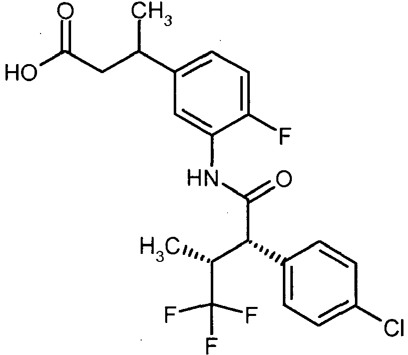 | ¹H-NMR (400 MHz, DMSO-*d₆*): δ [ppm] = 0.79 (d, 3H), 1.15/1.16 (2d, zus. 3H), 2.44 (d, 2H), 3.02-3.13 (m, 1H), 3.34-3.42 (m, 1H), 4.12 (d, 1H), 6.97-7.06 (m, 1H), 7.13 (dd, 1H), 7.40-7.49 (m, 4H), 7.67 (d, 1H), 10.03 (s, 1H), 12.05 (br. s, 1H). |
| **10** | (3*S*)-3-(4-Chlor-3-{[(4-chlorphenyl)(2,2-difluor-cyclopentyl)acetyl]amino}phenyl)butansäure (*Diastereomerengemisch*) | LC-MS (Methode 6): Rₜ = 1.17 min; m/z = 470 (M+H)⁺ und Rₜ = 1.19 min; m/z = 470 (M+H)⁺. |
| | 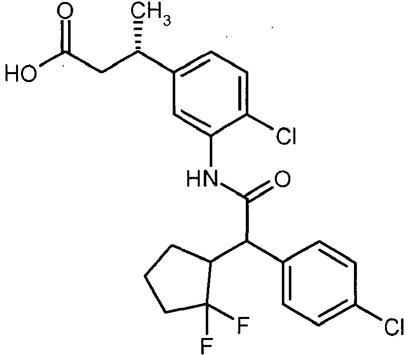 | |
| | | ¹H-NMR (400 MHz, DMSO-*d₆*): δ [ppm] = 1.11-1.19 (m, 3H), 1.45-1.78 (m, 3H), 2.00-2.26 (m, 2H), 2.45/2.46 (2d, zus. 2H), 2.88-3.17 (m, 2H), 4.04/4.07 (2d, zus. 1H), 6.99-7.13 (m, 1H), 7.30-7.44 (m, 3H), 7.44-7.52 (m, 2H), 9.64/ 9.84 (2s, zus. 1H), 12.07 (br. s, 1H). |
| **11** | 3-(4-Chlor-3-{[(4-chlorphenyl)(2,2-difluor-cyclopentyl)acetyl]amino}phenyl)propansäure (*Diastereomerengemisch*) | LC-MS (Methode 6): Rₜ = 1.16 min; m/z = 456 (M+H)⁺ und Rₜ = 1.18 min; m/z = 456 (M+H)⁺. |
| | 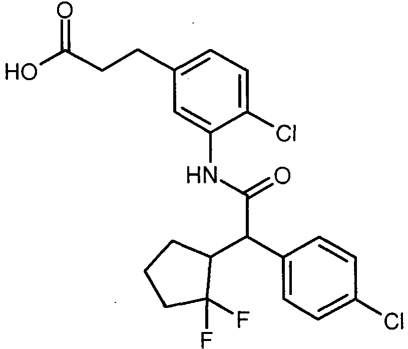 | |
| | | ¹H-NMR (400 MHz, DMSO-*d₆*): δ [ppm] = 1.11-1.81 (m, 3H), 1.96-2.26 (m, 2H), 2.48 (t, 2H), 2.76 (t, 2H), 2.86-3.20 (m, 1H), 4.03/4.07 (2d, zus. 1H), 6.98-7.09 (m, 1H), 7.29-7.54 (m, 6H), 9.64/9.85 (2s, zus. 1H), 12.09 (br. s, 1H). |

### Beispiel 12

### (+)-3-(4-Fluor-3-{[(2S,3R)-4,4,4-trifluor-3-methyl-2-(4-vinylphenyl)butanoyl]amino}phenyl)-propansäure

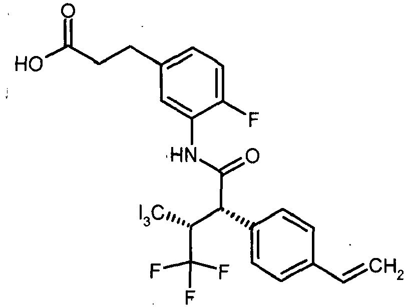

### Beispiel 13

### (+)-3-(4-Chlor-3-{[(2S,3R)-4,4,4-trifluor-3-methyl-2-(4-vinylphenyl)butanoyl]amino}phenyl)-propansäure

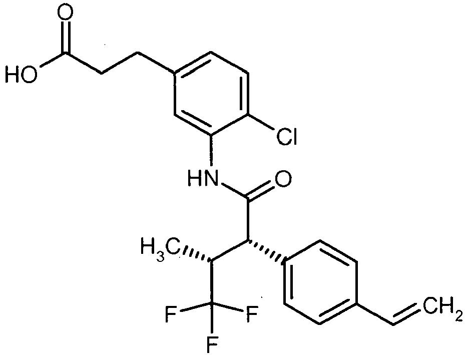

249.0 mg (0.502 mmol) (+)-3-(4-Chlor-3-{[(2*S*,3*R*)-4,4,4-trifluor-3-methyl-2-(4-vinylphenyl)-butanoyl]amino}phenyl)propansäure-*tert*.-butylester wurden in 3.8 ml einer 4 N Lösung von Chlorwasserstoff in Dioxan gelöst und 24 h bei RT gerührt. Anschließend wurde die Reaktionsmischung eingefroren (-78°C) und dann im Hochvakuum lyophilisiert. Der Rückstand wurde durch präparative RP-HPLC gereinigt (Eluent: Acetonitril/Wasser-Gradient). Es wurden 167.4 mg (75.8% d. Th.) der Titelverbindung erhalten.

LC-MS (Methode 6): Rₜ = 1.16 min; m/z = 440 (M+H)⁺.

¹H-NMR (400 MHz, DMSO-*d₆*): δ [ppm] = 0.80 (d, 3H), 2.48 (t, 2H), 2.76 (t, 2H), 3.35-3.42 (m, 1H), 4.09 (d, 1H), 5.27 (d, 1H), 5.84 (d, 1H), 6.73 (dd, 1H), 7.04 (dd, 1H), 7.34 (d, 1H), 7.39-7.52 (m, 5H), 9.79 (s, 1H), 12.14 (br. s, 1H).

[α]_{D}²⁰ = +88.8°, c = 0.325, Chloroform.

### Beispiel 14

### (+)-3-[4-Chlor-3-({(2S,3R)-4,4,4-trifluor-2-[4-(1-fluorvinyl)phenyl]-3-methylbutanoyl}amino)-phenyl]propansäure

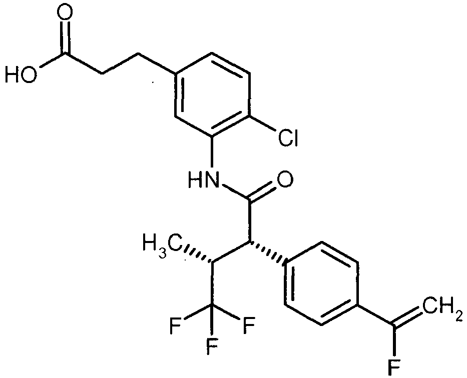

Eine auf 0°C gekühlte Lösung von 212 mg (0.449 mmol) (+)-3-[4-Chlor-3-({(2*S*,3*R*)-4,4,4-trifluor-2-[4-(1-fluorvinyl)phenyl]-3-methylbutanoyl} amino)phenyl]propansäuremethylester in einem Gemisch aus je 1.0 ml Methanol, THF und Wasser wurde mit 16.1 mg (0.674 mmol) Lithiumhydroxid versetzt. Die Mischung wurde anschließend auf RT erwärmt und 3 h bei RT gerührt, dann mit Wasser verdünnt und mit 1 N Salzsäure sauer gestellt (pH ca. 2). Es wurde dreimal mit Ethylacetat extrahiert. Die organischen Phasen wurden vereinigt und im Vakuum eingeengt. Das Rohprodukt wurde zunächst durch RP-HPLC vorgereinigt (Eluent: Acetonitril/Wasser-Gradient). Das im Zuge der basischen Hydrolyse entstandene 2*R*-Diastereomer wurde anschließend durch präparative HPLC an chiraler Phase abgetrennt [Säule: Daicel Chiralpak AD-H, 5 µm, 250 mm x 20 mm; Injektionsvolumen: 0.25 ml; Temperatur: 35°C; Eluent: 90% Isohexan / 10% Ethanol; Fluss: 15 ml/min; Detektion: 220 nm]. Es wurden so 74.0 mg (36.0% d. Th.) der Titelverbindung erhalten.

LC-MS (Methode 4): Rₜ = 1.33 min; m/z = 458 (M+H)⁺.

¹H-NMR (400 MHz, DMSO-d₆): δ [ppm] = 0.81 (d, 3H), 2.47 (t, 2H), 2.76 (t, 2H), 3.35-3.43 (m, 1H), 4.15 (d, 1H), 4.95 (dd, 1H), 5.39 (dd, 1 H), 7.04 (dd, 1H), 7.26-7.44 (m, 2H), 7.44-7.59 (m, 2H), 7.59-7.68 (m, 2H), 9.82 (s, 1H), 12.11 (br. s, 1H).

[α]_{D}²⁰ = +69.2°, c = 0.405, Chloroform.

### Beispiel 15

### (+)-3-(4-Chlor-3-{[(2S,3R)-2-(4-chlorphenyl)-4,4,4-trifluor-3-methylbutanoyl]amino}phenyl)-propansäure

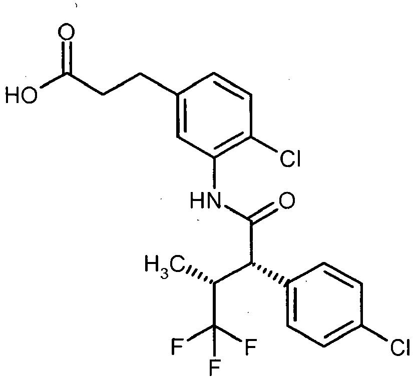

30.13 g (59.74 mmol)*tert*.-Butyl-3-(4-chlor-3-{[(2*S*,3*R*)-2-(4-chlorphenyl)-4,4,4-trifluor-3-methyl-butanoyl]amino}phenyl)propanoat wurden in 1000 ml Dichlormethan gelöst und bei RT mit 92 ml Trifluoressigsäure versetzt. Die Reaktionsmischung wurde 3.5 h bei RT gerührt. Dann wurde Dichlormethan und Wasser zugegeben. Die organische Phase wurde abgetrennt, mit Magnesium-sulfat getrocknet und im Vakuum eingeengt. Der Rückstand wurde gründlich im Hochvakuum getrocknet. Es wurden 26.31 g (98.3% d. Th.) der Zielverbindung erhalten.

LC-MS (Methode 7): Rₜ = 2.51 min; m/z = 446/448 (M-H)⁻.

¹H-NMR (400 MHz, DMSO-d₆, δ/ppm): 12.14 (1H, s), 9.83 (1H, s), 7.50-7.43 (4H, m), 7.39 (1H, d), 7.35 (1H, d), 7.05 (1H, dd), 4.12 (1H, d), 3.43-3.28 (1H, m), 2.76 (2H, t), 2.48 (2H, t), 0.80 (3H, d).

[α]_{D}²⁰ = +100.1°, c = 0.42, Methanol.

### Beispiel 16

### (+)-(2R)-3-(4-Chlor-3-{[(2S,3R)-2-(4-chlorphenyl)-4,4,4-trifluor-3-methylbutanoyl]amino}-phenyl)-2-methylpropansäure

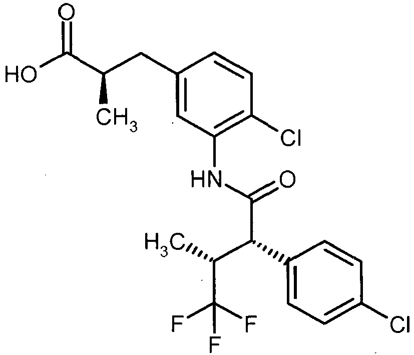

990 mg (2.02 mmol) Ethyl-(2*R*)-3-(4-chlor-3-{[(2*S*,3*R*)-2-(4-chlorphenyl)-4,4,4-trifluor-3-methyl-butanoyl]amino}phenyl)-2-methylpropanoat wurden in 5.7 ml Essigsäure gelöst und mit 2.7 ml konzentrierter Salzsäure versetzt. Die Mischung wurde 1 h bei 100°C gerührt. Nach dem Abkühlen wurde der Ansatz im Vakuum eingeengt. Der Rückstand wurde in Ethylacetat aufgenommen und mehrfach mit Wasser unter Zusatz von einigen Tropfen gesättigter NatriumhydrogencarbonatLösung gewaschen. Die organische Phase wurde über Magnesiumsulfat getrocknet und im Vakuum eingeengt. Der Rückstand wurde durch Chromatographie an Silicagel gereinigt (Laufmittel: zunächst Dichlormethan, dann Dichlormethan/Ethylacetat 10:1). Es wurden 652 mg (69.9% d. Th.) der Titelverbindung erhalten.

LC-MS (Methode 6): Rₜ = 1.21 min; m/z = 462 (M+H)⁺.

¹H-NMR (400 MHz, DMSO-d₆): δ [ppm] = 0.80 (d, 3H), 1.02 (d, 3H), 2.55-2.62 (m, 2H), 2.78-2.88 (m, 1H), 3.35-3.43 (m, 1H), 4.12 (d, 1H), 7.01 (dd, 1H), 7.32-7.39 (m, 2H), 7.42-7.50 (m, 4H), 9.83 (s, 1H), 12.16 (br. s, 1H).

[α]_{D}²⁰ = +60.56°, c = 0.530, Chloroform.

### Beispiel 17

### (+)-(2S)-3-(4-Chlor-3-{[(2S,3R)-2-(4-chlophenyl)-4,4,4-trifluor-3-methylbutanoyl]amino}-phenyl)-2-methylpropansäure

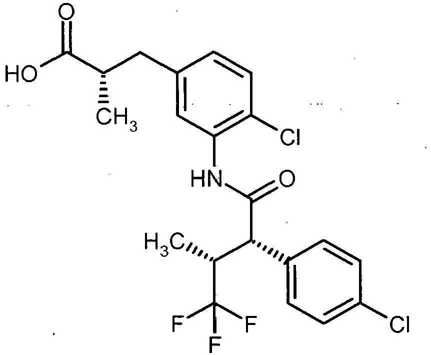

### Methode A:

Eine Mischung aus 2.45 g (5.0 mmol) Ethyl-(2*S*)-3-(4-chlor-3-{[(2*S*,3*R*)-2-(4-chlophenyl)-4,4,4-trifluor-3-methylbutanoyl]amino}phenyl)-2-methylpropanoat, 6.0 ml Essigsäure und 20 ml 20%-iger wässriger Schwefelsäure wurde 7 h unter Rückfluss gerührt. Nach dem Abkühlen wurde die Reaktionsmischung auf Wasser gegeben. Die wässrige Phase wurde dreimal mit Ethylacetat extrahiert, und die vereinigten organischen Phasen wurden im Vakuum eingeengt. Der Rückstand wurde erneut in Ethylacetat aufgenommen und mehrfach mit Wasser unter Zusatz von einigen Tropfen gesättigter Natriumhydrogencarbonat-Lösung gewaschen. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 10:1 → 4:1). Es wurden 1.88 g (81.4% d. Th.) der Titelverbindung erhalten.

LC-MS (Methode 6): Rₜ = 1.22 min; m/z = 462 (M+H)⁺.

¹H-NMR (400 MHz, DMSO-d₆): δ [ppm] = 0.80 (d, 3H), 1.02 (d, 3H), 2.55-2.61 (m, 2H), 2.78-2.88 (m, 1H), 3.35-3.43 (m, 1H), 4.12 (d, 1H), 7.01 (dd, 1H), 7.32-7.39 (m, 2H), 7.43-7.50 (m, 4H), 9.83 (s, 1H), 12.16 (br. s, 1H).

[α]_{D}²⁰ = +101.2°, c = 0.590, Chloroform.

### Methode B:

Eine Mischung aus 12.99 g (26.49 mmol) (+)-Ethyl-(2*S*)-3-(4-chlor-3-{[(2*S*,3*R*)-2-(4-chlorphenyl)-4,4,4-trifluor-3-methylbutanoyl]amino}phenyl)-2-methylpropanoat, 60 ml Essigsäure und 60 ml 30%-iger wässriger Schwefelsäure wurde 3 h unter Rückfluss gerührt (Badtemperatur 140°C). Nach dem Abkühlen wurde die Reaktionsmischung auf Wasser gegeben. Die wässrige Phase wurde dreimal mit Ethylacetat extrahiert, und die vereinigten organischen Phasen wurden mit gesättigter Natriumchlorid-Lösung gewaschen, über Natriumsulfat getrocknet und im Vakuum eingeengt. Der Rückstand wurde über Nacht im Hochvakuum getrocknet. Das so erhaltene Rohprodukt wurde mit 90 ml Diisopropylether zunächst 1 h bei 50°C und dann 4 h bei RT gerührt. Nach Filtration wurde der Feststoff im Hochvakuum getrocknet. Es wurden 7.84 g (64% d. Th.) der Zielverbindung erhalten (Fraktion 1). Aus dem Filtrat wurde nach Einengen und erneuter Behandlung mit 30 ml Diisopropylether eine weitere Charge isoliert. Nach Trocknen im Hochvakuum wurden 1.65 g (13.5% d. Th.) an leicht verunreinigter Zielverbindung erhalten (Fraktion 2).

LC-MS (Methode 4): Rₜ = 1.39 min; m/z = 461/463 (M+H)⁺.

¹H-NMR (400 MHz, DMSO-d₆): δ [ppm] = 0.80 (d, 3H), 1.02 (d, 3H), 2.55-2.61 (m, 2H), 2.77-2.88 (m, 1H), 3.34-3.43 (m, 1H), 4.12 (d, 1H), 7.01 (dd, 1H), 7.31-7.39 (m, 2H), 7.41-7.51 (m, 4H), 9.83 (s, 1H), 12.15 (s, 1H).

[α]_{D}²⁰ = +127.6°, c = 0.575, Chloroform.

### Beispiel 18

### (+)-[1-(3-{[(2S,3R)-2-(4-Chlorphenyl)-4,4,4-frifluor-3-methylbutanoyl]amino}-4-fluorphenyl)-cyclopropyl]essigsäure

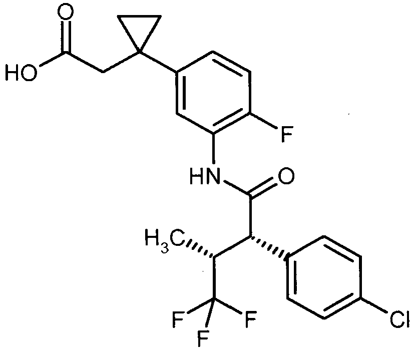

106 mg (0.23 mmol) Methyl-[1-(3-{[(2*S*,3*R*)-2-(4-chlorphenyl)-4,4,4-trifluor-3-methylbutanoyl]-amino}-4-fluorphenyl)cyclopropyl]acetat wurden in 4 ml Eisessig und 2 ml konzentrierter Salzsäure gelöst und 1 h bei 100°C gerührt. Anschließend wurde das Reaktionsgemisch mit 10 ml Wasser verdünnt und nachfolgend die wässrige Lösung dreimal mit jeweils 10 ml Essigsäureethylester extrahiert. Die vereinigten organischen Phasen wurden über Magnesiumsulfat getrocknet und im Vakuum eingeengt. Das erhaltene Rohprodukt wurde durch präparative RP-HPLC gereinigt (Eluent: Acetonitril/Wasser-Gradient). Es wurden 64 mg der Titelverbindung erhalten (0.14 mmol, 89% d. Th.).

LC-MS (Methode 4): Rₜ = 1.34 min; m/z = 458 (M+H)⁺.

¹H-NMR (400 MHz, DMSO-*d*₆, δ/ppm): 10.03 (1H, s), 7.69-7.59 (1H, m), 7.52-7.34 (4H, m), 7.11-7.00 (2H, m), 4.11 (1H, d), 3.43-3.27 (1H, m), 2.36 (2H, s), 0.88-0.82 (2H, m), 0.78 (3H, d), 0.72-0.64 (2H, m).

[α]_{D}²⁰ = +108.7°, c = 0.36, Methanol.

### Allgemeine Vorschrift 3: Spaltung von Ethyl- oder Methylestern zu den entsprechenden Carbonsäuren mit einem Gemisch von Salzsäure oder Schwefelsäure mit Essigsäure

Eine Lösung des betreffenden Ethyl- oder Methylesters in einem Gemisch aus Essigsäure und konzentrierter Salzsäure oder aus Essigsäure und 10%-iger oder halbkonzentrierter Schwefelsäure wird bei Temperaturen von 80°C bis 130°C (gegebenenfalls unter Rückfluss) für 30 min bis 12 h gerührt. Nach dem Abkühlen wird die Reaktionsmischung entweder direkt im Vakuum eingeengt oder auf Wasser gegeben, die wässrige Phase mit Ethylacetat oder Dichlormethan extrahiert und die vereinigten organischen Phasen im Vakuum eingeengt. Das Rohprodukt kann durch Chromatographie an Silicagel (Elution mit Dichlormethan/Ethylacetat- oder Cyclohexan/Ethylacetat-Gemischen, gegebenenfalls unter Zusatz geringer Mengen Essigsäure, oder mit Dichlormethan/ Methanol-Gemischen), durch Kristallisation aus Acetonitril oder Wasser/Acetonitril-Gemischen oder durch präparative RP-HPLC (Eluent: Acetonitril/Wasser-Gradient) gereinigt werden.

Die folgenden Beispiele wurden gemäß der Allgemeinen Vorschrift 3 hergestellt:

| **Beispiel** | **Name / Struktur / Edukt** | **Analytische Daten** |
|---|---|---|
| **19** | (+)-[1-(3-{[(2*S*,3*R*)-2-(4-Ethylphenyl)-4,4,4-trifluor-3-methylbutanoyl]amino}-4-fluorphenyl)cyclopropyl]-essigsäure | ¹H-NMR (400 MHz, DMSO-d₆, δ/ppm): 11.97 (1H, s), 9.96 (1H, s), 7.75 (1H, dd), 7.34 (2H, d), 7.20 (2H, d), 7.09 (1H, t), 7.05-6.99 (1H, m), 4.05 (1H, d), 3.40-3.27 (1H, m), 2.59 (2H, q), 2.55-2.48 (2H, m), 1.17 (3H, t), 0.88-0.82 (2H, m), 0.79-0.74 (5H, m). |
| | 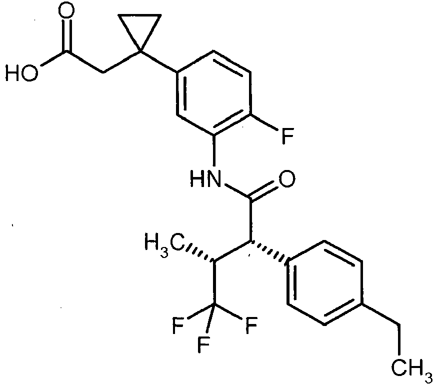 | |
| | | |
| | | LC-MS (Methode 5): Rₜ = 2.58 min; m/z = 452 (M+H)⁺. |
| | | |
| | (aus Methyl-[1-(3-{[(2*S*,3*R*)-2-(4-ethylphenyl)-4,4,4-trifluor-3-methylbutanoyl]amino}-4-fluorphenyl)cyclopropyl]acetat) | [α]_{D}²⁰ = +125.2°, c = 0.35, Methanol. |
| **20** | (2*S*)-3-(4-Chlor-3-{[(4-chlorphenyl)(3,3-difluor-cyclopentyl)acetyl]amino}phenyl)-2-methylpropansäure | LC-MS (Methode 6): Rₜ = 1.21 min; m/z = 470/472 (M)⁺. |
| | 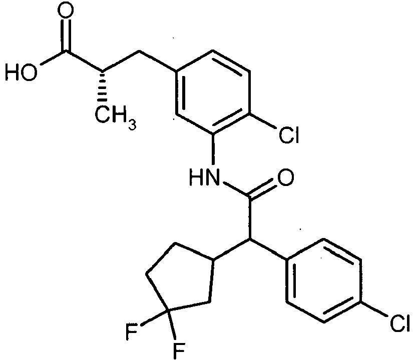 | |
| | (aus Ethyl-(2*S*)-3-(4-chlor-3-{[(4-chlorphenyl)-(3,3-difluorcyclopentyl)acetyl]amino}phenyl)-2-methylpropanoat) | |
| **21** | (2*R*)-3-(4-Chlor-3-{[(4-chlorphenyl)(3,3-difluor-cyclopentyl)acetyl]amino}phenyl)-2-methylpropansäure | LC-MS (Methode 6): Rₜ = 1.21 min; m/z = 470/472 (M)⁺. |
| | 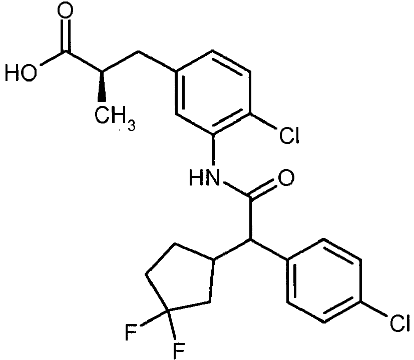 | |
| | (aus Ethyl-(2*R*)-3-(4-chlor-3-{[(4-chlorphenyl)-(3,3-difluorcyclopentyl)acetyl]amino}phenyl)-2-methylpropanoat) | |
| **22** | 3-(4-Chlor-3-{[(2*S*,3*R*)-2-(4-chlorphenyl)-4,4,4-trifluor-3-methylbutanoyl]amino}phenyl)-2-methylpropanoat (*Diastereomerengemisch*) | LC-MS (Methode 5): Rₜ = 2.57 min; m/z = 462 (M+H)⁺. |
| | | |
| | 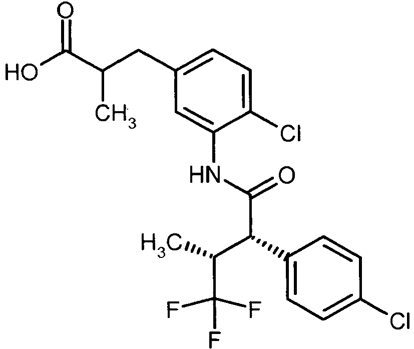 | ¹H-NMR (400 MHz, DMSO-*d₆*): δ [ppm] = 0.80 (d, 3H), 1.02 (d, 3H), 2.55-2.61 (m, 2H), 2.77-2.89 (m, 1H), 3.33-3.42 (m, 1H), 4.12 (d, 1H), 7.01 (dd, 1H), 7.30-7.40 (m, 2H), 7.41-7.51 (m, 4H), 9.82 (s, 1H), 12.14 (br. s, 1H). |
| **23** | 3-(3-{[(2*S*,3*R*)-2-(4-Ethylphenyl)-4,4,4-trifluor-3-methylbutanoyl]amino}-4-fluorphenyl)-2-methylpropansäure (*Diastereomerengemisch*) | LC-MS (Methode 6): Rₜ = 1.22 min; m/z = 440 (M+H)⁺. |
| | | |
| | 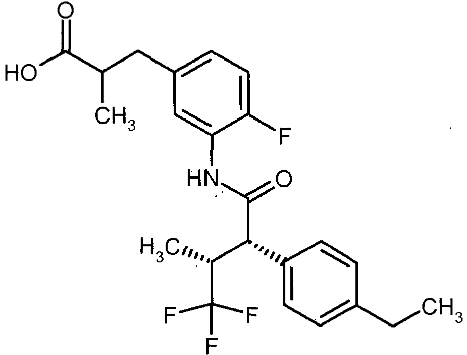 | ¹H-NMR (400 MHz, DMSO-*d₆*): δ [ppm] = 0.77 (d, 3H), 1.01 (d, 3H), 1.17 (t, 3H), 2.53-2.62 (m, ca. 4H), 2.77-2.87 (m, 1H), 3.27-3.38 (m, ca. 1H), 4.05 (d, 1H), 6.86-6.98 (m, 1H), 7.11 (dd, 1H), 7.16-7.23 (m, 2H), 7.29-7.37 (m, 2H), 7.66 (dt, 1H), 9.97 (s, 1H), 12.14 (br. s, 1H). |
| **24** | 3-(4-Chlor-3-{[(2*S*,3*R*)-2-(4-ethylphenyl)-4,4,4-trifluor-3-methylbutanoyl]amino}phenyl)-2-methylpropansäure (*Diastereomerengemisch*) | LC-MS (Methode 6): Rₜ = 1.27 min; m/z = 456 (M+H)⁺. |
| | | |
| | 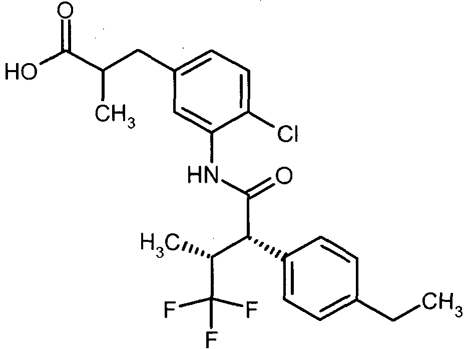 | ¹H-NMR (400 MHz, DMSO-*d₆*): δ [ppm] = 0.79 (d, 3H), 1.02 (d, 3H), 1.17 (t, 3H), 2.55-2.64 (m, ca. 4H), 2.77-2.88 (m, 1H), 3.27-3.38 (m, ca. 1H), 4.06 (d, 1H), 6.99 (dd, 1H), 7.21 (d, 2H), 7.34 (dd, 3H), 7.41 (d, 1H), 9.74 (s, 1H), 12.20 (br. s, 1H). |
| **25** | (2*S*)-3-(4-Chlor-3-{[(4-chlorphenyl)(2,2-difluor-cyclopentyl)acetyl]amino}phenyl)-2-methylpropansäure (*Diastereomerengemisch*) | LC-MS (Methode 6): Rₜ = 1.17 min; m/z = 470 (M+H)⁺ und Rₜ = 1.19 min; m/z = 470 (M+H)⁺. |
| | 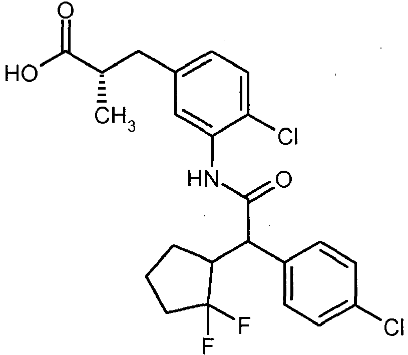 | |
| | | ¹H-NMR (400 MHz, DMSO-*d₆*): δ [ppm] = 1.02 (d, 3H), 1.11-1.26 (m, 1H), 1.48-1.78 (m, 3H), 1.99-2.24 (m, 2H), 2.55-2.62 (m, ca. 2H), 2.77-2.86 (m, 1H), 2.87-3.20 (m, 1H), 4.02/4.06 (2d, zus. 1H), 6.99/7.01 (2dd, zus. 1H), 7.30-7.45 (m, 4H), 7.45-7.51 (m, 2H), 9.64 (s, 1H), 12.14 (br. s, 1H). |
| **26** | 2-(4-Chlor-3-{[(2*S*,3*R*)-2-(4-chlorphenyl)-4,4,4-trifluor-3-methylbutanoyl]amino}benzyl)butansäure (*Diastereomerengemisch*) | LC-MS (Methode 6): Rₜ = 1.26 min; m/z = 476 (M+H)⁺. |
| | | |
| | 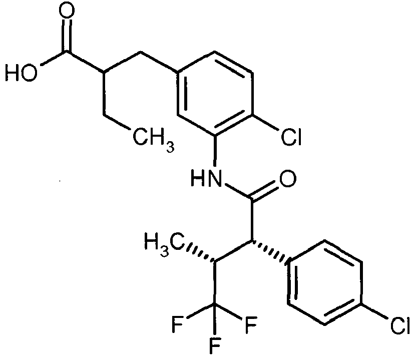 | ¹H-NMR (400 MHz, DMSO-*d₆*): δ [ppm] = 0.74-0.89 (m, 6H), 1.40-1.53 (m, 2H), 2.30-2.45 (m, 1H), 2.46-2.55 (m, ca. 1H), 2.57-2.68 (m, 1 H), 2.68-2.81 (m, 1H), 3.36-3.44 (m, ca. 1H), 4.12 (d, 1H), 7.00 (dd, 1H), 7.29-7.40 (m, 2H), 7.41-7.51 (m, 4H), 9.82 (s, 1H), 12.15 (br. s, 1H). |

### Beispiel 27

### (+)-[3-(4-Chlor-3-{[(2S,3R)-2-(4-chlorphenyl)-4,4,4-trifluor-3-methylbutanoyl]amino}phenyl)-oxetan-3-yl]essigsäure

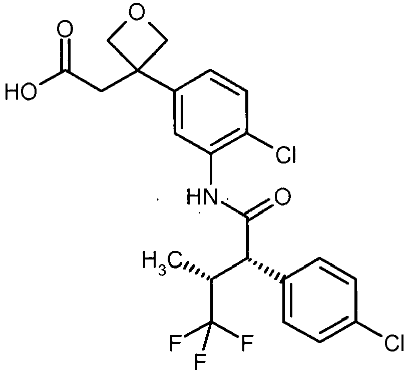

Eine Lösung von 120 mg (0.21 mmol) Benzyl-[3-(4-chlor-3-{[(2*S*,3*R*)-2-(4-chlorphenyl)-4,4,4-trifluor-3-methylbutanoyl]amino}phenyl)oxetan-3-yl]acetat in 15 ml Essigsäureethylester wurde mit 25 mg Palladium auf Kohle (10%) versetzt. Unter einer Wasserstoffatmosphäre wurde bei Normaldruck für 2 h hydriert. Das Reaktionsgemisch wurde danach über Tonsil filtriert, der FilterRückstand mit Essigsäureethylester nachgewaschen und die vereinigten Filtrate am Rotationsverdampfer eingeengt. Es wurden 98 mg (0.2 mmol, 97% d. Th.) der Titelverbindung erhalten.

LC-MS (Methode 4): Rₜ = 1.28 min; m/z = 488/490 (M-H)⁻.

¹H-NMR (400 MHz, DMSO-*d*₆, δ/ppm): 12.76-11.52 (1H, br. s), 9.88 (1H, s), 7.52 (1H, d), 7.50-7.39 (5H, m), 7.10 (1H, dd), 4.79-4.71 (2H, m), 4.71-4.64 (2H, m), 4.14 (1H, d), 3.42-3.28 (1H, m), 3.03 (2H, s), 0.80 (3H, d).

[α]_{D}²⁰ = +88.4°, c = 0.355, Methanol.

### Beispiel 28

### [1-(4-Chlor-3-{[(3R)-2-(4-chlorphenyl)-4,4,4-trifluor-3-methylbutanoyl]amino}phenyl)cyclobutyl]-essigsäure

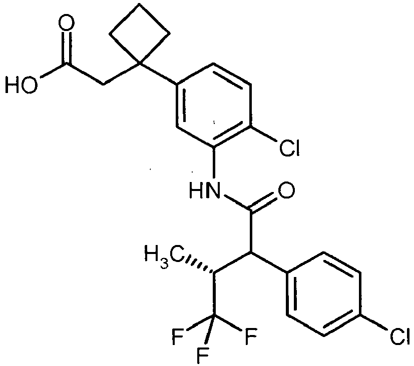

38 mg (0.08 mmol) Methyl-[1-(4-chlor-3-{[(3*R*)-2-(4-chlorphenyl)-4,4,4-trifluor-3-methylbutanoyl]-amino}phenyl)cyclobutyl]acetat wurden in 9.5 ml Dioxan gelöst und mit 0.15 ml 1 N Natronlauge versetzt. Der Ansatz wurde über Nacht bei 80°C gerührt. Das Reaktionsgemisch wurde dann mit 1 N Salzsäure auf pH 1 angesäuert und mehrmals mit Essigsäureethylester extrahiert. Die vereinigten organischen Phasen wurden mit gesättigter Natriumchlorid-Lösung gewaschen, über Natriumsulfat getrocknet und im Vakuum eingeengt. Das Rohprodukt wurde mittels präparativer HPLC gereinigt. Es wurden 22 mg (0.05 mmol, 60% d. Th.) der Zielverbindung erhalten.

LC-MS (Methode 6): Rₜ = 1.28 min; m/z = 488/490 (M+H)⁺.

¹H-NMR (400 MHz, DMSO-*d*₆, δ/ppm): 11.88 (1H, br. s), 9.95 (0.5H, s), 9.81 (0.5H, s), 7.54-7.31 (6H, m), 7.06-6.96 (1H, m), 4.14 (1H, d), 3.43-3.27 (0.5H, m), 3.27-3.14 (0.5H, m), 2.70 (1H, s), 2.69 (1H, s), 2.34-2.17 (4H, m), 2.10-1.95 (1H, m), 1.81-1.66 (1H, m), 1.25 (1.5H, d), 0.80 (1.5H, d).

### Beispiel 29

### (+)-(2R)-2-(4-Chlor-3-{[(2S,3R)-2-(4-chlorphenyl)-4,4,4-trifluor-3-methylbutanoyl]amino}benzyl)-butansäure

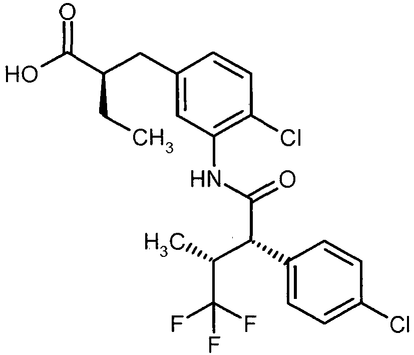

1.96 g (3.89 mmol) (+)-(2*R*)-2-(4-Chlor-3-{[(2*S*,3*R*)-2-(4-chlorphenyl)-4,4,4-trifluor-3-methyl-butanoyl]amino}benzyl)butansäureethylester wurden mit 15.2 ml Essigsäure und 7.6 ml konzentrierter Salzsäure versetzt. Die Reaktionsmischung wurde 5 h unter Rückfluss gerührt (Badtemperatur 140°C). Nach dem Abkühlen wurde Wasser zugesetzt. Es wurde mehrfach mit Dichlormethan extrahiert, und die vereinigten organischen Phasen wurden mit gesättigter Natriumchlorid-Lösung gewaschen, über Natriumsulfat getrocknet und im Vakuum eingeengt. Nach Chromatographie des Rückstands an Silicagel (Laufmittel Cyclohexan/Ethylacetat 10:1 → 2:1) wurden 1.46 g (78.6% d. Th.) der Titelverbindung erhalten.

LC-MS (Methode 6): Rₜ = 1.25 min; m/z = 476 (M+H)⁺.

¹H-NMR (400 MHz, DMSO-d₆): δ [ppm] = 0.80 (d, 3H), 0.82-0.89 (m, 3H), 1.42-1.54 (m, 2H), 2.41 (t, 1H), 2.64 (dd, 1H), 2.75 (dd, 1H), 4.12 (d, 1H), 7.00 (dd, 1H), 7.31-7.39 (m, 1H), 7.42-7.50 (m, 3H), 9.82 (s, 1H), 12.16 (br. s, 1H).

[α]_{D}²⁰ = +92.7°, c = 0.380, Methanol.

Nach analoger Vorgehensweise wurde die folgende Verbindung hergestellt:

### Beispiel 30

### (+)-(2S)-2-(4-Chlor-3-{[(2S,3R)-2-(4-chlorphenyl)-4,4,4-trifluor-3-methylbutanoyl]amino}benzyl)-butansäure

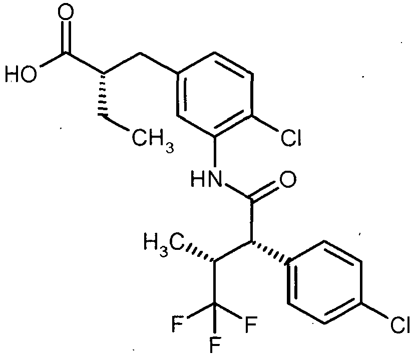

LC-MS (Methode 5): Rₜ = 2.66 min; m/z = 476 (M+H)⁺.

¹H-NMR (400 MHz, DMSO-d₆): δ [ppm] = 0.80 (d, 3H), 0.85 (t, 3H), 1.43-1.52 (m, 2H), 2.26-2.47 (m, 1H), 2.59-2.69 (m, 1H), 2.70-2.82 (m, 1H), 3.34-3.44 (m, 1H), 4.12 (d, 1H), 7.00 (dd, 1H), 7.30-7.39 (m, 2H), 7.40-7.52 (m, 4H), 9.82 (s, 1H), 12.13 (br. s, 1H).

[α]_{D}²⁰ = +143.1°, c = 0.380, Chloroform.

### Beispiel 31 und Beispiel 32

### 3-[4-Chlor-3-({4,4,4-trifluor-3-methyl-2-[4-(2,2,2-trifluorethyl)phenyl]butanoyl}amino)phenyl]-propansäure (Enantiomer 1 und 2)

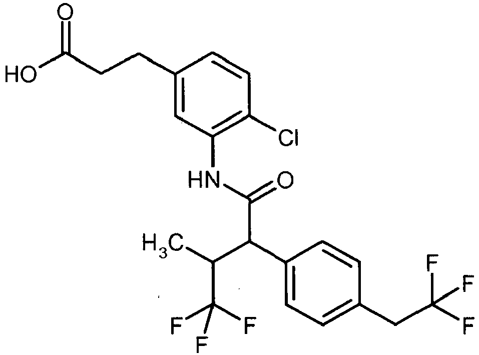

120 mg (0.24 mmol) der racemischen 3-[4-Chlor-3-({4,4,4-trifluor-3-methyl-2-[4-(2,2,2-trifluor-ethyl)phenyl]butanoyl}amino)phenyl]propansäure (Beispiel 5) wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/Ethanol 85:15 (v/v); Fluss: 15 ml/min; UV-Detektion: 220 nm; Temperatur: 35°C]:

### Beispiel 31

### (+)-3-[4-Chlor-3-({2S,3R)-4,4,4-trifluor-3-methyl-2-[4-(2,2,2-trifluorethyl)phenyl]butanoyl}-amino)phenyl]propansäure (Enantiomer 1)

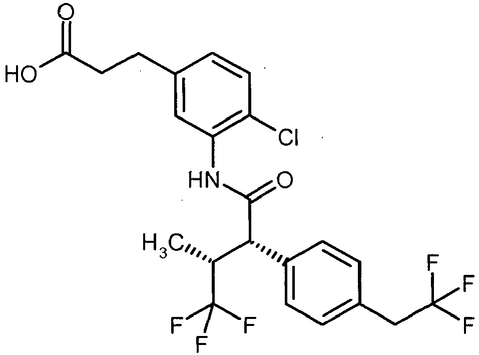

Ausbeute: 48 mg

Rₜ = 5.75 min; chemische Reinheit >99%; >99% ee [Säule: Daicel Chiralpak AD-H, 5 µm, 250 mm x 4.6 mm; Elutionsmittel: Isohexan/(Ethanol + 0.2% TFA + 1% Wasser) 85:15 (v/v); Fluss: 1 ml/min; Temperatur: 35°C; UV-Detektion: 220 nm].

[α]_{D}²⁰ = +91.8°, c = 0.405, Methanol.

LC-MS (Methode 4): Rₜ = 1.33 min; m/z = 496 (M+H)⁺.

¹H-NMR (400 MHz, DMSO-*d*₆, δ/ppm): 12.24-12.02 (1H, br. s), 9.80 (1H, s), 7.46 (2H, d), 7.43-7.39 (1H, m), 7.35 (3H, t), 7.04 (1H, dd), 4.11 (1H, d), 3.64 (2H, q), 3.44-3.27 (1H, m), 2.76 (2H, t), 2.48 (2H, t), 0.79 (3H, d).

### Beispiel 32

### (-)-3-[4-Chlor-3-({(2R,3S)-4,4,4-trifluor-3-methyl-2-[4-(2,2,2-trifluorethyl)phenyl]butanoyl}amino)-phenyl]propansäure (Enantiomer 2)

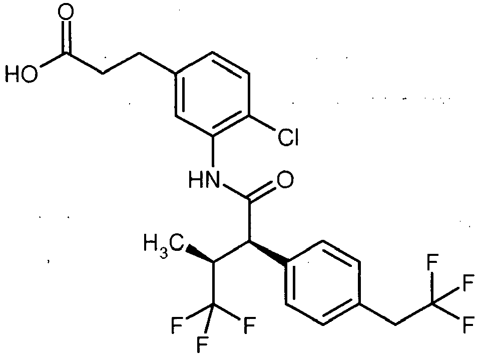

Ausbeute: 52 mg

Rₜ = 6.85 min; chemische Reinheit >97.4%; >99% ee [Säule: Daicel Chiralpak AD-H, 5 µm, 250 mm x 4.6 mm; Elutionsmittel: Isohexan/(Ethanol + 0.2% TFA + 1% Wasser) 85:15 (v/v); Fluss: 1 ml/min; Temperatur: 35°C; UV-Detektion: 220 nm].

[α]_{D}²⁰ = -94.3°, c = 0.40, Methanol.

LC-MS (Methode 4): Rₜ = 1.33 min; m/z = 496 (M+H)⁺.

### Beispiele 33 - 36

### 3-(4-Chlor-3-{[(4-chlorphenyl)(3,3-difluorcyclopentyl)acetyl]amino}phenyl)propansäure (Isomer 1 - 4)

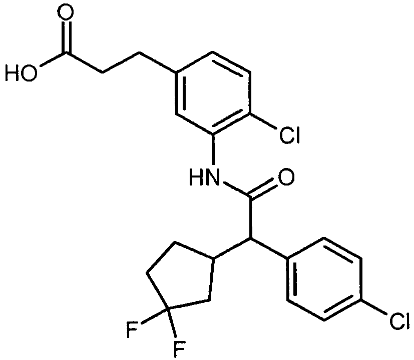

44 mg (0.096 mmol) des Diastereomerengemisches von 3-(4-Chlor-3-{[(4-chlorphenyl)(3,3-difluor-cyclopentyl)acetyl]amino}phenyl)propansäure (Beispiel 6) wurden mittels präparativer HPLC an chiraler Phase weiter aufgetrennt [Säule: Daicel Chiralcel OJ-H, 5 µm, 250 mm x 20 mm; Elutionsmittel: Isohexan/Ethanol 70:30 (v/v); Fluss: 15 ml/min; UV-Detektion: 220 nm; Temperatur: 35°C]. Hierbei wurden vier verschiedene Fraktionen erhalten, welche jeweils aus einem Gemisch von zwei Isomeren bestanden. Diese Fraktionen wurden durch nochmalige präparative HPLC an chiraler Phase in die einzelnen Isomeren aufgetrennt [*Fraktion 1 und 2:* Säule: Daicel Chiralpak AS-H, 5 µm, 250 mm x 20 mm; Elutionsmittel: Isohexan/Isopropanol 75:25 (v/v); Fluss: 15 ml/ min; UV-Detektion: 220 nm; Temperatur: 35°C. *Fraktion 3 und 4:* Säule: Daicel Chiralpak AD-H, 5 µm, 250 mm x 20 mm; Elutionsmittel: Isohexan/Ethanol 80:20 (v/v); Fluss: 15 ml/min; UV-Detektion: 220 nm; Temperatur: 35°C]:

### Beispiel 33 (Isomer 1):

Ausbeute: 8 mg

Rₜ = 6.49 min; chemische Reinheit >99%
[Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 35°C].

### Beispiel 34 (Isomer 2):

Ausbeute: 11 mg

Rₜ = 9.08 min; chemische Reinheit >98.5%
[Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 35°C].

### Beispiel 35 (Isomer 3):

Ausbeute: 12 mg

Rₜ = 7.19 min; chemische Reinheit >99%
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

### Beispiel 36 (Isomer 4):

### Ausbeute: 9 mg

Rₜ = 8.58 min; chemische Reinheit >97.5%
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

### Beispiel 37

### 3-(3-{[(3R)-2-(4-Chlorphenyl)-4,4,4-trifluor-3-methylbutanoyl]amino}-4-fluorphenyl)-2-methylpropansäure (Diastereomerengemisch)

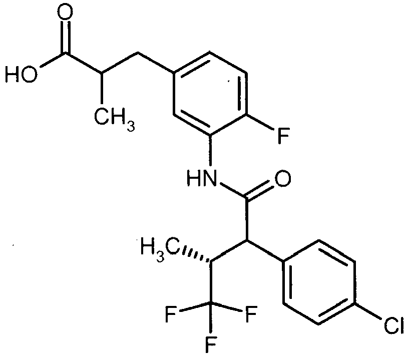

300 mg (0.633 mmol) Ethyl-3-(3-{[(2*S*,3*R*)-2-(4-chlorphenyl)-4,4,4-trifluor-3-methylbutanoyl]-amino}-4-fluorphenyl)-2-methylpropanoat (Diastereomerengemisch) wurden in einer Mischung aus je 1.0 ml Methanol, THF und Wasser gelöst und bei 0°C mit 265.5 mg (6.33 mmol) Lithiumhydroxid versetzt. Die Mischung wurde zunächst 1 h bei 0°C und anschließend 1 h bei RT gerührt. Danach wurde die Lösung mit Wasser verdünnt und mit 1 N Salzsäure sauer gestellt (pH ca. 2). Die wässrige Phase wurde dreimal mit Diethylether und einmal mit Ethylacetat extrahiert. Die vereinigten organischen Phasen wurden über Natriumsulfat getrocknet und im Vakuum eingeengt. Es wurden 294 mg (99.7% d. Th.) der Titelverbindung als Gemisch von vier Diastereomeren erhalten.

LC-MS (Methode 6): Rₜ = 1.18 min; m/z = 446 (M+H)⁺.

### Beispiel 38 und Beispiel 39

### 3-(3-{[(2S,3R)-2-(4-Chlorphenyl)-4,4,4-trifluor-3-methylbutanoyl]amino}-4-fluorphenyl)-2-methylpropansäure (Diastereomer 1 und 2)

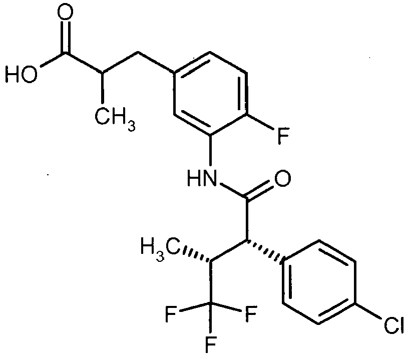

Das oben erhaltene Gemisch der diastereomeren 3-(3-{[(3*R*)-2-(4-Chlorphenyl)-4,4,4-trifluor-3-methylbutanoyl]amino}-4-fluorphenyl)-2-methylpropansäuren (Beispiel 37) wurde durch präparative HPLC an chiraler Phase weiter aufgetrennt [Säule: Daicel Chiralpak AS-H, 5 µm, 250 mm x 20 mm; Injektionsvolumen: 0.25 ml; Temperatur: 40°C; Eluent: 90% Isohexan / 10% (Ethanol + 0.2% TFA + 1% Wasser); Fluss: 15 ml/min; Detektion: 220 nm]. Ausgehend von 260 mg Diastereomerengemisch wurden neben zwei weiteren Isomeren 52 mg Isomer 1 (*Beispiel 38*) und 54 mg Isomer 2 (*Beispiel 39)* erhalten:

### Beispiel 38 (Diastereomer 1):

### (+)-(2S)-3-(3-{[(2S,3R)-2-(4-Chlorphenyl)-4,4,4-trifluor-3-methylbutanoyl]amino}-4-fluorphenyl)-2-methylpropansäure

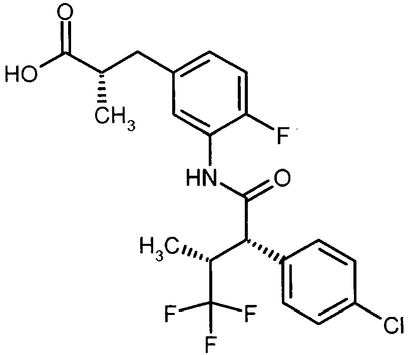

Das Isomer 1 wurde durch präparative RP-HPLC (Eluent Acetonitril/Wasser) nochmals nachgereinigt. Es wurden 32 mg erhalten.

LC-MS (Methode 6): Rₜ = 1.18 min; m/z = 446 (M+H)⁺.

¹H-NMR (400 MHz, DMSO-*d₆*): δ [ppm] = 0.79 (d, 3H), 1.01 (d, 3H), 2.51-2.58 (m, 2H), 2.76-2.86 (m, 1H), 3.35 (dd, 1H), 4.11 (d, 1H), 6.87-7.00 (m, 1H), 7.12 (dd, 1H), 7.41-7.49 (m, 4H), 7.63 (dd, 1H), 10.04 (s, 1H), 12.11 (br. s, 1H).

[α]_{D}²⁰ = +150.4°, c = 0.50, Chloroform.

### Beispiel 39 (Diastereomer 2):

### (+)-(2R)-3-(3-{[(2S,3R)-2-(4-Chlorphenyl)-4,4,4-trifluor-3-methylbutanoyl]amino}-4-fluophenyl)-2-methylpropansäure

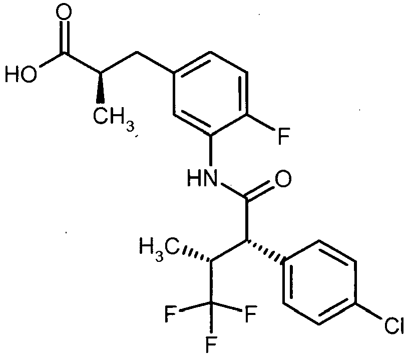

Das Isomer 2 wurde durch präparative RP-HPLC (Eluent Acetonitril/Wasser) nochmals nachgereinigt. Es wurden 21 mg erhalten.

LC-MS (Methode 6): Rₜ = 1.18 min; m/z = 446 (M+H)⁺.

¹H-NMR (400 MHz, DMSO-*d₆*): δ [ppm] = 0.79 (d, 3H), 1.02 (d, 3H), 2.53-2.58 (m, 2H), 2.77-2.87 (m, 1H), 3.30-3.41 (m, 1H), 4.11 (d, 1H), 6.89-7.00 (m, 1H), 7.12 (dd, 1H), 7.41-7.48 (m, 4H), 7.63 (dd, 1H), 10.04 (s, 1H), 12.12 (br. s, 1H).

[α]_{D}²⁰ = +131.6°, c = 0.530, Chloroform.

### Beispiel 40 und Beispiel 41

### 3-(4-Chlor-3-{[(2S,3R)-2-(4-ethylphenyl)-4,4,4-trifluor-3-methylbutanoyl]amino}phenyl)-2-methyl-propansäure (Diastereomer 1 und 2)

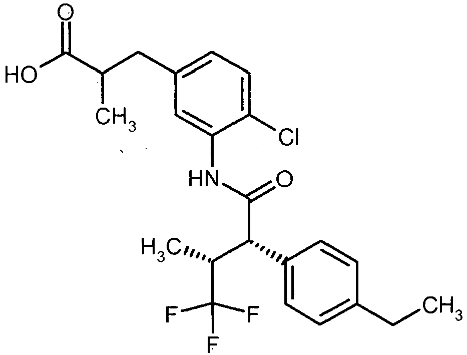

Das oben erhaltene Gemisch der diastereomeren 3-(4-Chlor-3- {[(2*S*,3*R*)-2-(4-ethylphenyl)-4,4,4-trifluor-3-methylbutanoyl]amino}phenyl)-2-methylpropansäuren (Beispiel 24) wurde durch präparative HPLC an chiraler Phase weiter aufgetrennt [Säule: chirale Kieselgel-Phase basierend auf dem Selektor Poly(*N*-methacryloyl-L-isoleucin-3-pentylamid), 430 mm x 40 mm; Injektionsvolumen: 2.0 ml; Temperatur: 24°C; Eluent: 40% Isohexan / 60% Ethylacetat; Fluss: 80 ml/min; Detektion: 265 nm]. Ausgehend von 514 mg Diastereomerengemisch wurden 178 mg Diastereomer 1 (*Beispiel 40)* und 218 mg Diastereomer 2 (*Beispiel 41*) erhalten:

### Beispiel 40 (Diastereomer 1):

### (+)-(2R)-3-(4-Chlor-3-{[(2S,3R)-2-(4-ethylphenyl)-4,4,4-trifluor-3-methylbutanoyl]amino}phenyl)-2-methylpropansäure

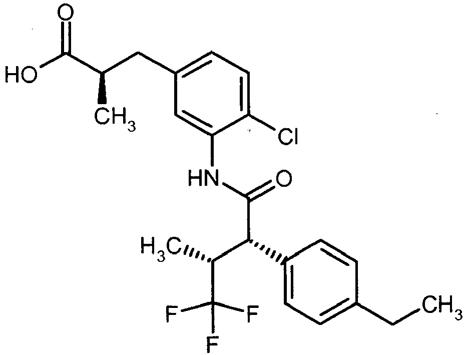

LC-MS (Methode 6): Rₜ = 1.25 min; m/z = 456 (M+H)⁺.

¹H-NMR (400 MHz, DMSO-*d₆*): δ [ppm] = 0.79 (d, 3H), 0.98-1.05 (m, 3H), 1.17 (t, 3H), 2.55-2.63 (m, 4H), 2.78-2.88 (m, 1H), 3.28-3.37 (m, 1H), 4.06 (d, 1H), 6.99 (dd, 1H), 7.20 (d, 2H), 7.34 (dd, 3H), 7.41 (d, 1H), 9.73 (s, 1H), 12.15 (s, 1H).

[α]_{D}²⁰ = +52°, c = 0.500, Chloroform.

### Beispiel 41 (Diastereomer 2):

### (+)-(2S)-3-(4-Chlor-3-{[(2S,3R)-2-(4-ethylphenyl)-4,4,4-trifluor-3-methylbutanoyl]amino}phenyl)-2-methylpropansäure

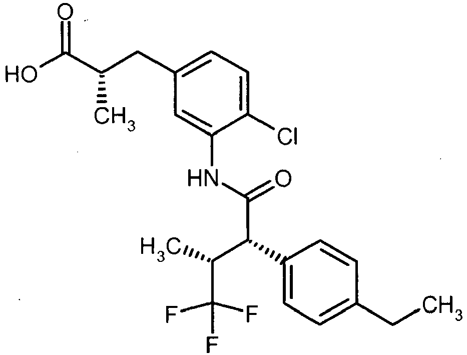

LC-MS (Methode 6): Rₜ = 1.27 min; m/z = 456 (M+H)⁺.

¹H-NMR (400 MHz, DMSO-*d₆*): δ [ppm] = 0.78 (d, 3H), 1.02 (d, 3H), 1.17 (t, 3H), 2.54-2.64 (m, 4H), 2.77-2.87 (m, 1H), 3.28-3.37 (m, 1H), 4.06 (d, 1H), 6.99 (dd, 1H), 7.21 (d, 2H), 7.34 (dd, 3H), 7.41 (d, 1H), 9.74 (s, 1H), 12.16 (br. s, 1H).

[α]_{D}²⁰ = +75.0°, c = 0.640, Chloroform.

### Beispiel 42 und Beispiel 43

### 3-(3-{[(2S,3R)-2-(4-Ethylphenyl)-4,4,4-trifluor-3-methylbutanoyl]amino}-4-fluorphenyl)-2-methylpropansäure (Diastereomer 1 und 2)

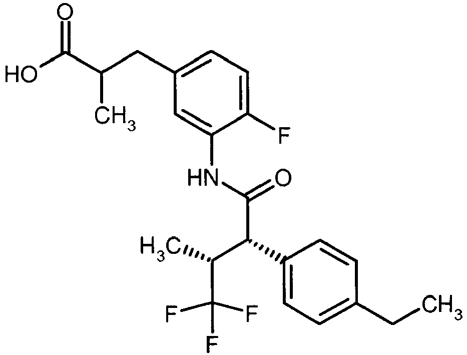

Das oben erhaltene Gemisch der diastereomeren 3-(3-{[(2*S*,3*R*)-2-(4-Ethylphenyl)-4,4,4-trifluor-3-methylbutanoyl]amino}-4-fluorphenyl)-2-methylpropansäuren (Beispiel 23) wurde durch präparative HPLC an chiraler Phase weiter aufgetrennt [Säule: Daicel Chiralpak AS-H, 5 µm, 250 mm x 20 mm; Injektionsvolumen: 0.30 ml; Temperatur: 30°C; Eluent: 92% Isohexan / 8% (Ethanol + 0.2% TFA + 1% Wasser); Fluss: 15 ml/min; Detektion: 220 nm]. Ausgehend von 509 mg Diastereomerengemisch wurden 209 mg Diastereomer 1 *(Beispiel 42)* und 220 mg Diastereomer 2 *(Beispiel 43*) erhalten:

### Beispiel 42 (Diastereomer 1):

### (+)-(2S)-3-(3-{[(2S,3R)-2-(4-Ethylphenyl)-4,4,4-trifluor-3-methylbutanoyl]amino}-4-fluorphenyl)-2-methylpropansäure

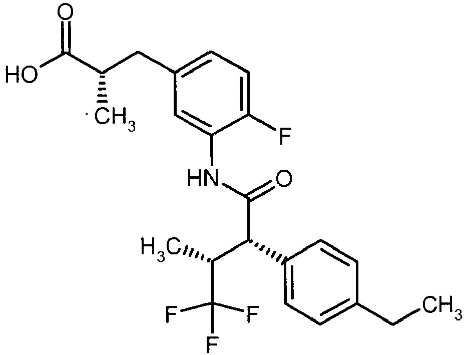

LC-MS (Methode 6): Rₜ = 1.22 min; m/z = 440 (M+H)⁺.

¹H-NMR (400 MHz, DMSO-*d₆*): δ [ppm] = 0.77 (d, 3H), 1.01 (d, 3H), 1.17 (t, 3H), 2.55-2.63 (m, 4H), 2.76-2.86 (m, 1H), 3.25-3.39 (m, 1H), 4.05 (d, 1H), 6.88-6.98 (m, 1H), 7.11 (dd, 1H), 7.17-7.24 (m, 2H), 7.29-7.38 (m, 2H), 7.66 (dd, 1H), 9.97 (s, 1H), 12.13 (br. s, 1H).

[α]_{D}²⁰ = +162.1°, c = 0.500, Chloroform.

### Beispiel 43 (Diastereomer 2):

### (+)-(2R)-3-(3-{[(2S,3R)-2-(4-Ethylphenyl)-4,4,4-trifluor-3-methylbutanoyl]amino}-4-fluorphenyl)-2-methylpropansäure

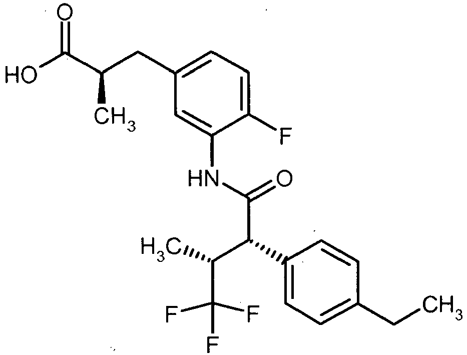

LC-MS (Methode 6): Rₜ = 1.22 min; m/z = 440 (M+H)⁺.

¹H-NMR (400 MHz, DMSO-*d₆*): δ [ppm] = 0.77 (d, 3H), 1.01 (d, 3H), 1.17 (t, 3H), 2.53-2.64 (m, 4H), 2.76-2.87 (m, 1H), 3.26-3.38 (m, 1H), 4.04 (d, 1H), 6.87-6.97 (m, 1H), 7.11 (dd, 1H), 7.17-7.23 (m, 2H), 7.28-7.38 (m, 2H), 7.65 (dd, 1H), 9.97 (s, 1H), 12.12 (br. s, 1H).

[α]_{D}²⁰ = +94.0°, c = 0.620, Chloroform.

### Beispiel 44 und Beispiel 45

### 3-(4-Chlor-3-{[(2S,3R)-2-(4-ethylphenyl)-4,4,4-trifluor-3-methylbutanoyl]amino}phenyl)butansäure (Diastereomer 1 und 2)

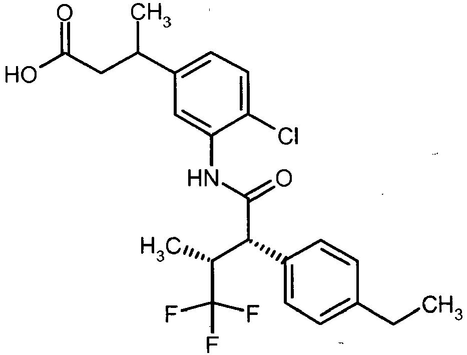

Das oben erhaltene Gemisch der diastereomeren 3-(4-Chlor-3-{[(2*S*,3*R*)-2-(4-ethylphenyl)-4,4,4-trifluor-3-methylbutanoyl]amino}phenyl)butansäuren (Beispiel 7) wurde durch präparative HPLC an chiraler Phase weiter aufgetrennt [Säule: Daicel Chiralpak AS-H, 5 µm, 250 mm x 20 mm; Injektionsvolumen: 0.20 ml; Temperatur: 30°C; Eluent: 90% Isohexan / 10% (Isopropanol + 0.2% TFA + 1% Wasser); Fluss: 15 ml/min; Detektion: 220 mm]. Ausgehend von 210 mg Diastereomerengemisch wurden 110 mg Diastereomer 1 *(Beispiel 44*) und 99 mg Diastereomer 2 (*Beispiel 45*) erhalten:

### Beispiel 44 (Diastereomer 1):

### (+)-(3S)-3-(4-Chlor-3-{[(2S,3R)-2-(4-ethylphenyl)-4,4,4-trifluor-3-methylbutanoyl]amino}-phenyl)-butansäure

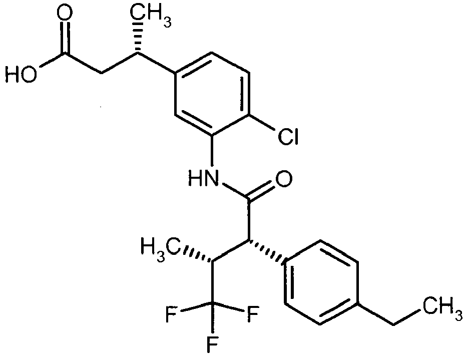

LC-MS (Methode 6): Rₜ = 1.26 min; m/z = 456 (M+H)⁺.

¹H-NMR (400 MHz, DM-SO-*d*₆): δ [ppm] = 0.79 (d, 3H), 1.13-1.21 (m, 6H), 2.45 (d, 2H), 2.59 (q, 2H), 3.08 (q, 1H), 3.27-3.38 (m, 1H), 4.07 (d, 1H), 7.06 (dd, 1H), 7.21 (d, 2H), 7.35 (dd, 3H), 7.46 (d, 1H), 9.72 (s, 1H), 12.05 (br. s, 1H).

[α]_{D}²⁰ = +86.8°, c = 0.440, Chloroform.

### Beispiel 45 (Diastereomer 2):

### (+)-(3R)-3-(4-Chlor-3-{[(2S,3R)-2-(4-ethylphenyl)-4,4,4-trifluor-3-methylbutanoyl]amino}-phenyl)-butansäure

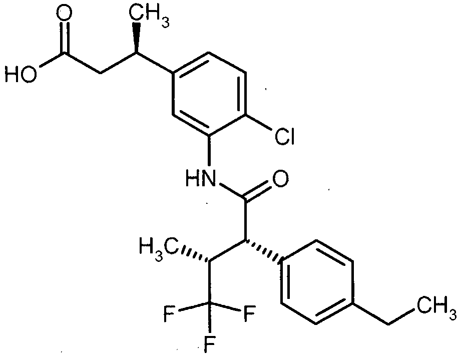

LC-MS (Methode 6): Rₜ = 1.26 min; m/z = 456 (M+H)⁺.

¹H-NMR (400 MHz, DMSO-*d₆*): δ [ppm] = 0.79 (d, 3H), 1.11-1.22 (m, 6H), 2.45 (d, 2H), 2.55-2.63 (m, 2H), 3.08 (q, 1H), 3.28-3.38 (m, 1H), 4.08 (d, 1H), 7.06 (dd, 1H), 7.20 (d, 2H), 7.35 (dd, 3H), 7.47 (d, 1H), 9.72 (s, 1H), 12.05 (br. s, 1H).

[α]_{D}²⁰ = +68.0°, c = 0.415, Chloroform.

### Beispiel 46 und Beispiel 47

### 3-(3-{[(2S,3R)-2-(4-Chlorphenyl)-4,4,4-trifluor-3-methylbutanoyl]amino}-4-chlorphenyl)butansäure (Diastereomer 1 und 2)

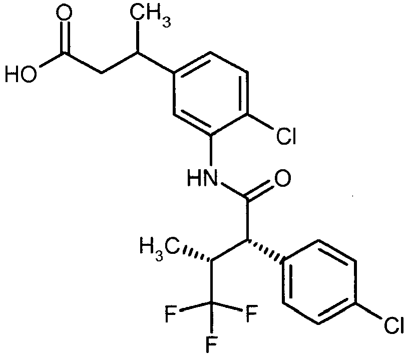

Das oben erhaltene Gemisch der diastereomeren 3-(3-{[(2*S*,3*R*)-2-(4-Chlorphenyl)-4,4,4-trifluor-3-methylbutanoyl]amino}-4-chlorphenyl)butansäuren (Beispiel 8) wurde durch präparative HPLC an chiraler Phase weiter aufgetrennt [Säule: Daicel Chiralpak AS-H, 5 µm, 250 mm x 20 mm; Injektionsvolumen: 0.30 ml; Temperatur: 30°C; Eluent: 90% Isohexan / 10% Isopropanol; Fluss: 15 ml/ min; Detektion: 220 nm]. Ausgehend von 250 mg Diastereomerengemisch wurden 116 mg Diastereomer 1 (*Beispiel 46)* und 113 mg Diastereomer 2 (*Beispiel 47*) erhalten:

### Beispiel 46 (Diastereomer 1):

### (+)-(3S)-3-(3-[(2S,3R)-2-(4-Chlophenyl)-4,4,4-trifluor-3-methylbutanoyl]amino}-4-chlorphenyl)-butansäure

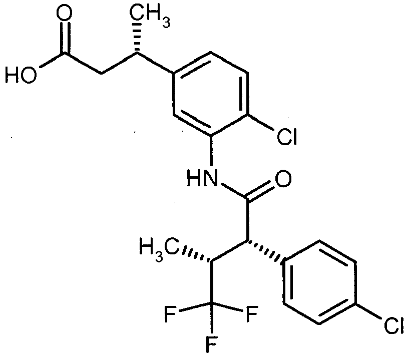

LC-MS (Methode 4): Rₜ = 1.36 min; m/z = 462 (M+H)⁺.

¹H-NMR (400 MHz; DMSO-*d*₆): δ [ppm] = 0.80 (d, 3H), 1.16 (d, 3H), 2.45 (d; 2H), 3.03-3.14 (m, 1H), 3.33-3.42 (m, 1H), 4.13 (d, 1H), 7.08 (dd, 1H), 7.36 (d, 1H), 7.41 (d, 1H), 7.43-7.51 (m, 4H), 9.81 (s, 1H), 12.05 (s, 1H).

[α]_{D}²⁰ = +88.6°, c = 0.435, Chloroform.

### Beispiel 47 (Diastereomer) 2):

### (+)-(3R)-3-(3-{[(2S,3R)-2-(4-Chlorphenyl)-4,4,4-trifluor-3-methylbutanoyl]amino}-4-chlorphenyl)-butansäure

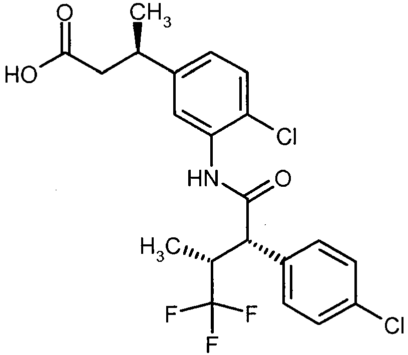

LC-MS (Methode 4): Rₜ = 1.36 min; m/z = 462 (M+H)⁺.

¹H-NMR (400 MHz, DMSO-*d*₆): δ [ppm] = 0.80 (d, 3H), 1.16 (d, 3H), 2.45 (d, 2H), 3.09 (q, 1H), 3.33-3.42 (m, 1H), 4.13 (d, 1H), 7.08 (dd, 1H), 7.35 (d, 1H), 7.42 (d, 1H), 7.43-7.51 (m, 4H), 9.81 (s, 1H), 12.05 (s, 1H).

[α]_{D}²⁰ = +57.9°, c = 0.365, Chloroform.

### Beispiel 48 und Beispiel 49

### 3-(3-{[(2S,3R)-2-(4-Chlophenyl)-4,4,4-trifluor-3-methylbutanoyl]amino}-4-fluorphenyl)butansäure (Diastereomer 1 und 2)

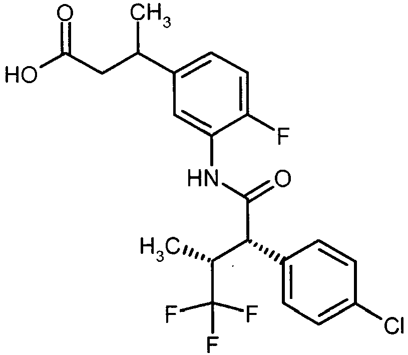

Das oben erhaltene Gemisch der diastereomeren 3-(3-{[(2*S*,3*R*)-2-(4-Chlorphenyl)-4,4,4-trifluor-3-methylbutanoyl]amino}-4-fluorphenyl)butansäuren (Beispiel 9) wurde durch präparative HPLC an chiraler Phase weiter aufgetrennt [Säule: Daicel Chiralpak AS-H, 5 µm, 250 mm x 20 mm; Injektionsvolumen: 0.25 ml; Temperatur: 30°C; Eluent: 85% Isohexan / 15% Isopropanol; Fluss: 15 ml/ min; Detektion: 220 nm]. Ausgehend von 295 mg Diastereomerengemisch wurden 121 mg Diastereomer 1 (*Beispiel 48)* und 111 mg Diastereomer 2 (*Beispiel 49)* erhalten:

### Beispiel 48 (Diastereomer 1):

### (+)-(3S)-3-(3-{[(2S,3R)-2-(4-Chlorphenyl)-4,4,4-trifluor-3-methylbutanoyl]amino}-4-fluorphenyl)-butansäure

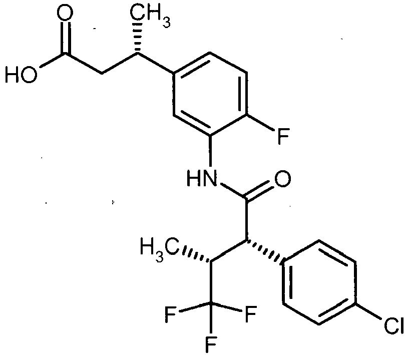

LC-MS (Methode 6): Rₜ = 1.14 min; m/z = 446 (M+H)⁺.

¹H-NMR (400 MHz, DMSO-*d*₆): δ [ppm] = 0.79 (d, 3H), 1.16 (d, 3H), 2.44 (d, 2H), 3.02-3.12 (m, 1H), 3.33-3.42 (m, 1H), 4.12 (d, 1H), 7.00-7.04 (m, 1H), 7.13 (dd, 1H), 7.43-7.48 (m, 4H), 7.68 (dd, 1H), 10.04 (s, 1H), 12.03 (s, 1H).

[α]_{D}²⁰ = +142.0°, c = 0.350, Chloroform.

### Beispiel 49 (Diastereomer 2):

### (+)-(3R)-3-(3-{[(2S,3R)-2-(4-Chlorphenyl)-4,4,4-trifluor-3-methylbutanoyl]amino}-4-fluorphenyl)-butansäure

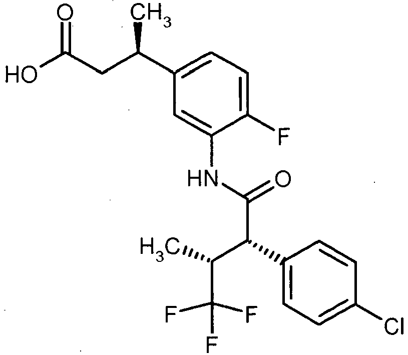

LC-MS (Methode 6): Rₜ = 1.14 min; m/z = 446 (M+H)⁺.

¹H-NMR (400 MHz, DMSO-*d*₆): δ [ppm] = 0.79 (d, 3H), 1.15 (d, 3H), 2.44 (d, 2H), 3.08 (q, 1H), 3.30-3.42 (m, 1H), 4.12 (d, 1H), 6.94-7.06 (m, 1H), 7.13 (dd, 1H), 7.40-7.50 (m, 4H), 7.68 (dd, 1H), 10.04 (s, 1H), 12.04 (br. s, 1H).

[α]_{D}²⁰ = +139.8°, c = 0.405, Chloroform.

### Allgemeine Vorschrift 4: Saure Hydrolyse von Ethylestern

Der betreffende Ethylester wird in einem 7:2-Gemisch aus Eisessig und konzentrierter Salzsäure (ca. 10 ml / mmol Substrat) gelöst und bis zur vollständigen Umsetzung auf 100°C erhitzt (im Allgemeinen zwischen 1 h und 8 h). Anschließend wird der Ansatz abgekühlt, auf Wasser gegossen und mehrfach mit Dichlormethan extrahiert. Die vereinigten organischen Phasen werden dreimal mit gesättigter Kochsalz-Lösung gewaschen, über Magnesiumsulfat getrocknet und eingeengt. Erforderlichenfalls wird der Rückstand mittels Flash-Chromatographie oder präparativer HPLC gereinigt.

Nach der Allgemeinen Vorschrift 4 wurden die folgenden Beispielverbindungen hergestellt:

| **Beispiel** | **Name / Struktur** | **Analytische Daten** |
|---|---|---|
| **50** | *threo-*3-(3-{[(2*S*,3*R*)-2-(4-Chlorphenyl)-4,4,4-trifluor-3-methylbutanoyl]amino}-4-fluorphenyl)-2-methylbutansäure | LC-MS (Methode 6): Rₜ = 1.21 min; m/z = 460 (M+H)⁺. |
| | | |
| | 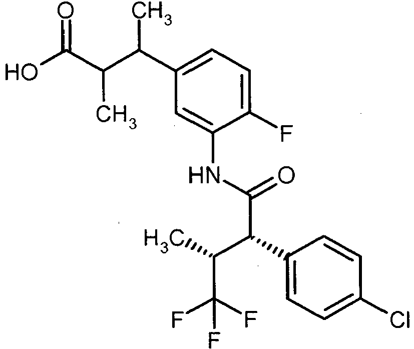 | ¹H-NMR (400 MHz, DMSO-d₆): δ [ppm] = 0.75-0.95 (m, 3H), 1.00-1.06 (m, 2H), 1.07-1.17 (m, 3H), 1.24 (s, 1H), 1.91 (s, 1H), 2.93 (t, 1H), 3.35-3.45 (m, 1H), 4.06-4.20 (m, 1H), 6.93-7.04 (m, 1H), 7.07-7.19 (m, 1H), 7.39-7.54 (m, 4H), 7.66 (dt, 1H), 9.98-10.09 (m, 1H), 11.96 (br. s, 1H). |
| **51** | *erythro*-3-(3-{[(2*S*,3*R*)-2-(4-Chlorphenyl)-4,4,4-trifluor-3-methylbutanoyl]amino}-4-fluorphenyl)-2-methylbutansäure | LC-MS (Methode 5): Rₜ = 2.52 min: m/z = 460 (M+H)⁺. |
| | | |
| | 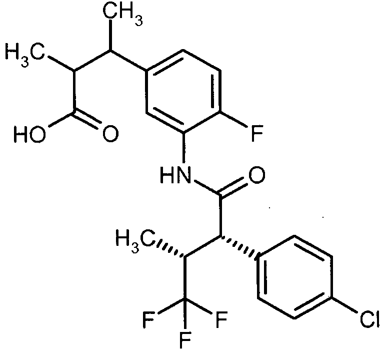 | ¹H-NMR (400 MHz, DMSO-d₆): δ [ppm] = 0.71-0.91 (m, 4H), 1.00-1.11 (m, 1H), 1.14 (d, 3H), 1.23 (br. s, 2H), 1.91 (s, 1H), 3.37-3.45 (m, 1H), 4.12 (d, 1H), 6.90-7.05 (m, 1H), 7.14 (t, 1H), 7.33-7.56 (m, 4H), 7.65 (d, 1H), 10.05 (s, 1H). |
| **52** | 2-(3-{[(2*S*,3*R*)-2-(4-Chlorphenyl)-4,4,4-trifluor-3-methylbutanoyl]amino}-4-fluorphenyl)-*trans*-cyclopropancarbonsäure | LC-MS (Methode 5): Rₜ = 2.45 min; m/z = 444 (M+H)⁺. |
| | | |
| | 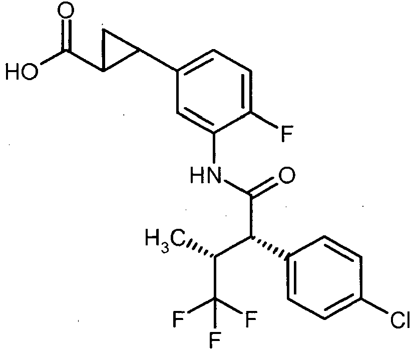 | ¹H-NMR (400 MHz, DMSO-d₆): δ [ppm] = 0.79 (d, 3H), 1.19-1.28 (m, 1H), 1.38 (dt, 1H), 1.65-1.75 (m, 1H), 2.30-2.40 (m, 1H), 4.12 (d, 1H), 6.88-6.97 (m, 1H), 7.13 (dd, 1H), 7.37-7.52 (m, 4H), 7.62 (dd, 1H), 10.06 (s, 1H), 12.30 (br. s, 1H). |

### Beispiel 53 und Beispiel 54

### 2-(3-{[(2S,3R)-2-(4-Chlorphenyl)-4,4,4-trifluor-3-methylbutanoyl]amino}-4-fluorphenyl)-trans-cyclopropancarbonsäure (Diastereomer 1 und 2)

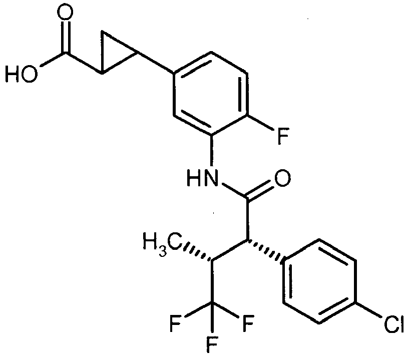

71 mg des Diastereomerengemisches von 2-(3-{[(2*S*,3*R*)-2-(4-Chlorphenyl)-4,4,4-trifluor-3-methyl-butanoyl]amino}-4-fluophenyl)-*trans*-cyclopropancarbonsäure (Beispiel 52) wurden in 2 ml Ethanol und 2 ml Isohexan gelöst und mittels präparativer HPLC an chiraler Phase weiter aufgetrennt [Säule: Daicel Chiralpak AD-H, 5 µm, 250 mm x 200 mm; Injektionsvolumen: 0.25 ml; Temperatur: 30°C; Eluent: 15% Isopropanol / 85% Isohexan; Fluss: 15 ml/min; Detektion: 220 nm]. Es wurden 36 mg Diastereomer 1 (*Beispiel 53)* und 37 mg Diastereomer 2 *(Beispiel 54)* erhalten:

### Beispiel 53 (Diastereomer 1):

LC-MS (Methode 6): Rₜ = 1.15 min; m/z = 444 (M+H)⁺.

¹H-NMR (400 MHz, DMSO-*d*₆): δ [ppm] = 0.79 (d, 3H), 1.24 (ddd, 1H), 1.38 (dt, 1H), 1.64-1.80 (m, 1H), 2.35 (ddd, 1H), 4.12 (d, 1H), 6.85-7.01 (m, 1H), 7.13 (dd, 1H), 7.37-7.56 (m, 4H), 7.62 (dd, 1H), 10.06 (s, 1H).

[α]_{D}²⁰ = +291.4°, c = 0.48, Chloroform.

### Beispiel 54 (Diastereomer 2):

LG-MS (Methode 6): Rₜ = 1.15 min; m/z = 444 (M+H)⁺.

¹H-NMR (400 MHz, DMSO-*d*₆): δ [ppm] =0.79 (d, 3H), 1.24 (ddd, 1H), 1.38 (dt, 1H), 1.64-1.76 (m, 1H), 2.29-2.40 (m, 2H), 4.12 (d, 1H), 6.92 (ddd, 1H), 7.13 (dd, 1H), 7.39-7.52 (m, 4H), 7.62 (dd, 1H), 10.06 (s, 1H).

[α]_{D}²⁰= +44.3°, c = 0.40, Chloroform.

### Beispiel 55

### 3-(3-{[(2S,3R)-2-(4-Chlorphenyl)-4,4,4-trifluor-3-methylbutanoyl]amino}-4-cyanophenyl)propansäure

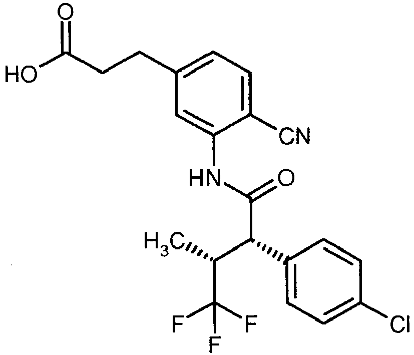

16.5 mg (33 µmol) *tert*.-Butyl-3-(3-{[(2*S*,3*R*)-2-(4-chlorphenyl)-4,4,4-trifluor-3-methylbutanoyl]-amino}-4-cyanophenyl)propanoat wurden in 1.1 ml Dichlormethan gelöst und mit 275 µl Trifluoressigsäure versetzt. Das Reaktionsgemisch wurde 1.5 h bei RT gerührt, anschließend mit 20 ml Dichlormethan verdünnt und im Vakuum eingeengt. Der Rückstand wurde über Nacht im Hochvakuum getrocknet. Es wurden 14.8 mg (97% d. Th.) der Titelverbindung erhalten.

LC-MS (Methode 6): Rₜ = 1.10 min; m/z = 439 (M+NH₄)⁺.

¹H-NMR (400 MHz, DMSO-*d*₆): δ [ppm] = 0.81 (d, 3H), 2.80-2.94 (m, 2H), 4.01 (d, 1H), 7.22 (dd, 1H), 7.32 (s, 1H), 7.40-7.52 (m, 4H), 7.69 (d, 1H), 10.48 (s, 1H).

### Beispiel 56

### (+/-)-3-(3-{[2-(4-Chlophenyl)-4,4,4-trifluor-3-methylbutanoyl]amino}-2-fluophenyl)propansäure (Diastereomer 1)

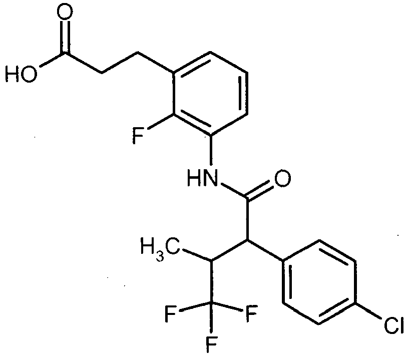

270 mg (0.553 mmol) (+/-)-3-(3-{[2-(4-Chlorphenyl)-4,4,4-trifluor-3-methylbutanoyl]amino}-2-fluorphenyl)propansäure-*tert*-butylester (Diastereomer 1, Beispiel 102A) wurden in 0.2 ml Dichlormethan gelöst und bei RT mit 0.85 ml Trifluoressigsäure versetzt. Die Reaktionsmischung wurde 4 h bei RT gerührt und dann im Vakuum eingeengt. Der Rückstand wurde durch präparative RP-HPLC (Acetonitril/Wasser-Gemisch) gereinigt. Es wurden 188 mg (78.7% d. Th.) der Zielverbindung erhalten.

LC-MS (Methode 6): Rₜ = 1.16 min; m/z = 432 (M+H)⁺.

¹H-NMR (400 MHz, DMSO-*d*₆): δ [ppm] = 0.79 (d, 3H), 2.48-2.53 (m, 2H), 2.82 (t, 2H), 3.35-3.48 (m, 1H), 4.13 (d, 1H), 6.88-7.13 (m, 2H), 7.37-7.51 (m, 4H), 7.54-7.76 (m, 1H), 10.04 (s, 1H), 12.19 (br. s, 1H).

Auf analoge Weise wurde die folgende Verbindung hergestellt:

### Beispiel 57

### (+/-)-3-(3-{[2-(4-Chlorphenyl)-4,4,4-trifluor-3-methylbutanoyl]amino}-2-fluorphenyl)propansäure (Diastereomer 2)

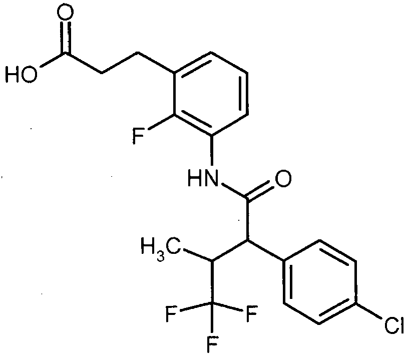

LC-MS (Methode 6): Rₜ = 1.15 min; m/z = 432 (M+H)⁺.

¹H-NMR (400 MHz, DMSO-*d*₆): δ [ppm] = 1.22 (d, 3H), 2.52-2.56 (m, 2H), 2.83 (t, 2H), 3.22 (dd, 1H), 4.15 (d, 1H), 6.98-7.10 (m, 2H), 7.36-7.43 (m, 2H), 7.45-7.53 (m, 2H), 7.62 (td, 1H), 10.13 (s, 1H), 12.19 (s, 1H).

Die folgenden Beispiele wurden gemäß der *Allgemeinen Vorschrift* 2 (Spaltung von *tert*.-Butylestern zu den entsprechenden Carbonsäuren mit Trifluoressigsäure) hergestellt:

| **Beispiel** | **Name / Struktur / Edukt** | **Analytische Daten** |
|---|---|---|
| **58** | (+)-3-(4-Chlor-3- {[(2*S*,3*R*)-2-(4-chlor-3-fluorphenyl)-4,4,4-trifluor-3-methylbutanoyl]amino}-phenyl)propansäure | LC-MS (Methode 6): Rₜ = 1.17 min; m/z = 466 (M+H)⁺. |
| | | |
| | 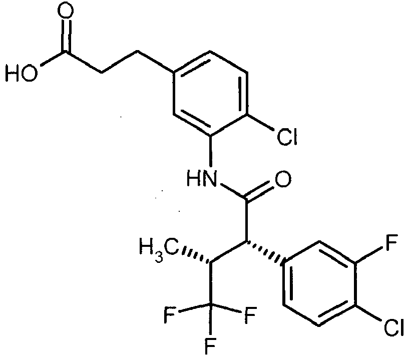 | ¹H-NMR (400 MHz, DMSO-d₆): δ [ppm] = 0.83 (d, 3H), 2.49 (t, 2H), 2.76 (t, 2H), 3.34-3.46 (m, 1H), 4.14 (d, 1H), 7.06 (dd, 1H), 7.28-7.41 (m, 3H), 7.49 (dd, 1H), 7.62 (t, 1H), 9.87 (s, 1H), 12.13 (s, 1H). |
| | | |
| | (aus (+)-*tert*.-Butyl-3-(4-chlor-3-{[(2*S*,3*R*)-2-(4-chlor-3-fluorphenyl)-4,4,4-trifluor-3-methyl-butanoyl]amino}phenyl)propanoat) | [α]_{D}²⁰ = +79.9°, c = 0.520, Chloroform. |
| **59** | (+)-3-(4-Chlor-3-{[(2*S*,3*R*)-2-(4-chlor-3-methylphenyl)-4,4,4-trifluor-3-methylbutanoyl]amino}-phenyl)propansäure | LC-MS (Methode 6): Rₜ = 1.20 min; m/z = 462 (M+H)⁺. |
| | | |
| | 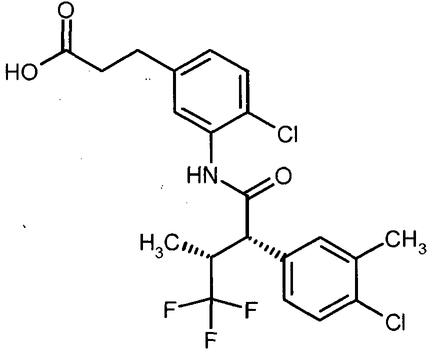 | ¹H-NMR (400 MHz, DMSO-*d*₆): δ [ppm] = 0.80 (d, 3H), 2.33 (s, 3H), 2.48 (t, 2H), 2.76 (t, 2H), 3.27-3.42 (m, 1H), 4.04-4.09 (m, 1H), 7.04 (dd, 1H), 7.29 (dd, 1H), 7.32-7.36 (m, 1H), 7.38-7.45 (m, 3H), 9.81 (s, 1H), 12.16 (br. s, 1H). |
| | | |
| | (aus *tert*.-Butyl-3-(4-chlor-3-{[(2*S*,3*R*)-2-(4-chlor-3-methylphenyl)-4,4,4-trifluor-3-methylbutanoyl]-amino}phenyl)propanoat) | [α]_{D}²⁰ = +86.0°, c = 0.250, Chloroform. |
| **60** | (+)-3-(4-Chlor-3-{[(2*S*,3*R*)-2-(3,4-dichlorphenyl)-4,4,4-trifluor-3-methylbutanoyl]amino}phenyl)-propansaure | LC-MS (Methode 6) Rₜ = 1.23 mm, m/z = 480/482 (M-H)⁻. |
| | | |
| | 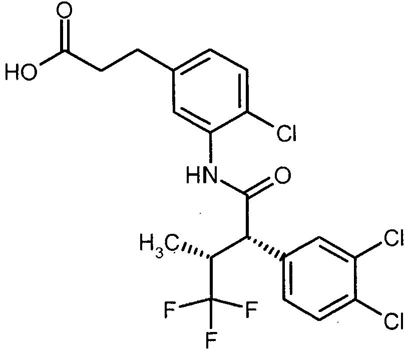 | ¹H-NMR (400 MHz, DMSO *d*₆): δ [ppm] = 0 83 (d, 3H), 2 47 (t, 2H), 2 72-2.81 (m, 2H), 3.35-3.47 (m, 1H), 4 09-4.17 (m, 1H), 7.06 (dd, 1H), 7.31-7 41 (m, 2H), 7 45 (dd, 1H), 7.67 (d, 1H), 7.72 (d, 1H), 9.88 (s, 1H). |
| | | |
| | (aus *tert*-Butyl-3-(4-chlor-3-{[(2*S*,3*R*)-2-(3,4-dichlorphenyl)-4,4,4-trifluor-3-methylbutanoyl] amino}phenyl)propanoat) | [α]_{D}²⁰ = +98 8°, c = 0.325, Methanol |
| **61** | 3-(4-Chlor-3-{[(2*S*,3*R*)-2-(4-chlorphenyl)-4,4,4-trifluor-3-methylbutanoyl]amino}-2-methylphenyl)-2-methylpropansäure *(Diastereomerengemisch)* | LC-MS (Methode 6): Rₜ = 1 20 min, m/z = 476 (M+H)⁺. |
| | | |
| | 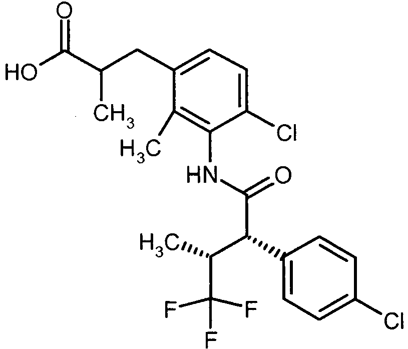 | ¹H-NMR (400 MHz, DMSO-d₆): beide Diastereomere δ [ppm] = 0.80 (d, 3H), 1.03 (br s, 3H), 1 51/1.57 (2 br s, zus. 2H), 2.15 (br, s, ca. 2H), 2.86 (br. s, ca 1H), 3.37-3 45 (m, ca. 1H), 3.90-4.00 (m, 1H), 6.99-7.12 (m, 1H), 7.16 (br. s, 1H), 7.25 (br. s, 1H), 7 35-7 54 (m, 5H), 9.88 (br. s, 1H), 12.18 (br s, 1H) [*Signale sind infolge Rotamere stark verbreitert*]. |
| | (aus *tert*.-Butyl-3-(4-chlor-3-{[(2*S*,3*R*)-2-(4-chlorphenyl)-4,4,4-trifluor-3-methylbutanoyl]amino}-2-methylphenyl)-2-methylpropanoat (*Diastereomerengemisch*)) | |
| **62** | (+)-(2*R*)-3-(4-Chlor-3-{[(2*S*,3*R*)-2-(4-chlorphenyl)-4,4,4-trifluor-3-methylbutanoyl]amino}-2-methylphenyl)-2-methylpropansäure | LC-MS (Methode 6): Rₜ = 1.26 min; m/z = 476 (M+H)⁺. |
| | | |
| | 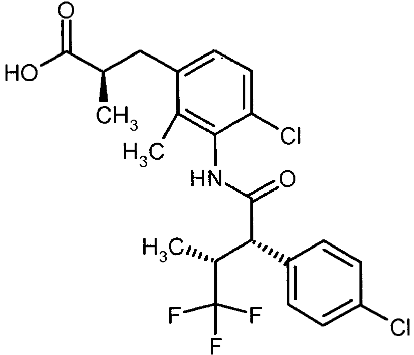 | ¹H-NMR (400 MHz, DMSO-*d*₆): δ [ppm] = 0.80 (d, 3H), 1.03 (br. s, 3H), 2.42-2.62 (br. s, ca. 2H, teilweise verdeckt), 3.30-3.44 (m, 1H), 3.94 (d, 1H), 7.05 (d, 1H), 7.23 (br. s, 1H), 7.45 (s, 4H), 12.15 (br. s, 1H) [*Signale sind infolge Rotamere stark verbreitert*]*.* |
| | | |
| | (aus (+)-*tert*.-Butyl-(2*R*)-3-(4-chlor-3-{[(2*S*,3*R*)-2-(4-chlorphenyl)-4,4,4-trifluor-3-methylbutanoyl]-amino}-2-methylphenyl)-2-methylpropanoat) | [α]_{D}²⁰ = + 108.9°, c = 0.510, Methanol. |
| **63** | (+)-(2*S*)-3-(4-Chlor-3-{[(2*S,*3*R*)-2-(4-chlorphenyl)-4,4,4-trifluor-3-methylbutanoyl]amino}-2-methylphenyl)-2-methylpropansäure | LC-MS (Methode 6): Rₜ = 1.26 min; m/z = 476 (M+H)⁺. |
| | | |
| | 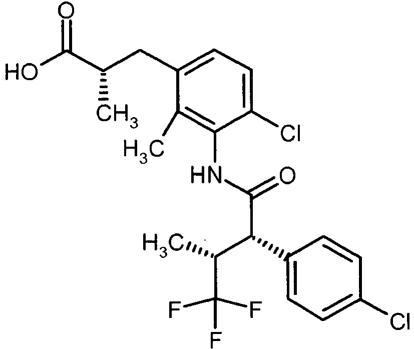 | ¹H-NMR (400 MHz, DMSO-*d*₆): δ [ppm] = 0.80 (d, 3H), 1.03 (br. s, 3H), 3.17 (d, 1H), 3.34-3.43 (m, 1H), 3.94 (d, 1H), 7.05 (d, 1H), 7.45 (br. s, 4H), 9.86 (br. s, 1H), 12.15 (br. s, 1H) *[Signale sind infolge Rotamere stark verbreitert*]*.* |
| | | |
| | (aus (+)-*tert*.-Butyl-(2*S*)-3-(4-chlor-3-{[(2*S*,3*R*)-2-(4-chlorphenyl)-4,4,4-trifluor-3-methylbutanoyl]-amino}-2-methylphenyl)-2-methylpropanoat) | [α]_{D}²⁰ = +143.7°, c = 0.505, Methanol. |
| **64** | 2-(4-Chlor-3-{[(2*S*,3*R*)-2-(4-chlorphenyl)-4,4,4-trifluor-3-methylbutanoyl]amino}benzyl)-2-methylbutansäure *(Diastereomerengemisch)* | LC-MS (Methode 6): Rₜ = 1.28 min; m/z = 490 (M+H)⁺. |
| | | |
| | 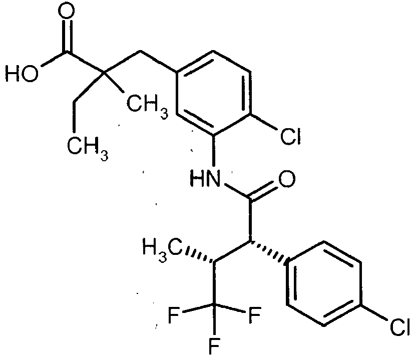 | ¹H-NMR (400 MHz, DMSO-d₆): beide Diastereomere δ [ppm] = 0.73-0.87 (m, 6H), 0.92 (d, 3H), 1.31-1.42 (m, 1H), 1.52-1.72 (m, 1H), 2.60 (d, 1H), 2.85/2.87 (2d, zus. 1H), 3.32-3.36 (m, 1H), 3.94-4.18 (m, 1H), 6.95 (dd, 1H), 7.27-7.37 (m, 2H), 7.39-7.53 (m, 4H), 9.84 (s, 1H). |
| | (aus *tert*.-Butyl-2-(4-chlor-3-{[(2*S*,3*R*)-2-(4-chlorphenyl)-4,4,4-trifluor-3-methylbutanoyl]amino}-benzyl)-2-methylbutanoat *(Diastereomerengemish*)) | |
| **65** | (+)-2-(4-Chlor-3-{[(2*S*,3*R*)-2-(4-chlorphenyl)-4,4,4-trifluor-3-methylbutanoyl]amino}benzyl)-2-methylbutansäure (*Diastereomer B*) | LC-MS (Methode 6): Rₜ = 1.26 min; m/z = 490 (M+H)⁺. |
| | | |
| | 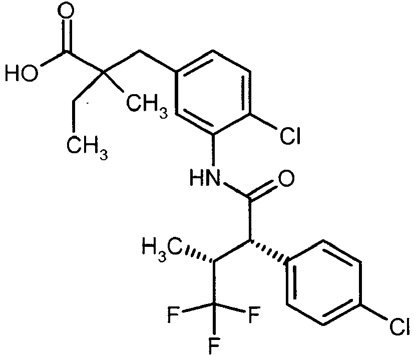 | ¹H-NMR (400 MHz, DMSO-d₆): δ [ppm] = 0.73-0.85 (m, 6H), 0.92 (s, 3H), 1.32-1.41 (m, 1H), 1.57-1.66 (m, 1H), 2.60 (d, 1H), 2.85 (d, 1H), 3.35-3.43 (m, 1H), 4.11 (d, 1H), 6.94 (d, 1H), 7.34 (d, 2H), 7.41-7.53 (m, 4H), 9.82 (s, 1H), 12.27 (br. s, 1H). |
| | | |
| | (aus *tert.* -Butyl-2-(4-chlor-3-{[(2*S*,3*R*)-2-(4-chlorphenyl)-4,4,4-trifluor-3-methylbutanoyl]amino}-benzyl)-2-methylbutanoat (*Diastereomer B*)) | [α]_{D}²⁰ = +80.6°, c = 0.350, Chlorofonn. |
| **66** | 3-(4-Chlor-3-{[(2*S*,3*R*)-2-(4-chlorphenyl)-4,4,4-trifluor-3-methylbutanoyl]amino}phenyl)-4,4,4-trifluorbutansäure *(Diastereomerengemisch)* | LC-MS (Methode 6): Rₜ = 1.18 min; m/z = 516 (M+H)⁺. |
| | | |
| | 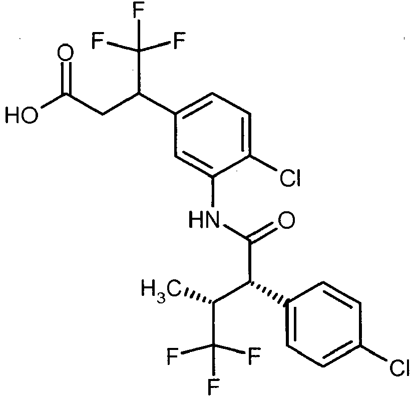 | ¹H-NMR (400 MHz, DMSO-*d*₆): beide Diastereomere δ [ppm] = 0.80 (d, 3H), 2.84 (dd, 1H), 2.95 (dd, 1H), 3.35-3.44 (m, 1H), 4.01-4.09 (m, 1H), 4.14 (d, 1H), 7.26 (dd, 1H), 7.39-7.52 (m, 5H), 7.61 (dd, 1H), 9.95 (s, 1H), 12.55 (br. s, 1H). |
| | (aus *tert*.-Butyl-3-(4-chlor-3-{[(2*S*,3*R*)-2-(4-chlorphenyl)-4,4,4-trifluor-3-methylbutanoyl]amino}-phenyl)-4,4,4-trifluorbutanoat *(Diastereomerengemisch*)) | |
| **67** | 3-(4-Chlor-3-{[(2*S*,3*R*)-2-(4-chlorphenyl)-4,4,4-trifluor-3-methylbutanoyl]amino}phenyl)-2-cyclo-butylpropansäure *(Diastereomerengemisch)* | LC-MS (Methode 6): Rₜ = 1.29 min; m/z = 502 (M+H)⁺. |
| | | |
| | 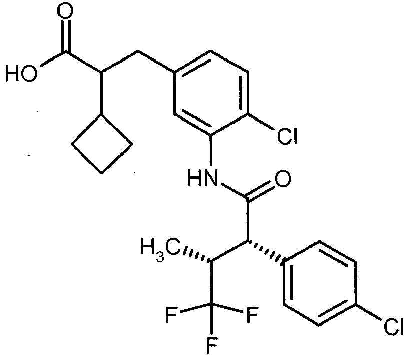 | ¹H-NMR (400 MHz, DMSO-*d*₆): beide Diastereomere δ [ppm] = 0.80 (d, 3H), 1.67-1.83 (m, 4H), 1.86-1.99 (m, 2H), 2.30-2.42 (m, 1H), 2.43-2.48 (m, 1H), 2.57-2.63 (m, 2H), 3.36-3.42 (m, 1H), 4.12 (d, 1H), 6.98 (d, 1H), 7.29-7.36 (m, 2H), 7.41-7.50 (m, 4H), 9.82 (s, 1H), 12.07 (s, 1H). |
| | (aus *tert*.-Butyl-3-(4-chlor-3-{[(2*S*,3*R*)-2-(4-chlorphenyl)-4,4,4-trifluor-3-methylbutanoyl]amino}-phenyl)-2-cyclobutylpropanoat *(Diastereomerengemish*)) | |
| **68** | (+)-3-(4-Chlor-3-{[(2*S*,3*R*)-2-(4-chlorphenyl)-4,4,4-trifluor-3-methylbutanoyl]amino}phenyl)-2-cyclo-butylpropansäure (*Diastereomer A)* | LC-MS (Methode 6): Rₜ = 1.32 min; m/z = 502 (M+H)⁺. |
| | | |
| | 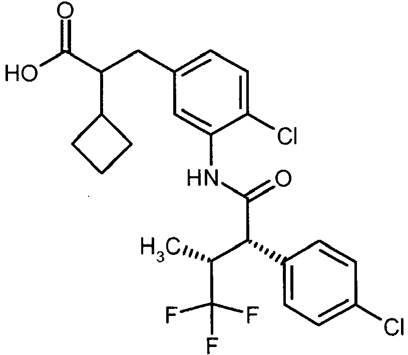 | ¹H-NMR (400 MHz, DMSO-d₆): δ [ppm] = 0.80 (d, 3H), 1.65-1.83 (m, 4H), 1.88-1.98 (m, 2H), 2.29-2.43 (m, 1H), 2.43-2.48 (m, 1H), 2.56-2.63 (m, 2H), 3.37-3.41 (m, 1H), 4.12 (d, 1H), 6.98 (dd, 1H), 7.28-7.36 (m, 2H), 7.42-7.55 (m, 4H), 9.82 (s, 1H), 12.08 (br. s, 1H). |
| | | |
| | (aus *tert*.-Butyl-3-(4-chlor-3-{[(2*S*,3*R*)-2-(4-chlorphenyl)-4,4,4-trifluor-3-methylbutanoyl]amino}-phenyl)-2-cyclobutylpropanoat (*Diastereomer A*)) | [α]_{D}²⁰ =+50.3°, c = 0.520, Chloroform. |
| **69** | (+)-3-(4-Chlor-3-{[(2*S*,3*R*)-2-(4-chlorphenyl)-4,4,4-trifluor-3-methylbutanoyl]amino}phenyl)-2-cyclo-butylpropansäure (*Diastereomer B)* | LC-MS (Methode 6): Rₜ = 1.29 min; m/z = 502 (M+H)⁺. |
| | | |
| | 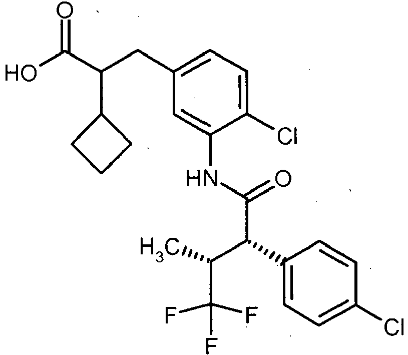 | ¹H-NMR (400 MHz, DMSO-d₆): δ [ppm] = 0.80 (d, 3H), 1.62-1.83 (m, 4H), 1.86-2.00 (m, 2H), 2.28-2.43 (m, 1H), 2.45-2.49 (m, 1H), 2.60 (d, 2H), 3.36-3.44 (m, 1H), 4.12 (d, 1H), 6.99 (dd, 1H), 7.24-7.37 (m, 2H), 7.41-7.53 (m, 4H), 9.82 (s, 1H), 12.07 (s, 1H). |
| | | |
| | (aus *tert*.-Butyl-3-(4-chlor-3-{[(2*S*,3*R*)-2-(4-chlorphenyl)-4,4,4-trifluor-3-methylbutanoyl]amino}-phenyl)-2-cyclobutylpropanoat (*Diastereomer B))* | [α]_{D}²⁰ = +97.8°, c = 0.445, Chlorofom. |
| **70** | 3-(4-Chlor-3- {[(2*S*,3*R*)-2-(4-chlorphenyl)-4,4,4-trifluor-3-methylbutanoyl]amino}phenyl)-2-cyclopropylpropansäure *(Diastereomerengemisch)* | LC-MS (Methode 4): Rₜ = 1.48 min; m/z = 488 (M+H)⁺. |
| | | |
| | 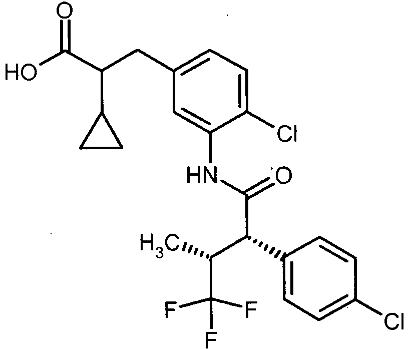 | ¹H-NMR (400 MHz, DMSO-*d*₆): beide Diastereomere δ [ppm] = 0.05-0.13 (m, 1H), 0.20-0.25 (m, 1H), 0.42 (d, 2H), 0.79 (d, 3H), 0.82-0.92 (m, 1H), 1.78 (td, 1H), 2.74-2.90 (m, 2H), 3.30-3.40 (m, 1H), 4.11 (d, 1H), 7.01 (d, 1H), 7.33 (d, 1H), 7.37 (d, 1H), 7.42-7.50 (m, 4H), 9.82 (s, 1H). |
| | (aus *tert*-Butyl-3-(4-chlor-3-{[(2*S*,3*R*)-2-(4-chlorphenyl)-4,4,4-trifluor-3-methylbutanoyl]amino}-phenyl)-2-cyclopropylpropanoat *(Diastereomerengemisch))* | |

### Beispiel 71

### (+)-2-(4-Chlor-3-{[(2S,3R)-2-(4-chlorphenyl)-4,4,4-trifluor-3-methylbutanoyl]amino}benzyl)-2-methylbutansäure (Diastereomer A)

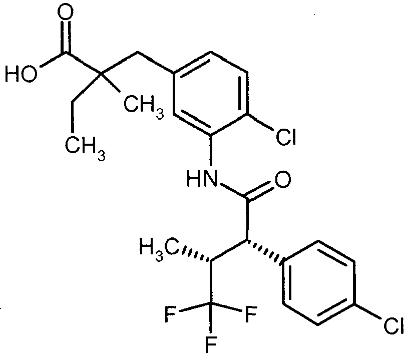

302 mg (0.553 mmol) (+)-*tert*.-Butyl-2-(4-chlor-3-{[(2*S*,3*R*)-2-(4-chlorphenyl)-4,4,4-trifluor-3-methylbutanoyl]amino}benzyl)-2-methylbutanoat *(Diastereomer A)* wurden in 2.3 ml Dichlormethan gelöst und bei RT mit 2 ml TFA versetzt. Nach 30 min wurde die Reaktionsmischung im Vakuum eingeengt und der Rückstand im Hochvakuum getrocknet. Der Rückstand wurde dann durch präparative RP-HPLC gereinigt (Eluent Acetonitril/Wasser). Es wurden 110.8 mg des Zielprodukts erhalten (40.9% d. Th.).

LC-MS (Methode 4): Rₜ = 1.50 min; m/z = 490 (M+H)⁺.

¹H-NMR (400 MHz, DMSO-*d*₆): δ [ppm] = 0.71-0.86 (m, 6H), 0.92 (s, 3H), 1.30-1.43 (m, 1H), 1.54-1.69 (m, 1H), 2.60 (d, 1H), 2.86 (d, 1H), 3.34-3.45 (m, 1H), 4.11 (d, 1H), 6.86-7.00 (m, 1H), 7.25-7.36 (m, 2H), 7.39-7.52 (m, 4H), 9.83 (s, 1H), 12.28 (s, 1H).

[α]_{D}²⁰ = +74.0°, c = 0.280, Chloroform.

Die folgenden Beispiele wurden gemäß der *Allgemeinen Vorschrift* 3 hergestellt (Spaltung von Methyl- oder Ethylestern zu den entsprechenden Carbonsäuren in Gemischen aus Salzsäure oder Schwefelsäure mit Essigsäure):

| **Beispiel** | **Name / Struktur / Edukt** | **Analytische Daten** |
|---|---|---|
| **72** | (+)-(2*S*)-3-(4-Chlor-3-{[(2*S*,3*R*)-2-(4-chlor-2-fluorphenyl)-4,4,4-trifluor-3-methylbutanoyl]amino}-phenyl)-2-methylpropansäure | LC-MS (Methode 6): Rₜ = 1.22 min; m/z = 480 (M+H)⁺. |
| | | |
| | 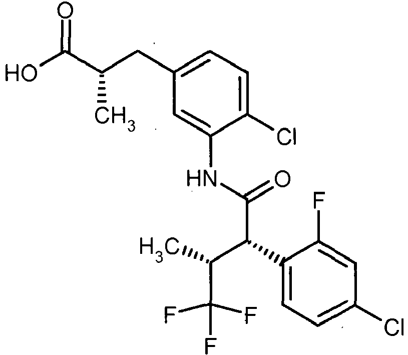 | ¹H-NMR (400 MHz, DMSO-*d*₆): δ [ppm] = 0.86 (d, 3H), 1.03 (d, 3H), 2.55-2.63 (m, 2H), 2.79-2.90 (m, 1H), 3.36-3.44 (m, 1H), 4.36 (d, 1H), 7.04 (dd, 1H), 7.26-7.39 (m, 3H), 7.52 (dd, 1H), 7.62 (t, 1H), 10.02 (s, 1H), 12.18 (br. s, 1H). |
| | | |
| | (aus (+)-Ethyl-(2*S*)-3-(4-chlor-3-{[(2*S*,3*R*)-2-(4-chlor-2-fluorphenyl)-4,4,4-trifluor-3-methyl-butanoyl]amino}phenyl)-2-methylpropanoat) | [α]_{D}²⁰ = +92.1 °, c = 0.365, Chloroform. |
| **73** | (+)-(2*S*)-3-(4-Chlor-3-{[(2*S*,3*R*)-2-(4-chlor-2-methylphenyl)-4,4,4-trifluor-3-methylbutanoyl]-amino}phenyl)-2-methylpropansäure | LC-MS (Methode 6): Rₜ = 1.26 min; m/z = 476 (M+H)⁺. |
| | | |
| | 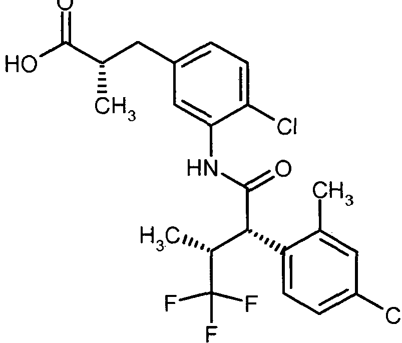 | ¹H-NMR (400 MHz, DMSO-d₆): δ [ppm] = 0.77 (d, 3H), 1.02 (d, 3H), 2.54-2.63 (m, 2H), 2.79-2.91 (m, 1H), 3.38 (br. s, 1H), 4.15 (d, 1H), 7.03 (dd, 1H), 7.22-7.38 (m, 4H), 7.52 (d, 1H), 9.89 (s, 1H), 12.18 (br. s, 1H). |
| | | |
| | (aus (+)-Ethyl-(2*S*)-3-(4-chlor-3-{[(2*S*,3*R*)-2-(4-chlor-2-methylphenyl)-4,4,4-trifluor-3-methyl-butanoyl]amino}phenyl)-2-methylpropanoat) | [α]_{D}²⁰ =124.3 °, c = 0.390, Chloroform. |
| **74** | (+)-(2*S*)-3-(4-Chlor-3-{[(2*S*,3*R*)-2-(4-chlor-3-fluophenyl)-4,4,4-trifluor-3-methylbutanoyl]-amino}phenyl)-2-methylpropansäure | LC-MS (Methode 6): Rₜ = 1.20 min; m/z = 480 (M+H)⁺. |
| | | |
| | 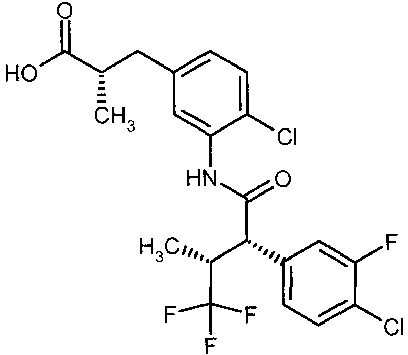 | ¹H-NMR (400 MHz, DMSO-*d*₆): δ [ppm] = 0.83 (d, 3H), 1.02 (d, 3H), 2.55-2.62 (m, 2H), 2.77-2.88 (m, 1H), 3.36-3.48 (m, 1H), 4.05-4.21 (m, 1H), 7.02 (dd, 1H), 7.25-7.41 (m, 3H), 7.49 (dd, 1H). 7.62 (t, 1H), 9.87 (s, 1H), 12.16 (br. s, 1H). |
| | | |
| | (aus (+)-Ethyl-(2*S*)-3-(4-chlor-3-{[(2*S*,3*R*)-2-(4-chlor-3-fluorphenyl)-4,4,4-trifluor-3-methyl-butanoyl]amino}phenyl)-2-methylpropanoat) | [α]_{D}²⁰ = +95.7°, c = 0.470, Chloroform. |
| **75** | (+)-(2*S*)-3-(4-Chlor-3-{[(2*S*,3*R*)-2-(4-chlor-3-methylphenyl)-4,4,4-trifluor-3-methylbutanoyl]-amino}phenyl)-2-methylpropansäure | LC-MS (Methode 4): Rₜ = 1.44 min; m/z = 476 (M+H)⁺. |
| | | |
| | 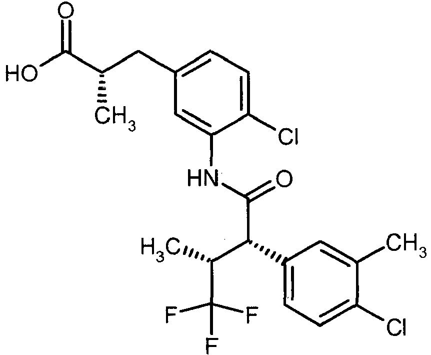 | ¹H-NMR (400 MHz, DMSO-*d*₆): δ [ppm] = 0.78-0.85 (m, 3H), 1.02 (d, 3H), 2.33 (s, 3H), 2.55-2.61 (m, 2H), 2.77-2.89 (m, 1H), 3.34-3.41 (m, ca. 1H, verdeckt), 4.04-4.10 (m, 1 H), 7.00 (dd, 1H), 7.26-7.45 (m, 5H), 9.81 (s, 1H), 12.17 (br. s, 1H). |
| | | |
| | (aus Ethyl-(2*S*)-3-(4-chlor-3-{[(2*S*,3*R*)-2-(4-chlor-3-methylphenyl)-4,4,4-trifluor-3-methyl-butanoyl]amino}phenyl)-2-methylpropanoat) | [α]_{D}²⁰ = +397.5°, c = 0.340, Chloroform. |
| **76** | (+)-(2*S*)-3-(4-Chlor-3-{[(2*S*,3*R*)-2-(3,4-dichlorphenyl)-4,4,4-trifluor-3-methylbutanoyl]amino}-phenyl)-2-methylpropansäure | LC-MS (Methode 6): Rₜ = 1.24 mim m/z = 496/498 (M+H)⁺. |
| | | |
| | 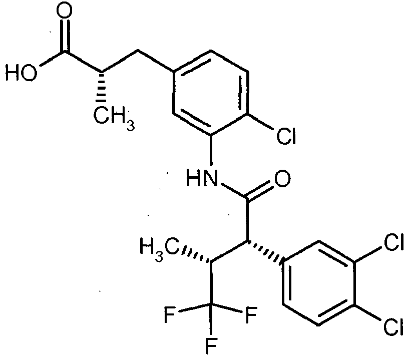 | ¹H-NMR (400 MHz, DMSO-*d*₆): δ [ppm] = 0.83 (d, 3H), 1.02 (d, 3H), 2.54-2.62 (m, 2H), 2.77-2.91 (m, 1H), 3.35-3.48 (m, 1H), 4.08-4.17 (m, 1H), 7.02 (dd, 1H), 7.31-7.39 (m, 2H), 7.45 (dd, 1H), 7.67 (d, 1H), 7.72 (d, 1H), 9.87 (s, 1H), 12.16 (br. s, 1H). |
| | | |
| | (aus Ethyl-(2*S*)-3-(4-chlor-3-{[(2*S*,3*R*)-2-(3,4-dichlophenyl)-4,4,4-trifluor-3-methylbutanoyl]-amino}phenyl)-2-methylpropanoat) | [α]_{D}²⁰ = +109.5°, c = 0.305, Methanol. |
| **77** | 3-(4-Chlor-3-{[(2*S*,3*R*)-2-(4-chlorphenyl)-4,4,4-trifluor-3-methylbutanoyl]amino}-2-methyl-phenyl)propansäure | LC-MS (Methode 6): Rₜ = 1.12 min; m/z = 462 (M+H)⁺. |
| | | |
| | 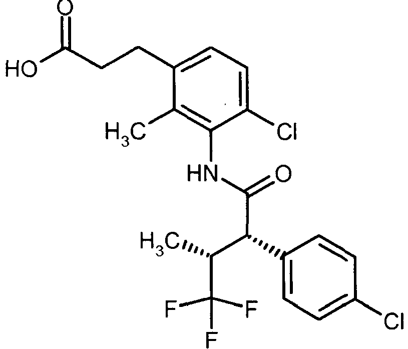 | ¹H-NMR (400 MHz, DMSO-*d*₆): δ [ppm] = 0.80 (d, 3H), 1.91/2.14 (2 br. s, zus. 3H), 2.42 (br. s, 2H), 2.73 (d, 2H), 3.34-3.43 (m, 1H), 3.96 (d, 1H), 7.04-7.14 (m, 1H), 7.14-7.35 (m, 1H), 7.45 (s, 4H), 9.91 (br. s, 1H), 12.13 (br. s, 1H) [*Signale sind infolge Rotamere stark verbreitert*]*.* |
| | (aus Methyl-3-(4-chlor-3-{[(2*S*,3*R*)-2-(4-chlorphenyl)-4,4,4-trifluor-3-methylbutanoyl]amino}-2-methylphenyl)propanoat) | |
| **78** | 3-(3-{[(2*S*,3*R*)-2-(4-Chlorphenyl)-4,4,4-trifluor-3-methylbutanoyl]amino}-4-fluorphenyl)-4,4,4-trifluorbutansäure *(Diastereomerengemisch)* | LC-MS (Methode 6): Rₜ = 1.14 min; m/z = 500 (M+H)⁺. |
| | | |
| | 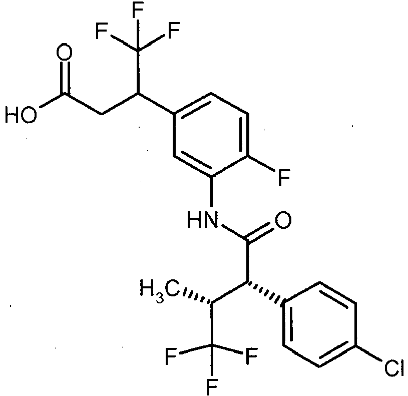 | ¹H-NMR (400 MHz, DMSO-d₆): beide Diastereomere δ [ppm] = 0.79 (d, 3H), 2.83 (dd, 1H), 2.95 (dd, 1H), 3.25-3.46 (m, 2H), 3.96-4.07 (m, 1H), 4.13 (d, 1H), 7.19-7.29 (m, 2H), 7.41-7.50 (m, 4H), 7.88 (d, 1H), 10.17 (s, 1H). |
| | (aus Ethyl-3-(3-{[(2*S*,3*R*)-2-(4-chlorphenyl)-4,4,4-trifluor-3-methylbutanoyl]amino}-4-fluorphenyl)-4,4,4-trifluorbutanoat) | |

### Beispiel 79 und Beispiel 80

### (+)-3-(3-{[(2S,3R)-2-(4-Chlorphenyl)-4,4,4-trifluor-3-methylbutanoyl]amino}-4-fluorphenyl)-4,4,4-trifluorbutansäure (Diastereomer 1 und 2)

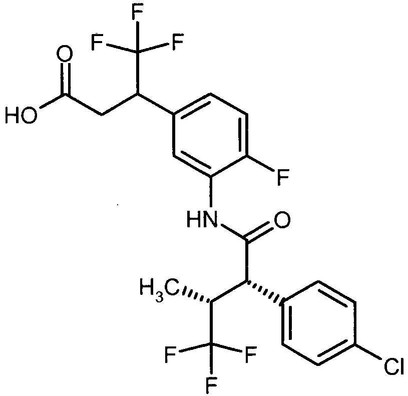

Das oben erhaltene Gemisch der diastereomeren 3-(3-{[(2*S*,3*R*)-2-(4-Chlorphenyl)-4,4,4-trifluor-3-methylbutanoyl]amino}-4-fluorphenyl)-4,4,4-trifluorbutansäuren (Beispiel 78) wurde durch präparative HPLC an chiraler Phase weiter aufgetrennt [Säule: Daicel Chiralpak AD-H, 5 µm, 250 mm x 20 mm; Fluss: 15 ml/min; Detektion: 230 nm; Injektionsvolumen: 0.80 ml; Temperatur: 45°C; Eluent: 92% Isohexan / 8% Isopropanol]. Ausgehend von 1.95 g Diastereomerengemisch wurden 556 mg Diastereomer 1 (*Beispiel 79*) und 730 mg Diastereomer 2 (*Beispiel 80*) erhalten:

### Beispiel 79 (Diastereomer 1):

Das Diastereomer 1 wurde durch präparative RP-HPLC (Eluent Methanol/Wasser) nochmals nachgereinigt. Es wurden 418 mg erhalten.

LC-MS (Methode 4): Rₜ = 1.49 min; m/z = 500 (M+H)⁺.

¹H-NMR (400 MHz, DMSO-*d*₆): δ [ppm] = 0.79 (d, 3H), 2.82 (dd, 1H), 2.94 (dd, 1H), 3.37-3.44 (m, 1H), 4.02 (td, 1H), 4.13 (d, 1H), 7.17-7.30 (m, 2H), 7.40-7.50 (m, 4H), 7.87 (d, 1H), 10.18 (s, 1H), 12.53 (br. s, 1H).

[α]_{D}²⁰ = +130°, c = 0.29, Chloroform.

### Beispiel 80 (Diastereomer 2):

Das Diastereomer 2 wurde durch präparative RP-HPLC (Eluent Methanol/Wasser) nochmals nachgereinigt. Es wurden 352 mg erhalten.

LC-MS (Methode 6): Rₜ = 1.18 min; m/z = 500 (M+H)⁺.

¹H-NMR (400 MHz, DMSO-*d*₆): δ [ppm] = 0.79 (d, 3H), 2.82 (dd, 1H), 2.94 (dd, 1H), 3.94-4.08 (m, 1H), 4.13 (d, 1H), 7.17-7.33 (m, 2H), 7.40-7.52 (m, 4H), 7.88 (d, 1H), 10.18 (s, 1H).

[α]_{D}²⁰=+104°, c = 0.260, Chloroform.

### Beispiel 81

### 3-(4-Chlor-3-{[(2S,3R)-2-(4-chlorphenyl)-4,4,4-trifluor-3-methylbutanoyl]amino}phenyl)hexansäure (Diastereomerengemisch)

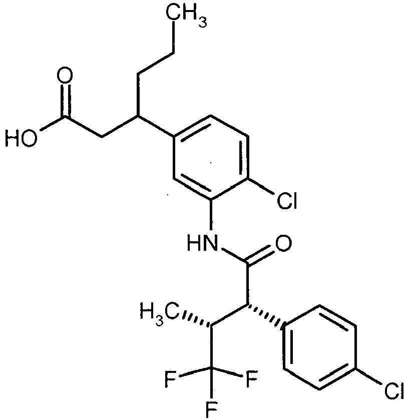

1.50 g (2.97 mmol) Methyl-3-(4-chlor-3-{[(2*S*,3*R*)-2-(4-chlorphenyl)-4,4,4-trifluor-3-methylbutanoyl]amino}phenyl)hexanoat (Diastereomerengemisch) wurden in 5 ml Essigsäure gelöst, mit 5 ml 30%-iger Schwefelsäure versetzt und auf Rückfluss erhitzt (Badtemperatur ca. 140°C). Nach 1.5 h wurden weitere 2.5 ml Essigsäure hinzugefügt, und die Reaktionsmischung wurde nochmals 2.5 h unter Rückfluss gerührt. Nach Abkühlen wurde das Gemisch über Nacht bei RT stehen gelassen, dann auf Wasser gegeben und dreimal mit Diethylether extrahiert. Die vereinigten organischen Phasen wurden mit 5%-iger Natriumhydrogencarbonat-Lösung und ges. Natriumchlorid-Lösung gewaschen, über Magnesiumsulfat getrocknet, im Vakuum eingeengt und der Rückstand im Hochvakuum getrocknet. Es wurden 1.44 g des Zielprodukts erhalten (98.8% d. Th).

LC-MS (Methode 6): Rₜ = 1.29 min; m/z = 490 (M+H)⁺.

¹H-NMR (400 MHz, DMSO-*d*₆): beide Diastereomere δ [ppm] = 0.74-0.83 (m, 6H), 0.99-1.14 (m, 2H), 1.37-1.60 (m, 2H), 2.39 (dd, 1H), 2.85-2.99 (m, 1H), 3.36-3.44 (m, 1H), 4.13 (d, 1H), 6.97-7.10 (m, 1H), 7.32-7.40 (m, 2H), 7.42-7.53 (m, 4H), 9.83 (s, 1H), 12.02 (br. s, 1H).

### Beispiel 82 und Beispiel 83

### (+)-3-(4-Chlor-3-{[(2S,3R)-2-(4-chlorphenyl)-4,4,4-trifluor-3-methylbutanoyl]amino}phenyl)hexansäure (Diastereomer 1 und 2)

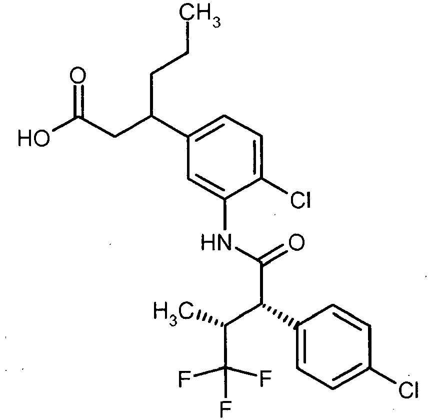

Das oben erhaltene Gemisch der diastereomeren 3-(4-Chlor-3-{[(2*S*,3*R*)-2-(4-chlophenyl)-4,4,4-trifluor-3-methylbutanoyl]amino}phenyl)hexansäuren (Beispiel 81) wurde durch präparative HPLC an chiraler Phase weiter aufgetrennt [Säule: Daicel Chiralpak AD-H, 5 µm, 250 mm x 20 mm; Fluss: 20 ml/min; Detektion: 230 nm; Injektionsvolumen: 0.60 ml; Temperatur: 25°C; Eluent: 95% Isohexan / 5% Isopropanol]. Ausgehend von 59.2 mg Diastereomerengemisch wurden 19 mg Diastereomer 1 (*Beispiel 82)* und 17 mg Diastereomer 2 (*Beispiel 83)* erhalten:

### Beispiel 82 (Diastereomer 1):

### (+)-(3S)-3-(4-Chlor-3-{[(2S,3R)-2-(4-chlorphenyl)-4,4,4-trifluor-3-methylbutanoyl]amino}-phenyl)-hexansäure

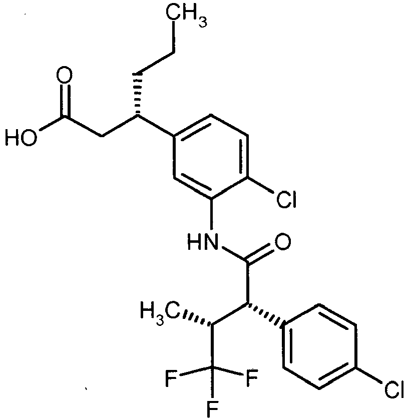

LC-MS (Methode 6): Rₜ = 1.27 min; m/z = 490 (M+H)⁺.

¹H-NMR (400 MHz, DMSO-*d*₆): δ [ppm] = 0.72-0.86 (m, 6H), 0.98-1.19 (m, 2H), 1.37-1.61 (m, 2H), 2.34-2.44 (m, 1H), 2.88-2.97 (m, 1H), 3.34-3.43 (m, 1H), 4.13 (d, 1H), 7.05 (dd, 1H), 7.26-7.40 (m, 2H), 7.41-7.63 (m, 4H), 9.82 (s, 1H), 12.02 (s, 1H).

[α]_{D}²⁰ = +52°, c = 0.30, Chloroform.

Nach der gleichen präparativen HPLC-Methode wurde auch eine größere Menge (1.40 g) der diastereomeren 3-(4-Chlor-3-{[(2*S*,3*R*)-2-(4-chlorphenyl)-4,4,4-trifluor-3-methylbutanoyl]amino}-phenyl)hexansäuren (Beispiel 81) aufgetrennt. Das erhaltene Diastereomer 1 wurde in diesem Fall durch RP-HPLC nochmals nachgereinigt [Säule: Sunfire 250 mm x 20 mm; Eluent: 80% Acetonitril / 5% aq. TFA (1%-ig) / 15% Wasser]. Es wurden so 337 mg an reinem Diastereomer 1 erhalten.

¹H-NMR (400 MHz, DMSO-*d*₆): δ [ppm] = 0.74-0.86 (m, 6H), 0.97-1.16 (m, 2H), 1.40-1.60 (m, 2H), 2.35-2.44 (m, 1H), 2.89-2.97 (m, 1H), 3.35-3.43 (m, 1H), 4.13 (d, 1H), 7.05 (dd, 1H), 7.30-7.40 (m, 2H), 7.41-7.54 (m, 4H), 9.83 (s, 1H), 12.02 (br. s, 1H).

[α]_{D}²⁰ = +86°, c = 0.480, Chloroform.

### Beispiel 83 (Diastereomer 2):

### (+)-(3R)-3-(4-Chlor-3-{[(2S,3R)-2-(4-chlorphenyl)-4,4,4-trifluor-3-methylbutanoyl]amino}-phenyl)-hexansäure

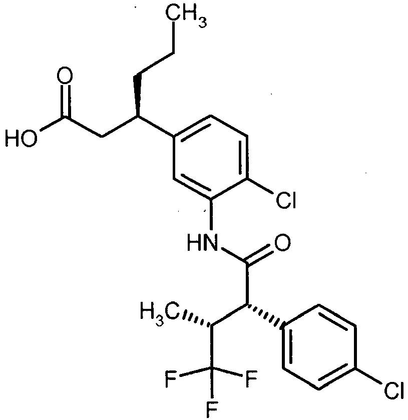

LC-MS (Methode 6): Rₜ = 1.27 min; m/z = 490 (M+H)⁺.

¹H-NMR (400 MHz, DMSO-*d*₆): δ [ppm] = 0.77-0.83 (m, 6H), 1.00-1.12 (m, 2H), 1.41-1.60 (m, 2H), 2.35-2.44 (m, 1H), 2.88-2.98 (m, 1H), 3.35-3.45 (m, 1H), 4.13 (d, 1H), 7.05 (dd, 1H), 7.32-7.40 (m, 2H), 7.43-7.64 (m, 4H), 9.82 (s, 1H), 12.04 (br. s, 1H).

[α]_{D}²⁰ = +22.1°, c = 0.40, Chloroform.

### Beispiel 84 und Beispiel 85

### (+)-3-(4-Chlor-3-{[(2S,3R)-2-(4-chlorphenyl)-4,4,4-trifluor-3-methylbutanoyl]amino}phenyl)-4,4,4-trifluorbutansäure (Diastereomer 1 und 2)

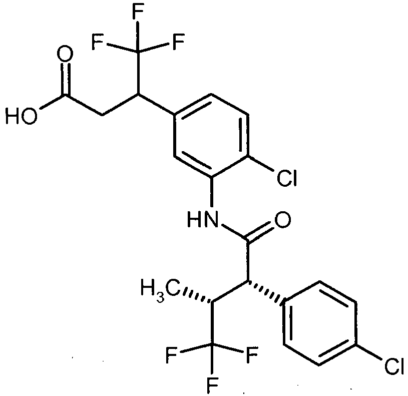

Das oben erhaltene Gemisch der diastereomeren 3-(4-Chlor-3-{[(2*S*,3*R*)-2-(4-chlorphenyl)-4,4,4-trifluor-3-methylbutanoyl]amino}phenyl)-4,4,4-trifluorbutansäuren (Beispiel 66) wurde durch präparative HPLC an chiraler Phase weiter aufgetrennt [Säule: Daicel Chiralpak AD-H, 5 µm, 250 mm x 20 mm; Fluss: 15 ml/min; Detektion: 220 nm; Injektionsvolumen: 0.25 ml; Temperatur: 30°C; Eluent: 93% Isohexan / 7% Isopropanol]. Ausgehend von 150 mg Diastereomerengemisch wurden 70 mg Diastereomer 1 (*Beispiel 84)* und 79 mg Diastereomer 2 *(Beispiel 85)* erhalten:

### Beispiel 84 (Diastereomer 1):

### (+)-(3S)-3-(4-Chlor-3-{[(2S,3R)-2-(4-chlorphenyl)-4,4,4-trifluor-3-methylbutanoyl]amino}phenyl)-4,4,4-trifluorbutansäure

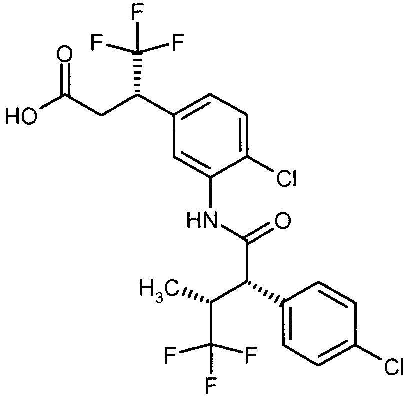

LC-MS (Methode 6): Rₜ = 1.18 min; m/z = 516 (M+H)⁺.

¹H-NMR (400 MHz, DMSO-*d*₆): δ [ppm] = 0.80 (d, 3H), 2.84 (dd, 1H), 2.95 (dd, 1H), 3.36-3.43 (m, 1H), 4.06 (td, 1H), 4.14 (d, 1H), 7.26 (dd, 1H), 7.40-7.52 (m, 5H), 7.60 (d, 1H), 9.95 (s, 1H), 12.54 (br. s, 1H).

[α]_{D}²⁰ = +78°, c = 0.52, Chloroform.

Alternativ konnte (+)-(3*S*)-3-(4-Chlor-3-{[(2*S*,3*R*)-2-(4-chlorphenyl)-4,4,4-trifluor-3-methylbutanoyl]amino}phenyl)-4,4,4-trifluorbutansäure auch auf folgendem Wege hergestellt werden:

1.76 g (3.08 mmol) (+)-*tert*.-Butyl-(3*S*)-3-(4-chlor-3-{[(2*S*,3*R*)-2-(4-chlorphenyl)-4,4,4-trifluor-3-methylbutanoyl]amino}phenyl)-4,4,4-trifluorbutanoat (Beispiel 203A) wurden in 4.9 ml Dichlormethan gelöst und bei RT mit 4.7 ml TFA versetzt. Die Reaktionsmischung wurde 2 h bei RT gerührt und dann im Vakuum eingeengt. Der Rückstand wurde in Ethylacetat aufgenommen und mit ges. Natriumhydrogencarbonat-Lösung und ges. Natriumchlorid-Lösung gewaschen, über Magnesiumsulfat getrocknet und im Vakuum eingeengt. Das Rohprodukt wurde durch präparative RP-HPLC gereinigt (Eluent Methanol/Wasser). Es wurden 1.30 g des Zielprodukts erhalten (81.9% d. Th.).

LC-MS (Methode 4): Rₜ = 1.46 min; m/z = 515 (M)⁺.

¹H-NMR (400 MHz, DMSO-*d*₆): δ [ppm] = 0.80 (d, 3H), 2.83 (dd, 1H), 2.95 (dd, 1H), 3.37-3.45 (m, 1H), 4.06 (td, 1H), 4.14 (d, 1H), 7.26 (dd, 1H), 7.43-7.52 (m, 5H), 7.60 (d, 1H), 9.95 (s, 1H), 12.56 (br. s, 1H).

[α]_{D}²⁰ = +79.9°, c = 0.475, Chloroform.

### Beispiel 85 (Diastereomer 2):

### (+)-(3R)-3-(4-Chlor-3-{[(2S,3R)-2-(4-chlorphenyl)-4,4,4-trifluor-3-methylbutanoyl]amino}phenyl)-4,4,4-trifluorbutansäure

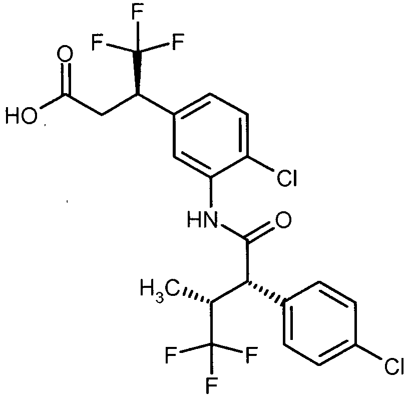

LC-MS (Methode 6): Rₜ = 1.19 min; m/z = 516 (M+H)⁺.

¹H-NMR (400 MHz, DMSO-*d*₆): δ [ppm] = 0.80 (d, 3H), 2.84 (dd, 1H), 2.95 (dd, 1H), 3.28-3.44 (m, 1H), 3.95-4.11 (m, 1H), 4.15 (d, 1H), 7.22-7.30 (m, 1H), 7.41-7.53 (m, 5H), 7.57-7.70 (m, 1H), 9.95 (s, 1H), 12.55 (br. s, 1H).

[α]_{D}²⁰ = +40.2°, c = 0.52, Chloroform.

Alternativ konnte (+)-(3*R*)-3-(4-Chlor-3-{[(2*S*,3*R*)-2-(4-chlorphenyl)-4,4,4-trifluor-3-methylbutanoyl]amino}phenyl)-4,4,4-trifluorbutansäure auch auf folgendem Wege hergestellt werden:

1.17 g (2.04 mmol) (+)-*tert*.-Butyl-(3*R*)-3-(4-chlor-3-{[(2*S*,3*R*)-2-(4-chlorphenyl)-4,4,4-trifluor-3-methylbutanoyl]amino}phenyl)-4,4,4-trifluorbutanoat (Beispiel 204A) wurden in 4.9 ml Dichlormethan gelöst und bei RT mit 3.2 ml TFA versetzt. Die Reaktionsmischung wurde 2 h bei RT gerührt und dann im Vakuum eingeengt. Der Rückstand wurde in Ethylacetat aufgenommen und mit ges. Natriumhydrogencarbonat-Lösung und ges. Natriumchlorid-Lösung gewaschen, über Magnesiumsulfat getrocknet und im Vakuum eingeengt. Das Rohprodukt wurde durch präparative RP-HPLC gereinigt (Eluent Methanol/Wasser). Es wurden 0.76 g des Zielprodukts erhalten (72% d. Th.).

LC-MS (Methode 6): Rₜ = 1.19 min; m/z = 516 (M+H)⁺.

¹H-NMR (400 MHz, DMSO-*d*₆): δ [ppm] = 0.80 (d, 3H), 2.83 (dd, 1H), 2.94 (dd, 1H), 3.37-3.47 (m, 1H), 3.93-4.10 (m, 1H), 4.15 (d, 1H), 7.26 (dd, 1H), 7.43-7.52 (m, 5H), 7.58-7.66 (m, 1H), 9.95 (s, 1H), 12.57 (br. s, 1H).

[α]_{D}²⁰ = +44.8°, c = 0.47, Chloroform.

### Beispiel 86

### 3-(4-Chlor-3-{[(2S,3R)-2-(4-chlorphenyl)-4,4,4-trifluor-3-methylbutanoyl]amino}phenyl)pentansäure (Diastereomerengemisch)

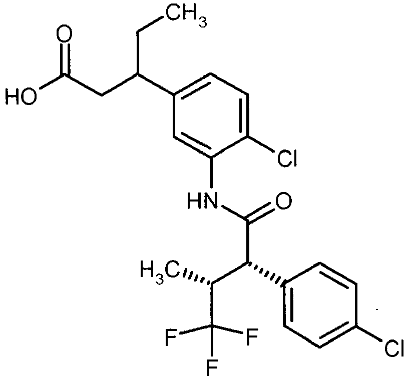

650 mg (1.33 mmol) Methyl-3-(4-chlor-3-{[(2*S*,3*R*)-2-(4-chlophenyl)-4,4,4-trifluor-3-methyl-butanoyl]amino}phenyl)pentanoat (Diastereomerengemisch) wurden in 2 ml Essigsäure gelöst, mit 1 ml 30%-iger Schwefelsäure versetzt und auf Rückfluss erhitzt (Badtemperatur ca. 140°C). Nach 1.5 h wurde die Reaktionsmischung abgekühlt und auf Wasser gegeben. Es wurde dreimal mit Ethylacetat extrahiert. Die vereinigten organischen Phasen wurden mit ges. Natriumhydrogencarbonat-Lösung und ges. Natriumchlorid-Lösung gewaschen, über Magnesiumsulfat getrocknet und im Vakuum eingeengt. Nach Trocknen im Hochvakuum wurde der Rückstand durch Chromatographie an Silicagel gereinigt (Laufmittel Cyclohexan/Ethylacetat 50:1). Es wurden 600 mg des Zielprodukts erhalten (95% d. Th).

LC-MS (Methode 6): Rₜ = 1.24 min; m/z = 476 (M+H)⁺.

¹H-NMR (400 MHz, DMSO-*d*₆): beide Diastereomere δ [ppm] = 0.69 (td, 3H), 0.80 (d, 3H), 1.40-1.52 (m, 1H), 1.55-1.68 (m, 1H), 2.40 (dd, 1H), 2.55-2.59 (m, 1H), 2.78-2.89 (m, 1H), 3.36-3.43 (m, 1H), 4.13 (d, 1H), 7.04 (dd, 1H), 7.31-7.41 (m, 2H), 7.42-7.57 (m, 4H), 9.84 (s, 1H), 12.04 (br. s, 1H).

### Beispiel 87 und Beispiel 88

### (+)-3-(4-Chlor-3-{[(2S,3R)-2-(4-chlophenyl)-4,4,4-trifluor-3-methylbutanoyl]amino}phenyl)pentansäure (Diastereomer 1 und 2)

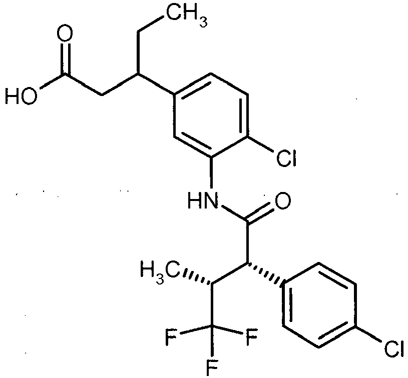

Das oben erhaltene Gemisch der diastereomeren 3-(4-Chlor-3-{[(2*S*,3*R*)-2-(4-chlorphenyl)-4,4,4-trifluor-3-methylbutanoyl]amino}phenyl)pentansäuren (Beispiel 86) wurde durch präparative HPLC an chiraler Phase weiter aufgetrennt [Säule: Daicel Chiralpak AD-H, 5 µm, 250 mm x 20 mm; Fluss: 18 ml/min; Detektion: 230 nm; Injektionsvolumen: 0.25 ml; Temperatur: 25°C; Eluent: 95% Isohexan / 5% Isopropanol]. Ausgehend von 545 mg Diastereomerengemisch wurden 140 mg Diastereomer 1 (*Beispiel 87*) und 156 mg Diastereomer 2 (*Beispiel 88)* erhalten:

### Beispiel 87 (Diastereomer 1):

### (+)-(3S)-3-(4-Chlor-3-{[(2S,3R)-2-(4-chlorphenyl)-4,4,4-trifluor-3-methylbutanoyl]amino}phenyl)-pentansäure

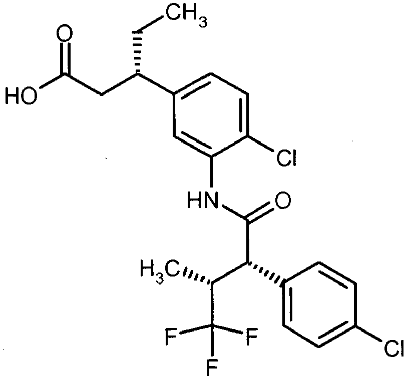

LC-MS (Methode 4): Rₜ = 1.47 min; m/z = 476 (M+H)⁺.

¹H-NMR (400 MHz, DMSO-*d*₆): δ [ppm] = 0.69 (t, 3H), 0.80 (d, 3H), 1.37-1.51 (m, 1H), 1.54-1.68 (m, 1H), 2.35-2.44 (m, 1 H), 2.55-2.59 (m, 1 H), 2.80-2.87 (m, 1 H), 3.36-3.40 (m, 1H), 4.13 (d, 1H), 7.04 (dd, 1H), 7.32-7.40 (m, 2H), 7.42-7.50 (m, 4H), 9.83 (s, 1H), 12.03 (br. s, 1H).

[α]_{D}²⁰ = +87.0, c = 0.47, Chloroform.

### Beispiel 88 (Diastereomer 2):

### (+)-(3R)-3-(4-Chlor-3-{[(2S,3R)-2-(4-chlorphenyl)-4,4,4-trifluor-3-methylbutanoyl]amino}phenyl)-pentansäure

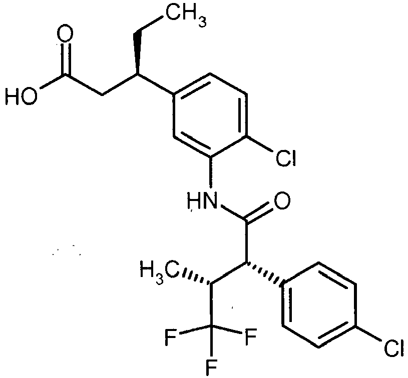

LC-MS (Methode 4): Rₜ = 1.47 min; m/z = 476 (M+H)⁺.

¹H-NMR (400 MHz, DMSO-*d*₆): δ [ppm] = 0.69 (t, 3H), 0.80 (d, 3H), 1.39-1.50 (m, 1H), 1.56-1.65 (m, 1H), 2.35-2.45 (m, 1H), 2.52-2.58 (m, 1H), 2.80-2.87 (m, 1H), 3.35-3.41 (m, 1H), 4.13 (d, 1H), 7.04 (dd, 1H), 7.31-7.41 (m, 2H), 7.42-7.52 (m, 4H), 9.83 (s, 1 H), 12.04 (br. s, 1H).

[α]_{D}²⁰ = +71.4, c = 0.48, Chloroform.

Die folgenden Beispiele wurden gemäß der *Allgemeinen Vorschrift* 2 hergestellt (Spaltung von *tert.-*Butylestem zu den entsprechenden Carbonsäuren mit Trifluoressigsäure):

| **Beispiel** | **Name / Struktur / Edukt** | **Analytische Daten** |
|---|---|---|
| **89** | 3-(4-Chlor-3-[(2*S*,3*R*)-2-(4-chlorphenyl)-4,4,4-trifluor-3-methylbutanoyl]amino}phenyl)-2,2-dimethylpropansäure | LC-MS (Methode 6): Rₜ = 1.24 min; m/z = 476/478 (M+H)⁺. |
| | 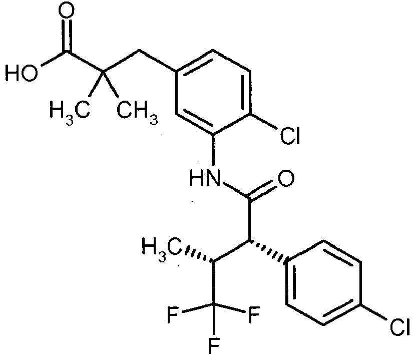 | |
| | | ¹H-NMR (400 MHz, DMSO-*d*₆): δ [ppm] = 0.80 (d, 3H), 1.046 (s, 3H), 1.051 (s, 3H), 2.74 (s, 2H), 3.29-3.45 (m, 1H, teilweise verdeckt durch H₂O-Signal), 4.11 (d, 1H), 6.95 (dd, 1H), 7.31-7.37 (m, 2H), 7.42-7.50 (m, 4H), 9.84 (s, 1H), 12.17-12.44 (br. s, 1H). |
| | (aus *tert*.-Butyl-3-(4-chlor-3-{[(2*S*,3*R*)-2-(4-chlor-phenyl)-4,4,4-trifluor-3-methylbutanoyl]amino}-phenyl)-2,2-dimethylpropanoat) | |
| **90** | [1-(4-Chlor-3- {[(2*S*,3*R*)-2-(4-chlorphenyl)-4,4,4-trifluor-3-methylbutanoyl]amino}phenyl)-cyclobutyl]essigsäure | MS: m/z = 488 (M+H)⁺. |
| | | |
| | 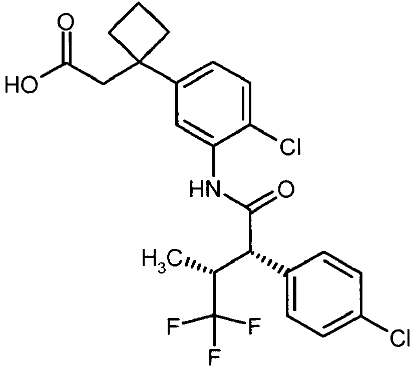 | ¹H-NMR (400 MHz, DMSO-*d*₆): δ [ppm] = 0.80 (d, 3H), 1.67-1.81 (m, 1H), 1.96-2.08 (m, 1H), 2.17-2.35 (m, 4H), 2.69 (s, 2H), 3.27-3.42 (m, 1H, teilweise verdeckt durch H₂O-Signal), 4.14 (d, 1H), 6.99 (dd, 1H), 7.35 (d, 1H), 7.41 (d, 1H), 7.43-7.50 (m, 4H), 9.81 (s, 1H), 11.88 (s, 1H). |
| | | |
| | (aus *tert*.-Butyl-[1-(4-chlor-3-{[(2*S*,3*R*)-2-(4-chlorphenyl)-4,4,4-trifluor-3-methylbutanoyl]amino}-phenyl)cyclobutyl] acetat) | [α]_{D}¹⁰ = +88°, c = 0.290, Methanol. |
| **91** | 3-(3-{[(2*S*,3*R*)-2-(4-Chlorphenyl)-4,4,4-trifluor-3-methylbutanoyl]amino}-2-methylphenyl)propansäure | LC-MS (Methode 4): Rₜ = 1.27 min; m/z = 428 (M+H)⁺. |
| | 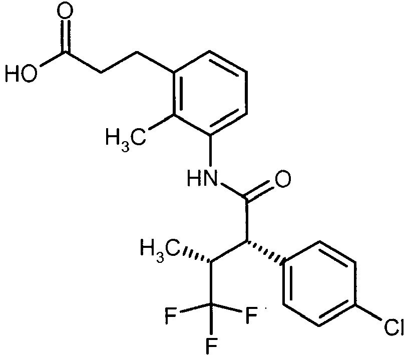 | |
| | | ¹H-NMR (400 MHz, DMSO-*d*₆): δ [ppm] = 0.80 (d, 3H), 1.92 (s, 3H), 2.43 (t, 2H), 2.79 (t, 2H), 3.28-3.44 (m, 1H, teilweise verdeckt durch H₂O-Signal), 3.94 (d, 1H), 6.93-7.09 (m, 3H), 7.39-7.52 (m, 4H), 9.69 (s, 1H), 12.14 (s, 1H). |
| | (aus *tert*.-Butyl-3-(3-{[(2*S*,3*R*)-2-(4-chlorphenyl)-4,4,4-trifluor-3-methylbutanoyl]amino}-2-methylphenyl)propanoat) | |
| **92** | 3-[4-Chlor-3-({(2*S*,3*R*)-2-[4-(3,3-difluorcyclobutyl)-phenyl]-4,4,4-trifluor-3-methylbutanoyl}amino)-phenyl]propansäure | LC-MS (Methode 6): Rₜ = 1.20 min; m/z = 502/504 (M-H)⁻. |
| | | |
| | 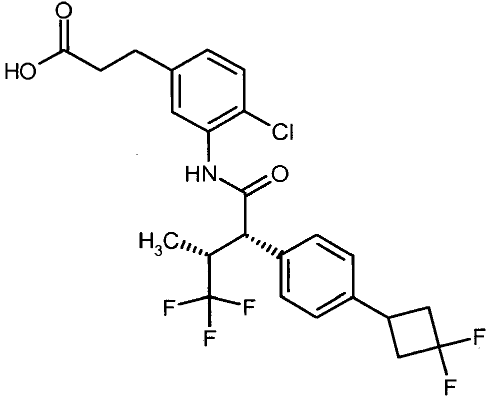 | ¹H-NMR (400 MHz, DMSO-*d*₆): δ [ppm] = 0.79 (d, 3H), 2.48 (t, 2H, teilweise verdeckt durch DMSO-Signal), 2.60-2.73 (m, 2H), 2.76 (t, 2H), 2.92-3.06 (m, 2H), 3.29-3.46 (m, 2H, teilweise verdeckt durch H₂O-Signal), 4.10 (d, 1H), 7.03 (dd, 1H), 7.32 (t, 3H), 7.41 (d, 3H), 9.76 (s, 1H), 12.02-12.26 (br. s, 1H). |
| | (aus *tert*.-Butyl-3-[4-chlor-3-({(2*S*,3*R*)-2-[4-(3,3-difluorcyclobutyl)phenyl]-4,4,4-trifluor-3-methyl-butanoyl}amino)phenyl]propanoat) | |
| **93** | 3-(4-Chlor-3-{[(2*S*,3*R*)-2-(4-chlor-3-methoxyphenyl)-4,4,4-trifluor-3-methylbutanoyl]amino} - phenyl)propansäure | LC-MS (Methode 6): Rₜ = 1.10 min; m/z = 476/478 (M-H)⁻. |
| | | |
| | 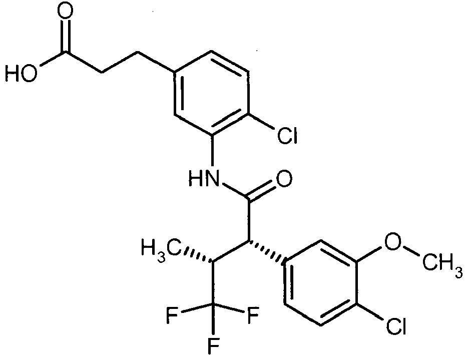 | ¹H-NMR (400 MHz, DMSO-*d*₆): δ [ppm] = 0.83 (d, 3H), 2.48 (t, 2H, teilweise verdeckt durch DMSO-Signal), 2.77 (t, 2H), 3.33-3.48 (m, 1H), 3.87 (s, 3H), 4.09 (d, 1H), 7.00-7.08 (m, 2H), 7.23 (d, 1H), 7.35 (d, 1H), 7.40 (d, 1H), 7.42 (d, 1H), 9.81 (s, 1H), 11.50-12.57 (br. s, 1H). |
| | (aus *tert*.-Butyl-3-(4-chlor-3-{[(2*S*,3*R*)-2-(4-chlor-3-methoxyphenyl)-4,4,4-trifluor-3-methylbutanoyl]-amino}phenyl)propanoat) | |
| **94** | 3-(3-{[(2*S*,3*R*)-2-(4-Chlor-3-methoxyphenyl)-4,4,4-trifluor-3-methylbutanoyl]amino}-4-fluorphenyl)-propansäure | LC-MS (Methode 6): Rₜ = 1.06 min; m/z = 460/462 (M-H)⁻. |
| | | |
| | 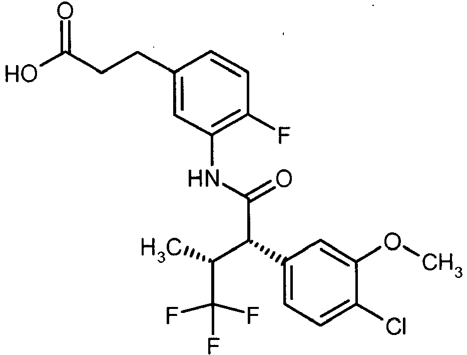 | ¹H-NMR (400 MHz, DMSO-*d*₆): δ [ppm] = 0.81 (d, 3H), 2.48 (t, 2H, teilweise verdeckt durch DMSO-Signal), 2.75 (t, 2H), 3.32-3.47 (m, 1H, teilweise verdeckt durch H₂O-Signal), 3.87 (s, 3H), 4.07 (d, 1H), 6.94-7.06 (m, 2H), 7.13 (t, 1H), 7.20 (s, 1H), 7.42 (d, 1H), 7.64 (d, 1H), 10.01 (s, 1H), 11.77-12.45 (br. s, 1H). |
| | (aus *tert*.-Butyl-3-(3-{[(2*S*,3*R*)-2-(4-chlor-3-methoxyphenyl)-4,4,4-trifluor-3-methylbutanoyl]-amino}-4-fluorphenyl)propanoat) | |
| **95** | 3-[4-Chlor-3-({(2*S*,3*R*)-2-[4-(2,2-difluorcyclopropyl)-phenyl]-4,4,4-trifluor-3-methyl-butanoyl} amino)phenyl]propansäure | LC-MS (Methode 7): Rₜ = 2.52 min; m/z = 488/490 (M-H)⁻. |
| | | |
| | 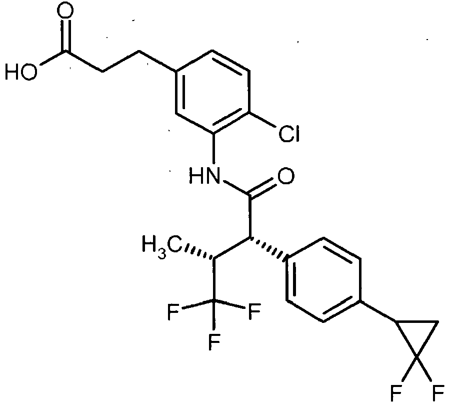 | ¹H-NMR (400 MHz, DMSO-*d*₆): δ [ppm] = 0.77 (d, 3H), 1.87-2.04 (m, 2H), 2.48 (t, 2H, teilweise verdeckt durch DMSO-Signal), 2.76 (t, 2H), 2.92-3.05 (m, 1H), 3.27-3.41 (m, 1H), 4.09 (d, 1H), 7.04 (dd, 1H), 7.28 (d, 2H), 7.34 (d, 1H), 7.38-7.44 (m, 3H), 9.78 (s, 1H), 11.60-12.59 (br. s, 1H). |
| | (aus *tert*.-Butyl-3-[4-chlor-3-({(2*S*,3*R*)-2-[4-(2,2-difluorcyclopropyl)phenyl]-4,4,4-trifluor-3-methylbutanoyl}amino)phenyl]propanoat) | |
| **96** | [1-(4-Chlor-3-{[(2*S*,3*R*)-2-(4-chlophenyl)-4,4,4-trifluor-3-methylbutanoyl]amino}phenyl)-cyclopropyl]essigsäure | LC-MS (Methode 6): Rₜ = 1.21 min; m/z = 474/476 (M+H)⁺. |
| | | |
| | 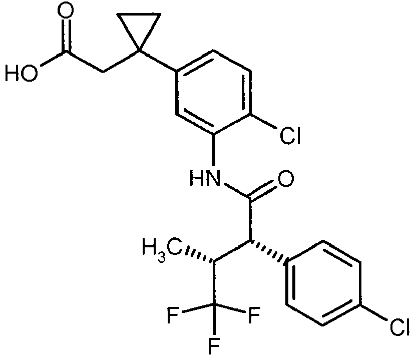 | ¹H-NMR (400 MHz, DMSO-*d*₆): δ [ppm] = 0.75-0.82 (m, 5H), 0.85-0.91 (m, 2H), 2.52 (s, 2H, teilweise verdeckt durch DMSO-Signal), 3.29-3.43 (m, 1H, teilweise verdeckt durch H₂O-Signal), 4.13 (d, 1H), 7.07 (dd, 1H), 7.32 (d, 1H), 7.42-7.50 (m, 5H), 9.82 (s, 1H), 11.89-12.10 (br. s, 1H). |
| | (aus *tert*.-Butyl-[1-(4-chlor-3-{[(2*S*,3*R*)-2-(4-chlorphenyl)-4,4,4-trifluor-3-methylbutanoyl]amino}phenyl)cyclopropyl]acetat) | |
| **97** | 3-(3-{[(2*S*,3*R*)-2-(4-Chlorphenyl)-4,4,4-trifluor-3-methylbutanoyl]amino}-4-cyanophenyl)-2-methylpropansäure | LC-MS (Methode 6): Rₜ = 1.07 min; m/z = 453/455 (M+H)⁺. |
| | | |
| | 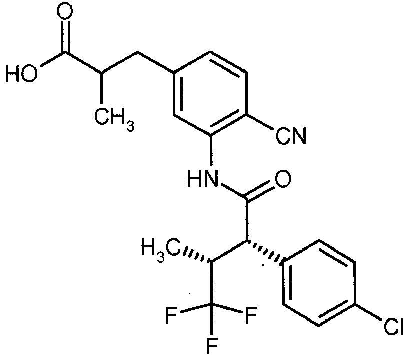 | ¹H-NMR (400 MHz, DMSO-*d*₆): δ [ppm] = 0.81 (d, 3H), 1.03 (d, 3H), 2.57-2.73 (m, 2H), 2.85-2.95 (m, 1H), 3.27-3.44 (m, 1H, teilweise verdeckt durch H₂O-Signal), 4.02 (d, 1H), 7.18 (d, 1H), 7.30 (s, 1H), 7.40-7.52 (m, 4H), 7.70 (d, 1H), 10.50 (s, 1H), 12.23 (br. s, 1H). |
| | (aus *tert.*-Butyl-3-(3-{[(2*S*,3*R*)-2-(4-chlorphenyl)-4,4,4-trifluor-3-methylbutanoyl]amino}-4-cyano-phenyl)-2-methylpropanoat) | |

Die folgenden Beispiele wurden gemäß der *Allgemeinen Vorschrift* 3 hergestellt (Spaltung von Methyl- oder Ethylestern zu den entsprechenden Carbonsäuren in Gemischen aus Salzsäure oder Schwefelsäure mit Essigsäure):

| **Beispiel** | **Name / Struktur / Edukt** | **Analytische Daten** |
|---|---|---|
| **98** | (2*S*)-3-[4-Chlor-3-({(2*S*,3*R*)-4,4,4-trifluor-3-methyl-2-[4-(1,1,1-trifluor-2-methylpropan-2-yl)phenyl]-butanoyl} amino)phenyl]-2-methylpropansäure | LC-MS (Methode 4): Rₜ = 1.49 min; m/z = 536/538 (M-H)⁻. |
| | | |
| | 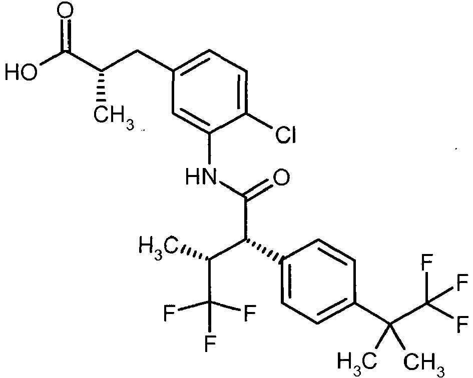 | ¹H-NMR (400 MHz, DMSO-*d*₆): δ [ppm] = 0.69 (d, 3H), 1.02 (d, 3H), 1.54 (s, 6H), 2.51-2.62 (m, 2H, teilweise verdeckt durch DMSO-Signal), 2.78-2.89 (m, 1H), 3.29-3.45 (m, 1H, teilweise verdeckt durch H₂O-Signal), 4.14 (d, 1H), 7.00 (dd, 1H), 7.35 (d, 1H), 7.45 (d, 1H), 7.48(d, 2H), 7.54 (d, 2H), 9.80 (s, 1H), 12.14 (s, 1H). |
| | (aus Ethyl-(2*S*)-3-[4-chlor-3-({(2*S*,3*R*)-4,4,4-trifluor-3-methyl-2-[4-(1,1,1-trifluor-2-methylpropan-2-yl)-phenyl]butanoyl}amino)phenyl]-2-methylpropanoat) | |
| **99** | (2*S*)-3-[4-Chlor-3-({4,4,4-trifluor-3-methyl-2-[4-(2,2,2-trifluorethyl)phenyl]butanoyl}amino)phenyl]-2-methylpropansäure | LC-MS (Methode 4): Rₜ = 1.39 min; m/z = 508/510 (M-H)⁻. |
| | | |
| | 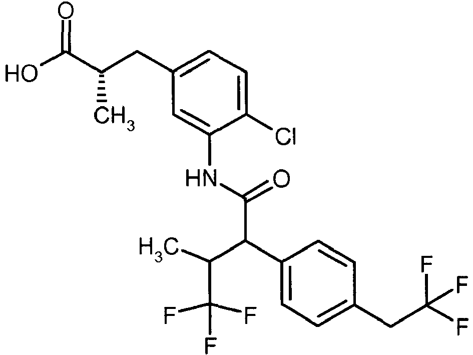 | ¹H-NMR (400 MHz, DMSO-*d*₆): δ [ppm] = 0.70 (d, 3H), 1.01 (d, 3H), 2.50-2.61 (m, 2H, teilweise verdeckt durch DMSO-Signal), 2.78-2.90 (m, 1H), 3.29-3.45 (m, 1H, teilweise verdeckt durch H₂O-Signal), 3.63 (q, 2H), 4.11 (d, 1H), 7.00 (dd, 1H), 7.35 (d, 2H), 7.39 (d, 2H), 7.46 (d, 2H), 9.80 (s, 1H), 12.15 (s, 1H). |
| | (aus Ethyl-(2*S*)-3-[4-chlor-3-({4,4,4-trifluor-3-methyl-2-[4-(2,2,2-trifluorethyl)phenyl]butanoyl}-amino)phenyl]-2-methylpropanoat) | |
| **100** | (2*S*)-3-[4-Chlor-3-({(2*S*,3*R*)-2-[4-(3,3-difluor-cyclobutyl)phenyl]-4,4,4-trifluor-3-methylbutanoyl} -amino)phenyl]-2-methylpropansäure | LC-MS (Methode 6): Rₜ = 1.24 min; m/z = 516/518 (M-H)⁻. |
| | | |
| | 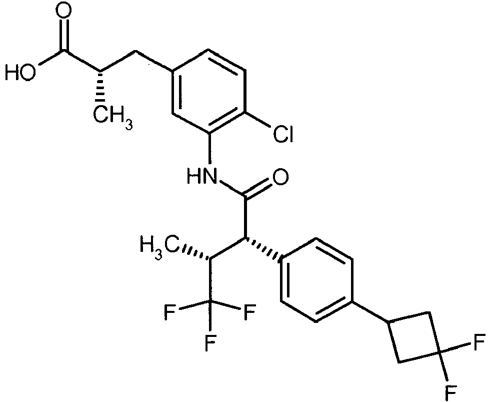 | ¹H-NMR (400 MHz, DMSO-*d*₆): δ [ppm] = 0.79 (d, 3H), 1.01 (d, 3H), 2.46-2.60 (m, 2H, teilweise verdeckt durch DMSO-Signal), 2.60-2.76 (m, 2H), 2.77-2.87 (m, 1H), 2.91-3.06 (m, 2H), 3.26-3.46 (m, 2H, teilweise verdeckt durch H₂O-Signal), 4.10 (d, 1H), 6.99 (dd, 1H), 7.33 (t, 3H), 7.42 (d, 3H), 9.76 (s, 1H), 11.86-12.37 (br. s, 1H). |
| | (aus Ethyl-(2*S*)-3-[4-chlor-3-({(2*S*,3*R*)-2-[4-(3,3-difluorcyclobutyl)phenyl]-4,4,4-trifluor-3-methyl-butanoyl}amino)phenyl]-2-methylpropanoat) | |
| **101** | (2S)-3-(4-Chlor-3-{[(2*S*,3*R*)-2-(4-chlor-3-methoxyphenyl)-4,4,4-trifluor-3-methylbutanoyl]amino}-phenyl)-2-methylpropansäure | LC-MS (Methode 6): Rₜ = 1.15 min; m/z = 492/494 (M+H)⁺. |
| | | |
| | 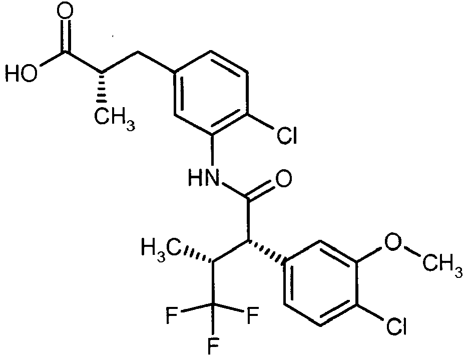 | ¹H-NMR (400 MHz, DMSO-*d*₆): δ [ppm] = 0.83 (d, 3H), 1.02 (d, 3H), 2.46-2.61 (m, 2H, teilweise verdeckt durch DMSO-Signal), 2.77-2.88 (m, 1H), 3.36-3.47 (m, 1H), 3.87 (s, 3H), 4.09 (d, 1H), 6.98-7.06 (m, 2H), 7.23 (d, 1H), 7.36 (d, 1H), 7.38 (d, 1H), 7.43 (d, 1H), 9.81 (s, 1H), 12.04-12.26 (br. s, 1H). |
| | (aus Ethyl-(2*S*)-3-(4-chlor-3-{[(2*S*,3*R*)-2-(4-chlor-3-methoxyphenyl)-4,4,4-trifluor-3-methylbutanoyl]-amino}phenyl)-2-methylpropanoat) | |
| **102** | [1-(4-Chlor-3-{[(2*S*,3*R*)-2-(4-chlorphenyl)-4,4,4-trifluor-3-methylbutanoyl]amino}phenyl)-3,3-difluorcyclobutyl]essigsäure | LC-MS-(Methode 6): Rₜ = 1.22 min; m/z = 524/526 (M+H)⁺. |
| | | |
| | 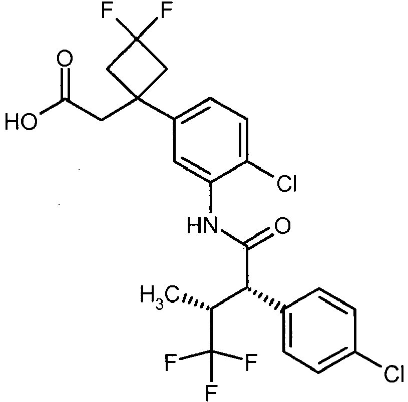 | ¹H-NMR (400 MHz, DMSO-*d*₆): δ [ppm] = 0.80 (d, 3H), 2.76 (s, 2H), 2.81-2.94 (m, 2H), 2.95-3.09 (m, 2H), 3.29-3.44 (m, 1H, teilweise verdeckt durch H₂O-Signal), 4.15 (d, 1H), 7.12 (dd, 1H), 7.41 (d, 1H), 7.42-7.49 (m, 4H), 7.50 (d, 1H), 9.88 (s, 1H), 12.09-12.24 (br. s, 1H). |
| | (aus Ethyl-[1-(4-chlor-3-{[(2*S*,3*R*)-2-(4-chlophenyl)-4,4,4-trifluor-3-methylbutanoyl]amino}phenyl)-3,3-difluorcyclobutyl]acetat) | |

### Beispiel 103 und Beispiel 104

### (2S)-3-[4-Chlor-3-({4,4,4-trifluor-3-methyl-2-[4-(2,2,2-trifluorethyl)phenyl]butanoyl}amino)-phenyl]-2-methylpropansäure (Diastereomer 1 und 2)

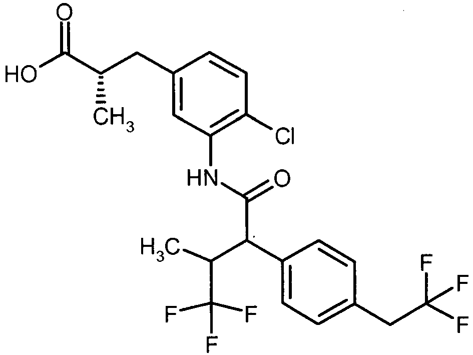

74 mg (0.15 mmol) des Isomerengemisches von (2*S*)-3-[4-Chlor-3-({4,4,4-trifluor-3-methyl-2-[4-(2,2,2-trifluorethyl)phenyl]butanoyl}amino)phenyl]-2-methylpropansäure (Beispiel 99) wurden mittels präparativer HPLC an chiraler Phase weiter aufgetrennt [Säule: Daicel Chiralpak AY-H, 5 µm, 250 mm x 20 mm; Elutionsmittel: Isohexan/Ethanol 1:1 (v/v); Fluss: 15 ml/min; UV-Detektion: 220 nm; Temperatur: 45°C]:

### Beispiel 103 (Diastereomer 1):

Ausbeute: 39 mg

Rₜ = 3.72 min; chemische Reinheit >98%; >99% de
[Säule: Daicel Chiralpak AY-H, 5 µm, 250 mm x 4 mm; Elutionsmittel: Isohexan/(Isopropanol + 0.2% TFA + 1% Wasser) 70:30 (v/v); Fluss: 1 ml/min; Temperatur: 45°C; UV-Detektion: 220 nm].

LC-MS (Methode 6): Rₜ = 1.16 min; m/z = 508/510 (M-H)⁻.

### Beispiel 104 (Diastereomer 2):

Ausbeute: 39 mg

Rₜ = 6.09 min; chemische Reinheit >98%; >99% de
[Säule: Daicel Chiralpak AY-H, 5 µm, 250 mm x 4 mm; Elutionsmittel: Isohexan/(Isopropanol + 0.2% TFA + 1% Wasser) 70:30 (v/v); Fluss: 1 ml/min; Temperatur: 45°C; UV-Detektion: 220 nm].

LC-MS (Methode 6): Rₜ = 1.16 min; m/z = 508/510 (M-H)⁻.

### Beispiele 105 - 108

### (2S)-3-(4-Chlor-3-{[(4-chlorphenyl)(3,3-difluorcyclopentyl)acetyl]amino}phenyl)-2-methylpropansäure (Isomer 1 - 4)

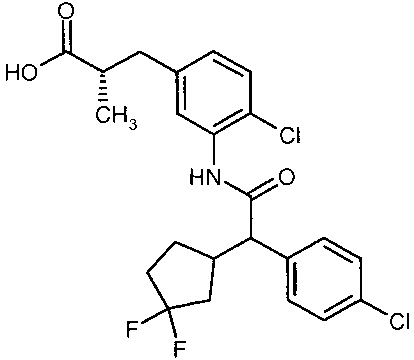

205 mg (0.44 mmol) des Diastereomerengemisches von (2*S*)-3-(4-Chlor-3-{[(4-chlorphenyl)(3,3-difluorcyclopentyl)acetyl]amino}phenyl)-2-methylpropansäure (Beispiel 20) wurden mittels präparativer HPLC an chiraler Phase weiter aufgetrennt [Säule: Daicel Chiralcel OJ-H, 5 µm, 250 mm x 20 mm; Elutionsmittel: Isohexan/Ethanol 70:30 (v/v); Fluss: 25 ml/min; UV-Detektion: 230 nm; Temperatur: 25°C]. Hierbei wurden zwei verschiedene Fraktionen erhalten, welche jeweils aus einem Gemisch von zwei Isomeren bestanden. Diese beiden Fraktionen wurden durch nochmalige präparative HPLC an chiraler Phase in die einzelnen Isomeren aufgetrennt [*Fraktion 1:* Säule: Daicel Chiralpak AD-H, 5 µm, 250 mm x 20 mm; Elutionsmittel: Isohexan/Isopropanol 80:20 (v/v); Fluss: 20 ml/min; UV-Detektion: 230 nm; Temperatur: 25°C. *Fraktion 2:* Säule: Daicel Chiralpak AD-H, 5 µm, 250 mm x 20 mm; Elutionsmittel: Isohexan/Ethanol 90:10 (v/v); Fluss: 20ml/min; UV-Detektion: 230 nm; Temperatur: 25°C]:

### Beispiel 105 (Isomer 1):

Ausbeute: 30 mg

Rₜ = 15.70 min; chemische Reinheit >89.8%
[Säule: Daicel Chiralpak AD-H, 5 µm, 250 mm x 4 mm; Elutionsmittel: Isohexan/Isopropanol 70:30 (v/v); Fluss: 1 ml/min; UV-Detektion: 230 nm; Temperatur: 25°C].

LC-MS (Methode 6): Rₜ = 1.22 min; m/z = 470/472 (M+H)⁺.

¹H-NMR (400 MHz, DMSO-*d*₆): δ [ppm] = 1.02 (d, 3H), 1.21-1.35 (m, 1H), 1.45-1.58 (m, 1H), 1.84-2.20 (m, 3H), 2.28-2.43 (m, 1H), 2.53-2.62 (m, 2H, teilweise verdeckt durch DMSO-Signal), 2.76-2.90 (m, 2H), 3.75 (d, 1H), 7.03 (dd, 1H), 7.32-7.49 (m, 6H), 9.74 (s, 1H), 12.04-12.35 (br. s, 1H).

### Beispiel 106 (Isomer 2):

Ausbeute: 35 mg

Rₜ = 20.07 min; chemische Reinheit >98.9%
[Säule: Daicel Chiralpak AD-H, 5 µm, 250 mm x 4 mm; Elutionsmittel: Isohexan/Isopropanol 70:30 (v/v); Fluss: 1 ml/min; UV-Detektion: 230 nm; Temperatur: 25°C].

LC-MS (Methode 5): Rₜ = 2.58 min; m/z = 470/472 (M+H)⁺.

¹H-NMR (400 MHz, DMSO-*d*₆): δ [ppm] = 1.02 (d, 3H), 1.52-1.69 (m, 2H), 1.81-1.96 (m, 1H), 1.98-2.29 (m, 3H), 2.52-2.62 (m, 2H, teilweise verdeckt durch DMSO-Signal), 2.78-2.92 (m, 2H), 3.78 (d, 1H), 7.03 (dd, 1H), 7.33-7.48 (m, 6H), 9.78 (s, 1H), 12.04-12.26 (br. s, 1H).

### Beispiel 107 (Isomer 3):

Ausbeute: 37 mg

Rₜ = 14.17 min; chemische Reinheit >95.7%
[Säule: Daicel Chiralpak AD-H, 5 µm, 250 mm x 4 mm; Elutionsmittel: Isohexan/Ethanol 90:10 (v/v); Fluss: 1 ml/min; UV-Detektion: 230 nm; Temperatur: 25°C].

LC-MS (Methode 5): Rₜ = 2.57 min; m/z = 470/472 (M+H)⁺.

¹H-NMR: siehe Beispiel 106 (*Isomer 2*)*.*

### Beispiel 108 (Isomer 4):

Ausbeute: 29 mg

Rₜ = 17.77 min; chemische Reinheit >99.5%
[Säule: Daicel Chiralpak AD-H, 5 µm, 250 mm x 4 mm; Elutionsmittel: Isohexan/Ethanol 90:10 (v/v); Fluss: 1 ml/min; UV-Detektion: 230 nm; Temperatur: 25°C].

LC-MS (Methode 5): Rₜ = 2.58 min; m/z = 470/472 (M+H)⁺.

¹H-NMR: siehe Beispiel 105 (*Isomer 1).*

### Beispiele 109 - 112

### (2R)-3-(4-Chlor-3-{[(4-chlorphenyl)(3,3-difluorcyclopentyl)acetyl]amino}phenyl)-2-methylpropansäure (Isomer 1 - 4)

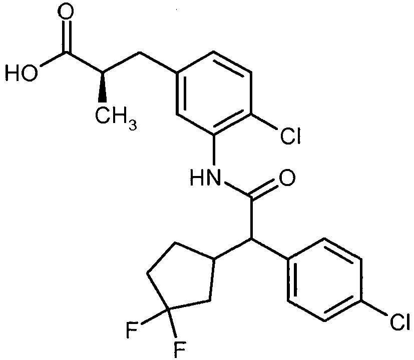

160 mg (0.32 mmol) des Diastereomerengemisches von (2*R*)-3-(4-Chlor-3-{[(4-chlorphenyl)(3,3-difluorcyclopentyl)acetyl]amino}phenyl)-2-methylpropansäure (Beispiel 21) wurden mittels präparativer HPLC an chiraler Phase in die vier Isomere aufgetrennt [Säule: Daicel Chiralcel OJ-H, 5 µm, 250 mm x 20 mm; Elutionsmittel: Isohexan/Ethanol/Methanol 90:5:5 (v/v); Fluss: 20 ml/ min; UV-Detektion: 230 nm; Temperatur: 25°C]:

### Beispiel 109 (Isomer 1):

Ausbeute: 16.7 mg

Rₜ = 10.49 min; chemische Reinheit >92.8%
[Säule: Daicel Chiralpak OJ-H, 5 µm, 250 mm x 4 mm; Elutionsmittel: Isohexan/Ethanol/ Methanol 90:5:5 (v/v); Fluss: 1 ml/min; UV-Detektion: 230 nm; Temperatur: 25°C].

LC-MS (Methode 4): Rₜ = 1.39 min; m/z = 470/472 (M+H)⁺.

### Beispiel 110 (Isomer 2):

Ausbeute: 24.4 mg

Rₜ = 12.26 min; chemische Reinheit >94.7%
[Säule: Daicel Chiralpak OJ-H, 5 µm, 250 mm x 4 mm; Elutionsmittel: Isohexan/Ethanol/ Methanol 90:5:5 (v/v); Fluss: 1 ml/min; UV-Detektion: 230 nm; Temperatur: 25°C].

LC-MS (Methode 4): Rₜ = 1.39 min; m/z = 470/472 (M+H)⁺.

### Beispiel 111 (Isomer 3):

Ausbeute: 22 mg

Rₜ = 18.89 min; chemische Reinheit >97.8%
[Säule: Daicel Chiralpak OJ-H, 5 µm, 250 mm x 4 mm; Elutionsmittel: Isohexan/Ethanol/ Methanol 90:5:5 (v/v); Fluss: 1 ml/min; UV-Detektion: 230 nm; Temperatur: 25°C].

LC-MS (Methode 4): Rₜ = 1.39 min; m/z = 470/472 (M+H)⁺.

### Beispiel 112 (Isomer 4):

Ausbeute: 25 mg

Rₜ = 28.37 min; chemische Reinheit >97.8%
[Säule: Daicel Chiralpak.OJ-H, 5 µm, 250 mm x 4 mm; Elutionsmittel: Isohexan/Ethanol/ Methanol 90:5:5 (v/v); Fluss: 1 ml/min; UV-Detektion: 230 nm; Temperatur: 25°C].

LC-MS (Methode 4): Rₜ = 1.39 min; m/z = 470/472 (M+H)⁺.

Das folgende Beispiel wurde gemäß der *Allgemeinen Vorschrift 3* hergestellt:

| **Beispiel** | **Name / Struktur / Edukt** | **Analytische Daten** |
|---|---|---|
| **113** | 3-(4-Chlor-3-{[(2*S*,3*R*)-2-(4-chlorphenyl)-4,4,4-trifluor-3-methylbutanoyl]amino}phenyl)-3-methylbutansäure | LC-MS (Methode 6): Rₜ = 1.23 min; m/z = 476/478 (M+H)⁺. |
| | | |
| | 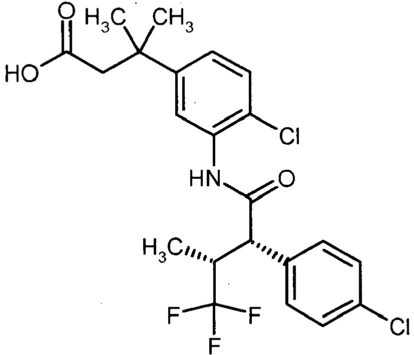 | ¹H-NMR (400 MHz, DMSO-*d*₆): δ [ppm] = 0.80 (d, 3H), 1.31 (s, 6H), 2.53 (s, 2H, teilweise verdeckt durch DMSO-Signal), 3.30-3.44 (m, 1H, teilweise verdeckt durch H₂O-Signal), 4.14 (d, 1H), 7.20 (dd, 1H), 7.35 (d, 1H), 7.42-7.50 (m, 4H), 7.56 (d, 1H), 9.83 (s, 1H), 11.85-11.97 (br. s, 1H). |
| | (aus Ethyl-3-(4-chlor-3-{[(2*S*,3*R*)-2-(4-chlorphenyl)-4,4,4-trifluor-3-methylbutanoyl]amino}phenyl)-3-methylbutanoat) | |

### B. Bewertung der pharmakologischen Wirksamkeit

Die pharmakologische Wirkung der erfindungsgemäßen Verbindungen kann in folgenden Assays gezeigt werden:

### B-1. Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und olme Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als x-fache Stimulation der Basalaktivität angegeben. Das Ergebnis für Beispiel 15 ist in Tabelle 1A und das für Beispiel 17 in Tabelle 1B gezeigt:

**Tabelle 1A: Stimulation (x-fach) der rekombinanten löslichen Guanylatcyclase (sGC) in vitro durch Beispiel 15**

| **Konzentration Beispiel 15 [µM]** | **Häm-haltige sGC** | | | **Häm-freie sGC** |
|---|---|---|---|---|
| | **Basal (n=8)** | **+ 0.01 µM DEA/NO** | **+ 10 µM ODQ** | **Basal (n=8)** |
| 0 | 1.0 ± 0.0 | 6.5 ± 0.8 | 4.4 ± 0.8 | 1.0 ± 0.0 |
| 0.01 | 1.1 ± 0.1 | 5.9 ± 0.7 | 4.6 ± 0.8 | 1.7 ± 0.3 |
| 0.1 | 1.0 ± 0.1 | 7.4 ± 0.8 | 4.5 ± 0.6 | 1.8 ± 0.2 |
| 1.0 | 0.9 ± 0.1 | 8.5 ± 0.7 | 4.8 ± 0.8 | 3.0 ± 0.5 |
| 10 | 3.1 ± 0.3 | 11.5 ± 0.9 | 16.3 ± 1.3 | 17.9 ± 3.1 |
| 100 | 31.6 ± 2.6 | 45.9 ± 3.1 | 97.3 ± 7.7 | 40.3 ± 6.6 |

**Tabelle 1B: Stimulation (x-fach) der rekombinanten löslichen Guanylatcyclase (sGC) in vitro durch Beispiel 17**

| **Konzentration Beispiel 17 [µM]** | **Häm-haltige sGC** | | | **Häm-freie sGC** |
|---|---|---|---|---|
| | **Basal (n=6)** | **+ 0.01 µM DEA/NO** | **+ 10 µM ODQ** | **Basal (n=6)** |
| 0 | 1.0 ± 0.0 | 7.9 ± 0.7 | 2.8 ± 0.6 | 1.0 ± 0.0 |
| 0.01 | 0.7 ± 0.2 | 9.6 ± 0.8 | 4.9 ± 1.2 | 1.7 ± 0.2 |
| 0.1 | 0.6 ± 0.1 | 8.8 ± 1.2 | 5.3 ± 1.3 | 2.0 ± 0.3 |
| 1.0 | 0.6 ± 0.1 | 9.8 ± 1.2 | 4.5 ± 1.1 | 4.1 ± 0.3 |
| 10 | 1.8 ± 0.3 | 10.7 ± 1.0 | 8.3 ± 1.4 | 22.9 ± 1.8 |
| 100 | 4.9 ± 0.7 | 11.4 ± 1.2 | 15.0 ± 2.0 | 33.5 ± 3.3 |

| | | | | |
|---|---|---|---|---|
| [DEA/NO = 2-(*N,N*-Diethylamino)diazenolat-2-oxid; ODQ = 1*H-*1,2,4-Oxadiazolo[4,3-a]chinoxalin-1-on]. | | | | |

Aus den Tabellen 1A und 1B ist ersichtlich, dass eine Stimulation sowohl des Häm-haltigen als auch des Häm-freien Enzyms erreicht wird. Weiterhin zeigt die Kombination von Beispiel 15 bzw. Beispiel 17 mit 2-(*N,N-*Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch 1*H*-1,2,4-Oxadiazolo[4,3-a]-chinoxalin-1-on (ODQ), einen Häm-abhängigen Inhibitor der löslichen Guanylatcyclase, nicht blockiert, sondern sogar gesteigert. Die Ergebnisse in den Tabellen 1A und 1B belegen somit den Wirkmechanismus der erfindungsgemäßen Verbindungen als Aktivatoren der löslichen Guanylatcyclase.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 2 aufgeführt:

**Tabelle 2: sGC-aktivierende Wirkung in der CHO-Reporterzelle in vitro**

| **Beispiel Nr.** | **MEC [nM]** |
|---|---|
| 2 | 0.3 |
| 5 | 3.0 |
| 7 | 0.2 |
| 10 | 5.2 |
| 15 | 10 |
| 17 | 4.8 |
| 18 | 10 |
| 28 | 1.0 |
| 30 | 0.3 |
| 37 | 10 |
| 50 | 30 |
| 53 | 300 |
| 71 | 10 |
| 81 | 0.3 |
| 82 | 0.23 |
| 84 | 1 |
| 87 | 1 |
| 89 | 3 |
| 90 | 1 |
| 96 | 1 |
| 99 | 10 |
| 100 | 3 |
| 102 | 1 |
| 105 | 30 |

| | |
|---|---|
| (MEC = minimale effektive Konzentration). | |

### B-3. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1 % BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 3 aufgeführt:

**Tabelle 3: Aktivierende Wirkung am sGC-Enzym in vitro**

| **Beispiel Nr.** | **MEC [nM]** | **EC₅₀ [nM]** |
|---|---|---|
| 15 | 10 | 290 |
| 17 | 7 | 130 |

| | | |
|---|---|---|
| (MEC = minimale effektive Konzentration; EC₅₀ = Konzentration bei 50% der maximalen Wirksamkeit). | | |

### B-4. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (1) Implantierbare Sender, (2) Empfänger, die über einen Multiplexer mit einem (3) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (1) systolischer Blutdruck (SBP), (2) diastolischer Blutdruck (DBP), (3) arterieller Mitteldruck (MAP) und (4) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 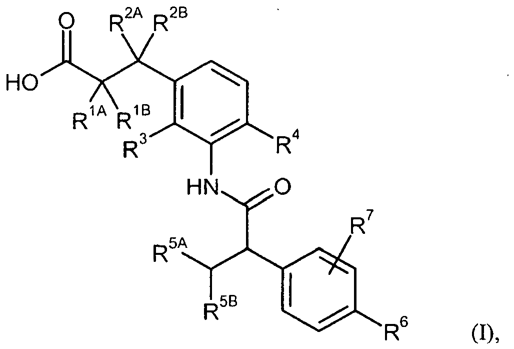 in welcher
R^{1A} für Wasserstoff, Fluor, Methyl, Trifluormethyl, Ethyl, 1,1-Difluorethyl, 2,2,2-Trifluorethyl, *n*-Propyl, Cyclopropyl oder Cyclobutyl steht,
R^{1B} für Wasserstoff oder Methyl steht,
R^{2A} für Wasserstoff, Methyl, Trifluormethyl, Ethyl, 1,1-Difluorethyl, 2,2,2-Trifluorethyl oder n-Propyl steht,
R^{2B} für Wasserstoff oder Methyl steht,
oder
R^{1A} und R^{2A} miteinander verknüpft sind und gemeinsam mit den Kohlenstoffatomen, an die sie gebunden sind, einen Cyclopropyl-Ring der Formel 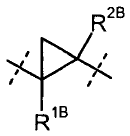 bilden, worin R^{1B} und R^{2B} die zuvor genannten Bedeutungen haben, oder
R^{2A} und R^{2B} miteinander verknüpft sind und gemeinsam mit dem Kohlenstoffatom, an das sie gebunden sind, eine cyclische Gruppe der Formel 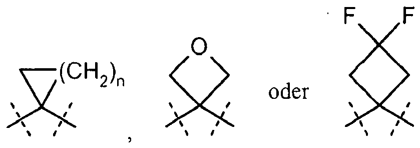 bilden, worin
n die Zahl 1, 2 oder 3 bedeutet,
R³ für Wasserstoff, Fluor, Methyl oder Trifluormethyl steht,
R⁴ für Wasserstoff, Fluor, Chlor, Cyano, Methyl, Trifluormethyl oder Ethyl steht,
R^{5A} für Methyl, Trifluormethyl oder Ethyl steht,
R^{5B} für Trifluormethyl steht, oder
R^{5A} und R^{5B} miteinander verknüpft sind und gemeinsam mit dem Kohlenstoffatom, an das sie gebunden sind, einen Difluor-substituierten Cycloalkyl-Ring der Formel 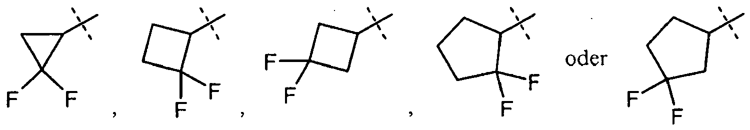 bilden,
R⁶ für Wasserstoff, Fluor, Chlor, Brom, Cyano, (C₁-C₄)-Alkyl, (C₂-C₄)-Alkenyl, Cyclopropyl oder Cyclobutyl steht, wobei (C₁-C₄)-Alkyl und (C₂-C₄)-Alkenyl bis zu dreifach mit Fluor und Cyclopropyl und Cyclobutyl bis zu zweifach mit Fluor substituiert sein können, und
R⁷ für Wasserstoff, Fluor, Chlor, Cyano, Methyl, Trifluormethyl, Ethyl, Methoxy oder Trifluormethoxy steht,
sowie ihre Salze, Solvate und Solvate der Salze.

2. Verbindung der Formel (I) nach Anspruch 1, in welcher
R^{1A} für Wasserstoff, Methyl, Trifluormethyl, Ethyl, *n*-Propyl, Cyclopropyl oder Cyclo-butyl steht,
R^{1B} für Wasserstoff oder Methyl steht,
R^{2A} für Wasserstoff, Methyl, Trifluormethyl, Ethyl oder *n*-Propyl steht,
R^{2B} für Wasserstoff oder Methyl steht, oder
R^{2A} und R^{2B} miteinander verknüpft sind und gemeinsam mit dem Kohlenstoffatom, an das sie gebunden sind, eine cyclische Gruppe der Formel 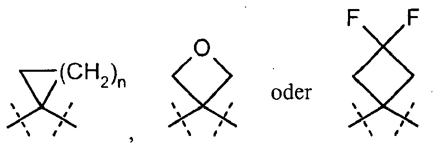 bilden, worin
n die Zahl 1 oder 2 bedeutet,
R³ für Wasserstoff, Fluor oder Methyl steht,
R⁴ für Wasserstoff, Fluor, Chlor, Cyano, Methyl oder Trifluormethyl steht,
R^{5A} für Methyl oder Ethyl steht,
R^{5B} für Trifluormethyl steht, oder
R^{5A} und R^{5B} miteinander verknüpft sind und gemeinsam mit dem Kohlenstoffatom, an das sie gebunden sind, einen Difluor-substituierten Cycloalkyl-Ring der Formel 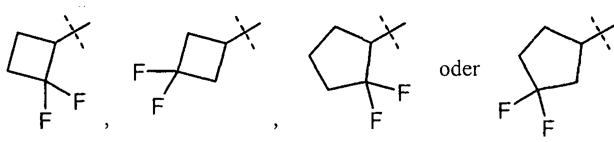 bilden,
R⁶ für Fluor, Chlor, (C₁-C₄)-Alkyl, (C₂-C₃)-Alkenyl, Cyclopropyl oder Cyclobutyl steht, wobei (C₁-C₄)-Alkyl und (C₂-C₃)-Alkenyl bis zu dreifach mit Fluor und Cyclopropyl und Cyclobutyl bis zu zweifach mit Fluor substituiert sein können, und
R⁷ für Wasserstoff, Fluor, Chlor, Methyl oder Methoxy steht,
sowie ihre Salze, Solvate und Solvate der Salze.

3. Verbindung der Formel (I) nach Anspruch 1 oder 2, in welcher
R^{1A} für Wasserstoff, Methyl oder Ethyl steht,
R^{1B} für Wasserstoff steht,
R^{2A} für Wasserstoff, Methyl, Trifluormethyl, Ethyl oder *n*-Propyl steht,
R^{2B} für Wasserstoff oder Methyl steht, oder
R^{2A} und R^{2B} miteinander verknüpft sind und gemeinsam mit dem Kohlenstoffatom, an das sie gebunden sind, eine cyclische Gruppe der Formel 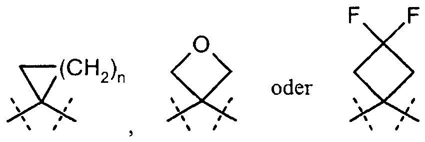 bilden, worin
n die Zahl 1 oder 2 bedeutet,
R³ für Wasserstoff steht,
R⁴ für Fluor, Chlor oder Methyl steht,
R^{5A} für Methyl steht,
R^{5B} für Trifluormethyl steht, oder
R^{5A} und R^{5B} miteinander verknüpft sind und gemeinsam mit dem Kohlenstoffatom, an das sie gebunden sind, einen Difluor-substituierten Cyclopentyl-Ring der Formel 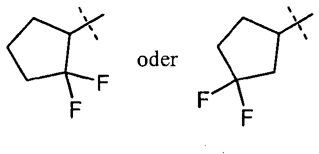 bilden,
R⁶ für Fluor, Chlor, Methyl, Trifluormethyl, Ethyl, 1,1-Difluorethyl, 2,2,2-Trifluorethyl, Isopropyl, *tert*.-Butyl, 1,1,1-Trifluor-2-methylpropan-2-yl, Vinyl, 1-Fluorvinyl, Cyclopropyl, 2,2-Difluorcyclopropyl, Cyclobutyl oder 3,3-Difluorcyclobutyl steht, und
R⁷ für Wasserstoff, Fluor, Chlor oder Methyl steht,
sowie ihre Salze, Solvate und Solvate der Salze.

4. Verfahren zur Herstellung einer Verbindung der Formel (I), wie in den Ansprüchen 1 bis 3
definiert, **dadurch gekennzeichnet, dass** man eine Carbonsäure der Formel (II) 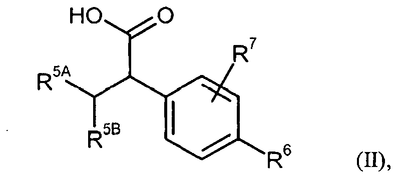 in welcher R^{5A}, R^{5B}, R⁶ und R⁷ die in den Ansprüchen 1 bis 3 angegebenen Bedeutungen haben,
in einem inerten Lösungsmittel mit Hilfe eines Kondensationsmittels oder über die Zwischenstufe des entsprechenden Carbonsäurechlorids in Gegenwart einer Base mit einem Amin der Formel (III) 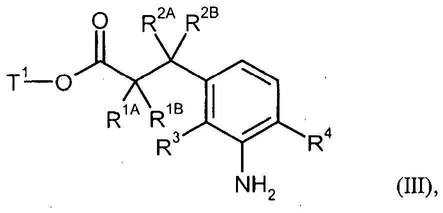 in welcher R^{1A}, R^{1B}, R^{2A}, R^{2B}, R³ und R⁴ die in den Ansprüchen 1 bis 3 angegebenen Bedeutungen haben
und
T¹ für (C₁-C₄)-Alkyl oder Benzyl steht,
zu einem Carbonsäureamid der Formel (IV) 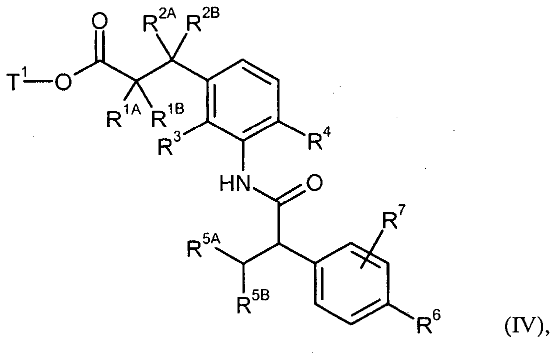 in welcher R^{1A}, R^{1B}, R^{2A}, R^{2B}, R³, R⁴, R^{5A}, R^{5B}, R⁶, R⁷ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen zu ihren Solvaten, Salzen und/oder Solvaten der Salze umsetzt.

5. Verbindung, wie in einem der Ansprüche 1 bis 3 definiert, zur Behandlung und/oder Prävention von Krankheiten.

6. Verbindung, wie in einem der Ansprüche 1 bis 3 definiert, zur Verwendung in einem Verfahren zur Behandlung und/oder Prävention von Herzinsuffizienz, Angina pectoris, Hypertonie, pulmonaler Hypertonie, Ischämien, Gefäßerkrankungen, Mikrozirkulationsstörungen, thromboembolischen Erkrankungen und Arteriosklerose.

7. Verwendung einer Verbindung, wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, Mikrozirkulationsstörungen, thromboembolischen Erkrankungen und Arteriosklerose.

8. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 3 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prävention von Herzinsuffizienz, Angina pectoris, Hypertonie, pulmonaler Hypertonie, Ischämien, Gefäßerkrankungen, Mikrozirkulationsstörungen, thromboembolischen Erkrankungen und Arteriosklerose.

## Claims

1. Compound of the formula (I) 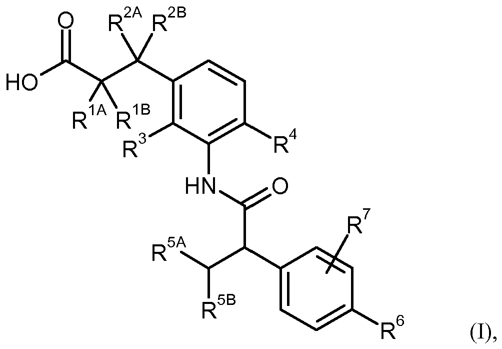 in which
R^{1A} represents hydrogen, fluorine, methyl, trifluoromethyl, ethyl, 1,1-difluoroethyl, 2,2,2-trifluoroethyl, *n*-propyl, cyclopropyl or cyclobutyl,
R^{1B} represents hydrogen or methyl,
R^{2A} represents hydrogen, methyl, trifluoromethyl, ethyl, 1,1-difluoroethyl, 2,2,2-trifluoroethyl or *n*-propyl,
R^{2B} represents hydrogen or methyl, or
R^{1A} and R^{2A} are attached to one another and together with the carbon atoms to which they are attached form a cyclopropyl ring of the formula 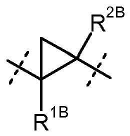 in which R^{1B} and R^{2B} have the meanings mentioned above, or
R^{2A} and R^{2B} are attached to one another and together with the carbon atom to which they are attached form a cyclic group of the formula 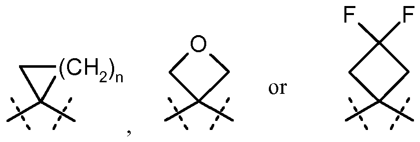 in which
n represents the number 1, 2 or 3,
R³ represents hydrogen, fluorine, methyl or trifluoromethyl,
R⁴ represents hydrogen, fluorine, chlorine, cyano, methyl, trifluoromethyl or ethyl,
R^{5A} represents methyl, trifluoromethyl or ethyl,
R^{5B} represents trifluoromethyl, or
R^{5A} and R^{5B} are attached to one another and together with the carbon atom to which they are attached form a difluoro-substituted cycloalkyl ring of the formula 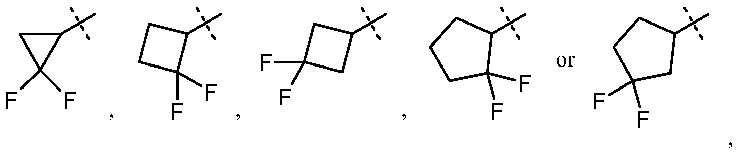
R⁶ represents hydrogen, fluorine, chlorine, bromine, cyano, (C₁-C₄)-alkyl, (C₂-C₄)-alkenyl, cyclopropyl or cyclobutyl, where (C₁-C₄)-alkyl and (C₂-C₄)-alkenyl may be substituted up to three times by fluorine and cyclopropyl and cyclobutyl may be substituted up to two times by fluorine, and
R⁷ represents hydrogen, fluorine, chlorine, cyano, methyl, trifluoromethyl, ethyl, methoxy or trifluoromethoxy,
or a salt, solvate or solvate of a salt thereof.

2. Compound of the formula (I) according to Claim 1 in which
R^{1A} represents hydrogen, methyl, trifluoromethyl, ethyl, *n*-propyl, cyclopropyl or cyclobutyl,
R^{1B} represents hydrogen or methyl,
R^{2A} represents hydrogen, methyl, trifluoromethyl, ethyl or *n*-propyl,
R^{2B} represents hydrogen or methyl, or
R^{2A} and R^{2B} are attached to one another and together with the carbon atom to which they are attached form a cyclic group of the formula 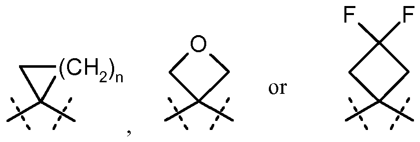 in which
n represents the number 1 or 2,
R³ represents hydrogen, fluorine or methyl,
R⁴ represents hydrogen, fluorine, chlorine, cyano, methyl or trifluoromethyl,
R^{5A} represents methyl or ethyl,
R^{5B} represents trifluoromethyl, or
R^{5A} and R^{5B} are attached to one another and together wtih the carbon atom to which they are attached form a difluoro-substituted cycloalkyl ring of the formula 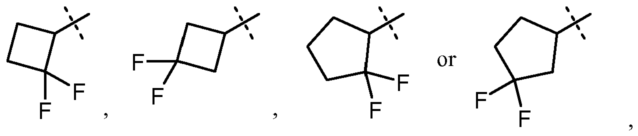
R⁶ represents fluorine, chlorine, (C₁-C₄)-alkyl, (C₂-C₃)-alkenyl, cyclopropyl or cyclobutyl, where (C₁-C₄)-alkyl and (C₂-C₃)-alkenyl may be substituted up to three times by fluorine and cyclopropyl and cyclobutyl may be substituted up to two times by fluorine, and
R⁷ represents hydrogen, fluorine, chlorine, methyl or methoxy,
or a salt, solvate or solvate of a salt thereof.

3. Compound of the formula (I) according to Claim 1 or 2 in which
R^{1A} represents hydrogen, methyl or ethyl,
R^{1B} represents hydrogen,
R^{2A} represents hydrogen, methyl, trifluoromethyl, ethyl or *n*-propyl,
R^{2B} represents hydrogen or methyl,
or
R^{2A} and R^{2B} are attached to one another and together with the carbon atom to which they are attached form a cyclic group of the formula 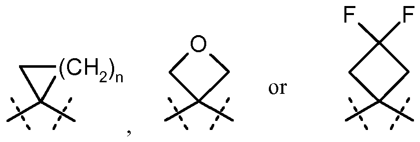 in which
n represents the number 1 or 2,
R³ represents hydrogen,
R⁴ represents fluorine, chlorine or methyl,
R^{5A} represents methyl,
R^{5B} represents trifluoromethyl, or
R^{5A} and R^{5B} are attached to one another and together with the carbon atom to which they are attached form a difluoro-substituted cyclopentyl ring of the formula 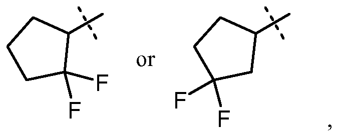
R⁶ represents fluorine, chlorine, methyl, trifluoromethyl, ethyl, 1,1-difluoroethyl, 2,2,2-trifluoroethyl, isopropyl, *tert*-butyl*,* 1,1,1-trifluoro-2-methylpropan-2-yl, vinyl, 1-fluorovinyl, cyclopropyl, 2,2-difluorocyclopropyl, cyclobutyl or 3,3-difluorocyclobutyl, and
R⁷ represents hydrogen, fluorine, chlorine or methyl,
or a salt, solvate or solvate of a salt thereof.

4. Process for preparing a compound of the formula (I) as defmed in any of Claims 1 to 3, **characterized in that** a carboxylic acid of the formula (II) 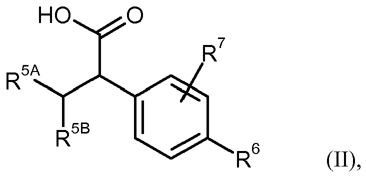 in which R^{5A}, R^{5B}, R⁶ and R⁷ have the meanings given in any of Claims 1 to 3
are coupled in an inert solvent with the aid of a condensing agent or via the intermediate of the corresponding carbonyl chloride in the presence of a base with
an amine of the formula (III) 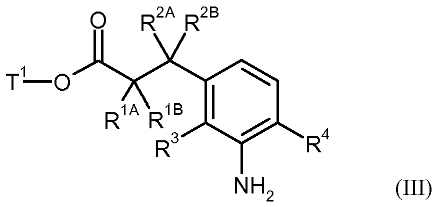 in which R^{1A}, R^{1B}, R^{2A}, R^{2B}, R³ and R⁴ have the meanings given in any of Claims 1 to 3
and
T¹ represents (C₁-C₄)-alkyl or benzyl,
to give a carboxamide of the formula (IV) 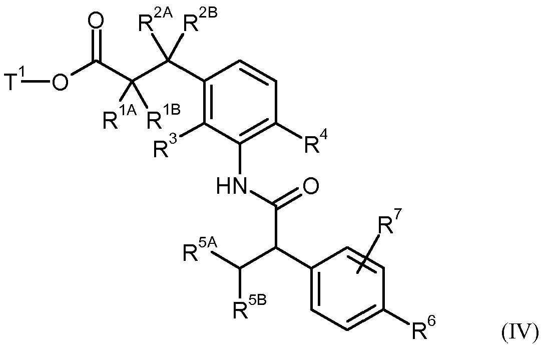 in which R^{1A}, R^{1B}, R^{2A}, R^{2B}, R³, R⁴, R^{5A}, R^{5B}, R⁶, R⁷ and T¹ have the meanings given above,
the ester radical T¹ is then removed by basic or acidic solvolysis or, if T¹ represents benzyl, also by hydrogenolysis, giving the carboxylic acid of the formula (I),
and the compounds of the formula (I) are separated where appropriate by methods known to the person skilled in the art into their enantiomers and/or diastereomers,
and/or where appropriate reacted with the appropriate (i) solvents and/or (ii) bases to give the solvates, salts and/or solvates of the salts thereof.

5. Compound as defined in any of Claims 1 to 3 for the treatment and/or prevention of diseases.

6. Compound as defined in any of Claims 1 to 3 for use in a method for the treatment and/or prevention of heart failure, angina pectoris, hypertension, pulmonary hypertension, ischemias, vascular disorders, impaired microcirculation, thromboembolic disorders and arteriosclerosis.

7. Use of a compound as defined in any of Claims 1 to 3 for the preparation of a medicament for the treatment and/or prevention of heart failure, angina pectoris, hypertension, pulmonary hypertension, ischemias, vascular disorders, impaired microcirculation, thromboembolic disorders and arteriosclerosis.

8. Medicament comprising a compound as defined in any of Claims 1 to 3 in combination with one or more inert, non-toxic, pharmaceutically suitable excipients.

9. Medicament comprising a compound as defined in any of Claims 1 to 3 in combination with one or more further active compounds selected from the group consisting of organic nitrates, NO donors, cGMP-PDE inhibitors, stimulators of guanylate cyclase, agents having antithrombotic activity, agents lowering blood pressure, and agents altering lipid metabolism

10. Medicament according to Claim 8 or 9 for the treatment and/or prevention of heart failure, angina pectoris, hypertension, pulmonary hypertension, ischemias, vascular disorders, impaired microcirculation, thromboembolic disorders and arteriosclerosis.

## Revendications

1. Composé de formule (I) 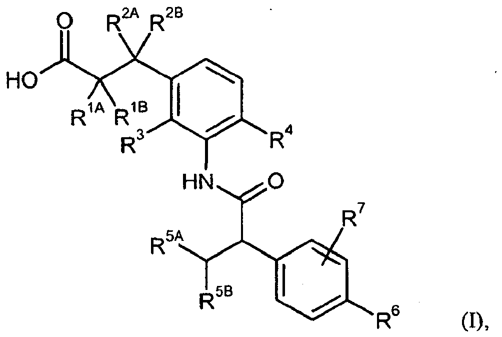 dans laquelle
R^{1A} représente hydrogène, fluor, méthyle, trifluorométhyle, éthyle, 1,1-difluoroéthyle, 2,2,2-trifluoroéthyle, n-propyle, cyclopropyle ou cyclobutyle,
R^{1B} représente hydrogène ou méthyle,
R^{2A} représente hydrogène, méthyle, trifluorométhyle, éthyle, 1,1-difluoroéthyle, 2,2,2-trifluoroéthyle ou n-propyle,
R^{2B} représente hydrogène ou méthyle, ou
R^{1A} et R^{2A} sont liés l'un à l'autre et forment, ensemble avec les atomes de carbone auxquels ils sont liés, un cycle cyclopropyle de formule 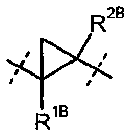 dans laquelle R^{1B} et R^{2B} ont les significations susmentionnées, ou
R^{2A} et R^{2B} sont liés l'un à l'autre et forment, ensemble avec l'atome de carbone auquel ils sont liés, un groupe cyclique de formule 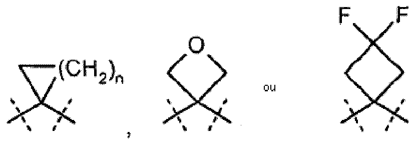 où
n vaut le nombre 1, 2 ou 3,
R³ représente hydrogène, fluor, méthyle ou trifluorométhyle,
R⁴ représente hydrogène, fluor, chlore, cyano, méthyle, trifluorométhyle ou éthyle,
R^{5A} représente méthyle, trifluorométhyle ou éthyle,
R^{5B} représente trifluorométhyle, ou
R^{5A} et R^{5B} sont liés l'un à l'autre et forment, ensemble avec l'atome de carbone auquel ils sont liés, un cycle cycloalkyle disubstitué par fluor de formule 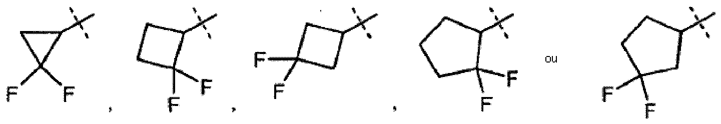 R⁶ représente hydrogène, fluor, chlore, brome, cyano, (C₁-C₄)-alkyle, (C₂-C₄)-alcényle, cyclopropyle ou cyclobutyle, où (C₁-C₄)-alkyle et (C₂-C₄)-alcényle peuvent être jusqu'à trisubstitués par fluor et cyclopropyle et cyclobutyle peuvent être jusqu'à disubstitués par fluor, et
R⁷ représente hydrogène, fluor, chlore, cyano, méthyle, trifluorométhyle, éthyle, méthoxy ou trifluorométhoxy, ainsi que ses sels, solvates et les solvates des sels.

2. Composé de formule (I) selon la revendication 1, dans laquelle
R^{1A} représente hydrogène, méthyle, trifluorométhyle, éthyle, n-propyle, cyclopropyle ou cyclobutyle,
R^{1B} représente hydrogène ou méthyle,
R^{2A} représente hydrogène, méthyle, trifluorométhyle, éthyle ou n-propyle,
R^{2B} représente hydrogène ou méthyle, ou
R^{2A} et R^{2B} sont liés l'un à l'autre et forment, ensemble avec l'atome de carbone auquel ils sont liés, un groupe cyclique de formule 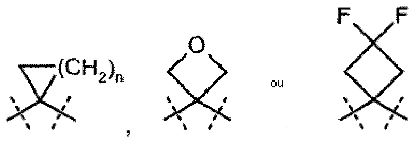 où
n vaut le nombre 1 ou 2,
R³ représente hydrogène, fluor ou méthyle,
R⁴ représente hydrogène, fluor, chlore, cyano, méthyle ou trifluorométhyle,
R^{5A} représente méthyle ou éthyle,
R^{5B} représente trifluorométhyle, ou
R^{5A} et R^{5B} sont liés l'un à l'autre et forment, ensemble avec l'atome de carbone auquel ils sont liés, un cycle cycloalkyle disubstitué par fluor de formule 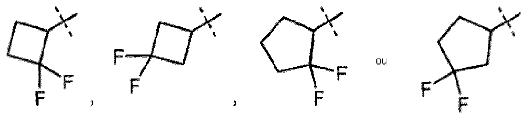
R⁶ représente fluor, chlore, (C₁-C₄)-alkyle, (C₂-C₃)-alcényle, cyclopropyle ou cyclobutyle, où (C₁-C₄)-alkyle et (C₂-C₃)-alcényle peuvent être jusqu'à trisubstitués par fluor et cyclopropyle et cyclobutyle peuvent être jusqu'à disubstitués par fluor, et
R⁷ représente hydrogène, fluor, chlore, méthyle ou méthoxy,
ainsi que ses sels, solvates et les solvates des sels.

3. Composé de formule (I) selon la revendication 1 ou 2, dans laquelle
R^{1A} représente hydrogène, méthyle ou éthyle,
R^{1B} représente hydrogène,
R^{2A} représente hydrogène, méthyle, trifluorométhyle, éthyle ou n-propyle,
R^{2B} représente hydrogène ou méthyle, ou
R^{2A} et R^{2B} sont liés l'un à l'autre et forment, ensemble avec l'atome de carbone auquel ils sont liés, un groupe cyclique de formule 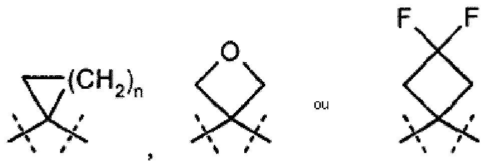 où
n vaut le nombre 1 ou 2,
R³ représente hydrogène,
R⁴ représente fluor, chlore ou méthyle,
R^{5A} représente méthyle,
R^{5B} représente trifluorométhyle, ou
R^{5A} et R^{5B} sont liés l'un à l'autre et forment, ensemble avec l'atome de carbone auquel ils sont liés, un cycle cyclopentyle disubstitué par fluor de formule 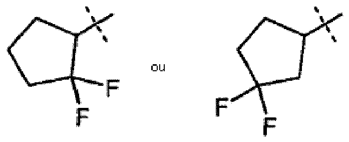
R⁶ représente fluor, chlore, méthyle, trifluorométhyle, éthyle, 1,1-difluoroéthyle, 2,2,2-trifluoroéthyle, isopropyle, tert-butyle, 1,1,1-trifluoro-2-méthylpropan-2-yle, vinyle, 1-fluorovinyle, cyclopropyle, 2,2-difluorocyclopropyle, cyclobutyle ou 3,3-difluorocyclobutyle, et
R⁷ représente hydrogène, fluor, chlore ou méthyle, ainsi que ses sels, solvates et les solvates des sels.

4. Procédé pour la préparation d'un composé de formule (I) tel que défini dans les revendications 1 à 3, **caractérisé en ce qu'**on couple un acide carboxylique de formule (II) 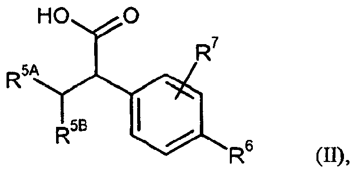 dans laquelle R^{5A}, R^{5B}, R⁶ et R⁷ présentent les significations indiquées dans les revendications 1 à 3, dans un solvant inerte à l'aide d'un agent de condensation ou via l'étape intermédiaire du chlorure d'acide carboxylique correspondant en présence d'une base avec une amine de formule (III) 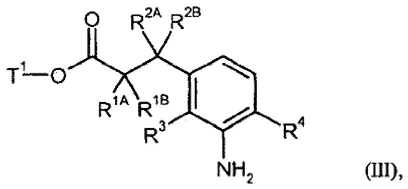 dans laquelle R^{1A}, R^{1B}, R^{2A}, R^{2B}, R³ et R⁴ présentent les significations indiquées dans les revendications 1 à 3 et
T¹ représente (C₁-C₄)-alkyle ou benzyle,
en un amide d'acide carboxylique de formule (IV) 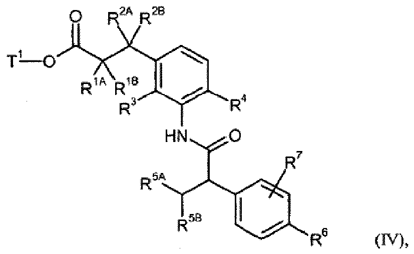 dans laquelle R^{1A}, R^{1B}, R^{2A}, R^{2B}, R³, R⁴, R^{5A}, R^{5B}, R⁶, R⁷ et T¹ présentent les significations indiquées ci-dessus,
puis on dissocie le radical ester T¹ par solvolyse basique ou acide ou, dans le cas où T¹ représente benzyle, également par hydrogénolyse avec obtention de l'acide carboxylique de formule (I)
et on sépare le cas échéant les composés de formule (I) selon des procédés connus par l'homme du métier en leurs énantiomères et/ou diastéréo-isomères et/ou on les transforme avec (i) des solvants et/ou (ii) des bases correspondant(e)s en leurs solvates, sels et/ou solvates des sels.

5. Composé, tel que défini dans l'une quelconque des revendications 1 à 3, destiné au traitement et/ou à la prévention de maladies.

6. Composé tel que défini dans l'une quelconque des revendications 1 à 3 destiné à être utilisé dans un procédé pour le traitement et/ou la prévention de l'insuffisance cardiaque, de l'angine de poitrine, de l'hypertonie, de l'hypertonie pulmonaire, des ischémies, des maladies vasculaires, des troubles microcirculatoires, des maladies thromboemboliques et de l'artériosclérose.

7. Utilisation d'un composé tel que défini dans l'une quelconque des revendications 1 à 3 pour la préparation d'un médicament destiné au traitement et/ou à la prévention de l'insuffisance cardiaque, de l'angine de poitrine, de l'hypertonie, de l'hypertonie pulmonaire, des ischémies, des maladies vasculaires, des troubles microcirculatoires, des maladies thromboemboliques et de l'artériosclérose.

8. Médicament contenant un composé, tel que défini dans l'une quelconque des revendications 1 à 3, en combinaison avec un ou plusieurs adjuvants inertes, non toxiques, pharmaceutiquement appropriés.

9. Médicament contenant un composé tel que défini dans l'une quelconque des revendications 1 à 3, en combinaison avec une ou plusieurs autres substances actives choisies dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

10. Médicament selon la revendication 8 ou 9, destiné au traitement et/ou à la prévention de l'insuffisance cardiaque, de l'angine de poitrine, de l'hypertonie, de l'hypertonie pulmonaire, des ischémies, des maladies vasculaires, des troubles microcirculatoires, des maladies thromboemboliques et de l'artériosclérose.
